(19) Europäisches Patentamt / European Patent Office / Office européen des brevets

(11) EP 3 077 382 B1

(12) EUROPÄISCHE PATENTSCHRIFT

(45) Veröffentlichungstag und Bekanntmachung des Hinweises auf die Patenterteilung:
**26.12.2018 Patentblatt 2018/52**

(21) Anmeldenummer: **14818867.5**

(22) Anmeldetag: **18.11.2014**

(51) Int Cl.:
***C07D 313/06*** *(2006.01)*

(86) Internationale Anmeldenummer:
**PCT/EP2014/003071**

(87) Internationale Veröffentlichungsnummer:
**WO 2015/082046 (11.06.2015 Gazette 2015/23)**

# (54) SUBSTITUIERTE OXEPINE

SUBSTITUTED OXEPINES

OXÉPINES SUBSTITUÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.12.2013 EP 13005698**

(43) Veröffentlichungstag der Anmeldung:
**12.10.2016 Patentblatt 2016/41**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **BUESING, Arne**
  **65929 Frankfurt am Main (DE)**
- **STOESSEL, Philipp**
  **60389 Frankfurt am Main (DE)**
- **GERHARD, Anja**
  **63329 Egelsbach (DE)**
- **PFLUMM, Christof**
  **64291 Darmstadt (DE)**
- **EBERLE, Thomas**
  **76829 Landau (DE)**
- **JATSCH, Anja**
  **60489 Frankfurt am Main (DE)**
- **KROEBER, Jonas Valentin**
  **60311 Frankfurt am Main (DE)**
- **DOBELMANN, Lars**
  **64295 Darmstadt (DE)**

(56) Entgegenhaltungen:
**WO-A1-2010/119274**

- **YI WEI, SHINGO SAMORI, SACHIKO TOJO, MAMORU FUJITSUKA, JIN-SHENG LIN ET AL.: "Emission mechanism of doubly ortho-linked quinoxaline / diphenylfluorene or cis-stilbene / fluorene hybrid compounds based on the transient absorption and emission measurements during pulse radiolysis", J. AM. CHEM. SOC., Bd. 131, Nr. 19, 22. April 2009 (2009-04-22), Seiten 6698-6707, XP002739995,**
- **JIYE LUO, XIAOMIN XU, RENXIN MAO, QIAN MIAO: "Curved polycyclic aromatic molecules that are pi-isoelectronic to hexabenzocoronene", J. AM. CHEM. SOC., Bd. 134, Nr. 33, 8. August 2012 (2012-08-08), Seiten 13796-13803, XP002739996,**
- **OHISHI T ET AL: "High-yielding TfOH-catalyzed condensation of phenols with aromatic aldehydes at high pressure. A model synthesis of the benzylidene biphenol key skeleton of blepharismins", TETRAHEDRON LETTERS, PERGAMON, GB, Bd. 42, Nr. 13, 26. März 2001 (2001-03-26) , Seiten 2493-2496, XP004232280, ISSN: 0040-4039, DOI: 10.1016/S0040-4039(01)00208-8**
- **CASEXANNEX vom 29.05.2015: Resultate aus der Anfangsphase der Recherche in der Datenbank Reaxys vom 20.05.2015, Seiten 1-87.**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine neue Klasse organischer Verbindungen gemäß Anspruch 13 sowie deren Herstellung, Zusammensetzungen gemäß den Ansprüchen 9 bis 12 und optoelektronische Vorrichtungen gemäß Anspruch 1.

Opto-elektronische Vorrichtungen sind seit einigen Jahren Gegenstand intensiver Forschung. Unter den optoelektronischen Vorrichtungen haben insbesondere die organischen Elektrolumineszenzvorrichtungen das Interesse von Forschung und Entwicklung in Industrie und Hochschulen geweckt. Hierzu zählen, beispielsweise, die organischen lichtemittierenden Dioden (OLEDs und PLEDs) sowie die organischen lichtemittierenden elektrochemischen Zellen (OLECs, LECs oder LEECs).

Der Aufbau organischer Elektrolumineszenzvorrichtungen (z.B. OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es sowohl bei OLEDs, die Singulettemission zeigen, wie auch bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.

Die Eigenschaften von OLEDs werden nicht nur durch die eingesetzten Emitter bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen.

Allerdings besteht bei Verwendung dieser Host- und Matrixmaterialien ebenso wie bei anderen Matrixmaterialien noch Verbesserungsbedarf, insbesondere in Bezug auf die Effizienz und die Lebensdauer der Vorrichtung.

Obwohl die auf kleinen Molekülen basierenden OLEDs (SMOLEDs) teilweise recht gute Effizienzen, Lebensdauern und/oder Betriebsspannung zeigen, sind thermische Aufdampfungsmethoden im Vakuum nötig, die auf eine bestimmte Device-Größe beschränkt sind. Für die Massenproduktion und für größere Displays ist es allerdings wünschenswert, die organischen Materialien aus Lösung aufzubringen, z.B. mittels Spin-coating- oder Inkjet-Verfahren, wodurch zusätzlich die Produktionskosten gesenkt werden können. Zumeist werden lichtemittierende Polymere, Oligomere und/oder Dendrimere verwendet, um elektrolumineszierende Vorrichtungen aus Lösung zu prozessieren. Diese Verbindungen zeigen oft eine gute Löslichkeit in organischen aromatischen Lösemitteln und weisen gute Filmbildungseigenschaften auf. Eine weitere Möglichkeit die Verarbeitbarkeit zu verbessern, besteht beispielsweise darin, lange Alkyl-Ketten als löslichkeitsvermittelnde Gruppen in ein Molekül einzubauen. Leider weisen die aus Lösung prozessierten Vorrichtungen unter Verwendung von Polymeren, Oligomeren und/oder Dendrimeren oder Molekülen mit Alkyl-Ketten meist eine schlechtere Performance auf, als vergleichbare kleine Moleküle, was Effizienz, Lebensdauer und Betriebsspannung betrifft.

Aufgabe der vorliegenden Erfindung ist die Bereitstellung von Verbindungen, welche sich für den Einsatz in einer fluoreszierenden oder phosphoreszierenden OLED eignen, beispielsweise als Matrixmaterial oder als Lochtransport-/Elektronenblockiermaterial bzw. Exzitonenblockiermaterial oder als Elektronentransport- bzw. Lochblockiermaterial, und welche bei Verwendung in einer OLED zu guten Device-Eigenschaften führen, sowie die Bereitstellung der entsprechenden elektronischen Vorrichtung.

Eine weitere Aufgabe der vorliegenden Erfindung besteht darin, Moleküle bereitzustellen, die eine verbesserte Löslichkeit aufweisen und daher bei der Herstellung einer lichtemittierenden Vorrichtung aus Lösung prozessiert werden können. Noch eine weitere Aufgabe der vorliegenden Erfindung besteht in der Bereitstellung von Molekülen, die sich besonders gut dazu eignen, lichtemittierende Vorrichtungen aus der Gasphase herzustellen, d.h. Moleküle bereitzustellen, die besonders gut aufgedampft werden können.

Überraschend wurde gefunden, dass bestimmte, unten näher beschriebene Verbindungen diese Aufgaben lösen und zu guten Eigenschaften der organischer elektronischer Vorrichtungen, insbesondere in organischen Elektrolumineszenzvorrichtung führen, insbesondere hinsichtlich der Lebensdauer, der Effizienz, der Betriebsspannung und Prozessierbarkeit. Elektronische Vorrichtungen, insbesondere organische Elektrolumineszenzvorrichtungen, welche derartige Verbindungen enthalten, sowie bevorzugten Verbindungen sind daher der Gegenstand der vorliegenden Erfindung.

In WO 2010/119274 werden Monomere mit einem Grundgerüst basierend auf 4,6-Dihydro-10H-Fluoreno[4,5-cde]oxepin und entsprechende Polymere beschrieben.

Die vorliegende Erfindung betrifft eine elektronische Vorrichtung enthaltend wenigstens eine Verbindung der allgemeinen Formel (5) oder (7),

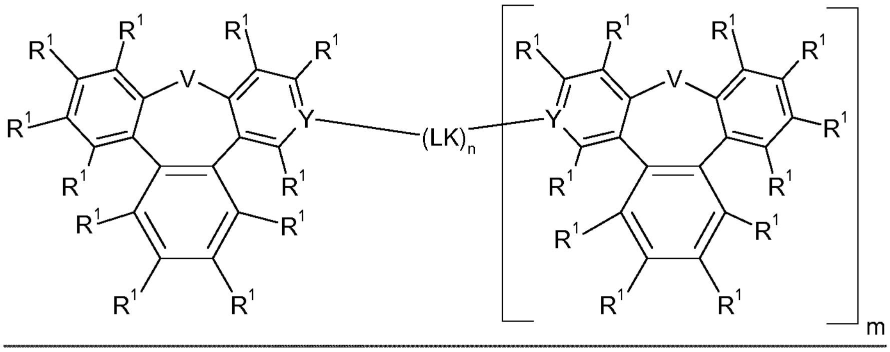

Formel (5)

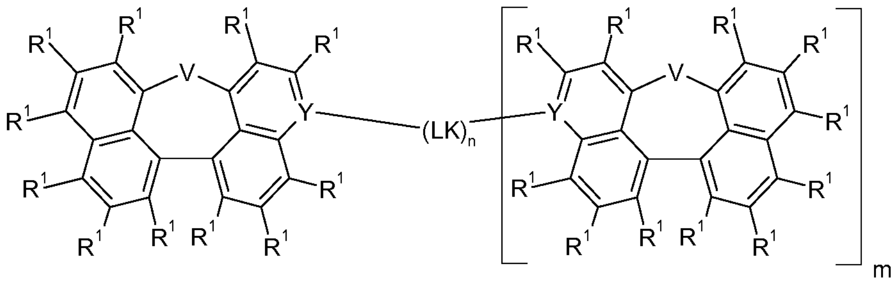

Formel (7),

wobei für die Symbole und Indizes gilt:

V ist O oder S und ganz bevorzugt O;

X ist gleich oder verschieden bei jedem Auftreten N oder $CR^1$, bevorzugt $CR^1$;

m ist 0 (Monomer), 1 (Dimer) oder 2 (Trimer);

n ist 0 oder 1, wobei gilt:

n = 0 für m = 0 und
n = 1 für m = 1 oder für m = 2;

Y ist gleich ein sp2-hybridisiertes Kohlenstoffatom für m ungleich 0 oder gleich X für m = 0;

LK ist für den Fall m=1 eine Einfachbindung oder ein bifunktioneller Linker; LK kann mit einem oder mehreren Resten $R^1$ substituiert sein, wobei die Reste $R^1$ bei jedem Auftreten gleich oder verschieden sein können; ist für m = 2 ein trifunktioneller Linker, wobei der Linker mit einem oder mehreren Resten $R^1$ substituiert sein kann, wobei die Reste $R^1$ bei jedem Auftreten gleich oder verschieden sein können; ist, wenn n = 0 ist, nicht vorhanden, so dass ein Monomer vorliegt;

$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit

10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann, wobei bevorzugt ist, wenn zwei oder mehrere benachbarte Reste $R^1$ miteinander kein mono- oder polycyclisches, aliphatisches oder aromatisches oder heteroaromatisches Ringsystem bilden; das mit einem oder mehreren Resten $R^2$ substituiert sein kann;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkyl-alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches oder heteroaromatisches Ringsystem bilden, wobei bevorzugt ist, wenn zwei oder mehrere benachbarte Reste $R^2$ miteinander kein mono- oder polycyclisches, aliphatisches oder aromatisches oder heteroaromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 40 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches oder heteroaromatisches Ringsystem bilden, wobei bevorzugt ist, wenn zwei oder mehrere benachbarte Reste $R^3$ miteinander kein mono- oder polycyclisches, aliphatisches oder aromatisches oder heteroaromatisches Ringsystem bilden;

mit der Maßgabe, dass wenn m = 0 ist, wenigstens einer der Reste $R^1$ ungleich H ist.

Sofern m gleich 0 ist, ist ferner bevorzugt, wenn wenigstens einer der Reste $R^1$ ungleich H ist, wobei gilt:

$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q; dabei können zwei oder mehrere benachbarte Reste $R^1$ miteinander kein mono- oder polycyclisches, aliphatisches oder aromatisches oder heteroaromatisches Ringsystem bilden, das mit einem oder mehreren Resten $R^2$ substituiert sein kann;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkyl-alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$

ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander kein mono- oder polycyclisches, aliphatisches oder aromatisches oder heteroaromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 40 C-Atomen, in dem auch ein oder mehrere H-Atome durch F ersetzt sein können; dabei können zwei oder mehrere Substituenten $R^3$ auch miteinander kein mono- oder polycyclisches, aliphatisches oder aromatisches oder heteroaromatisches Ringsystem bilden.

Es ist bevorzugt, wenn m gleich 0 oder 1 ist, ganz bevorzugt ist wenn m gleich 0 ist, d.h., wenn die Verbindungen als Monomere vorliegen.

In einer bevorzugten Ausführungsform gilt m = n = 0 (Monomer). In einer weiteren bevorzugten Ausführungsform gilt m = n = 1 (Dimer). In noch einer weiteren bevorzugten Ausführungsform gilt m = 2 und n = 1 (Trimer). Ganz besonders bevorzugt ist in diesem Zusammenhang wenn m = n = 0 ist.

Bevorzugt betrifft die vorliegende Erfindung eine Verbindung der allgemeinen Formel
(5) nach Anspruch 13, wobei an anderer Stelle genannte bevorzugte Ausführungsformen der verwendeten Indizes und Symbole auch bevorzugte Ausführungsformen für die Verbindung der Formel (5) darstellen.

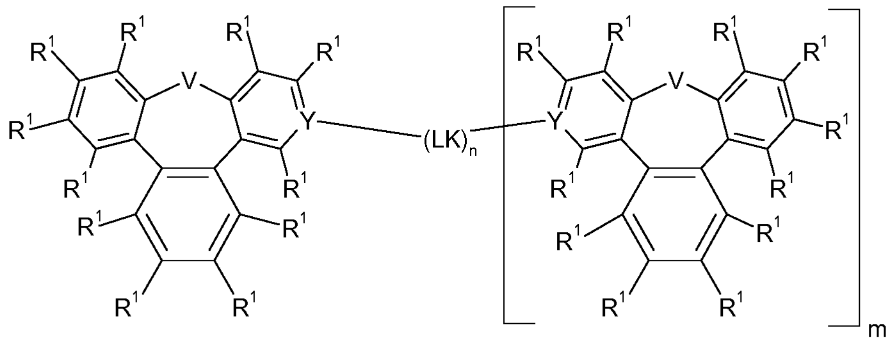

Formel (5)

Weiter bevorzugt ist dabei, wenn wenigstens ein oder zwei der Reste $R^1$ in Formel (5) ungleich H sind.

Gganz bevorzugt ist in diesem Zusammenhang auch eine Verbindung der allgemeinen Formel (7),wobei an anderer Stelle genannte bevorzugte Ausführungsformen der verwendeten Indizes und Symbole auch bevorzugte Ausführungsformen für die Verbindung der Formel (7) darstellen.

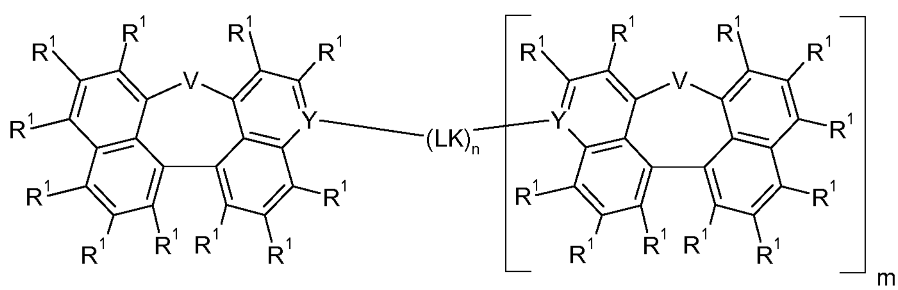

Formel (7)

In einer weiteren bevorzugten Ausführungsform betrifft die vorliegende Erfindung eine Verbindung der allgemeinen Formel (8), wobei an anderer Stelle genannte bevorzugte Ausführungsformen der verwendeten Indizes und Symbole auch bevorzugte Ausführungsformen für die Verbindung der Formel (8) darstellen.

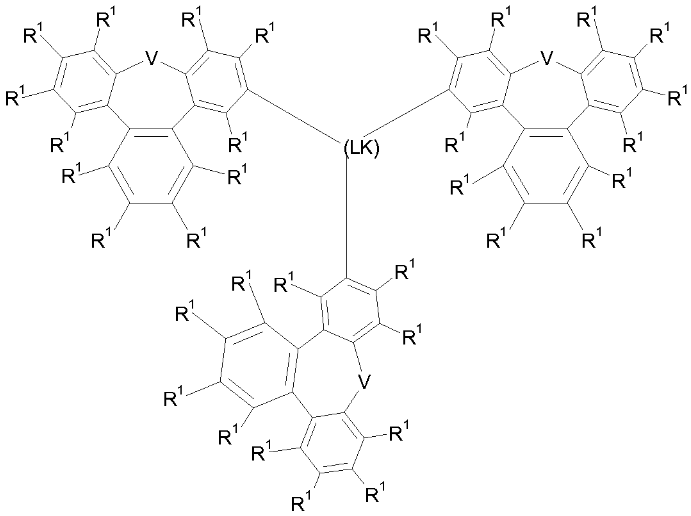

Formel (8)

In noch einer weiteren bevorzugten Ausführungsform betrifft die vorliegende Erfindung eine Verbindung der allgemeinen Formel (9), wobei an anderer Stelle genannte bevorzugte Ausführungsformen der verwendeten Indizes und Symbole auch bevorzugte Ausführungsformen für die Verbindung der Formel (9) darstellen.

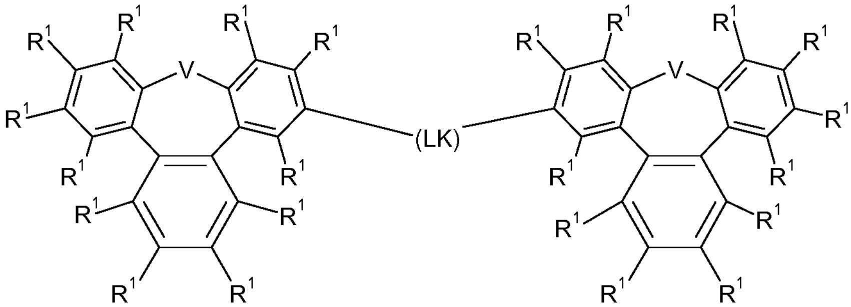

Formel (9)

In einer ganz bevorzugten Ausführungsform betrifft die vorliegende Erfindung eine Verbindung der allgemeinen Formel (10), wobei an anderer Stelle genannte bevorzugte Ausführungsformen der verwendeten Indizes und Symbole auch bevorzugte Ausführungsformen für die Verbindung der Formel (10) darstellen.

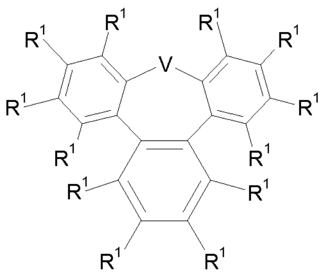

Formel (10)

In einer weiteren bevorzugten Ausführungsform betrifft die vorliegende Erfindung eine Verbindung der allgemeinen Formel (10a),

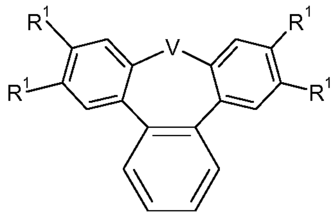

Formel (10a)

wobei für die Verbindung der allgemeinen Formel (10a) insbesondere bevorzugt ist, wenn die benachbarten Reste $R^1$ einen Ringschluss eingehen.

In einer weiteren bevorzugten Ausführungsform betrifft die vorliegende Erfindung eine Verbindung der allgemeinen Formel (10b),

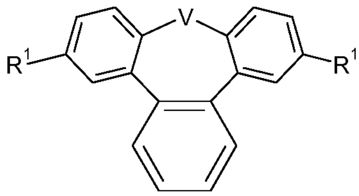

Formel (10b)

Es ist bevorzugt, wenn beide Reste $R^1$ in der Verbindung der Formel (10b) ungleich H sind.

In einer weiteren bevorzugten Ausführungsform betrifft die vorliegende Erfindung eine Verbindung der allgemeinen Formel (10c),

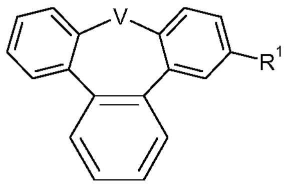

Formel (10c)

In einer weiteren bevorzugten Ausführungsform betrifft die vorliegende Erfindung eine Verbindung der allgemeinen Formel (10d)

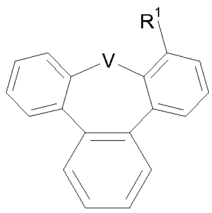

Formel (10d)

In einer weiteren bevorzugten Ausführungsform betrifft die vorliegende Erfindung eine Verbindung der allgemeinen Formel (11), wobei an anderer Stelle genannte bevorzugte Ausführungsformen der verwendeten Indizes und Symbole auch bevorzugte Ausführungsformen für die Verbindung der Formel (11) darstellen.

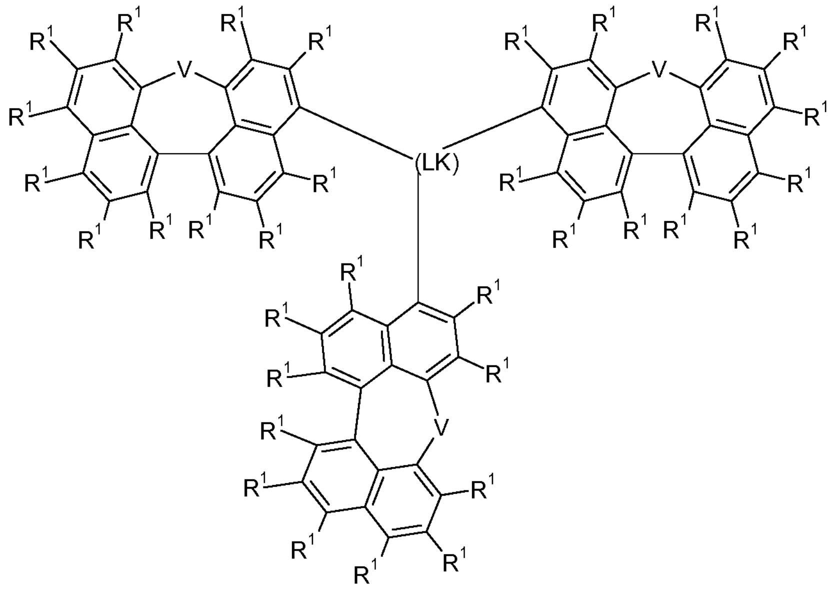

Formel (11)

In noch einer weiteren bevorzugten Ausführungsform betrifft die vorliegende Erfindung eine Verbindung der allgemeinen Formel (12), wobei an anderer Stelle genannte bevorzugte Ausführungsformen der verwendeten Indizes und Symbole auch bevorzugte Ausführungsformen für die Verbindung der Formel (12) darstellen.

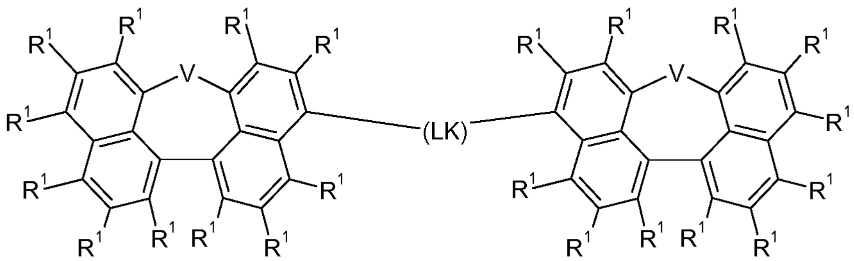

Formel (12)

In einer ganz bevorzugten Ausführungsform betrifft die vorliegende Erfindung eine Verbindung der allgemeinen Formel (13), wobei an anderer Stelle genannte bevorzugte Ausführungsformen der verwendeten Indizes und Symbole auch bevorzugte Ausführungsformen für die Verbindung der Formel (13) darstellen.

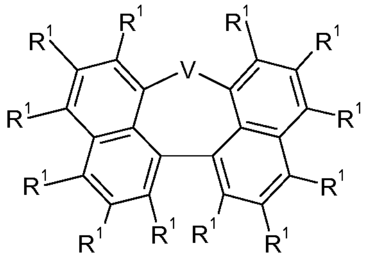

Formel (13)

In einer ganz besonders bevorzugten Ausführungsform betrifft die vorliegende Erfindung eine Verbindung der allgemeinen Formel (14), wobei an anderer Stelle genannte bevorzugte Ausführungsformen der verwendeten Indizes und Symbole auch bevorzugte Ausführungsformen für die Verbindung der Formel (14) darstellen.

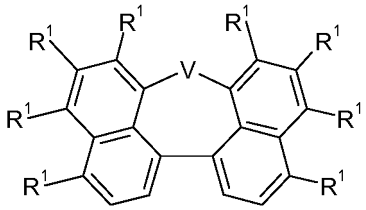

Formel (14)

In einer weiterhin ganz besonders bevorzugten Ausführungsform betrifft die vorliegende Erfindung eine Verbindung der allgemeinen Formel (15), wobei an anderer Stelle genannte bevorzugte Ausführungsformen der verwendeten Indizes und Symbole auch bevorzugte Ausführungsformen für die Verbindung der Formel (15) darstellen.

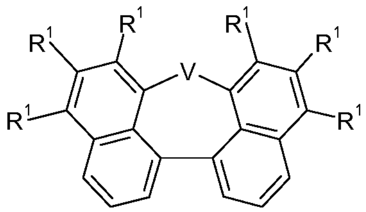

Formel (15)

In einer weiterhin ganz besonders bevorzugten Ausführungsform betrifft die vorliegende Erfindung eine Verbindung der allgemeinen Formel (16), wobei an anderer Stelle genannte bevorzugte Ausführungsformen der verwendeten Indizes und Symbole auch bevorzugte Ausführungsformen für die Verbindung der Formel (16) darstellen.

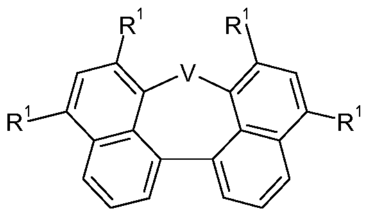

Formel (16)

In einer weiterhin ganz besonders bevorzugten Ausführungsform betrifft die vorliegende Erfindung eine Verbindung der allgemeinen Formel (17), wobei an anderer Stelle genannte bevorzugte Ausführungsformen der verwendeten Indizes und Symbole auch bevorzugte Ausführungsformen für die Verbindung der Formel (17) darstellen.

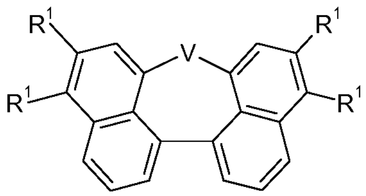

Formel (17)

In einer insbesondere bevorzugten Ausführungsform betrifft die vorliegende Erfindung eine Verbindung der allgemeinen Formel (18), wobei an anderer Stelle genannte bevorzugte Ausführungsformen der verwendeten Indizes und Symbole auch bevorzugte Ausführungsformen für die Verbindung der Formel (18) darstellen.

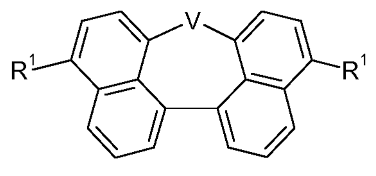

Formel (18)

In noch einer insbesondere bevorzugten Ausführungsform betrifft die vorliegende Erfindung eine Verbindung der allgemeinen Formel (19), wobei an anderer Stelle genannte bevorzugte Ausführungsformen der verwendeten Indizes und Symbole auch bevorzugte Ausführungsformen für die Verbindung der Formel (19) darstellen.

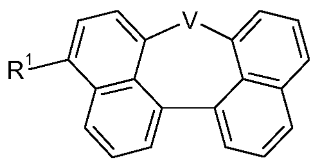

Formel (19)

LK in den hierin genannten Verbindungen ist entweder eine Einfachbindung, ein bifunktioneller oder trifunktioneller Linker. Der Fachmann hat dabei keinerlei Schwierigkeiten geeignete Linker auszuwählen.

In einer bevorzugten Ausführungsform der vorliegenden Erfindung ist der Linker LK eine Einfachbindung oder eine bifunktionelle Gruppe, d.h., n = 1 und m = 1.

Bevorzugte bifunktionelle Linker LK im Sinne der vorliegenden Erfindung sind ausgewählt aus $NR^1$, C(=O), P(=O), $P(=O)R^1$, eine geradkettige Alkyl-, Alkoxy- oder Thioalkoxygruppe mit 1 bis 10 C-Atomen oder eine geradkettige Alkenyl- oder Alkinylgruppe mit 2 bis 10 C-Atomen oder eine verzweigte oder cyclische Alkyl-, Alkenyl-, Alkinyl-, Alkoxy-, Alkylalkoxy- oder Thioalkoxygruppe mit 3 bis 20 C-Atomen, die jeweils mit einem oder mehreren Resten $R^1$ substituiert sein kann, wobei eine oder mehrere nicht benachbarte $CH_2$-Gruppen durch $R^1C=CR^1$, C=C, $Si(R^1)_2$, $Ge(R^1)_2$, $Sn(R^1)_2$, C=O, C=S, C=Se, $C=NR^1$, $P(=O)(R^1)$, SO, $SO_2$, $NR^1$, O, S oder $CONR^1$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 30 aromatischen Ringatomen, das jeweils durch einen oder mehrere Reste $R^1$ substituiert sein kann, oder eine Aryloxy-, Arylalkoxy- oder Heteroaryloxygruppe mit 5 bis 30 aromatischen Ringatomen, die durch einen oder mehrere Reste $R^1$ substituiert sein kann.

Ganz bevorzugte bifunktionelle Linker LK sind ausgewählt aus $NR^1$, C(=O), P(=O), $P(=O)R^1$ oder ein aromatisches oder heteroaromatisches Ringsystem mit 5 bis 30 aromatischen Ringatomen, das jeweils durch einen oder mehrere Reste $R^1$ substituiert sein kann.

Unter den aromatischen Ringen und heteroaromatischen Ringen als bifunktionelle Gruppe LK sind ganz besonders bevorzugt eine Phenylen-, Biphenylen, Terphenylen-, Naphthylen-, Anthracenylen-, Pyridylen-, Pyridazylen-, Pyrimidylen-, Pyrazinylen-, Triazylen-, Fluorenylen-, Indenofluorenylen-, Dibenzofuranylen-, Dibenzothiophenylen-, Carbazoylen-, Indenocarbazoylen- und Indolocarbazoylen-Gruppe, die jeweils durch einen oder mehrere, voneinander unabhängige Reste $R^1$ substituiert sein können, wobei die Phenylene ganz besonders bevorzugte bifunktionelle Linker darstellen. Insbesondere bevorzugt ist eine meta oder para Verknüpfung an dem Phenylen.

Bevorzugte trifunktionelle Linker im Sinne der vorliegenden Erfindung sind ausgewählt aus N, P(=O), eine verzweigte oder cyclische Alkyl-, Alkenyl-, Alkinyl-, Alkoxy-, Alkylalkoxy- oder Thioalkoxygruppe mit 3 bis 20 C-Atomen, die jeweils mit einem oder mehreren Resten $R^1$ substituiert sein kann, wobei eine oder mehrere nicht benachbarte $CH_2$-Gruppen durch $R^1C=CR^1$, C=C, $Si(R^1)_2$, $Ge(R^1)_2$, $Sn(R^1)_2$, C=O, C=S, C=Se, $C=NR^1$, $P(=O)(R^1)$, SO, $SO_2$, $NR^1$, O, S oder $CONR^1$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 30 aromatischen Ringatomen, das jeweils durch einen oder mehrere Reste $R^1$ substituiert sein kann, oder eine Aryloxy-, Arylalkoxy-oder Heteroaryloxygruppe mit 5 bis 30 aromatischen Ringatomen, die durch einen oder mehrere, voneinander unabhängige Reste $R^1$ substituiert sein kann.

Ganz bevorzugte trifunktionelle Linker LK sind ausgewählt aus N, P(=O), oder ein aromatisches oder heteroaromatisches Ringsystem mit 5 bis 30 aromatischen Ringatomen, das jeweils durch einen oder mehrere, voneinander unabhängige Reste $R^1$ substituiert sein kann.

Unter den aromatischen Ringen und heteroaromatischen Ringen als bifunktionelle Gruppe LK sind ganz besonders bevorzugt eine Phenylen-, Biphenylen, Terphenylen-, Naphthylen-, Anthracenylen-, Pyridylen-, Pyridazylen-, Pyrimidylen-, Pyrazinylen-, Triazylen-, Fluorenylen-, Indenofluorenylen-, Dibenzofuranylen-, Dibenzothiophenylen-, Carbazoylen-, Indenocarbazoylen- und Indolocarbazoylen-Gruppe, die jeweils durch einen oder mehrere, voneinander unabhängige Reste $R^1$ substituiert sein können, wobei die Phenylene und Triazinylene ganz besonders bevorzugte trifunktionelle Linker darstellen. Insbesondere bevorzugt ist eine 1, 3, 5-Verknüpfung an dem Phenylen.

In den erfindungsgemäßen Verbindungen können die angegebenen Reste $R^1$ alle auch gleich H sein. In einer bevorzugten Ausführungsform ist jedoch wenigstens einer der Reste $R^1$ ungleich H.

Bevorzugt ist, wenn der Rest $R^1$ gleich oder verschieden bei jedem Auftreten ausgewählt ist aus der Gruppe bestehend aus H, D, $N(R^2)_2$, CN, $B(OR^2)_2$, $C(=O)R^2$, $P(=O)(R^2)_2$, $S(=O)R^2$, $S(=O)_2R^2$, $OSO_2R^2$, einer geradkettigen Alkyl-, Alkoxy- oder Thioalkoxygruppe mit 1 bis 40 C-Atomen oder einer verzweigten oder cyclischen Alkyl-, Alkoxy-, Alkylalkoxy- oder Thioalkoxygruppe mit 3 bis 40 C-Atomen, die jeweils mit einem oder mehreren Resten $R^2$ substituiert sein kann oder einem aromatischen oder heteroaromatischen Ringsystem mit 5 bis 60 aromatischen Ringatomen, das jeweils durch einen oder mehrere Reste $R^2$ substituiert sein kann, oder einer Aryloxy-, Arylalkoxy- oder Heteroaryloxygruppe mit 5 bis 60 aromatischen Ringatomen, die durch einen oder mehrere Reste $R^2$ substituiert sein kann, oder einer Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder einer Kombination aus zwei oder mehr dieser Gruppen; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann.

Ganz bevorzugt ist, wenn der Rest $R^1$ gleich oder verschieden bei jedem Auftreten ausgewählt ist aus der Gruppe bestehend aus H, D, $N(R^2)_2$, einer geradkettigen Alkyl-, Alkoxy- oder Thioalkoxygruppe mit 1 bis 40 C-Atomen oder einer verzweigten oder cyclischen Alkyl-, Alkoxy-, Alkylalkoxy- oder Thioalkoxygruppe mit 3 bis 40 C-Atomen, die jeweils mit einem oder mehreren Resten $R^2$ substituiert sein kann oder einem aromatischen oder heteroaromatischen Ringsystem mit 5 bis 60 aromatischen Ringatomen, das jeweils durch einen oder mehrere Reste $R^2$ substituiert sein kann oder einer Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder einer Kombination aus zwei oder mehr dieser Gruppen; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann.

Besonders bevorzugt ist, wenn der Rest $R^1$ gleich oder verschieden bei jedem Auftreten ausgewählt ist aus der Gruppe bestehend aus H, D, einer geradkettige Alkylgruppe mit 1 bis 40 C-Atomen, einem aromatischen oder heteroaromatischen Ringsystem mit 5 bis 60 aromatischen Ringatomen, das jeweils durch einen oder mehrere Reste $R^2$ substituiert sein kann; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann.

Ganz besonders bevorzugt ist, wenn der Rest $R^1$ gleich oder verschieden bei jedem Auftreten ausgewählt ist aus der Gruppe bestehend aus H, einer geradkettige Alkylgruppe mit 1 bis 40 C-Atomen, einem aromatischen oder heteroaromatischen Ringsystem mit 5 bis 60 aromatischen Ringatomen, das jeweils durch einen oder mehrere Reste $R^2$ substituiert sein kann; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann.

Insbesondere bevorzugt ist, wenn der Rest $R^1$ gleich oder verschieden bei jedem Auftreten ausgewählt ist aus der Gruppe bestehend aus H, einem aromatischen oder heteroaromatischen Ringsystem mit 5 bis 60 aromatischen Ringatomen, das jeweils durch einen oder mehrere Reste $R^2$ substituiert sein kann; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann.

Die folgende Übersicht zeigt einige besonders bevorzugte Reste $R^1$, wobei die gezeigten Reste noch mit einem oder mehreren Reste $R^2$ substituiert sein können, die gleich oder verschieden bei jedem Auftreten sein können.

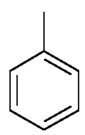
Formel (R1-1)

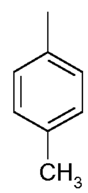
Formel (R1-2)

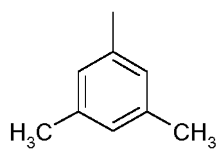
Formel (R1-3)

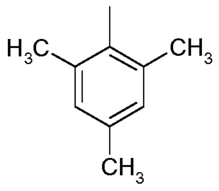

Formel (R1-4)

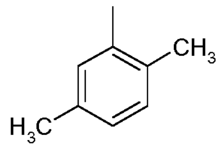

Formel (R1-5)

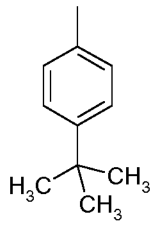

Formel (R1-6)

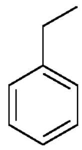

Formel (R1-7)

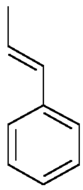

Formel (R1-8)

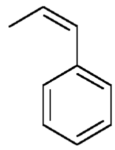

Formel (R1-9)

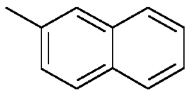

Formel (R1-10)

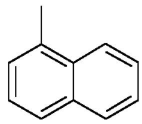

Formel (R1-11)

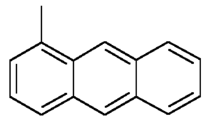

Formel (R1-12)

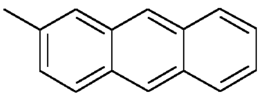

Formel (R1-13)

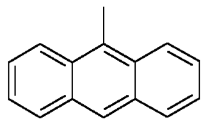

Formel (R1-14)

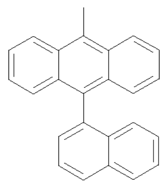

Formel (R1-15)

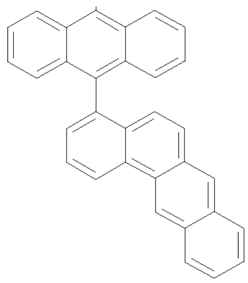

Formel (R1-16)

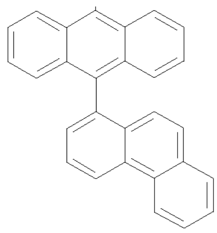

Formel (R1-17)

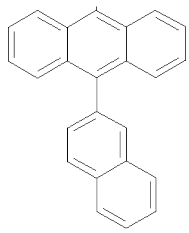

Formel (R1-18)

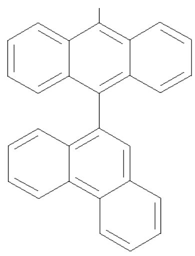

Formel (R1-19)

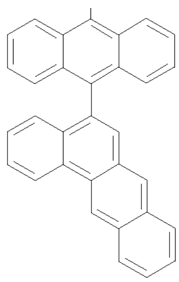

Formel (R1-20)

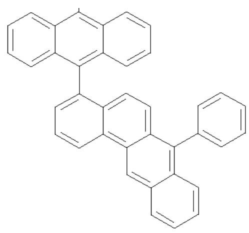

Formel (R1-21)

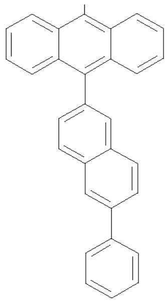

Formel (R1-22)

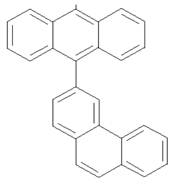

Formel (R1-23)

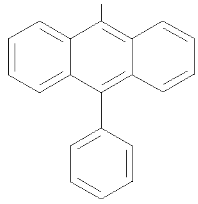

Formel (R1-24)

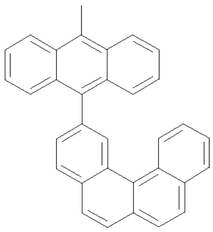

Formel (R1-25)

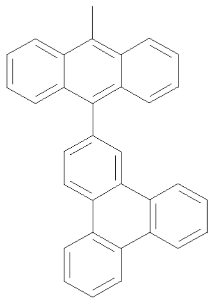

Formel (R1-26)

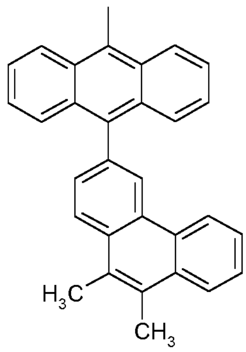

Formel (R1-27)

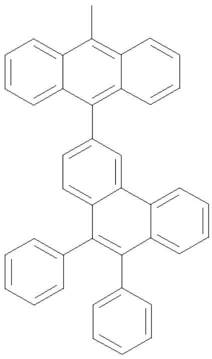

Formel (R1-28)

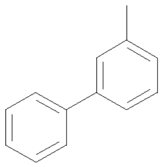

Formel (R1-29)

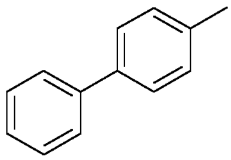

Formel (R1-30)

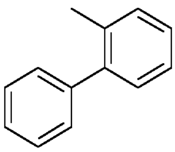

Formel (R1-31)

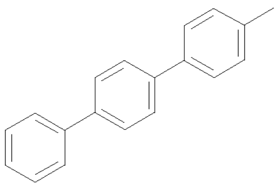

Formel (R1-32)

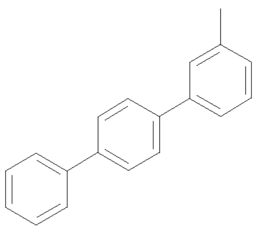

Formel (R1-33)

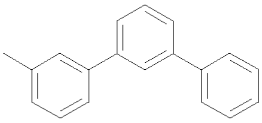

Formel (R1-34)

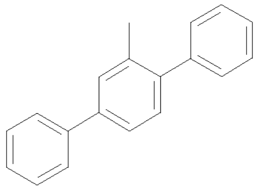

Formel (R1-35)

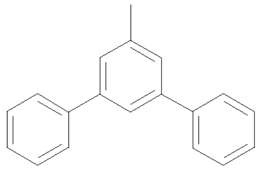

Formel (R1-36)

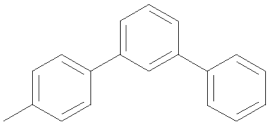

Formel (R1-37)

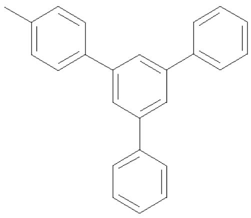

Formel (R1-38)

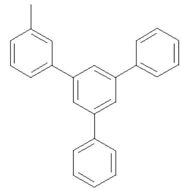

Formel (R1-39)

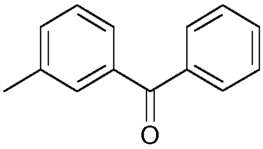

Formel (R1-40)

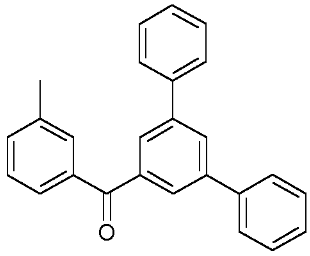

Formel (R1-41)

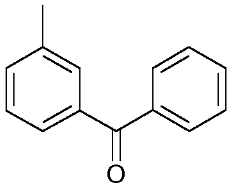

Formel (R1-42)

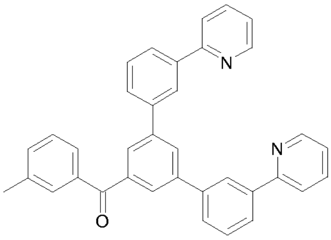

Formel (R1-43)

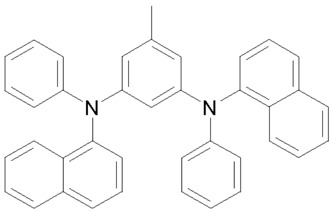

Formel (R1-44)

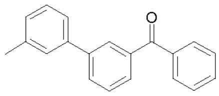

Formel (R1-45)

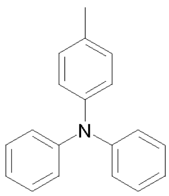

Formel (R1-46)

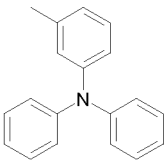

Formel (R1-47)

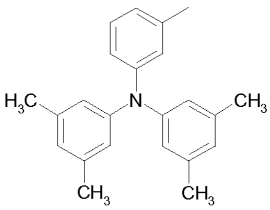

Formel (R1-48)

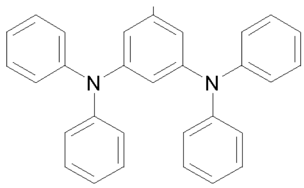

Formel (R1-49)

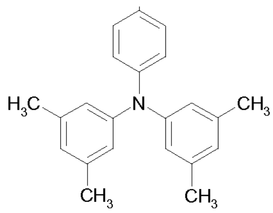

Formel (R1-50)

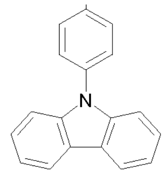

Formel (R1-51)

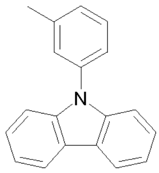

Formel (R1-52)

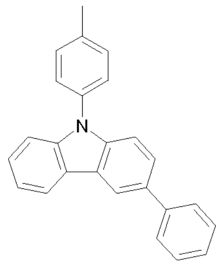

Formel (R1-53)

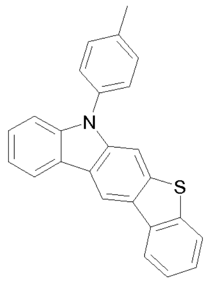

Formel (R1-54)

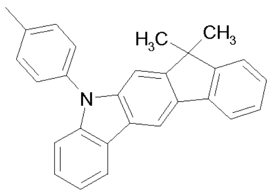

Formel (R1-55)

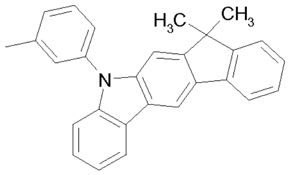

Formel (R1-56)

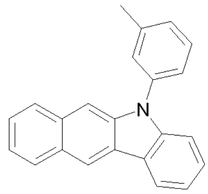

Formel (R1-57)

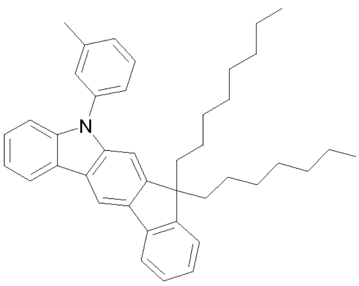

Formel (R1-58)

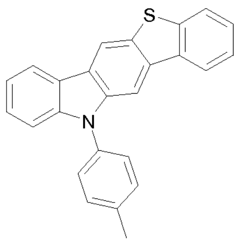

Formel (R1-59)

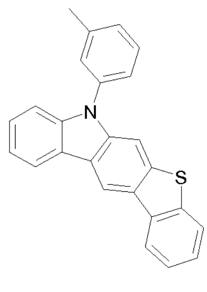

Formel (R1-60)

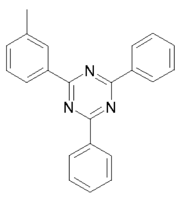

Formel (R1-61)

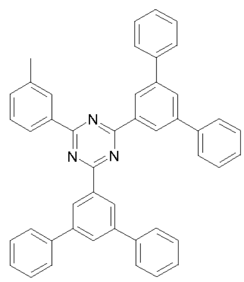

Formel (R1-62)

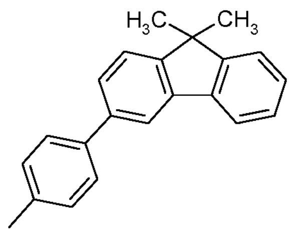

Formel (R1-63)

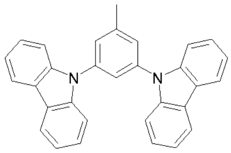

Formel (R1-64)

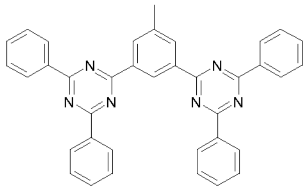

Formel (R1-65)

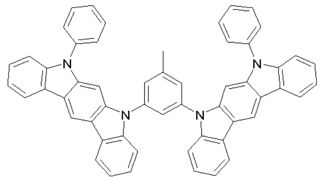

Formel (R1-66)

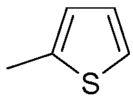

Formel (R1-67)

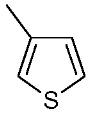

Formel (R1-68)

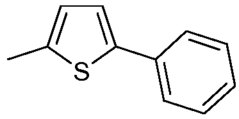

Formel (R1-69)

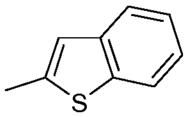

Formel (R1-70)

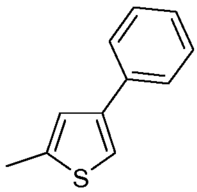

Formel (R1-71)

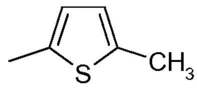

Formel (R1-72)

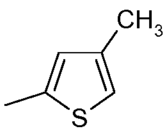

Formel (R1-73)

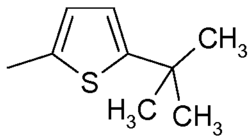

Formel (R1-74)

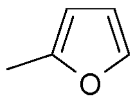

Formel (R1-75)

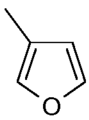

Formel (R1-76)

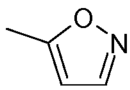

Formel (R1-77)

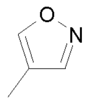

Formel (R1-78)

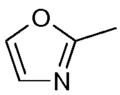

Formel (R1-79)

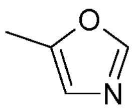

Formel (R1-80)

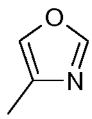

Formel (R1-81)

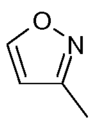

Formel (R1-82)

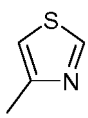

Formel (R1-83)

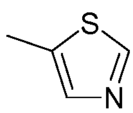

Formel (R1-84)

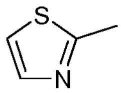
Formel (R1-85)

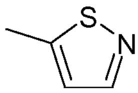
Formel (R1-86)

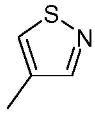
Formel (R1-87)

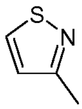
Formel (R1-88)

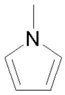
Formel (R1-89)

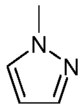
Formel (R1-90)

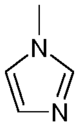
Formel (R1-91)

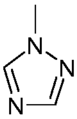
Formel (R1-92)

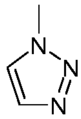
Formel (R1-93)

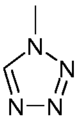
Formel (R1-94)

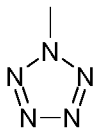
Formel (R1-95)

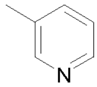
Formel (R1-96)

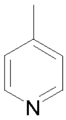
Formel (R1-97)

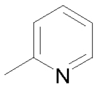
Formel (R1-98)

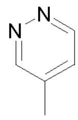
Formel (R1-99)

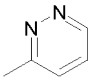
Formel (R1-100)

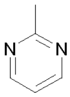
Formel (R1-101)

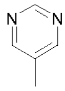
Formel (R1-102)

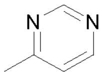
Formel (R1-103)

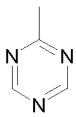
Formel (R1-104)

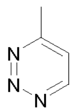
Formel (R1-105)

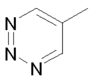
Formel (R1-106)

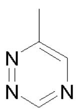
Formel (R1-107)

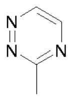
Formel (R1-108)

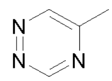
Formel (R1-109)

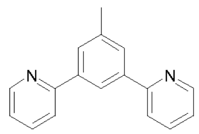
Formel (R1-110)

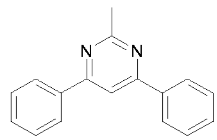
Formel (R1-111)

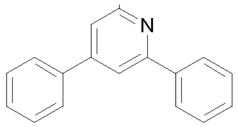
Formel (R1-112)

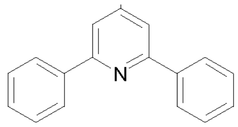
Formel (R1-113)

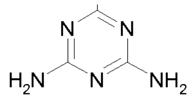
Formel (R1-114)

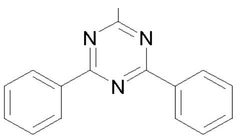
Formel (R1-115)

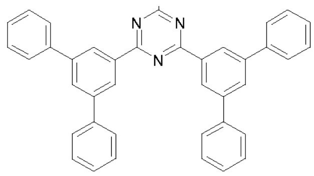
Formel (R1-116)

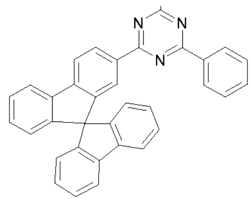
Formel (R1-117)

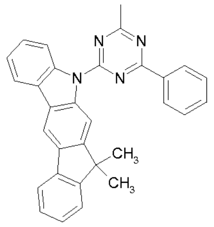
Formel (R1-118)

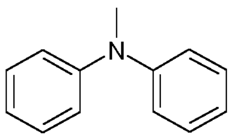
Formel (R1-119)

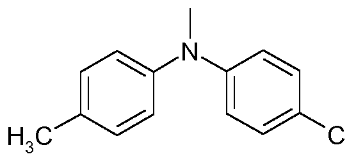
Formel (R1-120)

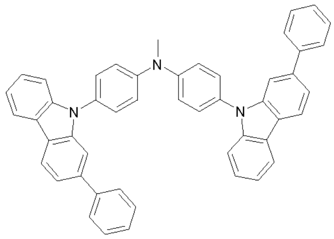
Formel (R1-121)

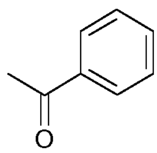
Formel (R1-122)

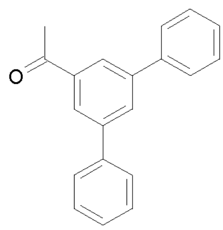
Formel (R1-123)

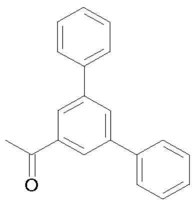
Formel (R1-124)

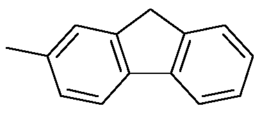
Formel (R1-125)

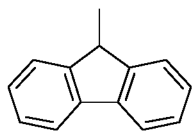
Formel (R1-126)

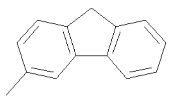
Formel (R1-127)

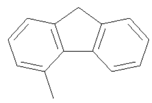
Formel (R1-128)

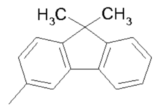
Formel (R1-129)

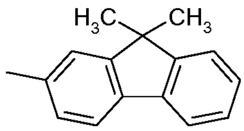
Formel (R1-130)

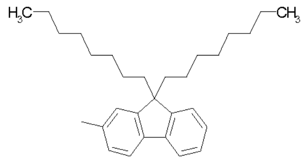
Formel (R1-131)

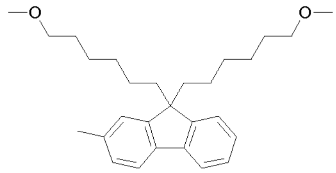
Formel (R1-132)

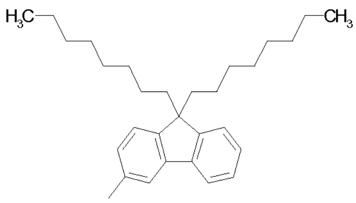
Formel (R1-133)

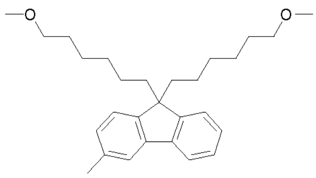
Formel (R1-134)

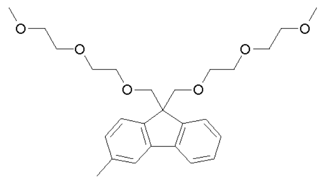
Formel (R1-135)

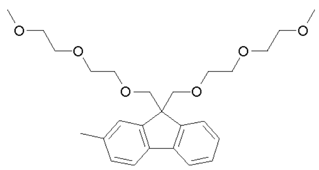
Formel (R1-136)

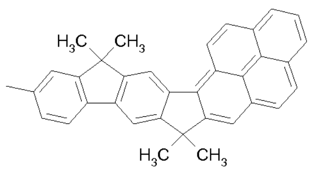
Formel (R1-137)

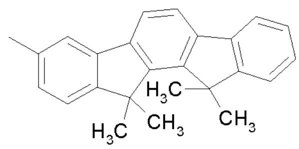
Formel (R1-138)

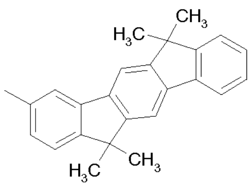
Formel (R1-139)

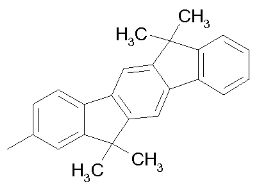
Formel (R1-140)

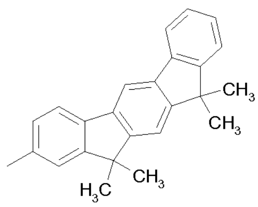
Formel (R1-141)

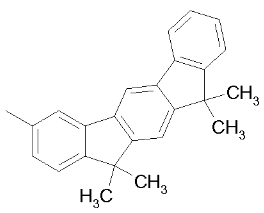
Formel (R1-142)

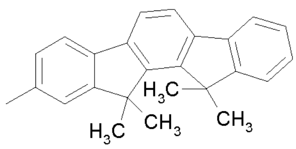
Formel (R1-143)

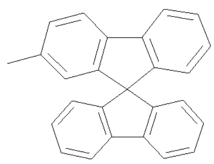
Formel (R1-144)

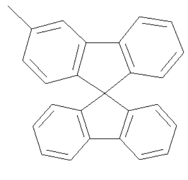

Formel (R1-145)

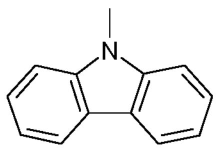

Formel (R1-146)

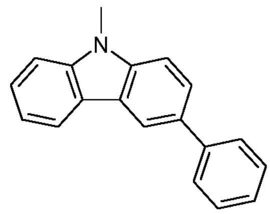

Formel (R1-147)

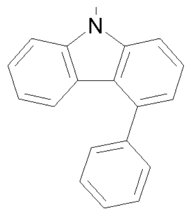

Formel (R1-148)

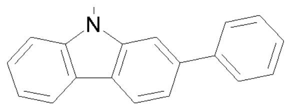

Formel (R1-149)

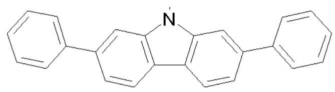

Formel (R1-150)

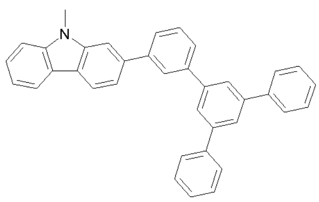

Formel (R1-151)

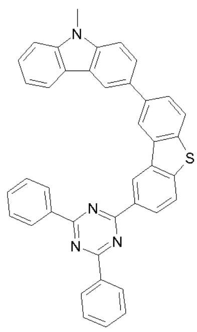

Formel (R1-152)

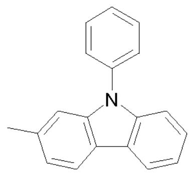

Formel (R1-153)

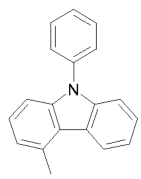

Formel (R1-154)

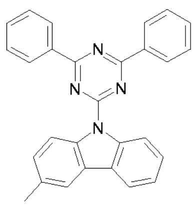

Formel (R1-155)

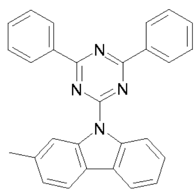

Formel (R1-156)

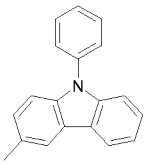

Formel (R1-157)

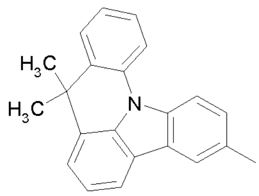

Formel (R1-158)

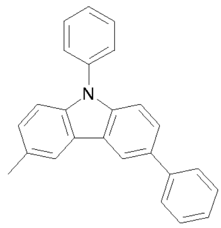

Formel (R1-159)

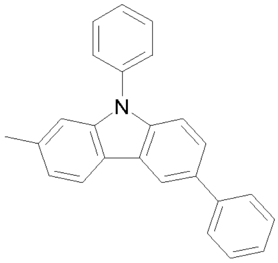

Formel (R1-160)

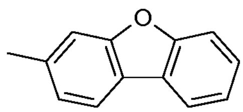

Formel (R1-161)

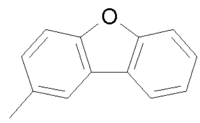

Formel (R1-162)

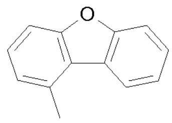

Formel (R1-163)

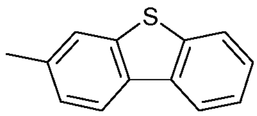

Formel (R1-164)

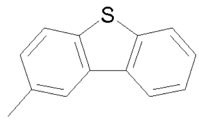

Formel (R1-165)

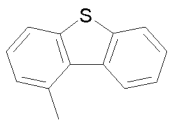

Formel (R1-166)

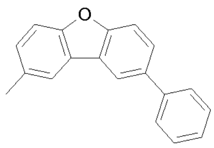

Formel (R1-167)

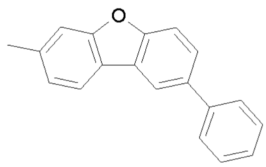

Formel (R1-168)

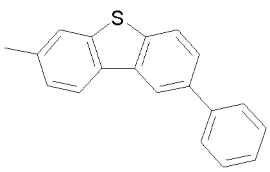

Formel (R1-169)

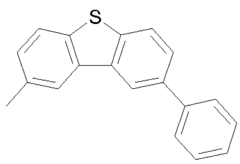

Formel (R1-170)

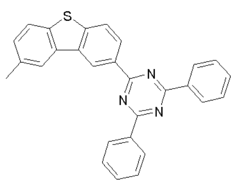

Formel (R1-171)

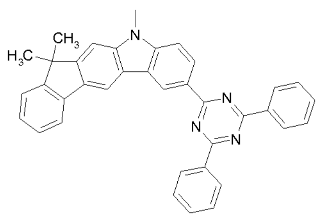

Formel (R1-172)

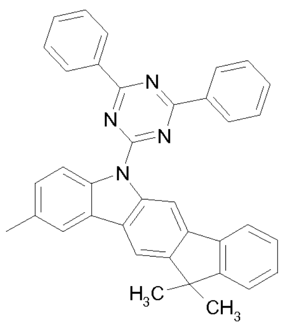

Formel (R1-173)

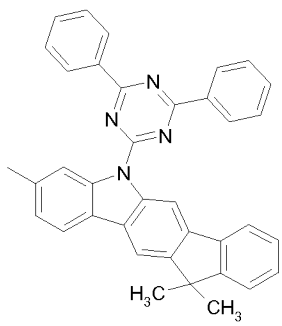

Formel (R1-174)

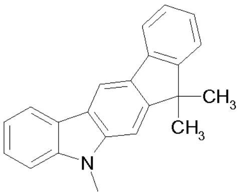

Formel (R1-175)

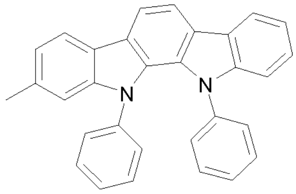

Formel (R1-176)

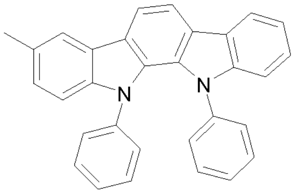

Formel (R1-177)

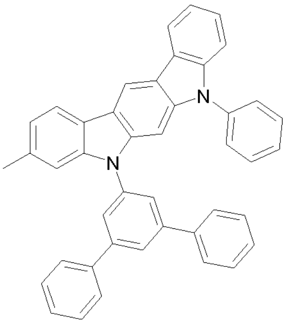

Formel (R1-178)

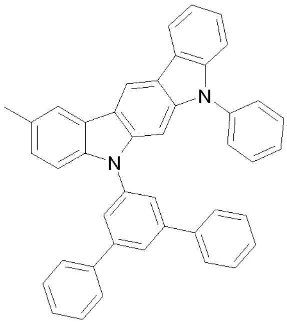

Formel (R1-179)

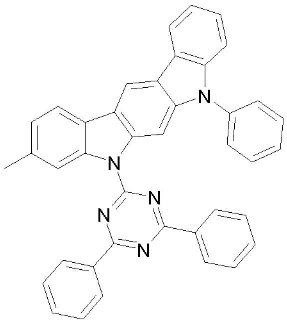

Formel (R1-180)

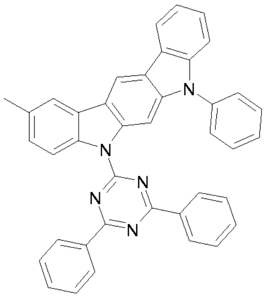

Formel (R1-181)

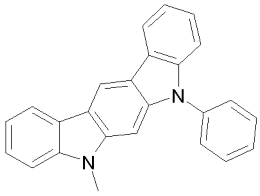

Formel (R1-182)

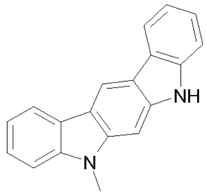

Formel (R1-183)

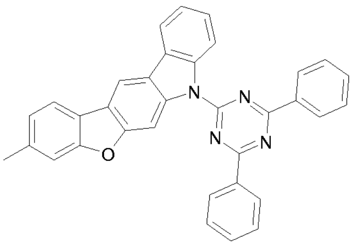

Formel (R1-184)

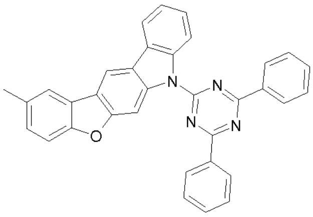

Formel (R1-185)

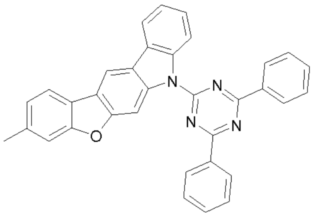

Formel (R1-186)

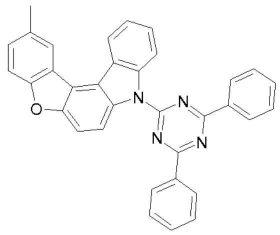

Formel (R1-187)

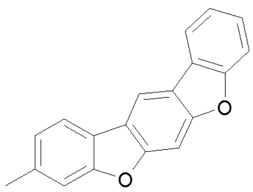

Formel (R1-188)

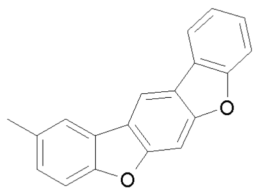

Formel (R1-189)

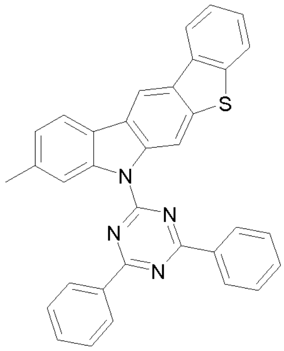

Formel (R1-190)

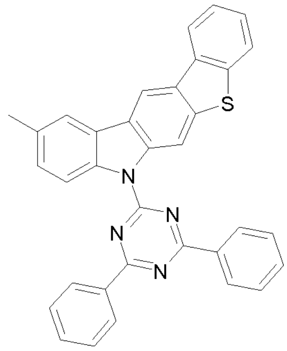

Formel (R1-191)

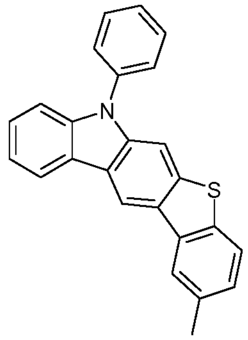

Formel (R1-192)

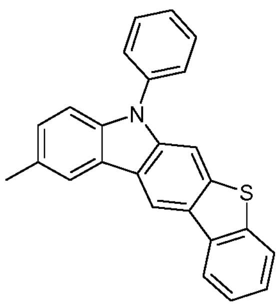

Formel (R1-193)

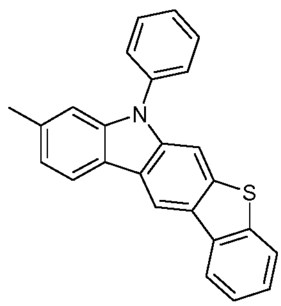

Formel (R1-194)

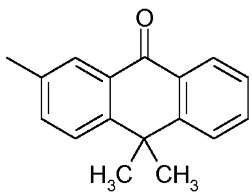

Formel (R1-195)

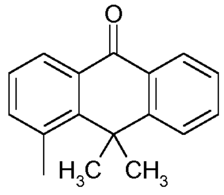

Formel (R1-196)

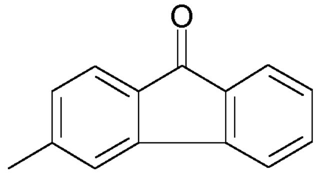

Formel (R1-197)

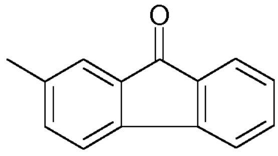

Formel (R1-198)

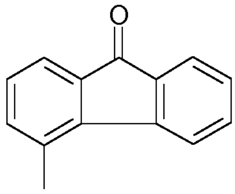

Formel (R1-199)

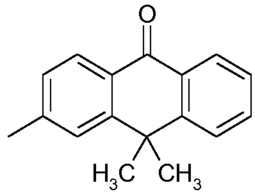

Formel (R1-200)

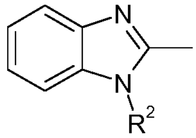

Formel (R1-201)

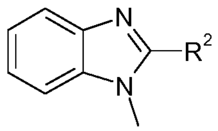

Formel (R1-202)

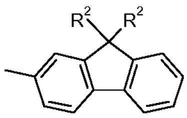

Formel (R1-203)

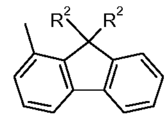

Formel (R1-204)

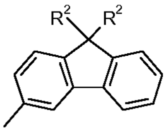

Formel (R1-205)

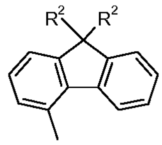

Formel (R1-206)

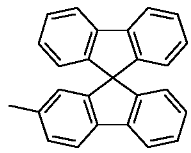

Formel (R1-207)

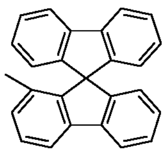

Formel (R1-208)

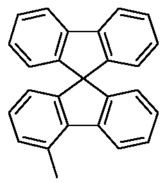

Formel (R1-209)

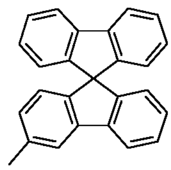

Formel (R1-210)

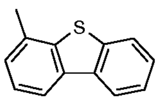

Formel (R1-211)

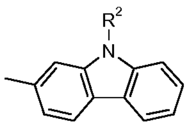

Formel (R1-212)

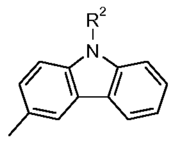

Formel (R1-213)

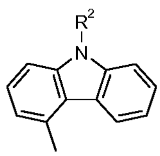

Formel (R1-214)

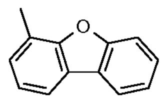

Formel (R1-215)

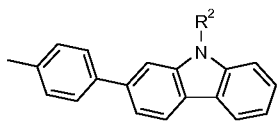

Formel (R1-216)

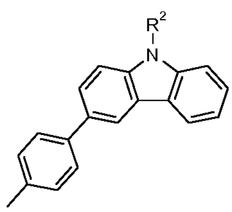

Formel (R1-217)

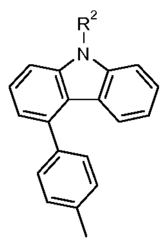

Formel (R1-218)

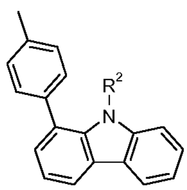

Formel (R1-219)

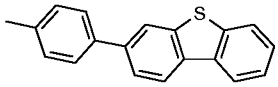

Formel (R1-220)

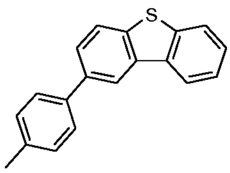

Formel (R1-221)

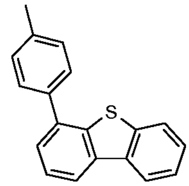

Formel (R1-222)

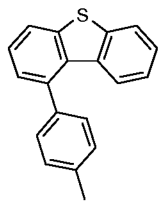

Formel (R1-223)

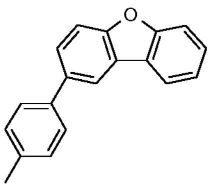

Formel (R1-224)

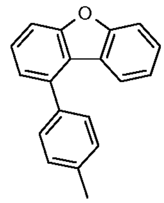

Formel (R1-225)

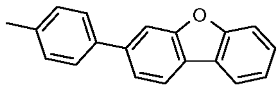

Formel (R1-226)

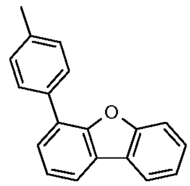

Formel (R1-227),

wobei der Bindungsstrich die Position der Verknüpfung des Restes $R^1$ kennzeichnet.

Besonders bevorzugte Reste stellen die der Formeln (R1-211), (R1-219), (R1-227) dar.

Die erfindungsgemäßen Verbindungen können unterschiedliche Funktionen in einer organischen elektronischen Vorrichtung wahrnehmen. Die Verbindungen können, beispielsweise, in der Emissionsschicht als fluoreszierender Emitter oder als Matrixmaterial eingesetzt werden. Weiterhin können die erfindungsgemäßen Verbindungen als Lochblockiermaterial (HBM) in einer Lochblockierschicht (HBL) oder in einer Elektronentransportschicht (ETL) als Elektronentransportmaterial (ETM) eingesetzt werden. Weiterhin können die erfindungsgemäßen Verbindungen als Lochinjektionsmaterial (HIM),
als Lochtransportmaterial (HTM) oder als Elektronenblockiermaterial (EBM) eingesetzt werden. Eine weitere typische Anwendung für die erfindungsgemäßen Verbindungen ist die Verwendung in einem Mixed-Matrix System für fluoreszierende und besonders bevorzugt für phosphoreszierende Emissionsschichten.

Die Verbindung kann dabei als Reinmaterial oder in Kombination mit anderen Materialien eingesetzt werden.

Der Fachmann weiß aufgrund des allgemeinen Fachwissens auf dem vorliegenden technischen Gebiet, dass in Abhängigkeit der Verwendung der erfindungsgemäßen Verbindung die Reste $R^1$ bis $R^3$ so gewählt werden müssen, dass die opto-elektronischen Eigenschaften der Verbindungen an die jeweiligen Anwendungen angepasst werden.

Wird die erfindungsgemäße Verbindung als elektronentransportierende Verbindung, beispielsweise in einer ETM, oder als elektronentransportierende Matrix in einer Emissionsschicht eingesetzt, dann enthält wenigstens einer der Reste $R^1$ bis $R^3$ eine elektronentransportierende Gruppe ETG.

Eine ETG ist eine organische elektronentransportierende Gruppe (ETG) aus der Gruppe der elektronenarmen heteroaromatischen Gruppen, wobei die ETG bevorzugt eine Heteroarylgruppe mit 5 bis 60 aromatischen Ringatomen, wobei N ganz bevorzugte Heteroatme darstellen und ganz besonders bevorzugte ETGs aus der Gruppe Triazine, Pyrimidine, Pyrazine, Pyrazole, Pyridazine, Chinole, Isochionoline, Thiazole, Benzothiazole, Oxazole, Benzoxazole, Imidazole, Benzimidazole und Pyridine ausgewählt sind.

Bevorzugte ETGs sind heteroaromatische Gruppen mit 6 aromatischen Ringatomen, von denen mindestens eines, bevorzugt 2 und ganz bevorzugt mindestens drei ein N-Atom ist, oder heteroaromatische Gruppen mit 5 aromatischen Ringatomen, von denen mindestens 2 Heteroatome sind, bevorzugt mindestens eines davon ein N-Atom, wobei an diese Gruppen jeweils auch weitere Aryl- oder Heteroarylgruppen ankondensiert sein können.

Bevorzugte Beispiele für elektronenarme heteroaromatische Gruppen sind: Pyridine, Pyrazine, Pyrimidine, Pyridazine, 1,2,4-Triazine, 1,3,5-Triazine, Chinoline, Isochinoline, Chinoxaline, Pyrazole, Imidazole, Benzimidazole, Thiazole, Benzothiazole, Oxazole oder Benzooxazole, die jeweils mit $R^1$ substituiert sein können. Noch mehr bevorzugt ist die elektronentransportierende Gruppe ein mit einem oder mehreren Resten $R^1$ substituiertes Pyridin, Pyrazin, Pyrimidin, Pyridazin und 1,3,5-Triazin.

Sofern die erfindungsgemäße Verbindung als elektronentransportierende Verbindung, beispielsweise als Matrix oder in einer ETL, eingesetzte wird ist weiterhin bevorzugt, wenn die Verbindung eine LUMO (lowest unoccupied molecular orbital) Energie aufweist, die niedriger als -1.3 eV ist, ganz bevorzugt niedriger als -2.5 eV und ganz besonders bevorzugt niedriger als -2.7 eV ist.

Sofern die erfindungsgemäße Verbindung als elektronentransportierende Verbindung, beispielsweise als Matrix oder in einer ETL, eingesetzte wird ist weiterhin bevorzugt wenn die Elektronenmobilität $\mu_-$ $10^{-6}$ $cm^2/(Vs)$ oder mehr beträgt, ganz bevorzugt beträgt sie $10^{-5}$ $cm^2/(Vs)$ oder mehr und ganz besonders bevorzugt beträgt sie $10^{-4}$ $cm^2/(Vs)$ oder mehr.

Wird die erfindungsgemäße Verbindung als lochtransportierende Verbindung, beispielsweise in einer HTM, oder als lochtransportierende Matrix in einer Emissionsschicht eingesetzt, dann enthält wenigstens einer der Reste $R^1$ bis $R^3$ eine lochtransportierende Gruppe LTG.

Eine LTG ist eine organische lochtransportierende Gruppe aus der Gruppe der elektronenreichen organische Gruppen, wobei die LTG bevorzugt ausgewählt ist aus der Gruppe der Arylamine, Triaryl-amine, der verbrückten Amine, bevorzugte verbrückte Amine sind dabei Dihydroacridine, Dihydrophenazine, Phenoxazione und Phenothiazine, Carbazole, verbrückten Carbazole, Biscarbazole, Indenocarbazole und Indolocarbazole.

Ist die erfindungsgemäße Verbindung eine lochtransportierende Verbindung, dann weist die Verbindung vorzugsweise eine HOMO Energie ($HOMO_L$) auf, die im Bereich der Elektronenausrittsarbeit der verwendeten Anode ($\phi_{Anode}$) zuzüglich +1.5 eV oder niedriger liegt, d.h. es gilt:

$$HOMO_L \leq (\phi_{Anode} + 1.5\ eV).$$

Wenn die verwendete Anode eine Elektronenaustrittsarbeit von -5 eV hat, so ist $HOMO_L$ -3.5 eV oder niedriger (d.h. negativer als -3.5 eV). Ganz bevorzugt ist, wenn $HOMO_L$ Energie gleich der Elektronenaustrittsarbeit der Anode oder niedriger ist, ganz besonders bevorzugt ist sie niedriger.

Ist die erfindungsgemäße Verbindung eine lochtransportierende Verbindung, dann weist die Verbindung eine Lochmobilität von $\mu_+$ $10^{-6}$ $cm^2/(Vs)$ oder mehr auf, ganz bevorzugt beträgt sie $10^{-5}$ $cm^2/(Vs)$ oder mehr und ganz besonders bevorzugt beträgt sie $10^{-4}$ $cm^2/(Vs)$ oder mehr.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands

mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

"Vernetzbare Gruppe" in Sinne der vorliegenden Erfindung bedeutet eine funktionelle Gruppe, die in der Lage ist, irreversibel zu reagieren. Dadurch wird ein vernetztes Material gebildet, das unlöslich ist. Die Vernetzung kann gewöhnlich durch Wärme oder durch UV-, Mikrowellen-, Röntgen-oder Elektronenstrahlung unterstützt werden. Durch die hohe Stabilität des Polymers kommt es bei der Vernetzung zu weniger Nebenproduktbildung. Zudem vernetzen die vernetzbaren Gruppen im Polymer sehr leicht, so dass geringere Energiemengen für die Vernetzung erforderlich sind (z.B. < 200°C bei der thermischen Vernetzung).

Beispiele für vernetzbare Gruppen Q sind Einheiten, die eine Doppelbindung, eine Dreifachbindung, eine Vorstufe, die zu einer in situ Bildung einer Doppel- bzw. Dreifachbindung in der Lage ist, oder einen heterocyclischen additionspolymerisierbaren Rest enthalten. Bevorzugte Reste Q umfassen Vinyl, Alkenyl, vorzugsweise Ethenyl und Propenyl, $C_{4-20}$-Cycloalkenyl, Azid, Oxiran, Oxetan, Di(hydrocarbyl)amino, Cyanatester, Hydroxy, Glycidylether, $C_{1-10}$-Alkylacrylat, $C_{1-10}$-Alkylmethacrylat, Alkenyloxy, vorzugsweise Ethenyloxy, Perfluoralkenyloxy, vorzugsweise Perfluorethenyloxy, Alkinyl, vorzugsweise Ethinyl, Maleimid, Tri($C_{1-4}$)-alkylsiloxy und Tri($C_{1-4}$)-alkylsilyl. Besonders bevorzugt ist Vinyl und Alkenyl.

Eine Arylgruppe im Sinne dieser Erfindung enthält 6 bis 40 C-Atome; eine Heteroarylgruppe im Sinne dieser Erfindung enthält 1 bis 39 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (annellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 59 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verknüpft sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe unterbrochen sind. Weiterhin sollen Systeme, in denen mehrere Aryl- und/oder Heteroarylgruppen durch eine Einfachbindung miteinander verknüpft sind, wie z. B. Biphenyl, Terphenyl oder Bipyridin, als aromatisches bzw. heteroaromatisches Ringsystem verstanden werden.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder $CH_2$-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte $CH_2$-Gruppen durch $R^1C=CR^1$, $C\equiv C$, $Si(R^1)_2$, $Ge(R^1)_2$, $Sn(R^1)_2$, C=O, C=S, C=Se, $C=NR^1$, $P(=O)(R^1)$, SO, $SO_2$, $NR^1$, O, S oder $CONR^1$ ersetzt sein können; weiterhin können auch ein oder mehrere H-Atome durch D, F, Cl, Br, I, CN oder $NO_2$, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches

noch jeweils mit den oben genannten Resten $R^1$ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

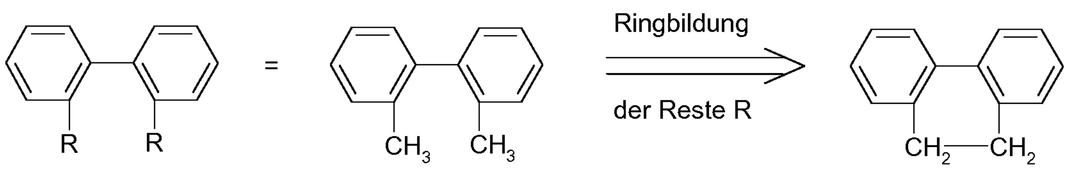


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

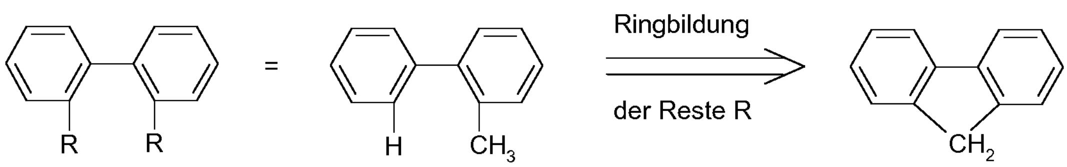


Die erfindungsgemäßen Verbindungen können gemäß der folgenden Schemata 1 bis 7 dargestellt werden, wobei sich die ersten drei Schemata auf die Herstellung von Dinaphthooxepinen beziehen und sich die Schemata 4 bis 6 auf die Herstellung von Tribenzoxepinen beziehen. Der Fachmann hat dabei keinerlei Schwierigkeiten, die hierin angegebenen Syntheserouten im Rahmen des allgemeinen Fachwissen auf die Synthese weiterer Derivate erfolgreich anzuwenden.

Die entsprechenden Mono- und Dibromide können durch NBS-Bromierung hergestellt werden. Reaktion von diesen Mono- und Dibromide via Suzuki-Kupplung mit den entsprechenden Arylboronsäuren oder via Buchwald-Kupplung oder Ullmann-Kupplung mit entsprechende Arylhalogeniede führt zu gewünschten Zielverbindungen.

Aus den Bromiden können auch die entsprechende Boronsäuren hergestellt werden, die wiederum via Suzuki-Kupplung weiter umgesetzt werden können.

**Schema 1**: X bedeutet $CR^1$

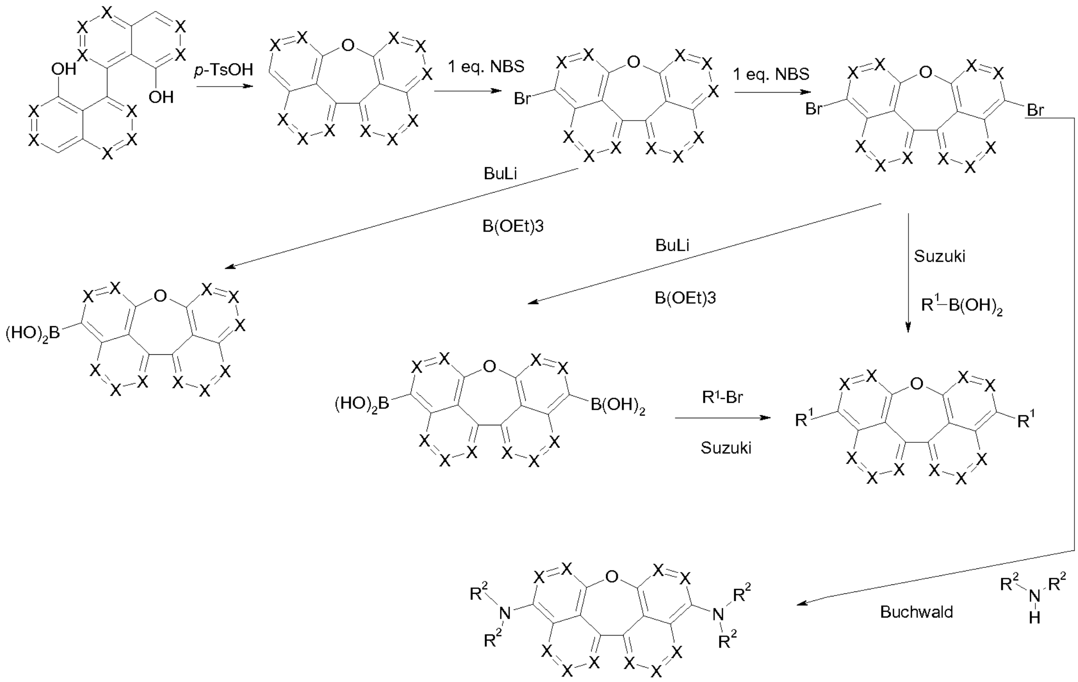


In einem weiterem Schritte können so erhaltene Verbindungen wieder selektive mit NBS bromiert werden und via Suzuki oder Buchwald-Kupplung oder Ullmann-Kupplung zu weitergewünschten Zielverbindungen umgesetzt werden (Schema 2 und 3).

**Schema 2**: X bedeutet $CR^1$

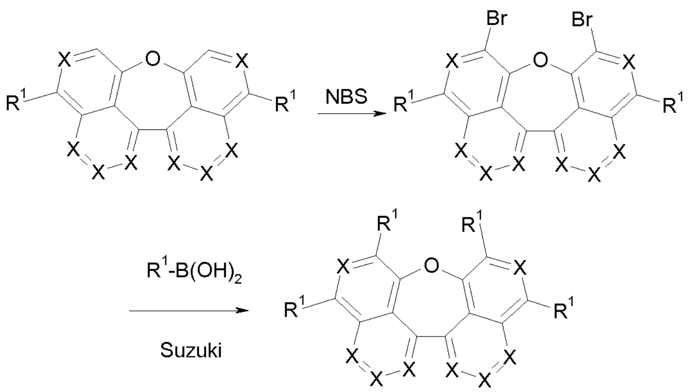

**Schema 3**: X bedeutet $CR^1$

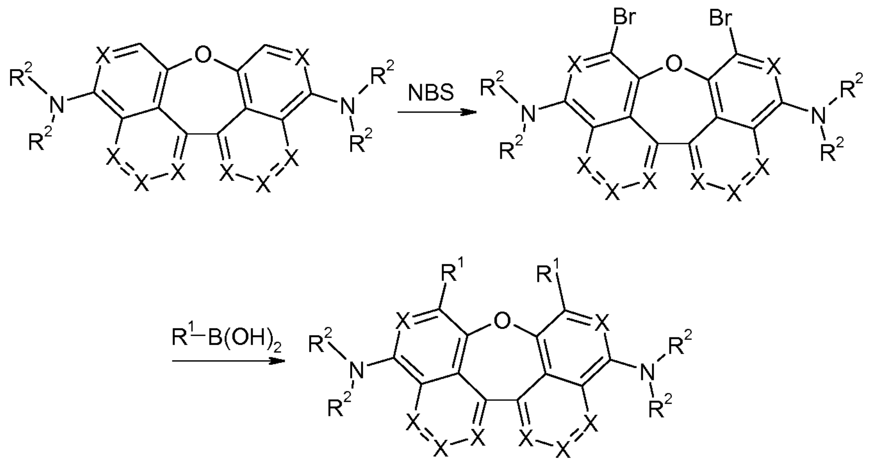


Erfindungsgemäße Tribenz[a,c,e]-oxepine können nach dem folgenden allgemeinen Schema hergestellt werden.

**Schema 4**: X bedeutet $CR^1$

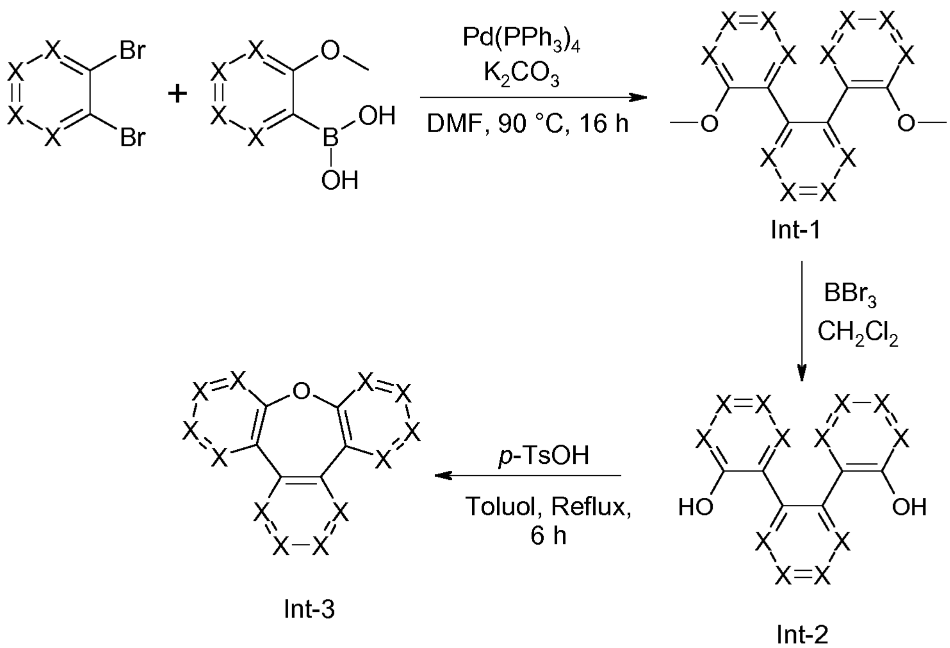


Int-1

Int-2

Int-3

**Schema 5:** X bedeutet $CR^1$

Eine Funktionalisierung zur weiteren Substitution erfolgt durch ortho-Metallierung mit n Butyllithium und durch Bromierung mit N-Bromsuccinimid.

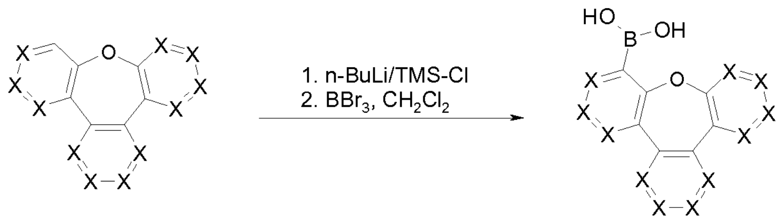


Int-3 Int-4

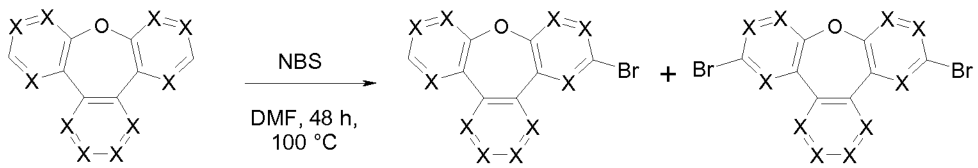


Int-3 Int-5 Int-6

**Schema 6**: X bedeutet $CR^1$

Weitere Derivatisierungen von Tribenz[a,c,e]oxepin können wie folgt vorgenommen werden.

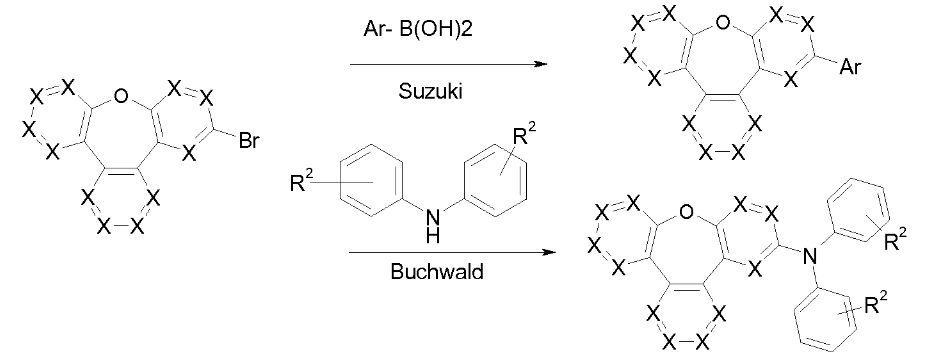


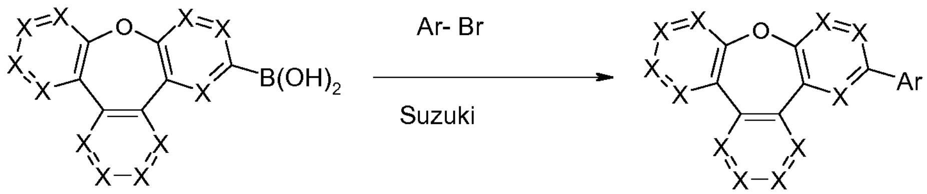


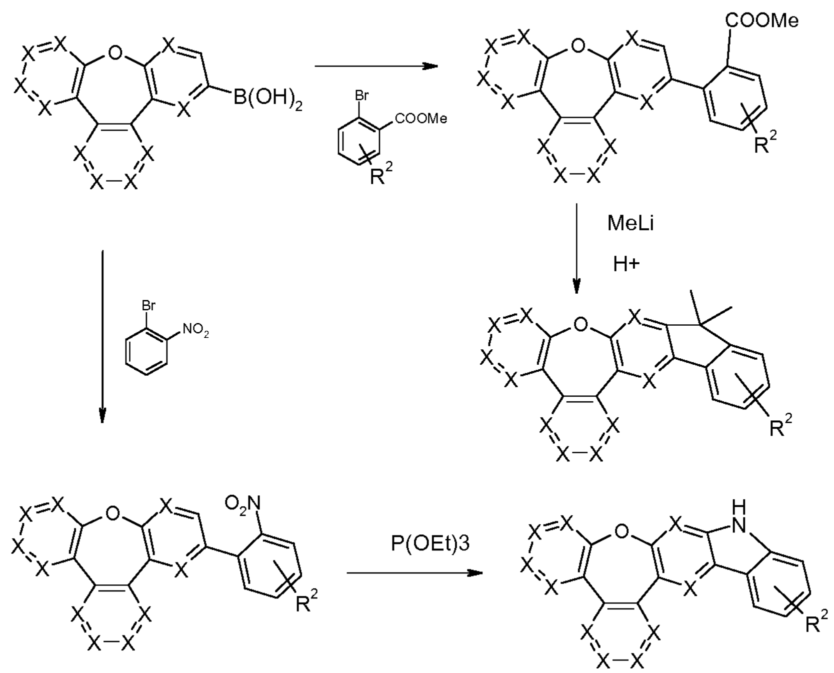


wobei Ar für eine aromatische oder heteroaromatische Gruppe gemäß der Definition von $R^1$ steht.

Die Herstellung der entsprechenden Dibromide bzw. Diboronsäuren zeigt das folgende Schema.

**Schema 7:** X bedeutet $CR^1$

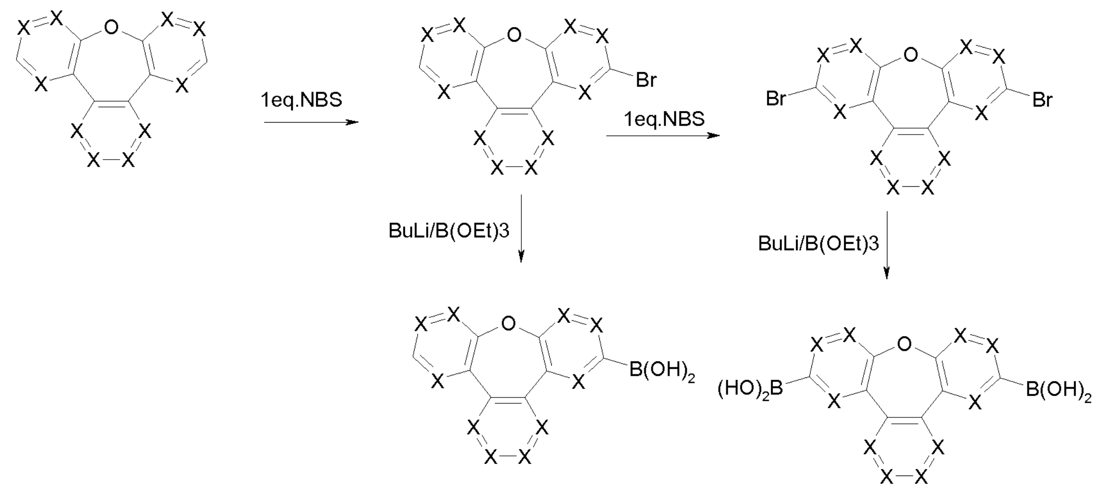


Die folgende Übersicht enthält eine beispielhafte Darstellung erfindungsgemäßer Verbindungen, die nach einem der hierin beschriebenen Verfahren hergestellt werden können.

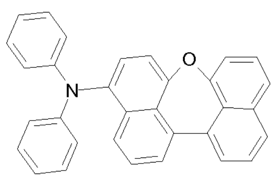
Formel (A-1)

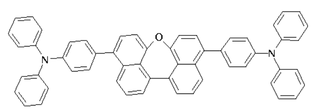
Formel (A-2)

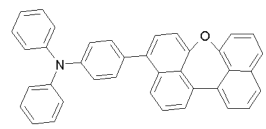
Formel (A-3)

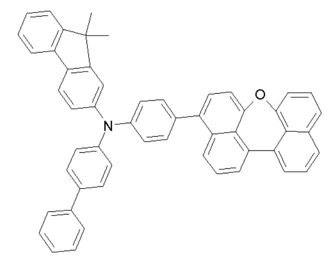
Formel (A-4)

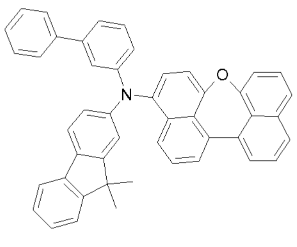
Formel (A-5)

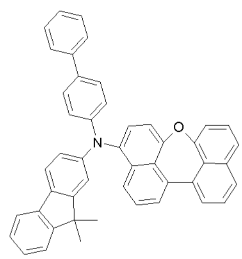
Formel (A-6)

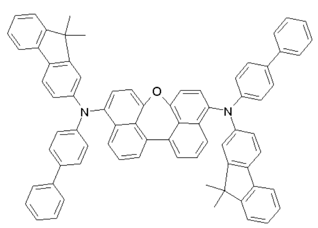
Formel (A-7)

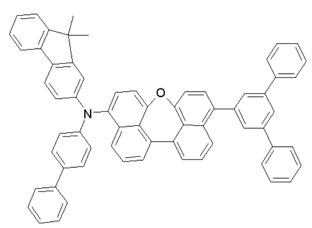
Formel (A-8)

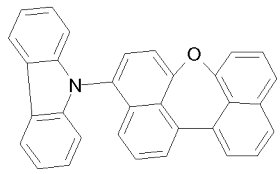
Formel (A-9)

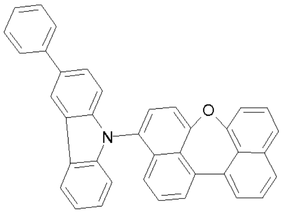

Formel (A-10)

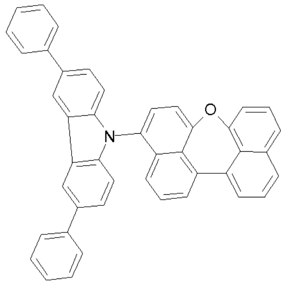

Formel (A-11)

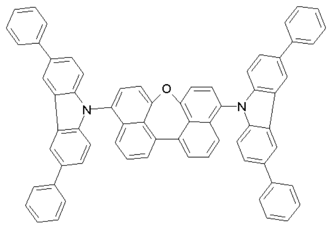

Formel (A-12)

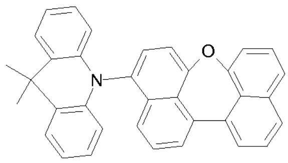

Formel (A-13)

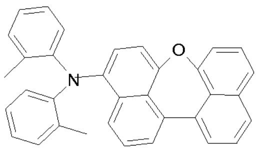

Formel (A-14)

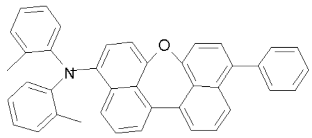

Formel (A-15)

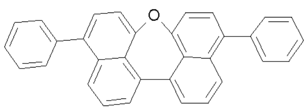

Formel (A-16~~7~~)

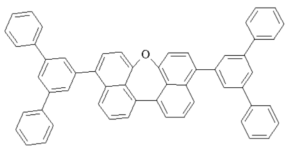

Formel (A-17)

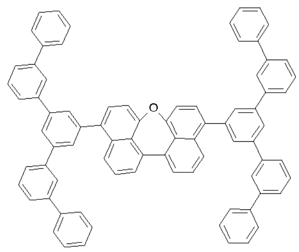

Formel (A-18)

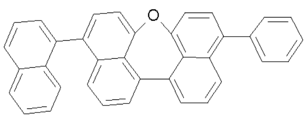

Formel (A-19)

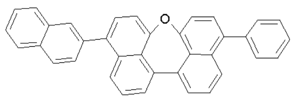

Formel (A-20)

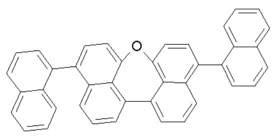

Formel (A-21)

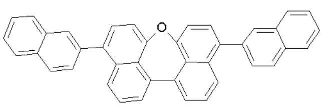

Formel (A-22)

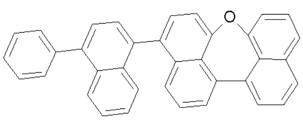

Formel (A-23)

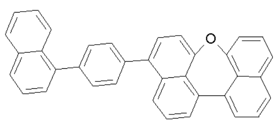

Formel (A-24)

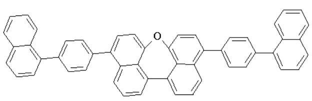

Formel (A-25)

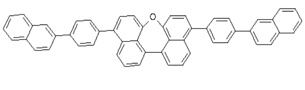

Formel (A-26)

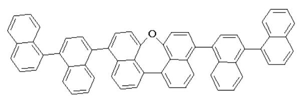

Formel (A-27)

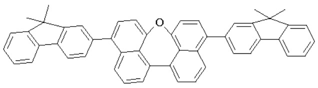

Formel (A-28)

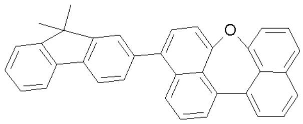

Formel (A-29)

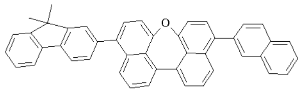

Formel (A-30)

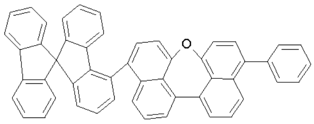

Formel (A-31)

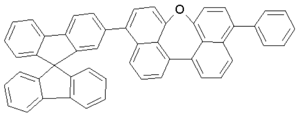

Formel (A-32)

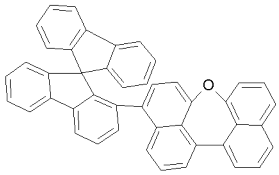

Formel (A-33)

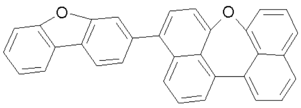

Formel (A-34)

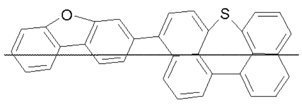

~~Formel (A-35)~~

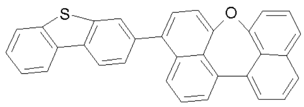

Formel (A-36)

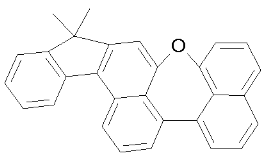

Formel (A-40)

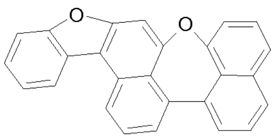

Formel (A-41)

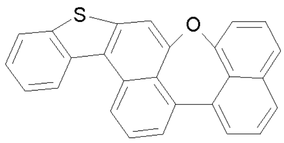

Formel (A-42)

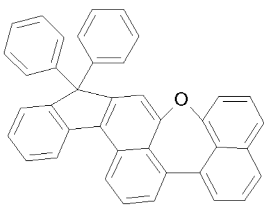

Formel (A-43)

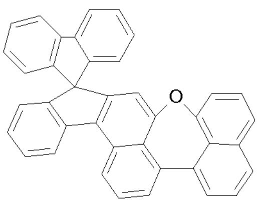

Formel (A-44)

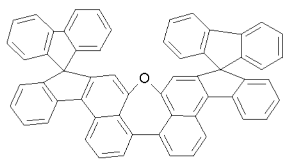

Formel (A-45)

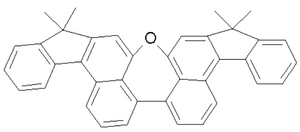

Formel (A-46)

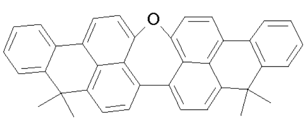

Formel (A-49)

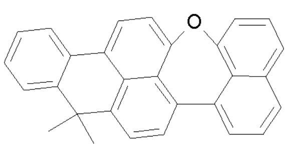

Formel (A-50)

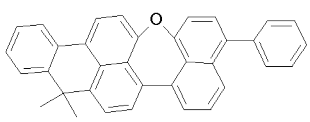

Formel (A-51)

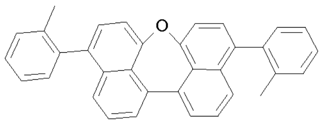
Formel (A-5~~4~~2)

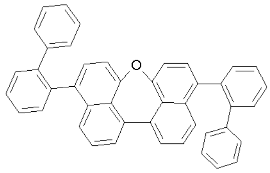
Formel (A-53)

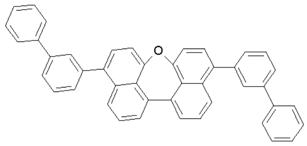
Formel (A-54)

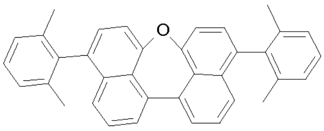
Formel (A-55)

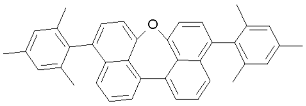
Formel (A-56)

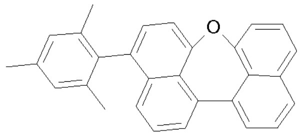
Formel (A-57)

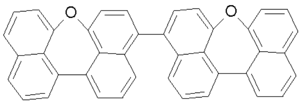
Formel (A-58)

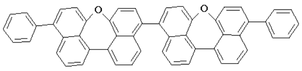
Formel (A-59)

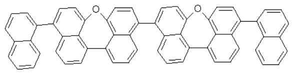
Formel (A-60)

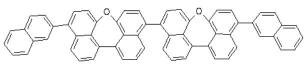
Formel (A-61)

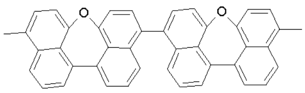
Formel (A-62)

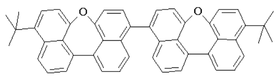
Formel (A-63)

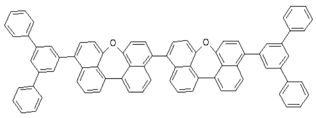
Formel (A-64)

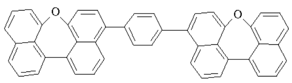
Formel (A-65)

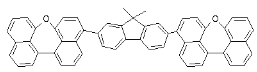
Formel (A-66)

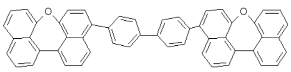
Formel (A-67)

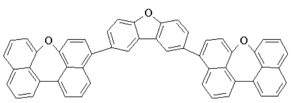
Formel (A-68)

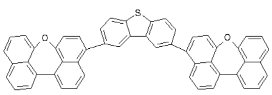
Formel (A-69)

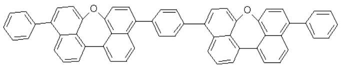
Formel (A-69a~~50~~)

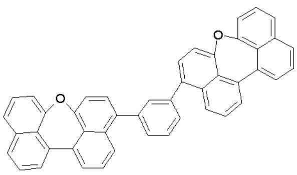
Formel (A-69b~~51~~)

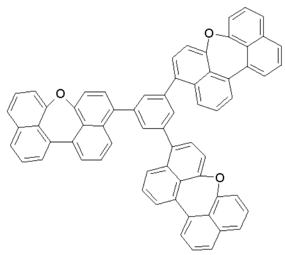
Formel (A-69c~~52~~)

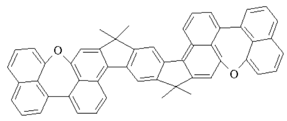

Formel (A-569d3)

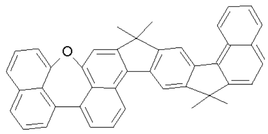

Formel (A-69e54)

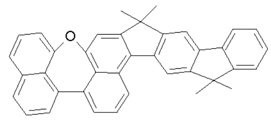

Formel (A-69f55)

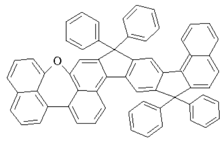

Formel (A-56g)

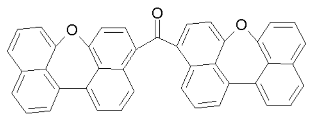

Formel (A-5769h)

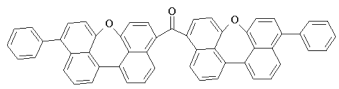

Formel (A-69i58)

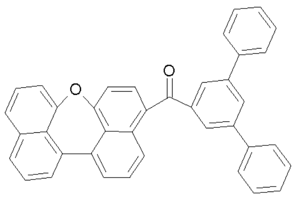

Formel (A-659j)

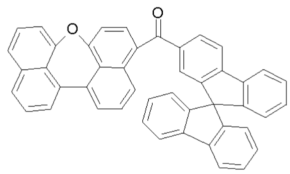

Formel (A-69k0)

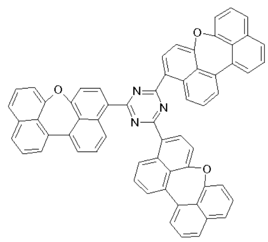

Formel (A-69l1)

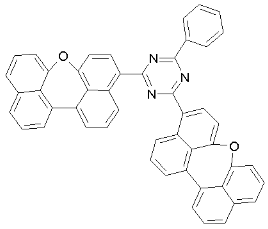

Formel (A-69m2)

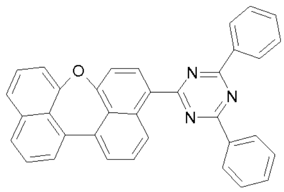

Formel (A-69n3)

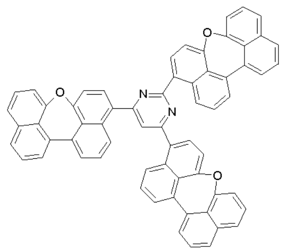

Formel (A-69o4)

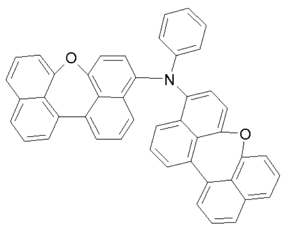

Formel (A-69q5)

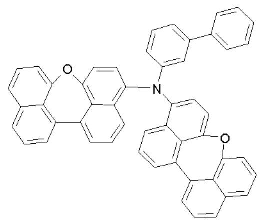

Formel (A-69r6)

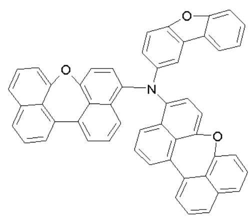

Formel (A-69s7)

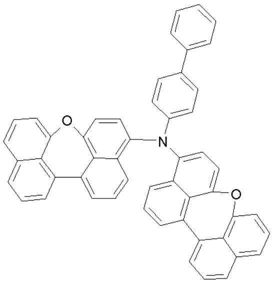

Formel (A-69t8)

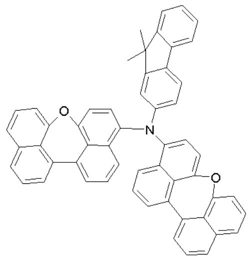

Formel (A-69u)

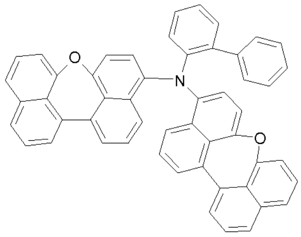

Formel (A-70)

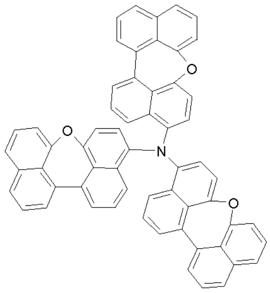

Formel (A-71)

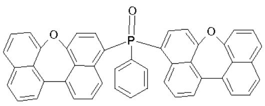

Formel (A-72)

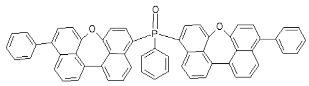

Formel (A-73)

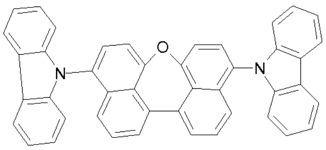

Formel (A-74)

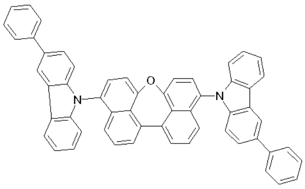

Formel (A-75)

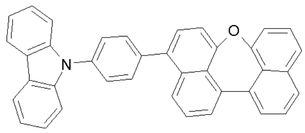

Formel (A-76)

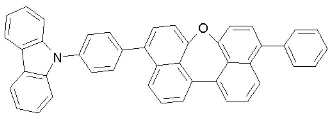

Formel (A-77)

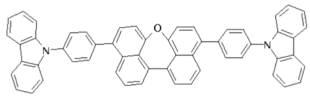

Formel (A-78)

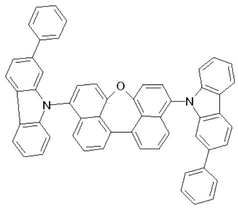

Formel (A-79)

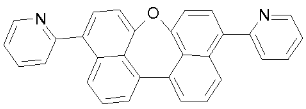

Formel (A-80)

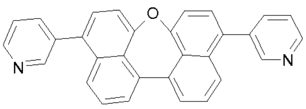

Formel (A-81)

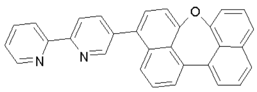

Formel (A-82)

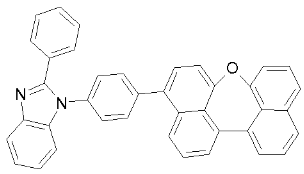
Formel (A-83)

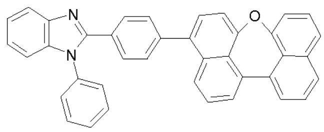
Formel (A-84)

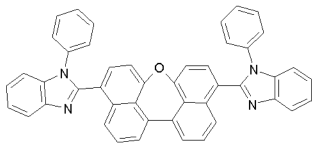
Formel (A-85)

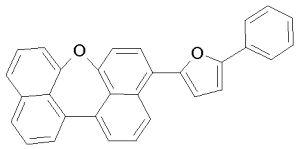
Formel (A-86)

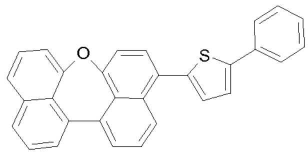
Formel (A-87)

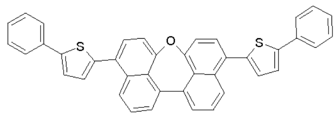
Formel (A-88)

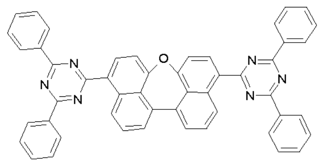
Formel (A-89)

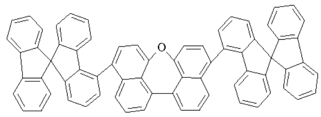
Formel (A-90)

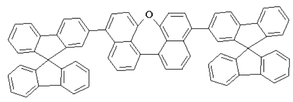
Formel (A-91)

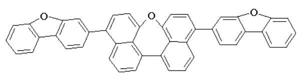
Formel (A-92)

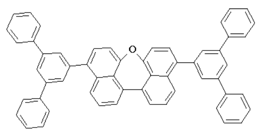
Formel (A-93)

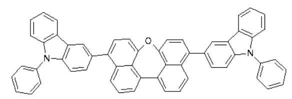
Formel (A-94)

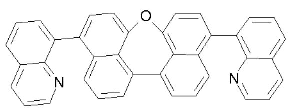
Formel (A-95)

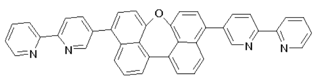
Formel (A-96)

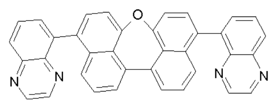
Formel (A-97)

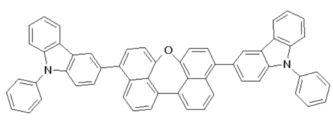
Formel (A-98)

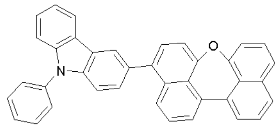
Formel (A-99)

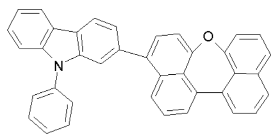
Formel (A-100)

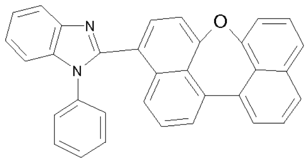
Formel (A-101)

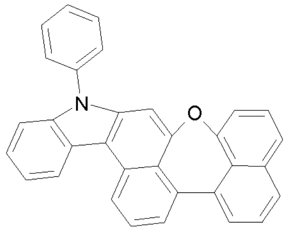
Formel (A-102)

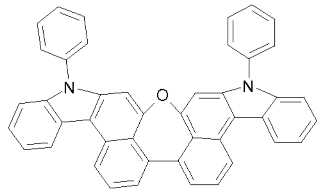
Formel (A-103)

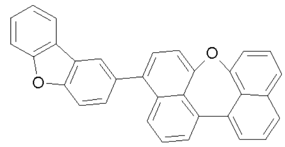
Formel (A-104)

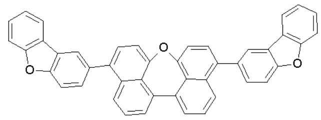
Formel (A-105)

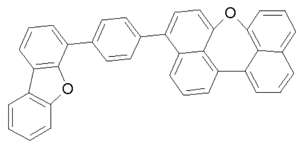
Formel (A-106)

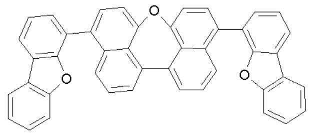
Formel (A-107)

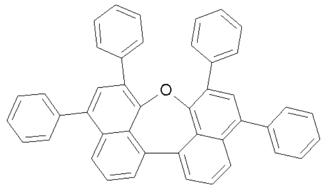
Formel (A-108)

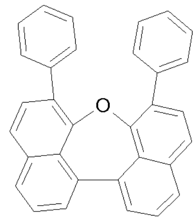
Formel (A-109)

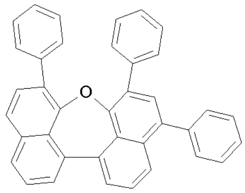
Formel (A-110)

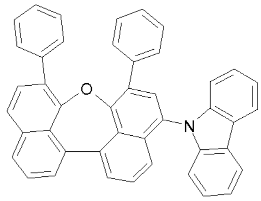
Formel (A-111)

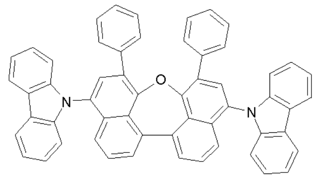
Formel (A-112)

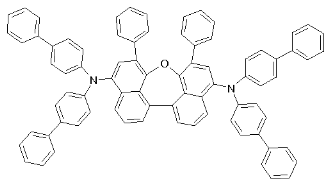
Formel (A-113)

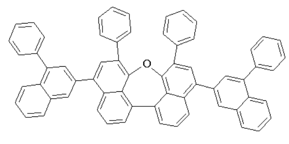
Formel (A-114)

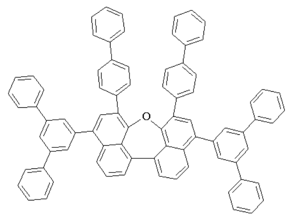
Formel (A-115)

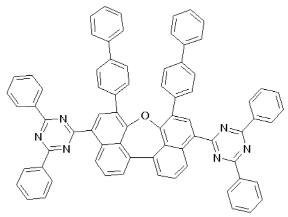
Formel (A-116)

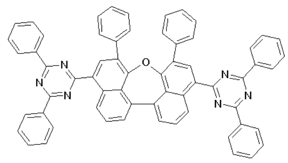
Formel (A-117)

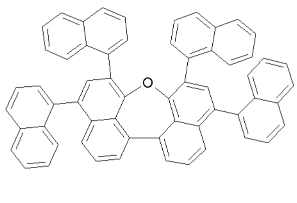
Formel (A-118)

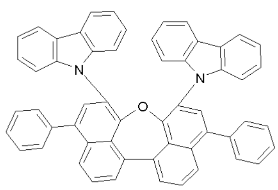
Formel (A-119)

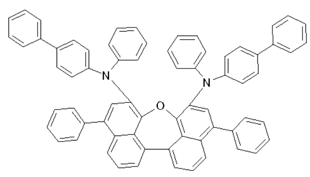
Formel (A-120)

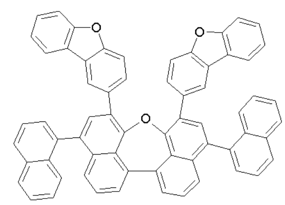
Formel (A-121)

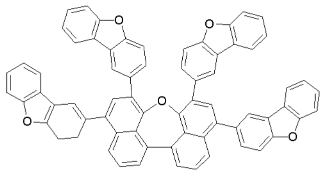
Formel (A-122)

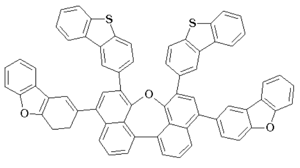
Formel (A-123)

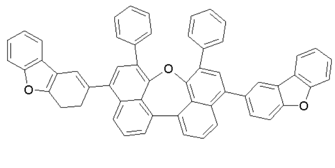
Formel (A-124)

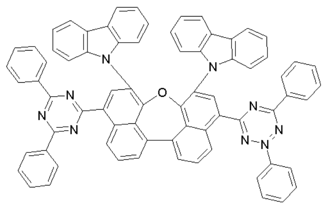
Formel (A-125)

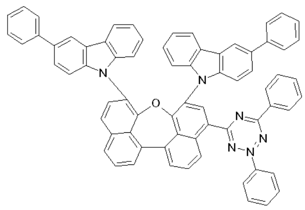
Formel (A-126)

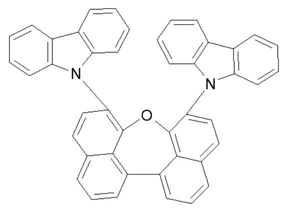
Formel (A-127)

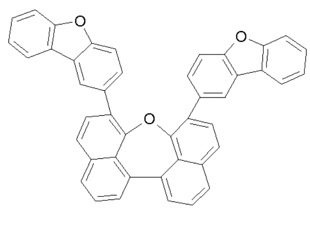
Formel (A-128)

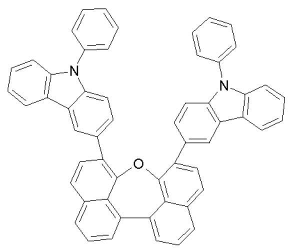
Formel (A-129)

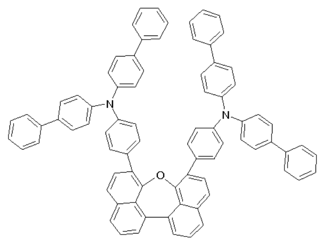
Formel (A-130)

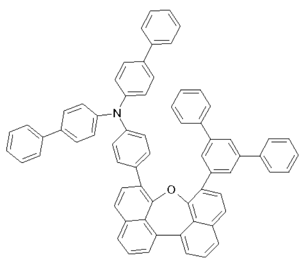
Formel (A-131)

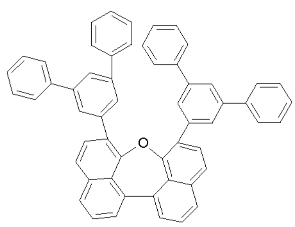
Formel (A-132)

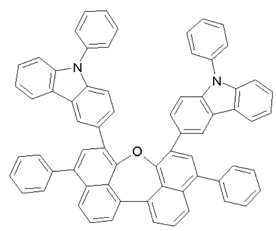
Formel (A-133)

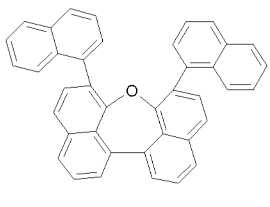
Formel (A-134)

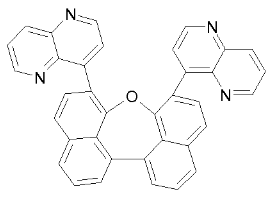
Formel (A-135)

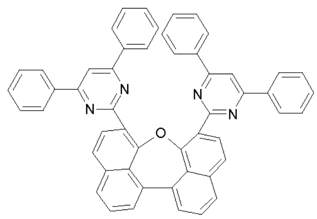
Formel (A-136)

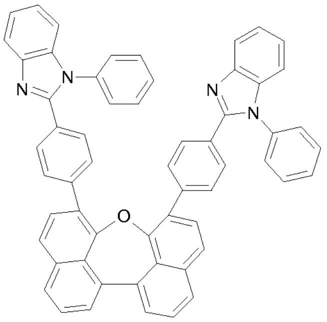
Formel (A-137)

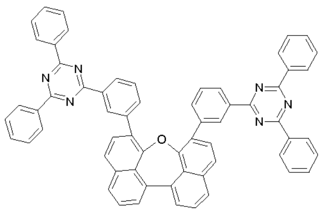
Formel (A-138)

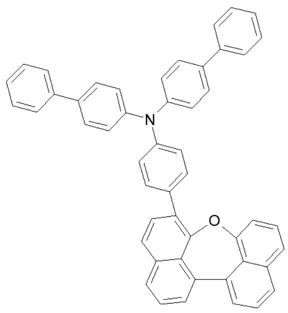
Formel (A-139)

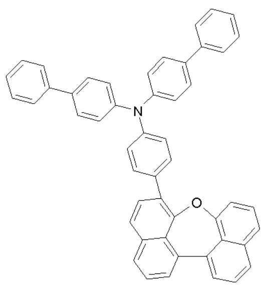
Formel (A-140)

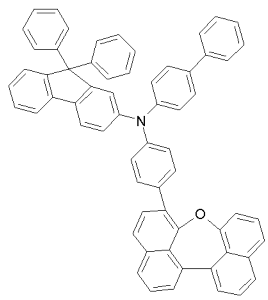
Formel (A-141)

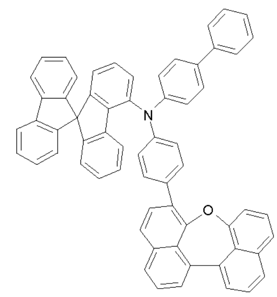
Formel (A-142)

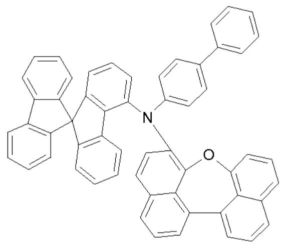
Formel (A-143)

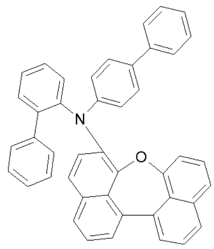
Formel (A-144)

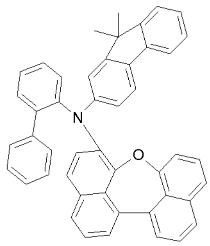
Formel (A-145)

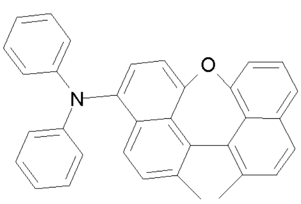
Formel (A-146)

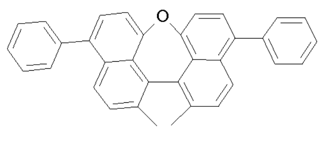
Formel (A-147)

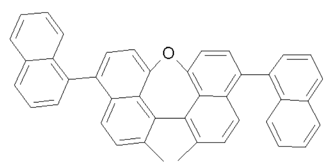
Formel (A-148)

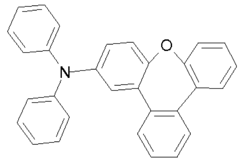
Formel (A-149)

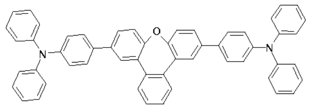
Formel (A-149a)

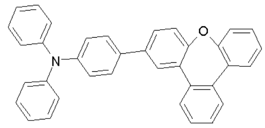
Formel (A-149b)

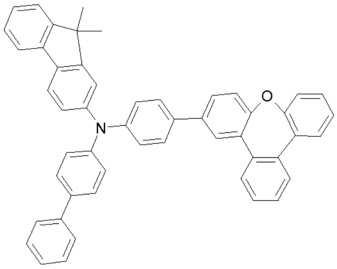

Formel (A-150)

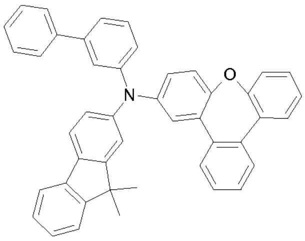

Formel (A-151)

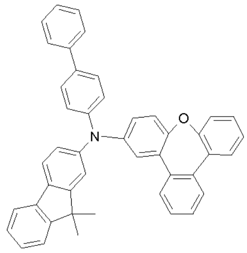

Formel (A-152)

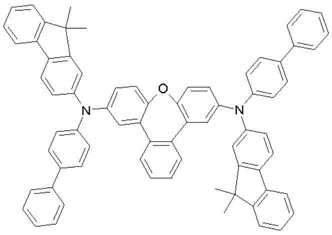

Formel (A-153)

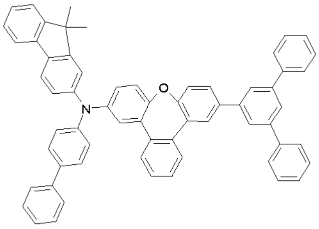

Formel (A-154)

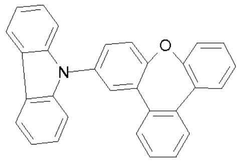

Formel (A-155)

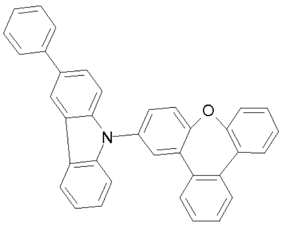

Formel (A-156)

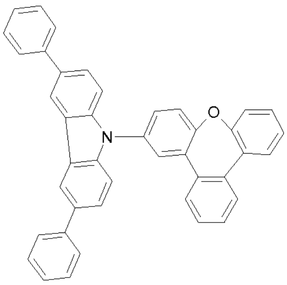

Formel (A-157)

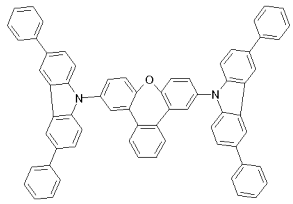

Formel (A-158)

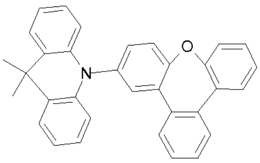

Formel (A-159)

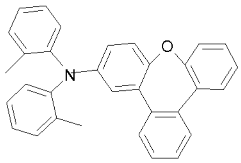

Formel (A-160)

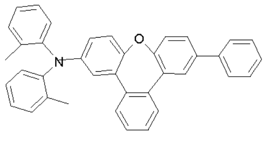

Formel (A-161)

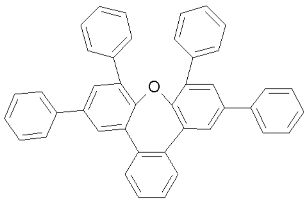

Formel (A-162)

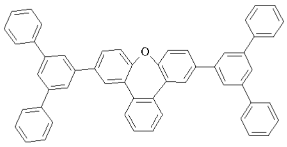

Formel (A-163)

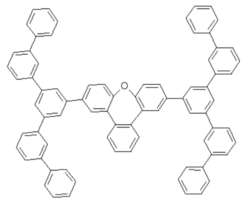

Formel (A-164)

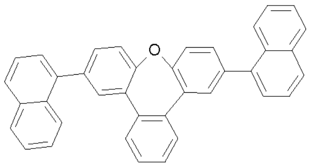

Formel (A-165)

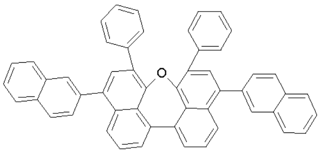

Formel (A-166)

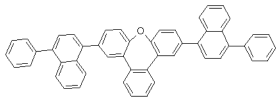

Formel (A-167)

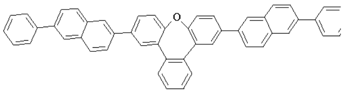

Formel (A-168)

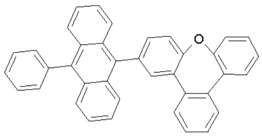

Formel (A-169)

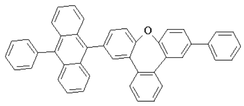

Formel (A-170)

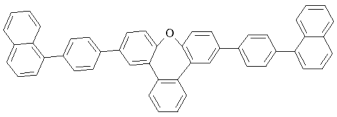

Formel (A-171)

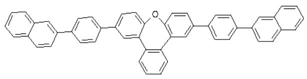

Formel (A-172)

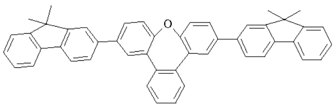

Formel (A-174)

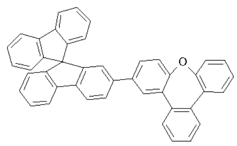

Formel (A-175)

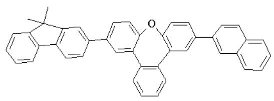

Formel (A-176)

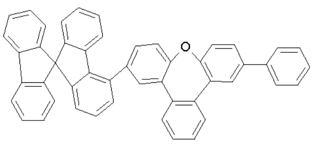

Formel (A-177)

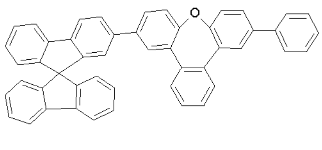

Formel (A-178)

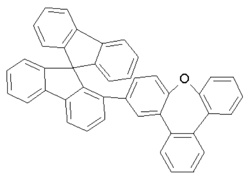

Formel (A-179)

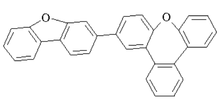

Formel (A-180)

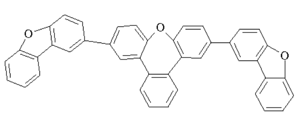

Formel (A-181)

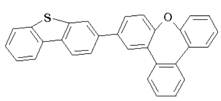

Formel (A-182)

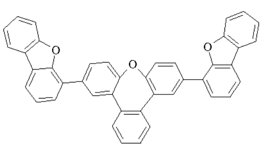

Formel (A-183)

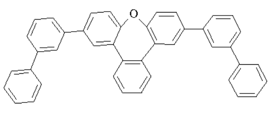

Formel (A-184)

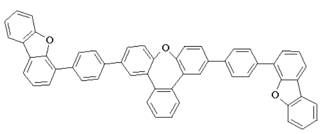

Formel (A-185)

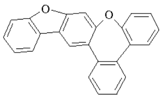

Formel (A-187)

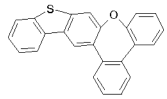

Formel (A-188)

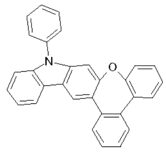

Formel (A-190)

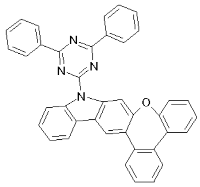

Formel (A-193)

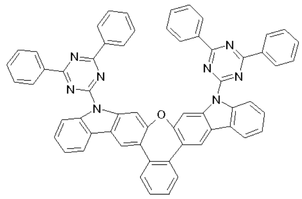

Formel (A-194)

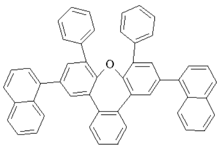

Formel (A-195)

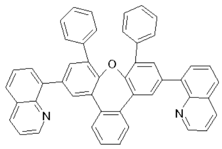

Formel (A-196)

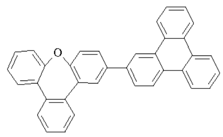

Formel (A-197)

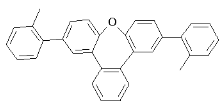

Formel (A-198)

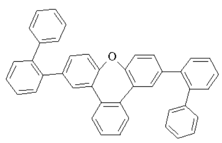

Formel (A-199)

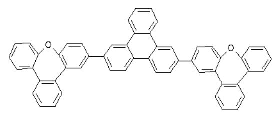

Formel (A-200)

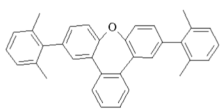

Formel (A-201)

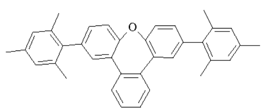

Formel (A-202)

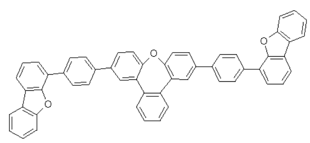

Formel (A-203)

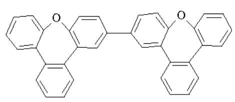

Formel (A-204)

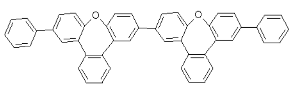

Formel (A-205)

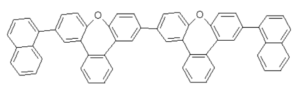

Formel (A-206)

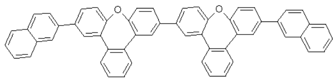
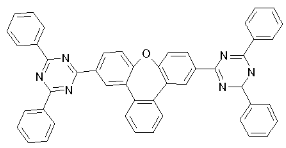
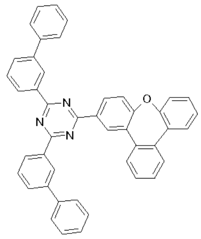

Formel (A-207) Formel (A-208) Formel (A-209)

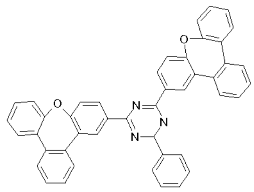
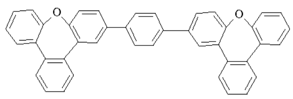
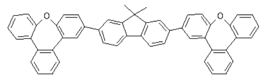

Formel (A-210) Formel (A-211) Formel (A-212)

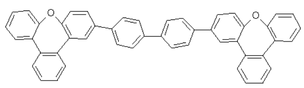
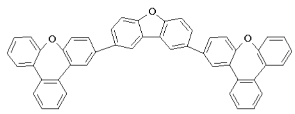
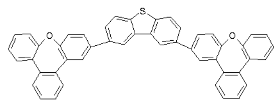

Formel (A-213) Formel (A-214) Formel (A-215)

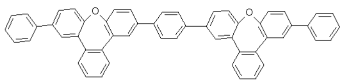
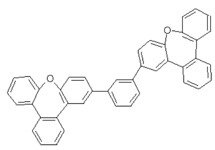
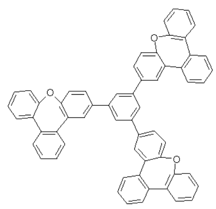

Formel (A-216) Formel (A-217) Formel (A-218)

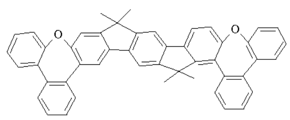
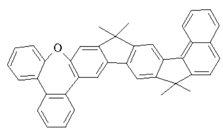
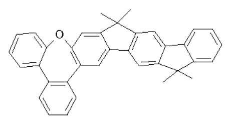

Formel (A-219) Formel (A-220) Formel (A-221)

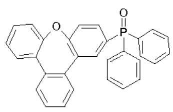
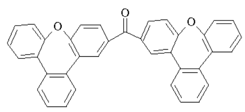
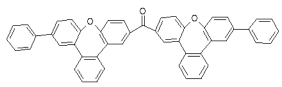

Formel (A-222) Formel (A-223) Formel (A-224)

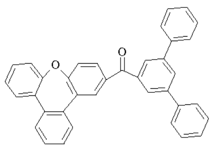
Formel (A-225)

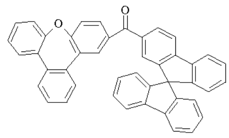
Formel (A-226)

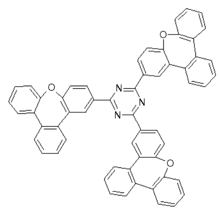
Formel (A-227)

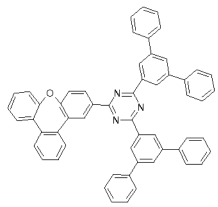
Formel (A-228)

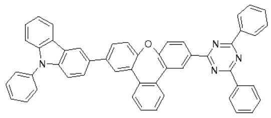
Formel (A-229)

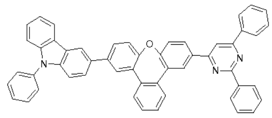
Formel (A-230)

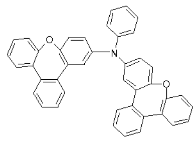
Formel (A-231)

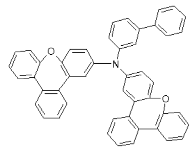
Formel (A-232)

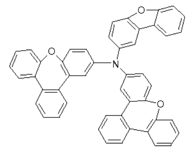
Formel (A-233)

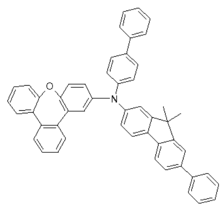
Formel (A-234)

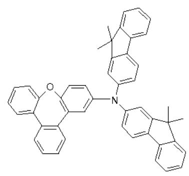
Formel (A-235)

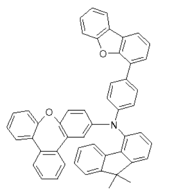
Formel (A-236)

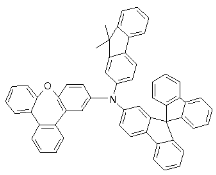
Formel (A-237)

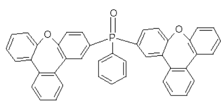
Formel (A-238)

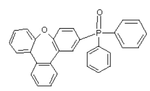
Formel (A-239)

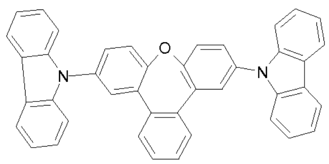
Formel (A-240)

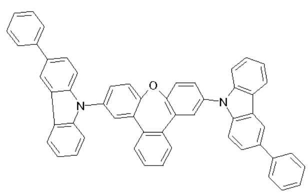
Formel (A-241)

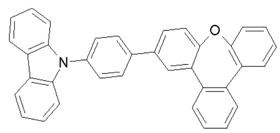
Formel (A-242)

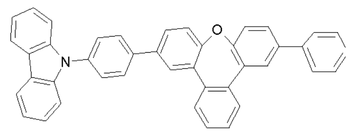

Formel (A-243)

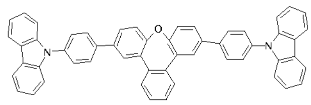

Formel (A-244)

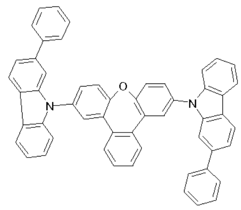

Formel (A-245)

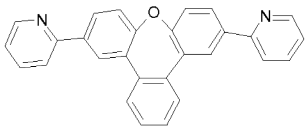

Formel (A-246)

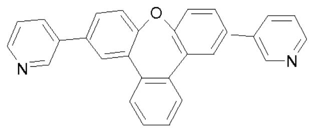

Formel (A-247)

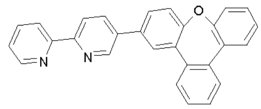

Formel (A-248)

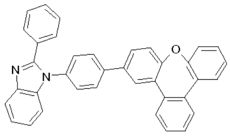

Formel (A-249)

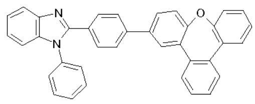

Formel (A-250)

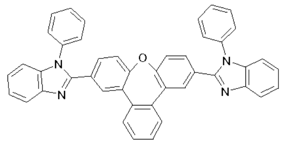

Formel (A-251)

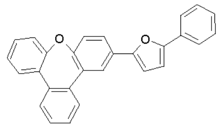

Formel (A-252)

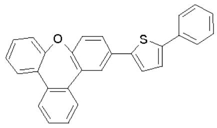

Formel (A-253)

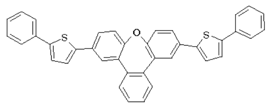

Formel (A-254)

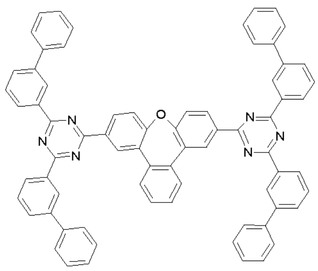

Formel (A-255)

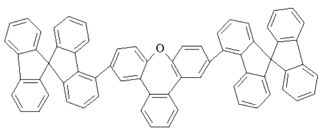

Formel (A-256)

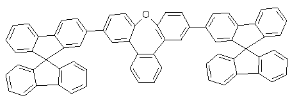

Formel (A-257)

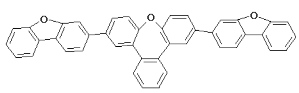

Formel (A-258)

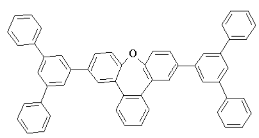

Formel (A-259)

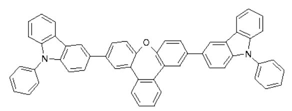

Formel (A-260)

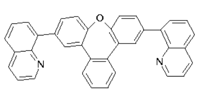

Formel (A-261)

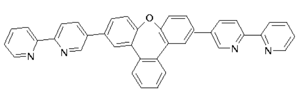

Formel (A-262)

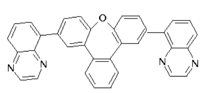

Formel (A-263)

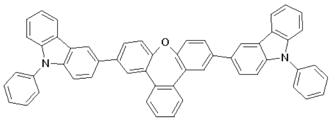
Formel (A-264)

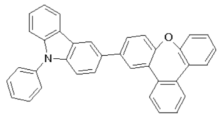
Formel (A-265)

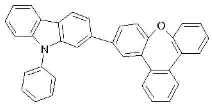
Formel (A-266)

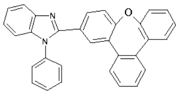
Formel (A-267)

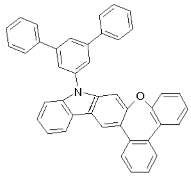
Formel (A-268)

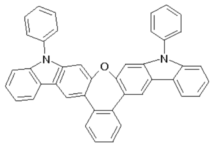
Formel (A-269)

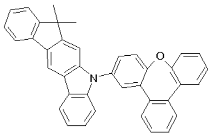
Formel (A-270)

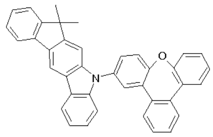
Formel (A-271)

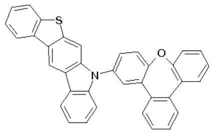
Formel (A-272)

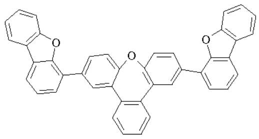
Formel (A-273)

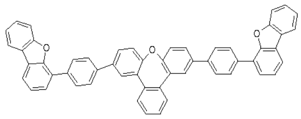
Formel (A-274)

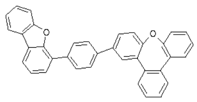
Formel (A-275)

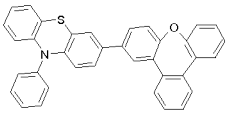
Formel (A-276)

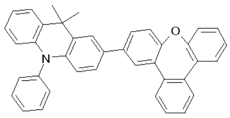
Formel (A-277)

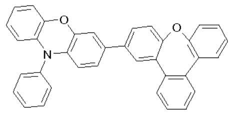
Formel (A-278)

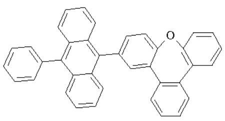
Formel (A-279)

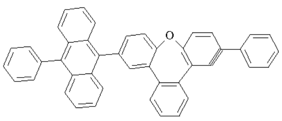
Formel (A-280)

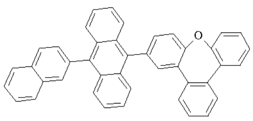
Formel (A-281)

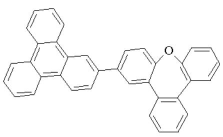
Formel (A-282)

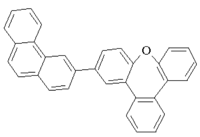
Formel (A-283)

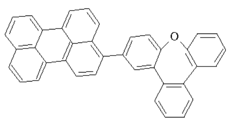
Formel (A-284)

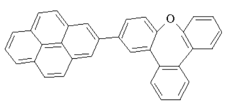
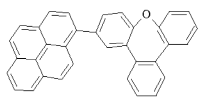
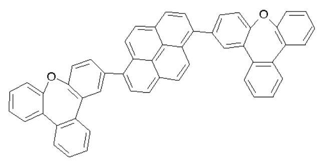

Formel (A-285) Formel (A-286) Formel (A-287)

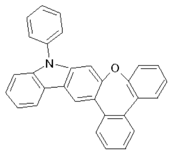
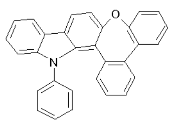
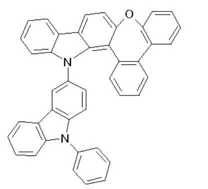

Formel (A-288) Formel (A-289) Formel (A-290)

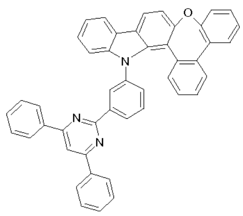
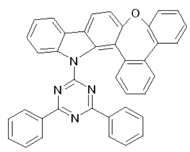
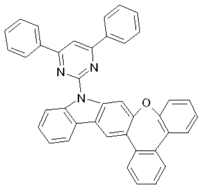

Formel (A-291) Formel (A-292) Formel (A-293).

Die erfindungsgemäßen Verbndungen können in einer organischen elektronischen Vorrichtung, bevorzugt in einer ladungstransportierenden und/oder in einer emittierenden Schicht verwendet werden.
Die vorliegende Erfindung betrifft daher auch eine organische elektronische Vorrichtung enthaltend wenigstens eine Verbindung der allgemeinen Formel (5) oder (7) nach Anspruch 1.

Die erfindungsgemäße elektronische Vorrichtung ist bevorzugt gewählt aus den organischen integrierten Schaltungen (OICs), organischen Feld-Effekt-Transistoren (OFETs), organischen Dünnfilmtransistoren (OTFTs), organischen Solarzellen (OSCs), organischen optischen Detektoren, organischen Photorezeptoren, organischen Feld-Quench-Devices (OFQDs), organischen Elektrolumineszenzvorrichtungen, wobei die organischen Elektrolumineszenzvorrichtungen ganz bevorzugt sind.
Zu den organischen Elektrolumineszenzvorrichtungen zählen insbesondere die hierin bevorzugten organischen lichtemittierenden Transistoren (OLETs), die organischen lichtemittierenden elektrochemischen Zellen (OLECs, LECs oder LEECs), die organischen Laserdioden (O-Laser), die polymeren lichtemittierenden Dioden (PLED) und die organischen lichtemittierenden Dioden (OLEDs), wobei die OLECs, PLEDs und OLEDs ganz bevorzugt im Sinne der vorliegenden Erfindung sind und wobei die PLEDs und OLEDs besonders bevorzugt und die OLEDs ganz besonders bevorzugt sind.
Die organische Schicht enthaltend die Verbindung der Formel (5) oder (7) ist bevorzugt eine Schicht mit ladungstransportierender Funktion. Besonders bevorzugt ist sie eine Elektroneninjektionsschicht, Elektronentransportschicht, eine Lochblockier- und Lochtransportschicht oder eine emittierende Schicht.
Die Verbindung der Formel (5) oder (7) kann als Emitter in einer Emissionsschicht eingesetzt werden. Daher betrifft die vorliegende Erfindung eine organische elektronische Vorrichtung, bevorzugt eine organische Elektrolumineszenzvorrichtung, enthaltend wenigstens eine Verbindung der
allgemeinen Formel (5) oder (7) in der Emissionsschicht als fluoreszierenden Emitter. Die Emissionsschicht kann dabei weitere Materialien enthalten. Typischerweise handelt es sich dabei um Matrix-oder Hostmaterialien oder Mischungen aus Hostmaterialien.

Wenn die Verbindung der allgemeinen Formel (5) oder (7) als Emitter in einer emittierenden Schicht, insbesondere in einer fluoreszierenden Schicht, einer organischen Elektrolumineszenzvorrichtung eingesetzt wird, dann beträgt ihr Anteil an der gesamte Schicht bevorzugt zwischen 0,1 und 50 Vol.-%, ganz bevorzugt zwischen 0,5 und 20 Vol.-% und besonders bevorzugt zwischen 1,0 und 10 Vol.-%. Entsprechend beträgt der Anteil der Matrix oder des Hosts bevorzugt zwischen 50 und 99,9 Vol.-%, ganz bevorzugt zwischen 80 und 99,5 Vol.-% und besonders bevorzugt zwischen 90 und 99 Vol.-%.

Weiterhin betrifft die vorliegende Erfindung auch eine organische Elektrolumineszenzvorrichtung enthaltend wenigstens eine Verbindung der Formel (5) oder (7) als Matrix in einer Emissionsschicht sowie wenigstens einen fluoreszierenden Emitter, auch fluoreszierender Dotand genannt.

Wenn die Verbindung der allgemeinen Formel (5) oder (7) als Matrix oder Host in einer emittierenden Schicht, insbesondere in einer fluoreszierenden Schicht, einer organischen Elektrolumineszenzvorrichtung eingesetzt wird, dann beträgt ihr Anteil an der gesamte Schicht bevorzugt zwischen 50 und 99,9 Vol.-%, ganz bevorzugt zwischen 80 und 99,5 Vol.-% und besonders bevorzugt zwischen 90 und 99 Vol.-%. Entsprechend beträgt der Anteil des Emitters (Dotanden) bevorzugt zwischen 0,01 und 50 Vol.-%, ganz bevorzugt zwischen 0,1 und 20 Vol.-%,besonders bevorzugt zwischen 0,5 und 15 Vol.-% und ganz besonders bevorzugt zwischen 1 und 10 Vol.-% und

Weiterhin betrifft die vorliegende Erfindung auch eine elektronische Vorrichtung, insbesondere eine organische Elektrolumineszenzvorrichtung, enthaltend wenigstens eine Verbindung der Formel (5) oder (7) in einer Elektronentransportschicht (ETL).

Die ETL kann dabei weitere Materialien enthalten. Insbesondere kommen dabei weitere ETMs sowie n-Dotanden in Betracht. Unter n-Dotanden werden hierin Reduktionsmittel, d.h. Elektronendonatoren verstanden. Bevorzugte Beispiele für n-Dotanden sind $W(hpp)_4$ und weitere elektronenreiche Metallkomplexe gemäß WO 2005/086251 A2, P=N-Verbindungen (z.B. WO 2012/175535 A1, WO 2012/175219 A1), Naphthylencarbodiimide (z.B. WO 2012/168358 A1), Fluorene (z.B. WO 2012/031735 A1), Radikale und Diradikale (z.B. EP 1837926 A1, WO 2007/107306 A1), Pyridine (z.B. EP 2452946 A1, EP 2463927 A1), N-heterocyclische Verbindungen (z.B. WO 2009/000237 A1) und Acridine sowie Phenazine (z.B. US 2007/145355 A1).

Die vorliegende Erfindung betrifft daher auch eine elektronische Vorrichtung, insbesondere eine organische Elektrolumineszenzvorrichtung, enthaltend wenigstens eine Verbindung der Formel (5) oder (7) in einer Elektronentransportschicht (ETL) sowie wenigstens eine weitere Verbindung, die bevorzugt ausgewählt ist aus den Elektronentransportmaterialien und n-Dotanden.

Weiterhin kann es auch eine elektronische Vorrichtung, insbesondere eine organische Elektrolumineszenzvorrichtung, sein enthaltend wenigstens eine Verbindung der Formel (5) oder (7) in einer Lochtransportschicht (HTL).

Die HTL kann dabei weitere Materialien enthalten. Insbesondere kommen dabei weitere HTMs sowie p-Dotanden in Betracht.

Unter p-Dotanden werden hierin Oxidationsmittel, d.h. Elektronenakzeptoren verstanden. Bevorzugte Beispiele für p-Dotanden sind $F_4$-TCNQ, $F_6$-TNAP, NDP-2 (Fa. Novaled), NDP-9 (Fa. Novaled), Chinone (z.B. EP 1538684 A1, WO 2006/081780 A1, WO 2009/003455 A1, WO 2010/097433 A1), Radialene (z.B. EP 1988587 A1, US 2010/102709 A1, EP 2180029 A1, WO 2011/131185 A1, WO 2011134458 A1, US 2012/223296 A1), S-haltige Übergangsmetallkomplexe (z.B. WO 2007/134873 A1, WO 2008/061517 A2, WO 2008/061518 A2, DE 102008051737 A1, WO 2009/089821 A1, US 2010/096600 A1), Bisimidazole (z.B. WO 2008/138580 A1), Phthalocyanine (z.B. WO 2008/058525 A2), Bora-Tetraazapentalene (z.B. WO 2007/115540 A1) Fullerene (z.B. DE 102010046040 A1) und Hauptgruppenhalogenide (z.B. WO 2008/128519 A2)

Eine Lochtransportschicht ist eine Schicht mit lochtransportierender Funktion, welche sich zwischen Anode und emittierender Schicht befindet.

Eine Elektronenransportschicht gemäß der vorliegenden Anmeldung ist eine Schicht mit elektronentransportierender Funktion, welche sich zwischen Kathode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wie oben bereits erwähnt, wird die Verbindung der Formel (5) oder (7) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED, PLED oder OLEC, eingesetzt. Dabei wird das Matrixmaterial der Formel (5) oder (7) in der elektronischen Vorrichtung in Kombination mit einem oder mehreren fluoreszierenden oder phosphoreszierenden Dotanden vorhanden.

Besonders bevorzugt ist, wenn die Verbindung der Formel (5) oder (7) eine lochtransportierende Matrix darstellt und in Kombination mit einem anderen Matrixmaterial als Mixed Matrix für phosphoreszierende Emitter in der

Emissionsschicht verwendet wird. Lochtransportierende Verbindungen der Formel (5) oder (7) weisen hierfür besonders vorteilhafte, hohe $T_1$-Energieniveaus auf.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als

phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Weiterhin betrifft die vorliegende Erfindung auch eine organische Elektrolumineszenzvorrichtung enthaltend wenigstens eine Verbindung der Formel (5) oder (7) als Matrix in einer Emissionsschicht sowie wenigstens ein weiteres Matrix- oder Hostmaterial.

Weiterhin betrifft die vorliegende Erfindung auch eine organische Elektrolumineszenzvorrichtung enthaltend wenigstens eine Verbindung der Formel (5) oder (7) als Matrix in einer Emissionsschicht sowie wenigstens einen fluoreszierenden Emitter und wenigstens ein weiteres Matrix- oder Hostmaterial.

Wie bereits ausgeführt, kann eine emittierende Schicht einer organischen Elektrolumineszenzvorrichtung auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (5) oder (7) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im Mixed-Matrix-System eingesetzt wird.

Weiterhin betrifft die vorliegende Erfindung eine Zusammensetzung enthaltend wenigstens eine Verbindung gemäß Formel (5) oder (7) sowie wenigstens ein weiteres organisches Halbleitermaterial ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien, der n-Dotanden und der p-Dotanden.

Unter n-Dotanden werden hierbei, wie weiter oben bereits definiert, Reduktionsmittel verstanden. Bevorzugte n-Dotanden sind die weiter oben aufgeführten Verbindungen.

Unter p-Dotanden werden hierbei, wie weiter oben bereits definiert, Oxidationsmittel verstanden. Bevorzugte p-Dotanden sind die weiter oben aufgeführten Verbindungen.

Die vorliegenden Erfindung betrifft auch eine Zusammensetzung enthaltend wenigstens eine Verbindung gemäß Formel (1) sowie wenigstens ein weiteres Matrixmaterial.

Die vorliegende Erfindung betrifft auch eine Zusammensetzung enthaltend wenigstens eine Verbindung gemäß Formel (5) oder (7) sowie wenigstens ein wide band gap Material, wobei unter wide band gap Material ein Material im Sinne der Offenbarung von US 7,294,849 verstanden wird. Diese Systeme zeigen besondere vorteilhafte Leistungsdaten in elektrolumineszierenden Vorrichtungen.

Die vorliegende Erfindung betrifft auch eine Zusammensetzung enthaltend wenigstens eine Verbindung gemäß Formel (5) oder (7) sowie wenigstens ein weiteres Matrixmaterial sowie wenigstens einen phosphoreszierenden Emitter.

Die vorliegende Erfindung betrifft auch eine Zusammensetzung enthaltend wenigstens eine Verbindung gemäß Formel (5) oder (7) sowie wenigstens ein wide band gap Material sowie wenigstens einen phosphoreszierenden Emitter.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

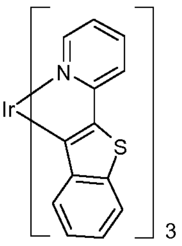
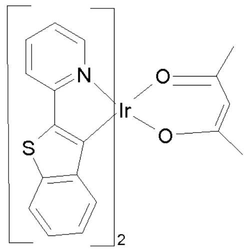
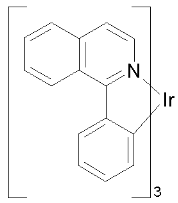
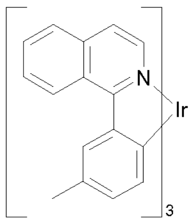
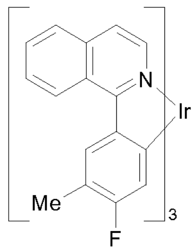
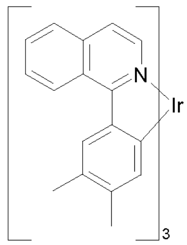
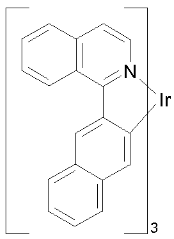
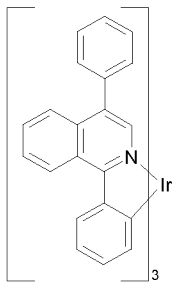
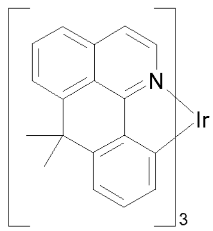
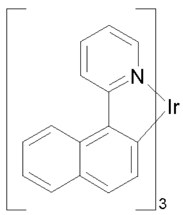
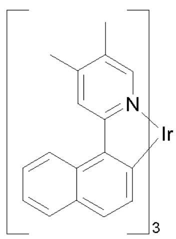
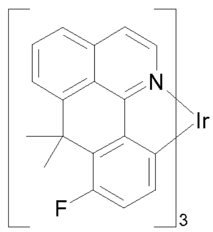
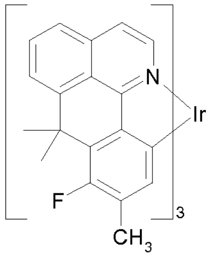
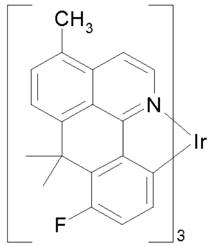
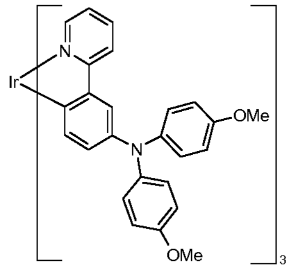

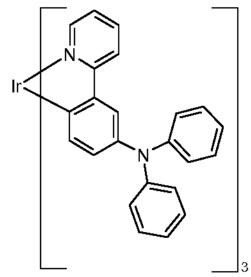 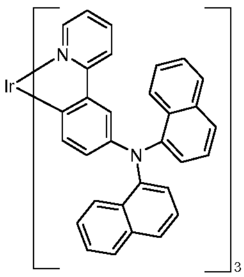 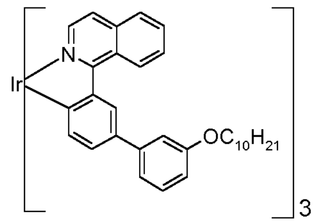

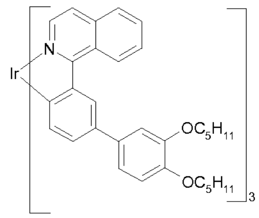 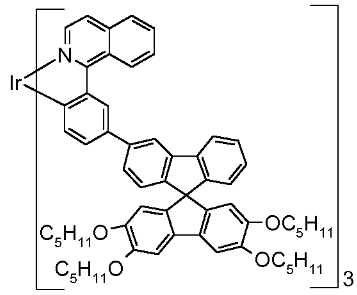 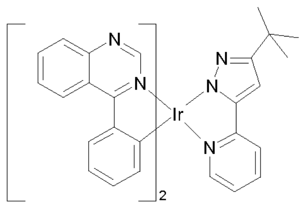

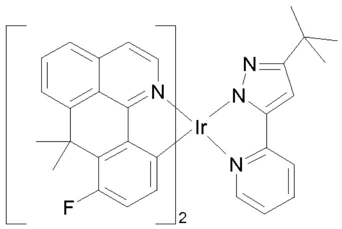 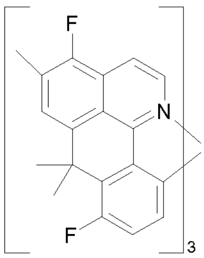 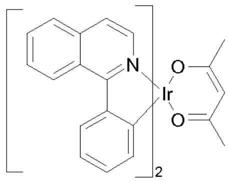

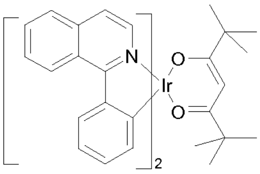 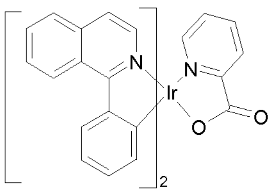 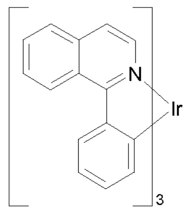

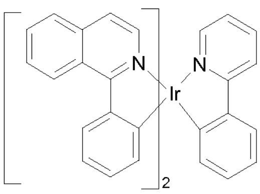 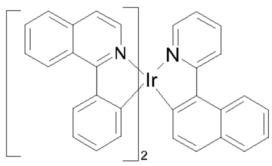 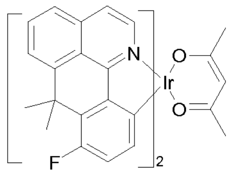

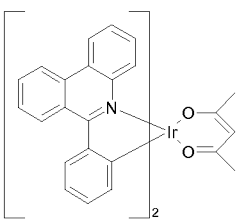 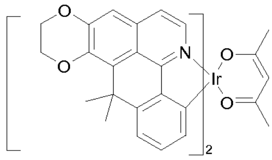 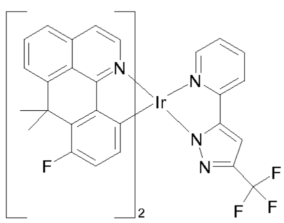

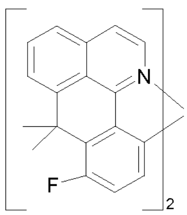
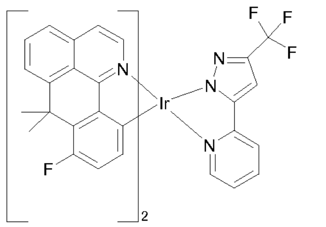
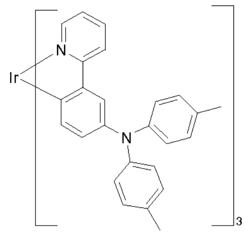

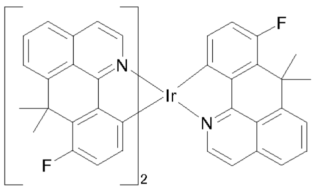
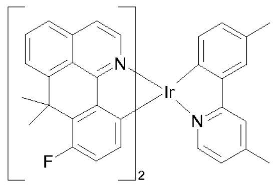
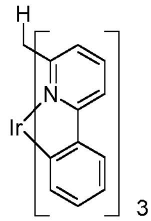

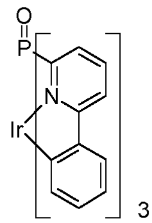
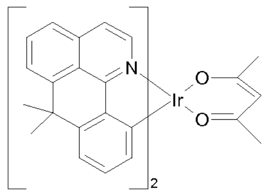
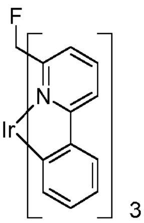

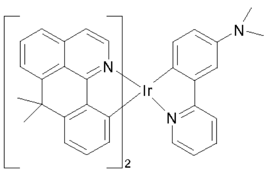
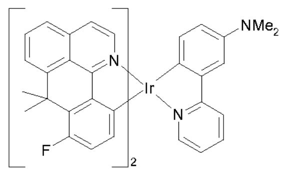
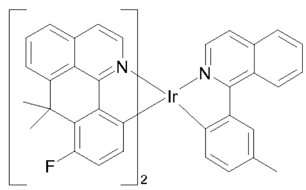

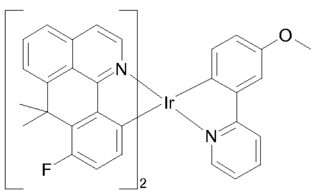
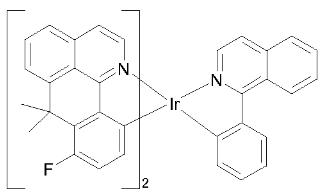
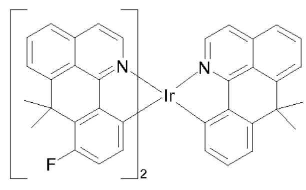

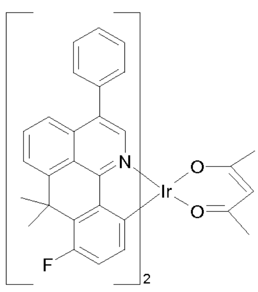
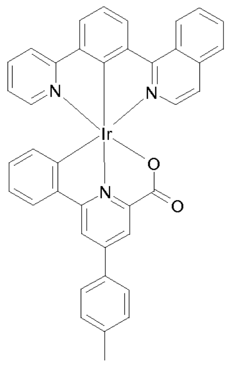
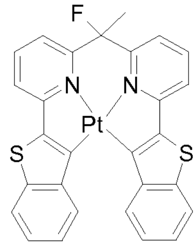

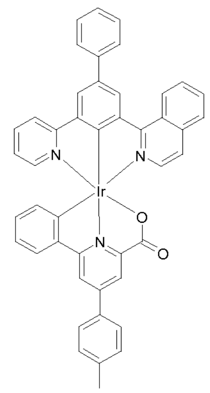
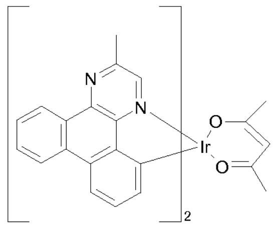
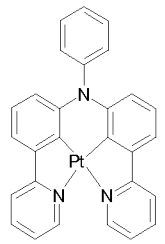

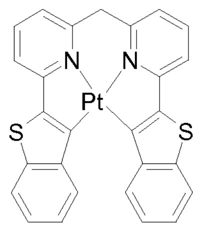
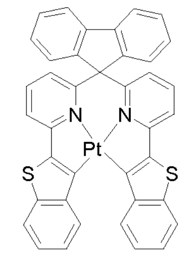
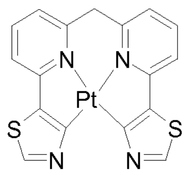

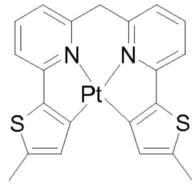
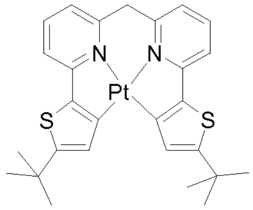
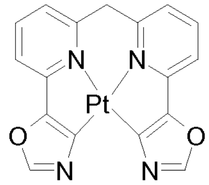

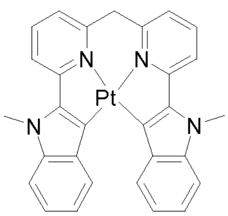
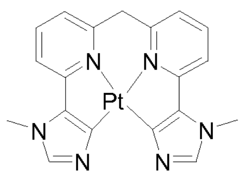
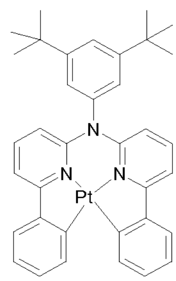

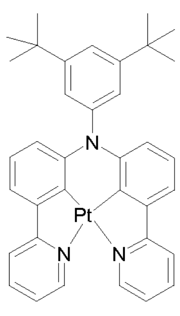
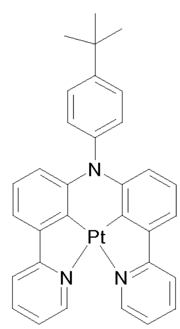
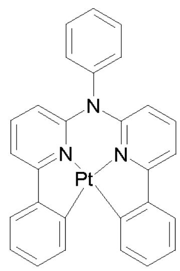

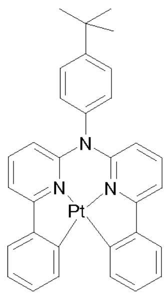

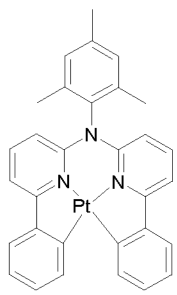

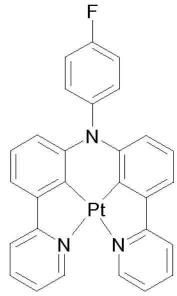

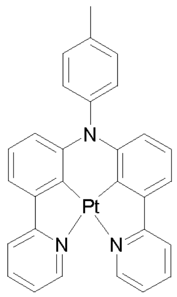

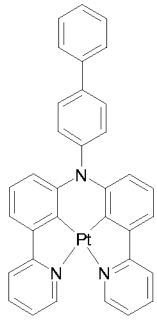

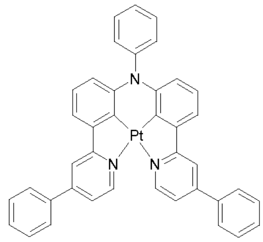

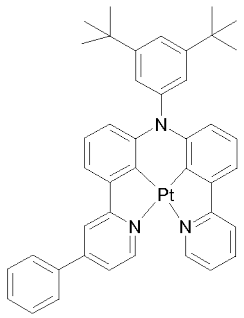

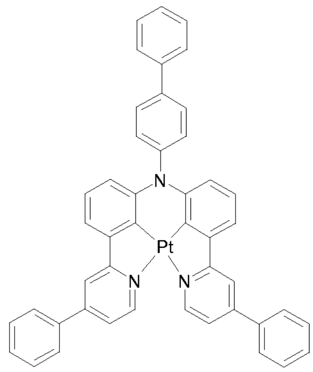

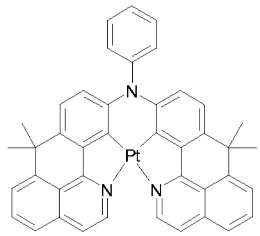

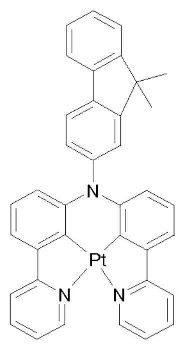

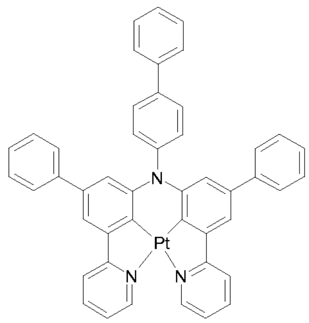

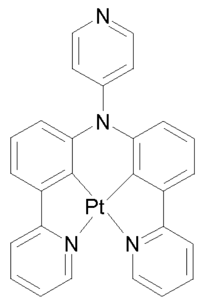

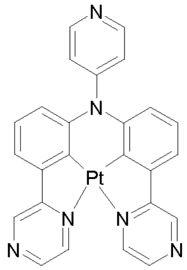

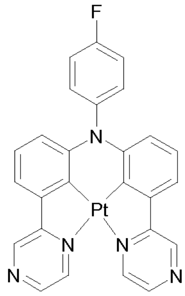

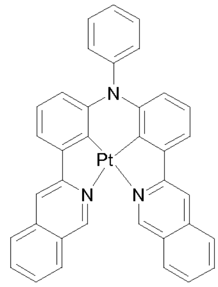

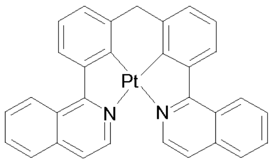
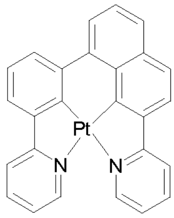
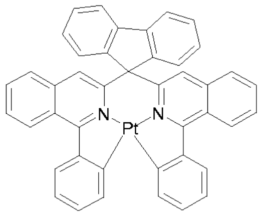

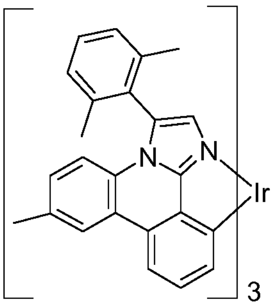
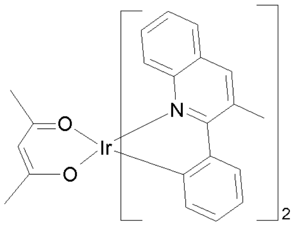
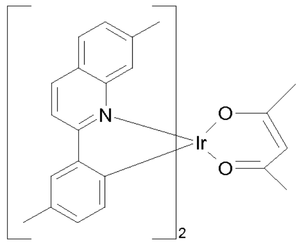

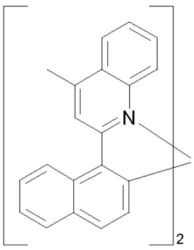
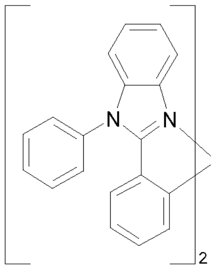
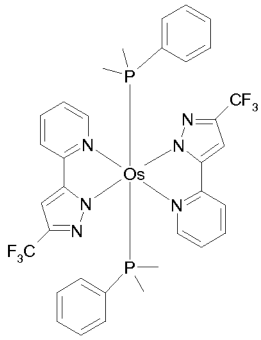

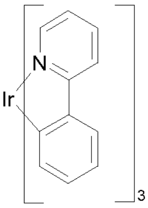
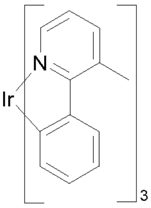
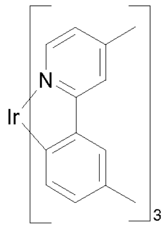

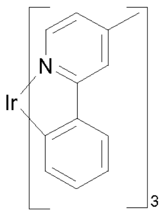
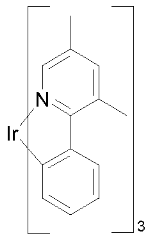
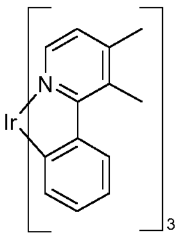

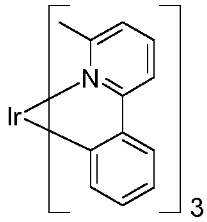
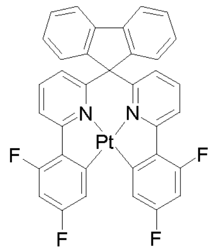
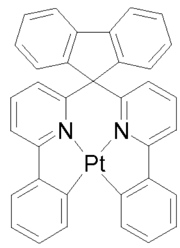
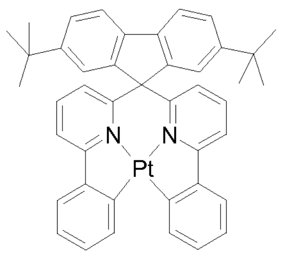
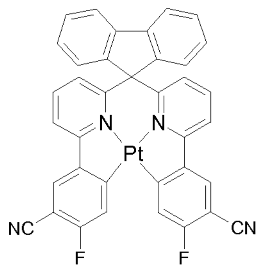
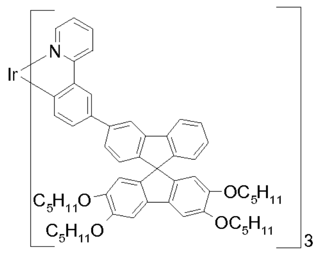
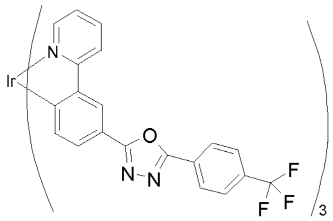
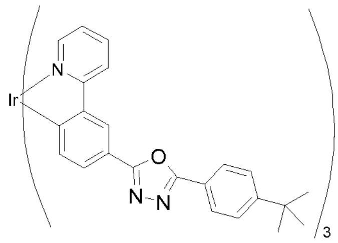
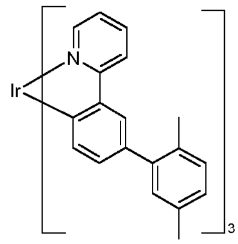
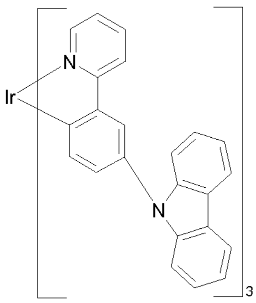
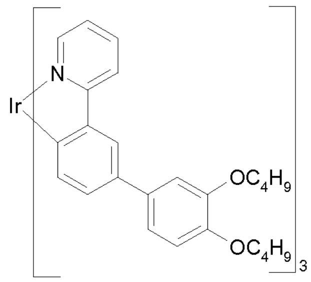
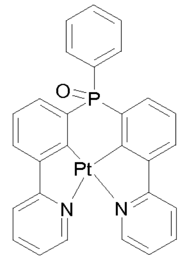
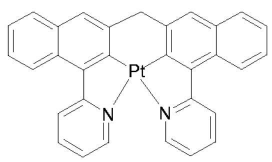
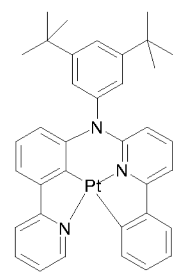
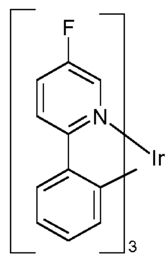

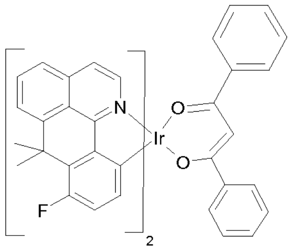

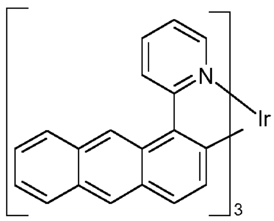

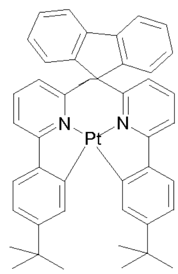

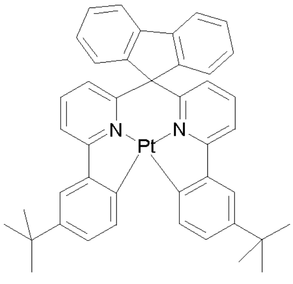

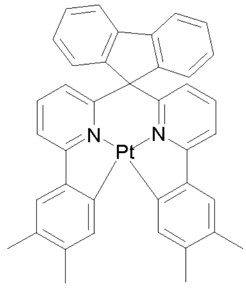

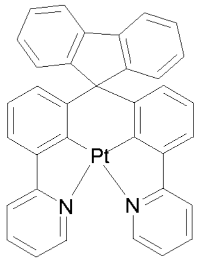

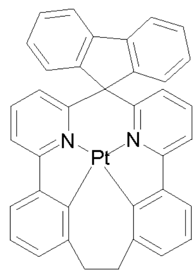

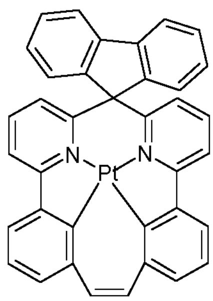

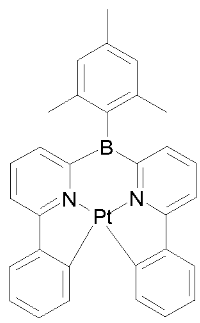

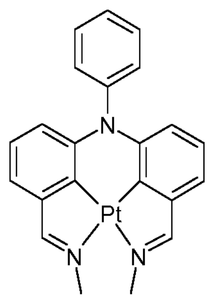

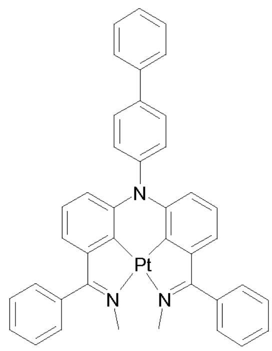

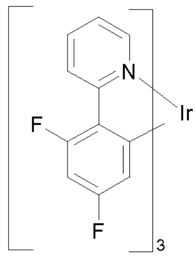

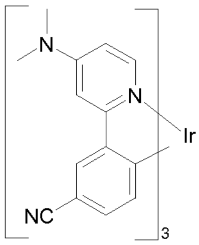

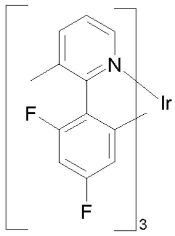

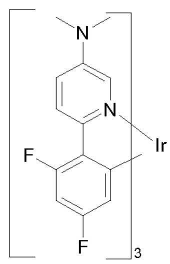

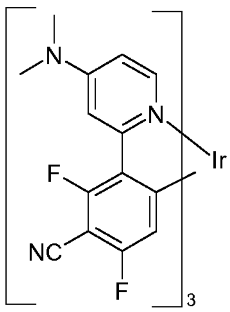

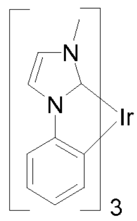

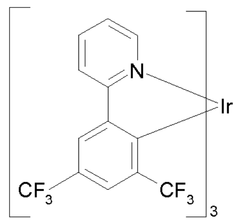

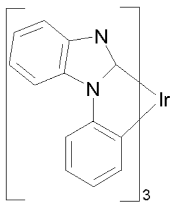

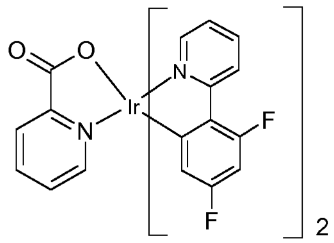

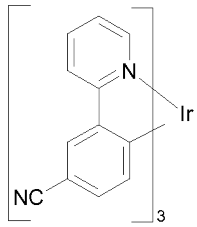

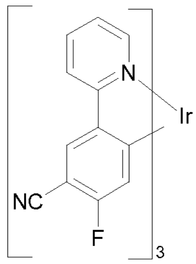

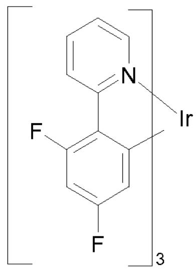

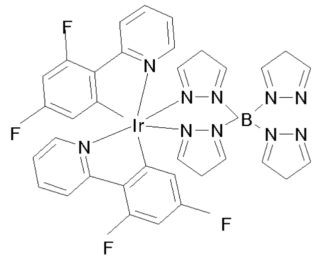

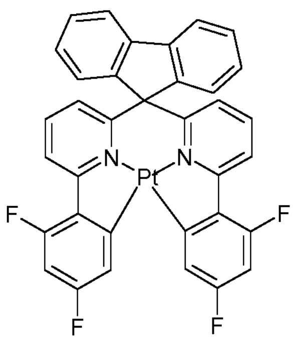

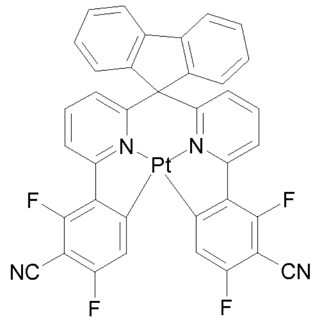

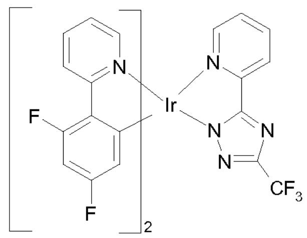

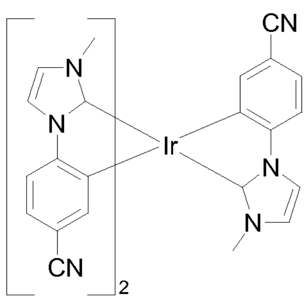

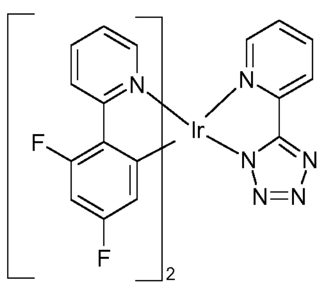

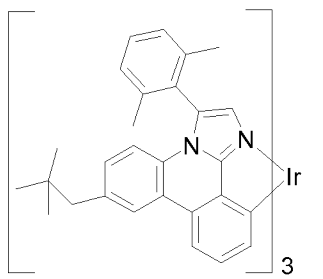

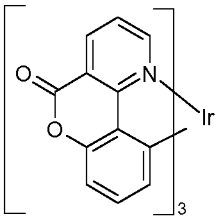
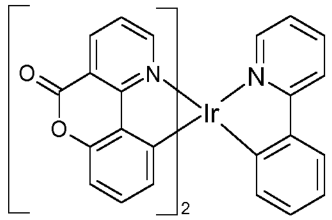
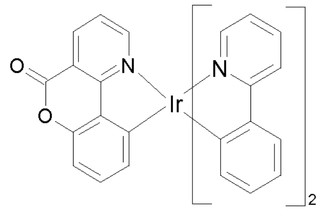

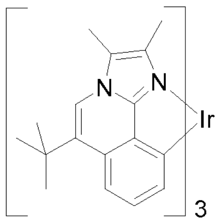
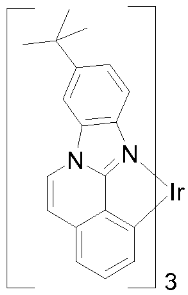
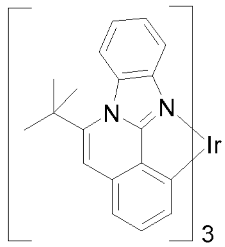

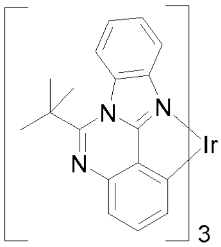
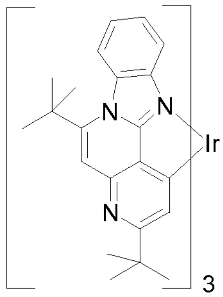
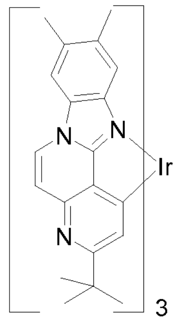
.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Als Matrixmaterialien, bevorzugt für fluoreszierende Dotanden, kommen neben den Verbindungen der Formel (5) oder (7) Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrix-materialien sind ausgewählt aus den

Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den Verbindungen der Formel (5) oder (7) aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, DiazaphospholDerivate, z. B. gemäß WO 2010/054730 und Aluminiumkomplexe, z. B. BAlQ.

Außer Kathode, Anode und der Schicht enthaltend die Verbindung der Formel (5) oder (7) kann die elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss.

Die Abfolge der Schichten der organischen Elektrolumineszenzvorrichtung ist bevorzugt die folgende: Anode/Lochinjektionsschicht/Lochtransportschicht/emittierende Schicht/Elektronentransportschicht/Elektroneninjektionsschicht/Kathode.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere bevorzugt Materialien, die in einer Lochtransport-, Lochinjektions- oder Elektronenblockierschicht verwendet werden können, Indenofluorenamin-Derivate (z. B. gemäß WO 2006/122630 oder WO 2006/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 2007/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß der noch nicht offengelegten Anmeldung EP 11009127.9) und Dihydroacridin-Derivate (z. B. gemäß der noch nicht offen gelegten EP 11007067.9).

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (5) oder (7) nötig. Hohe Löslichkeit lässt sich durch geeignete Substitution der Verbindungen erreichen. Die erfindungsgemäßen Verbindungen können weiterhin aus Lösung aufgebracht werden und durch anschließende Vernetzung oder Fixierung in einem Polymernetzwerk in der jeweiligen Schicht fixiert werden.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Gegenstand der Erfindung ist somit weiterhin ein Verfahren zur Herstellung der erfindungsgemäßen elektronischen Vorrichtung, dadurch gekennzeichnet, dass mindestens eine organische Schicht durch Gasphasenabscheidung oder aus Lösung aufgebracht wird.

Erfindungsgemäß können die elektronischen Vorrichtungen enthaltend eine oder mehrere Verbindungen gemäß Formel (5) oder (7) in Displays, als Lichtquellen in Beleuchtungsanwendungen sowie als Lichtquellen in medizinischen und/oder kosmetischen Anwendungen (z.B. Lichttherapie) eingesetzt werden.

Eine Formulierung enthaltend wenigstens eine Verbindung gemäß Formel (5) oder (7) oder wenigstens eine der oben genannten Zusammensetzungen sowie wenigstens ein Lösungsmittel kann bei der Herstellung verwendet werden.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Vorrichtungen enthaltend die Verbindungen nach Formel (5) oder (7) können sehr vielseitig eingesetzt werden. So können, beispielsweise, elektrolumineszierende Vorrichtuungen enthaltend eine oder mehrere Verbindungen nach Formel (5) oder (7) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach Formel (5) oder (7) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Gegenstand der vorliegenden Erfindung ist daher eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (5) oder (7) zur Verwendung in der Medizin zur Phototherapie.

Ein weiterer bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (5) oder (7) zur Verwendung zur phototherapeutischen Behandlung von Hautkrankheiten.

Ein weiterer ganz bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (5) oder (7) zur Verwendung zur phototherapeutischen Behandlung von Psoriasis, atopische Dermatitis, Entzündungserkrankungen, Vitiligo, Wundheilung und Hautkrebs.

Die vorliegende Erfindung betrifft weiterhin die Verwendung der elektronischen Vorrichtung, bevorzugt einer organischen Elektrolumineszenzvorrichtung, ganz bevorzugt einer OLED oder OLEC und ganz besonders bevorzugt einer OLED enthaltend wenigstens eine Verbindung gemäß Formel (5) oder (7) in der Kosmetik, bevorzugt zur Behandlung von Akne, alternder Haut (Skin Ageing), und von Zellulithe.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:

1. Die erfindungsgemäßen Verbindungen eignen sich sehr gut für den Einsatz in einer Emissionsschicht und zeigen verbesserte Leistungsdaten gegenüber Verbindungen aus dem Stand der Technik.

2. Die erfindungsgemäßen Verbindungen weisen eine relativ niedrige Sublimationstemperatur, eine hohe Temperaturstabilität und lassen sich daher unzersetzt und rückstandsfrei sublimieren. Weiterhin weisen sie eine hohe Oxidationsstabilität und eine hohe Glasübergangstemperatur auf, was sowohl für die Prozessierbarkeit, beispielweise aus Lösung oder aus der Gasphase, als auch für die Verbendung in elektronischen Vorrichtungen vorteilhaft ist.

3. Die Verwendung der erfindungsgemäßen Verbindungen in elektronischen Vorrichtungen, insbesondere eingesetzt als Matrixmaterial, aber auch als Elektronentransport-, Elektronenblockier- oder Elektroneninjektionsmaterial, Lochtransportmaterial oder Lochblockiermaterial führen zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit

anderen Beispielen kombiniert werden.

Die Erfindung wird durch die nachfolgenden Beispiele näher erläutert, ohne sie dadurch einschränken zu wollen.

## Beispiele

Die nachfolgenden Synthesen werden, sofern nicht anders angegeben, unter einer Schutzgasatmosphäre in getrockneten Lösungsmitteln durchgeführt. Die Edukte können von *Aldrich* (*p*-Toluolsulfonsäure, *N*-Bromsuccinimid, Benzolboronsäure, Tri(*o*-tolyl)phosphin, Kaliumphosphat, Paliadium(II)acetat) bezogen werden. 8,8'-Dihydroxy-1,1'-binaphtyl kann nach einer Literaturvorschrift [J. Org. Chem., 1985, 50, 1486-1496] dargestellt werden. Die Angaben in eckigen Klammern zu literaturbekannten chemischen Verbindungen beziehen sich auf die CAS-Nummer.

### Beispiel 1

**Synthese von Dinaphth[1,8-*bc*:1',8'-*ef*]oxepin**

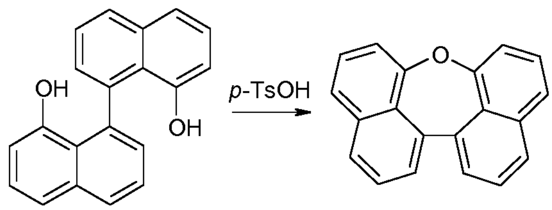


Zu einer siedenden Lösung von 24,4 g 8,8'-Dihydroxy-1,1'-binaphtyl (85,2 mmol) in 500 mL Toluol werden über 2 h in kleinen Portionen 17,8 g (190 mmol) p-Toluolsulfonsäure gegeben. Es wird anschließend 1 h gerührt. Nach dem Abkühlen versetzt man die Reaktionsmischung mit einer Lösung von 25,9 g (187,5 mmol) $K_2CO_3$ in 25 mL Wasser und rührt 1 h. Die organische Phase wird abgetrennt, mit 100 mL Wasser gewaschen, mit $Mg_2SO_4$ getrocknet und dann eingeengt. Ausbeute: 19,8 g (73,8 mmol), 87% der Theorie.

Analog kann die folgende Verbindung erhalten werden.

| Edukt 1 | Produkt | Ausbeute |
|---|---|---|
| 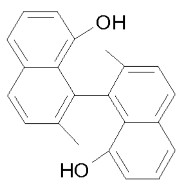 [250716-74-6] | 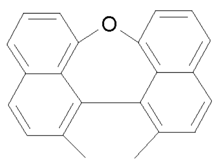 | 49% |

### Beispiel 2

**Synthese von 4,4'-Dibrom-dinaphth[1,8-*bc*:1',8'-*ef*]oxepin**

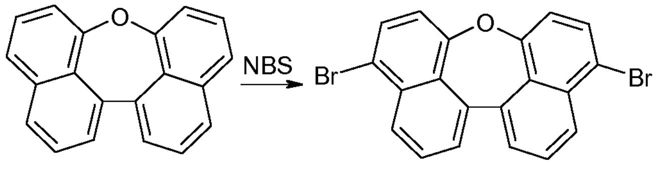


Man löst 19,6 g (73,0 mmol) Dinaphth[1,8-*bc*:1',8'-*ef*]oxepin in 200 mL Dimethylformamid und gibt dazu 27,3 g (178,0 mmol) *N*-Bromsuccinimid und erhitzt die Reaktionsmischung auf 40°C. Nach 30 min lässt man den Ansatz

erkalten und filtriert den ausgefallenen gelben Feststoff ab, wäscht mit zweimal 40 mL Ethanol nach. Ausbeute: 31,1 g (63,4 mmol), 87% der Theorie; Reinheit ca. 99% n. $^{1}$H-NMR.

Analog kann die folgende Verbindung erhalten werden.

| Edukt 1 | Produkt | Ausbeute |
|---|---|---|
| 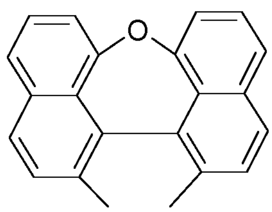 | 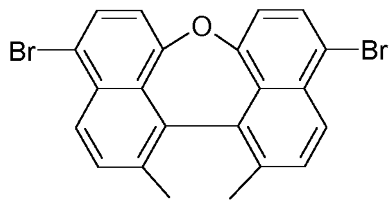 | 78% |

## Beispiel 3

**Synthese von 4-Brom-dinaphth[1,8-*bc*:1',8'-*ef*]oxepin**

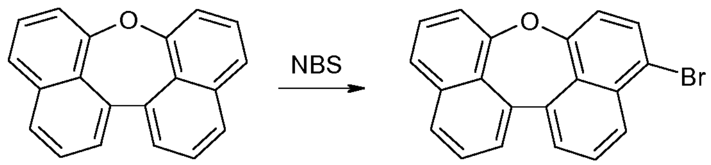


Man löst 30 mg (0,11 mmol) Dinaphth[1,8-*bc*:1',8'-*ef*]oxepin in 5 mL Chloroform und gibt dazu 40 mg (0,22 mmol) *N*-Bromsuccinimid und rührt die Reaktionsmischung 6 h bei Raumtemperatur. Danach erweitert man mit 5 mL Dichlormethan, versetzt die Reaktionsmischung mit Wasser und trennt die organische Phase ab. Die säulenchromatographische Aufreinigung ergibt eine Ausbeute von 25,3 mg (0,07 mmol, 65,2% der Theorie).

Analog kann die folgende Verbindung erhalten werden.

| Edukt 1 | Produkt | Ausbeute |
|---|---|---|
| 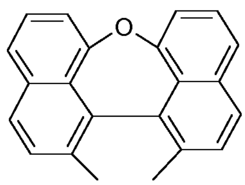 | 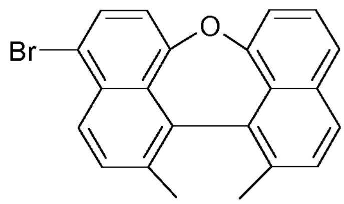 | 78% |

## Beispiel 4

**Synthese von Dinaphth[1,8-*bc*:1',8'-*ef*]oxepin-4-boronsäure**

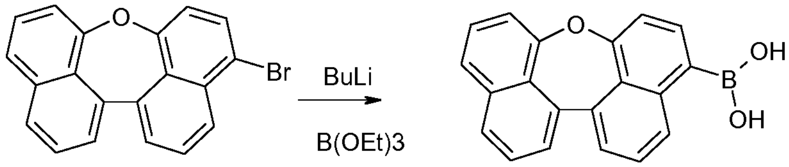


Eine auf -78°C gekühlte Lösung von 93,6 g (270 mmol) 4-Brom-Dinaphth[1,8-*bc*:1',8'-*ef*]oxepin in 1500 mL Diethylether wird tropfenweise mit 110 mL (276 mmol) n-Buthyllithium (2.5 M in Hexan) versetzt. Die Reaktionsmischung

wird 30 min. bei -78°C gerührt. Man lässt auf Raumtemperatur kommen, kühlt erneut auf -78°C und versetzt dann schnell mit einer Mischung von 40 mL (351 mmol) Trimethylborat in 50 mL Diethylether. Nach Erwärmen auf -10°C wird mit 135 mL 2 N Salzsäure hydrolysiert. Die organische Phase wird abgetrennt, mit Wasser gewaschen, über Natriumsulfat getrocknet und zur Trockene eingeengt. Der Rückstand wird in 300 mL n-Heptan aufgenommen, der farblose Feststoff wird abgesaugt, mit n-Heptan gewaschen und im Vakuum getrocknet. Ausbeute: 77,6 g (248 mmol), 92% der Theorie.

Analog kann mit 0,5 eq. Bromid die folgende Verbindung erhalten werden.

| Edukt 1 | Produkt | Ausbeute |
|---|---|---|
| 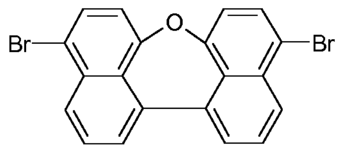 | 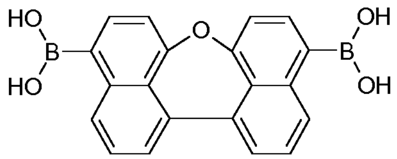 | 78 % |

**Beispiel 5**

**Synthese von 4,4'-Diphenyl-dinaphth[1,8-*bc*:1',8'-*ef*]oxepin**

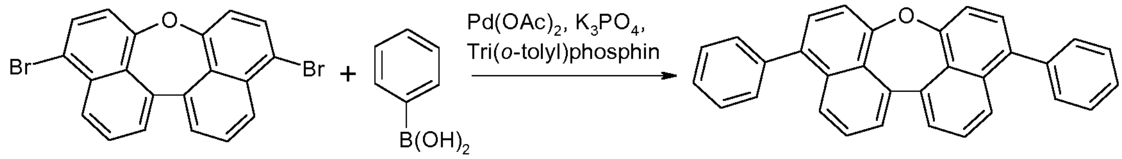


35 g (82,1 mmol) 4,4'-Dibrom-dinaphth[1,8-bc:1',8'-ef]oxepin, 22,0 g (180,7 mmol) Benzolboronsäure und 38,3 g (180,7 mmol) Kaliumphosphat werden in 500 mL Toluol, 250 mL 1,4-Dioxan und 120 mL Wasser suspendiert. Zu der Mischung werden 1,3 g (4,1 mmol) Tri(o-tolyl)phosphin und dann 461 mg (2 mmol) Palladium(II)acetat gegeben und die Reaktionsmischung 48 h unter Rückfluss erhitzt. Nach dem Erkalten wird die organische Phase abgetrennt, dreimal mit je 100 mL Wasser gewaschen und eingeengt. Nach der säulenchromatographischen Reinigung ($SiO_2$, n-Heptan/Dichlormethan 3:1) wird der erhaltene Schaum in Dichlormethan gelöst und mit Ethanol gefällt. Der Rückstand wird aus Toluol und aus Dichlormethan umkristallisiert und abschließend im Hochvakuum ($p = 5 \times 10^{-5}$ mbar) sublimiert. Ausbeute: 18,2 g (43,2 mmol), 53% der Theorie. Reinheit ca. 99,9% n. HPLC.

Analog können folgende Verbindungen erhalten werden.

| Edukt 1 | Edukt 2 | Produkt | Ausbeute |
|---|---|---|---|
| 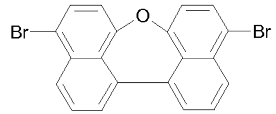 | 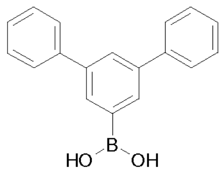 [128388-54-5] | 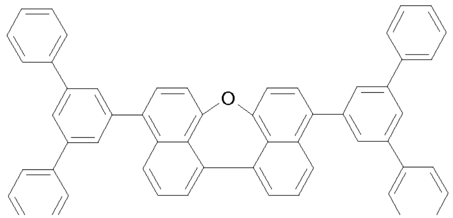 | 59% |
| 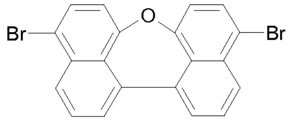 | 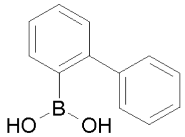 4688-76-0] | 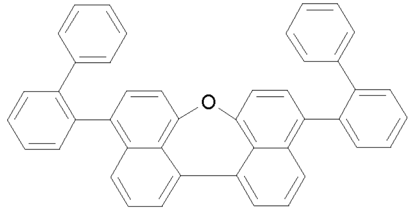 | 62% |
| 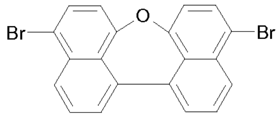 | 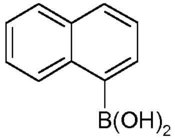 [13922-41-3] | 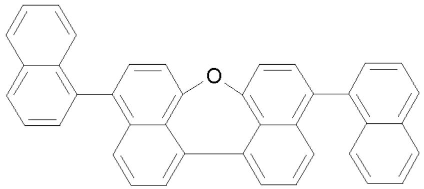 | 71% |
| 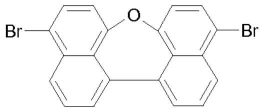 | 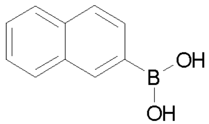 [32316-92-0] | 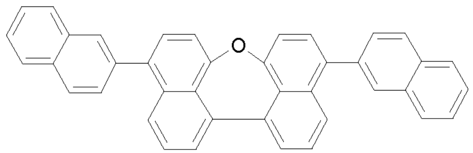 | 73% |
| 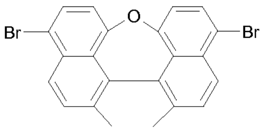 | 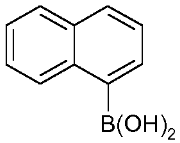 [13922-41-3] | 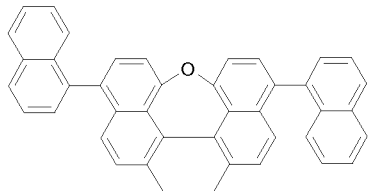 | 69% |

(fortgesetzt)

| Edukt 1 | Edukt 2 | Produkt | Ausbeute |
| --- | --- | --- | --- |
| 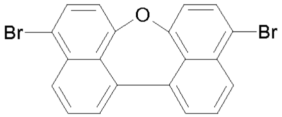 | 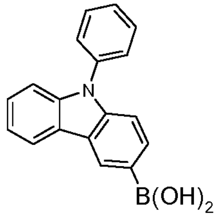 [854952-58-2] | 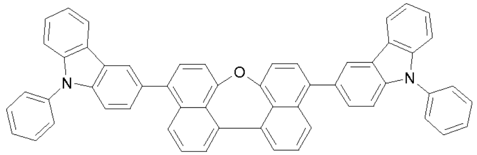 | 71% |
| 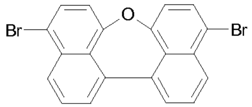 | 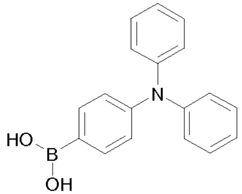 [201802-67-7] | 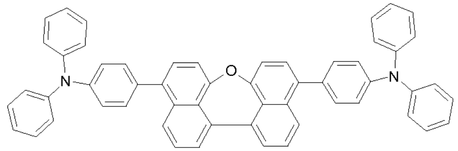 | 65% |
| 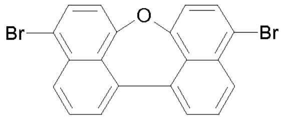 | 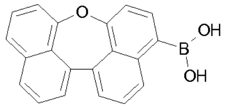 | 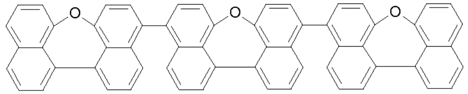 | 78% |
| 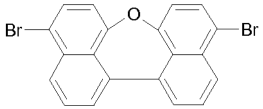 | 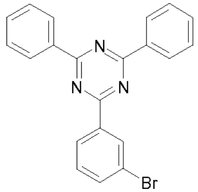 | 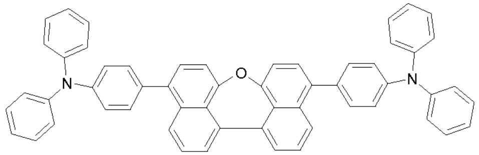 | 69% |

**Beispiel 6**

**Synthese von 4 Naphthalen-2-yl--diphenyl-dinaphth[1,8-*bc*:1',8'-*ef*]oxepin**

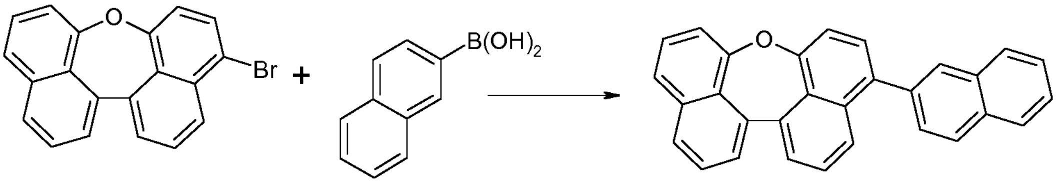

61 g (177 mmol) 4-Brom-dinaphth[1,8-*bc*:1',8'-*ef*]oxepin 25,5 g (180,7 mmol) 2-Naphtylboronsäure und 38,3 g (180,7 mmol) Kaliumphosphat werden in 500 mL Toluol, 250 mL 1,4-Dioxan und 120 mL Wasser suspendiert. Zu der Mischung werden 1,3 g (4,1 mmol) Tri(o-tolyl)phosphin und dann 461 mg (2 mmol) Palladium(II)acetat gegeben und die Reaktionsmischung 48 h unter Rückfluss erhitzt. Nach dem Erkalten wird die organische Phase abgetrennt, dreimal mit je 100 mL Wasser gewaschen und eingeengt. Nach der säulenchromatographischen Reinigung ($SiO_2$, *n*-Heptan/Dichlormethan 3:1) wird der erhaltene Schaum in Dichlormethan gelöst und mit Ethanol gefällt. Der Rückstand wird aus Toluol und aus Dichlormethan umkristallisiert und abschließend im Hochvakuum (p = 5 x $10^{-5}$ mbar) sublimiert. Ausbeute: 24 g (61,8 mmol), 59% der heorie. Reinheit ca. 99.9 % n. HPLC.

Analog können die folgenden Verbindungen erhalten werden.

| Edukt 1 | Edukt 2 | Produkt | Ausbeute |
|---|---|---|---|
| 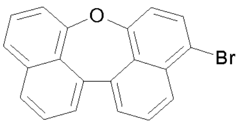 | 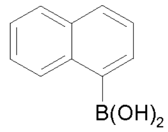 [13922-41-3] | 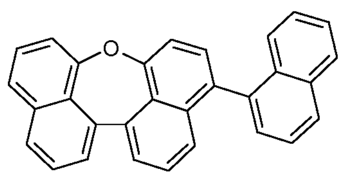 | 58% |
| 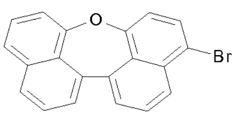 | 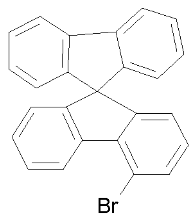 [1161009-88-6] | 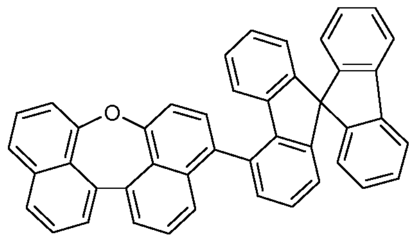 | 63% |
| 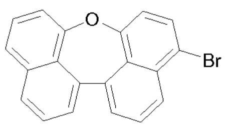 | 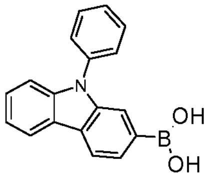 [1001911-63-2] | 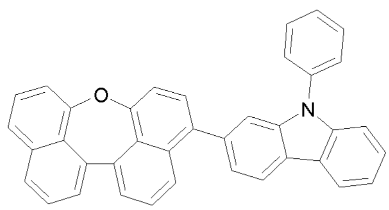 | 62% |
| 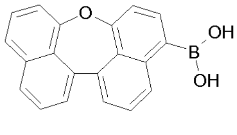 | 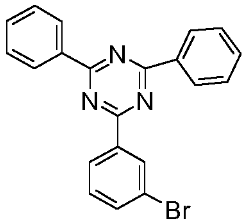 [64377-31-1] | 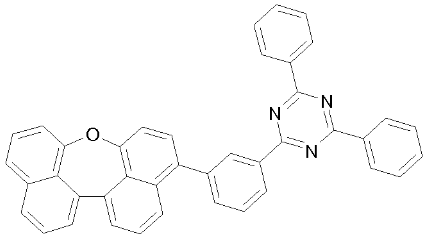 | 64% |

(fortgesetzt)

| Edukt 1 | Edukt 2 | Produkt | Ausbeute |
|---|---|---|---|
| 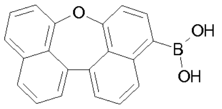 | 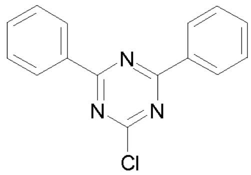 | 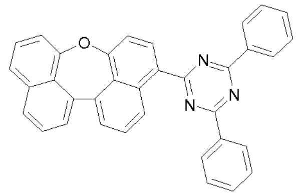 | 59% |
| 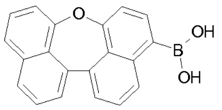 | 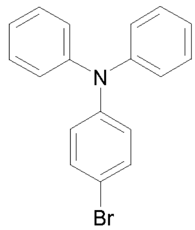 [201802-67-7] | 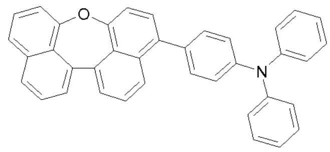 | 64% |
| 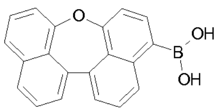 | 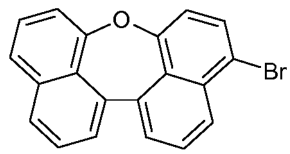 | 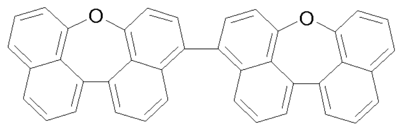 | 75% |

**Beispiel 7**

**Synthese von 2,2'-Dibrom-4,4'-Diphenyl-dinaphth[1,8-*bc*:1',8'-*ef*]oxepin**

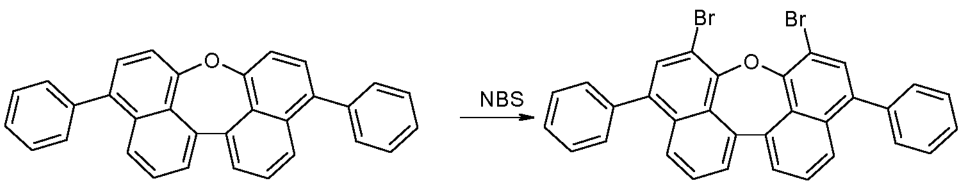


Man löst 2 g (4,8 mmol) 4,4'-Diphenyl-dinaphth[1,8-bc:1',8'-ef]oxepin in 20 mL Dimethylformamid und gibt dazu 1,8 g (10,0 mmol) N-Bromsuccinimid und erhitzt die Reaktionsmischung auf 80°C. Nach 1 h lässt man den Ansatz erkalten und filtriert den ausgefallenen grünen Feststoff ab, wäscht mit je 5 mL kaltem DMF und Wasser nach. Ausbeute: 1,2 g (2,1 mmol), 44% der Theorie; Reinheit ca. 96% n. $^1$H-NMR.

**Beispiel 8**

**Synthese von 2,2',4,4'-Tetraphenyl-dinaphth[1,8-*bc*:1',8'-*ef*]oxepin**

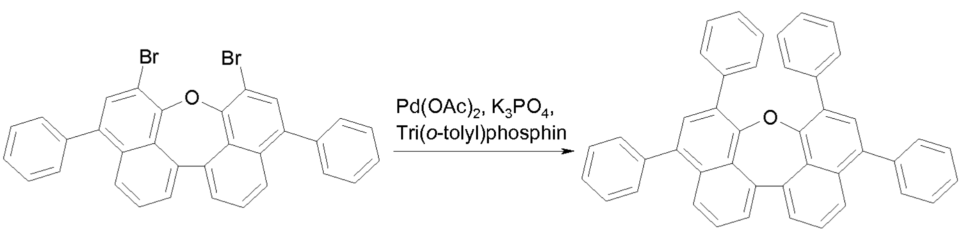

5 g (8,6 mmol) 4,4'-Dibrom-dinaphth[1,8-bc:1',8'-ef]oxepin, 2,3 g (19,0 mmol) Benzolboronsäure und 4,0 g (19,0 mmol) Kaliumphosphat werden in 55 mL Toluol, 26 mL 1,4-Dioxan und 12,5 mL Wasser suspendiert. Zu der Mischung werden 130 mg (0,43 mmol) Tri(o-tolyl)phosphin und dann 48 mg (0,21 mmol) Palladium(II)acetat gegeben und die Reaktionsmischung 48 h unter Rückfluss erhitzt. Nach dem Erkalten wird die organische Phase abgetrennt, dreimal mit je 50 mL Wasser gewaschen und eingeengt. Nach der säulenchromatographischen Reinigung ($SiO_2$, n-Heptan/Dichlormethan 3:1) wird der Rückstand aus Toluol und aus Dichlormethan umkristallisiert und abschließend im Hochvakuum (p = 5 x $10^{-5}$ mbar) sublimiert. Ausbeute: 3,2 g (5,6 mmol), 65% der Theorie. Reinheit ca. 99,9% n. HPLC.

Analog können die folgenden Verbindungen erhalten werden.

| Edukt 1 | Edukt 2 | Produkt | Ausbeute |
|---|---|---|---|
| 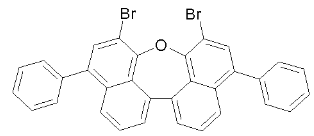 | 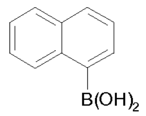 [13922-41-3] | 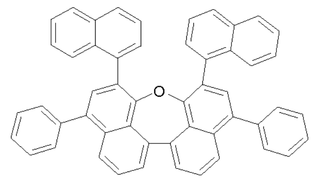 | 59% |
| 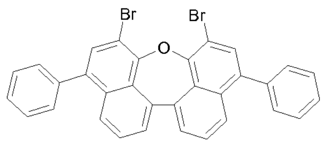 | 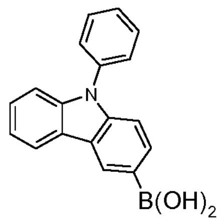 [854952-58-2] | 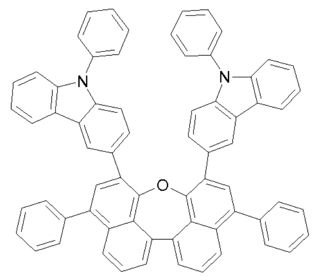 | 76% |

## Beispiel 9

### Synthese von N,N,N',N'-Tetrakis-biphenyl-4-yl-dinaphth[1,8-*bc*:1',8'-*ef*]oxepin-4,4'-diamin

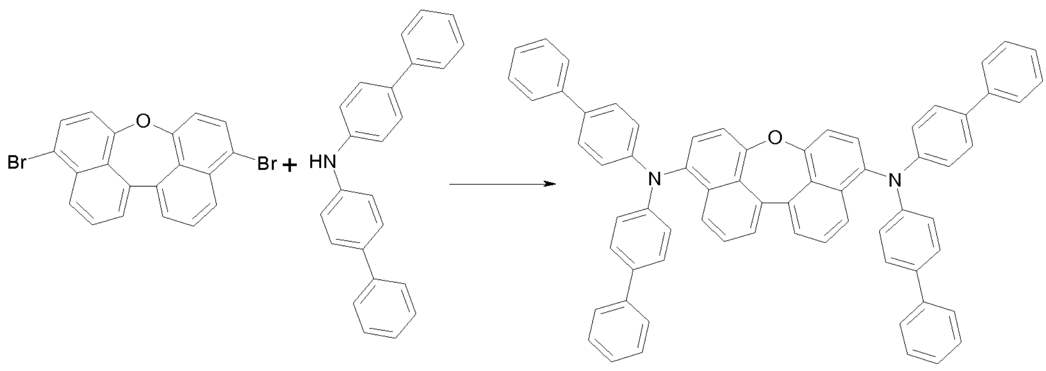

[102113-98-4 ]

Ein Gemisch aus 12,3 g (50 mmol) 4,4'-Dibrom-dinaphth[1,8-*bc*:1',8'-*ef*]oxepin, 19,2 g (60 mmol) Bis-biphenyl-4-yl-amin, 7.7 g (80 mmol) Natrium-tert-butylat, 1.4 g (5 mmol) Tricyclohexylamin, 561 mg (2.5 mmol) Palladium(II)acetat und 300 mL Mesitylen wird 24 h unter Rückfluss erhitzt. Nach dem Erkalten versetzt man mit 200 mL Wasser, rührt 30 min. nach, trennt die org. Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. $10^{-6}$ mbar). Ausbeute: 20,9 g (23 mmol), 80% der Theorie; Reinheit: 99.9% n. HPLC.

Analog können die folgenden Verbindungen erhalten werden.

| Edukt 1 | Edukt 2 | Produkt | Ausbeute |
|---|---|---|---|
| 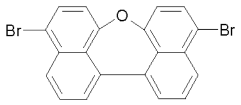 | 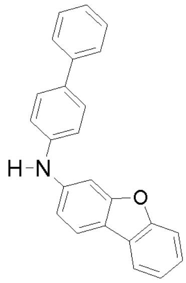 [1290039-85-8] | 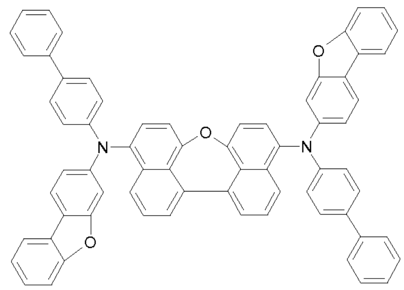 | 63% |
| 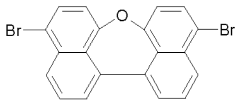 | 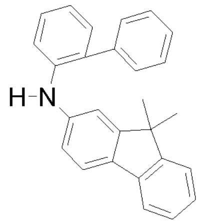 [1198395-24-2] | 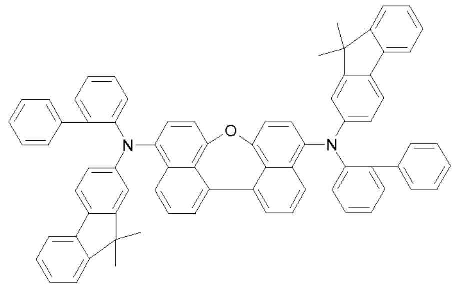 | 76% |
| 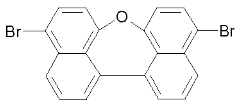 | 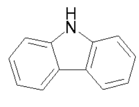 [86-74-8] | 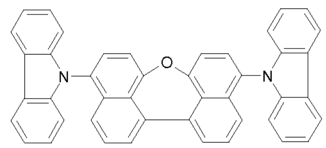 | 75% |

**Beispiel 10**

**Synthese von 8-Trimethylsilyl-tribenz[a,c,e]oxepin**

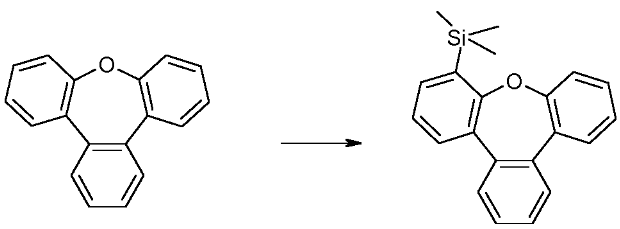

4,73 g (19,4 mmol) Tribenz[a,c,e]oxepin [2688-95-1] und 5,86 ml (4,57 g, 39,3 mmol, 2 mol%) Tetramethylethylenediamin (TMEDA) werden in 60 mL trockenem Diethylether gelöst. Dann werden 18,7 mL n-Butyllithium (2,5 M in Hexan, 46,9 mmol, 2,4 mol%) zugegeben und die Reaktionsmischung anschließend 2 h zum Rückfluss erhitzt. Dann wird die Reaktionsmischung auf 0°C abgekühlt, Chlortrimethylsilan (6,0 mL, 47,4 mmol) zugegeben und über Nacht gerührt, wobei man auf Raumtemperatur erwärmen lässt. Die Reaktionsmischung wird mit 60 mL Wasser versetzt und die organische Phase abgetrennt. Die wässrige Phase wird zweimal mit je 30 mL Diethylether extrahiert und die vereinigten organischen Phasen mit $MgSO_4$ getrocknet am Rotationsverdampfer eingeengt. Das erhaltene ölige Rohprodukt wird säulenchromatographisch ($SiO_2$, Heptan) gereinigt und ergibt 8-Trimethylsilyl-tribenz[a,c,e]oxepin in 94% Ausbeute (5,76 g, 18,2 mmol) in Form eines farblosen Öls.

**Beispiel 11**

**Synthese von Tribenz[a,c,e]oxepin-1-boronsäure (Int-4)**

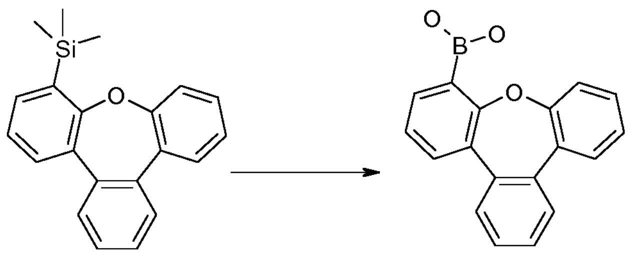

9,82 g (31,03 mmol)8-Trimethylsilyl-tribenz[a,c,e]oxepin werden in 50 mL trockenem Dichlormethan vorgelegt und dann langsam 37,2 mL Bortribromid (1 M in Dichlormethan (37,2 mmol 1,2 mol%) zugegeben. Die Reaktionsmischung wird 15 h gerührt und dann auf Eis gegeben. Die gelbe organische Phase wird abgetrennt, die Wässrige zweimal mit je 30 mL Ethylacetat extrahiert. Die vereinigten organischen Phasen werden mit je 30 mL Wasser und ges. NaCl-Lsg. nachgewaschen und dann mit $Na_2SO_4$ getrocknet, filtriert und eingeengt. Das erhaltene gelbe Öl von **Int-4** (8,4 g, 29,2 mmol, 94%) wird ohne weitere Aufreinigung weiter umgesetzt.

**Beispiel 12**

**Synthese von 6-Brom-tribenz[a,c,e]oxepin (Int-5)**

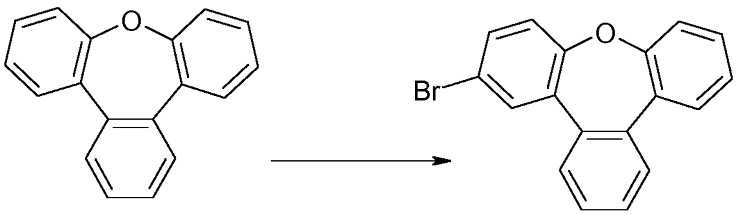

10 g (41 mmol) Tribenz[a,c,e]oxepin werden mit 8 mg N-Bromsuccinimid (45 mmol, 1,1 mol%) in 100 mL trockenem Dimethylformamid (DMF) vorgelegt. Die Reaktionsmischung wird 24 h auf 120°C erhitzt und danach das Lösungsmittel im Vakuum entfernt. Der Rückstand wird säulenchromatographisch an Kieselgel mit Heptan/DCM (2/1) als Eluent gereinigt. **Int-5** wird als leicht gelblicher Feststoff in 76% Ausbeute (10 g, 31 mmol) erhalten.

**Beispiel 13**

**Synthese von 6,12-Dibromo-tribenz[a,c,e]oxepin (Int-6)**

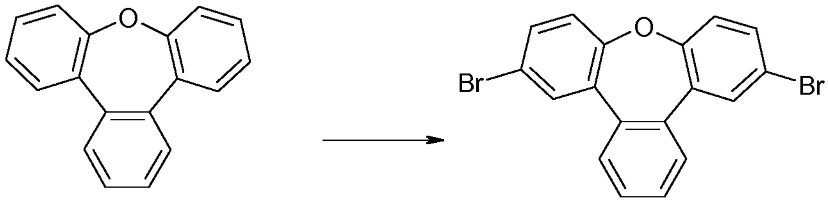

10 g (41 mmol) Tribenz[a,c,e]oxepin werden mit 16 g N-Bromsuccinimid (90 mmol, 2,2 mol%) in 150 mL trockenem Dimethylformamid (DMF) vorgelegt. Die Reaktionsmischung wird 24 h auf 120°C erhitzt und danach das

Lösungsmittel im Vakuum entfernt. Der Rückstand wird säulenchromatographisch an Kieselgel mit Heptan/DCM (2/1) als Eluent gereinigt. **Int-6** wird als gelber Feststoff in 91% Ausbeute (15 g, 37 mmol) erhalten.

## Beispiel 14

### Synthese von 2-(Tribenz[a,c,e]oxepin-8-yl)-4,6-diphenyl-[1,3,5]triazine

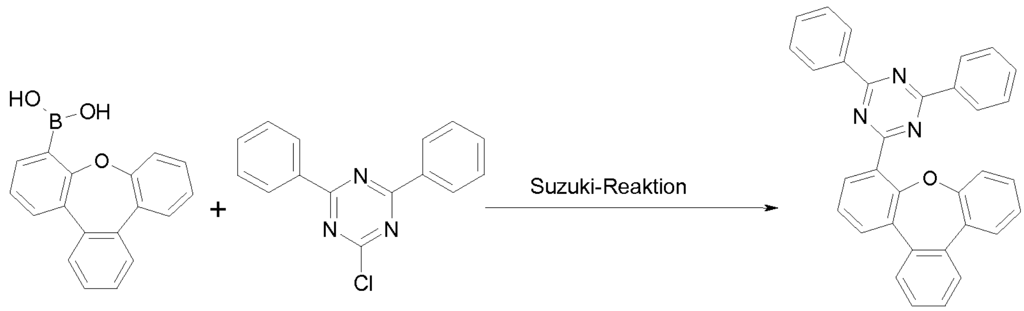


8,4 g (29,2 mmol)Tribenz[a,c,e]oxepin-1-boronsäure, 10,2 g 2-Chloro-4,6-diphenyl-[1,3,5]triazine (37,9 mmol, 1,3 mol%) und 6,8 g $Na_2CO_3$ (64,1 mmol, 2.2 mol%) warden in einer Mischung aus 122 mL Toluol, 60 mL 1,4-Dioxan und 30 mL Wasser vorgelegt. Dann werden 1,68 g $Pd(PPh_3)_4$ (1,46 mmol, 0,05 mol%) zugeben und die Reaktionsmischung 15 h auf 80°C erhitzt. Die organische Phase wird abgetrennt, dreimal mit je 100 mL Wasser extrahiert, mit $Na_2SO_4$ getrocknet und im Vakuum zur Trockene eingeengt. Das Rohprodukt wird durch Säulenchromatographie ($SiO_2$, Heptan/$CH_2Cl_2$ 2/1) gereinigt. Das Oxepin wird in 63% Ausbeute (8,74 g, 18,4 mmol) als sehr feine leicht gelbliche Kristalle erhalten.

Analog können folgende Verbindungen erhalten werden:

| Edukt 1 | Edukt 2 | Produkt | Ausbeute |
|---|---|---|---|
| 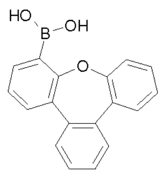 | 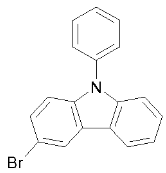 [1153-85-1] | 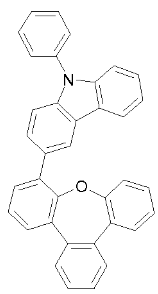 | 78% |
| 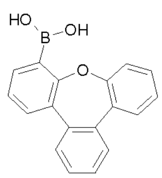 | 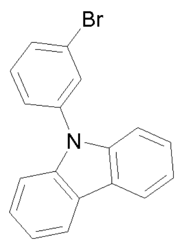 [185112-61-2] | 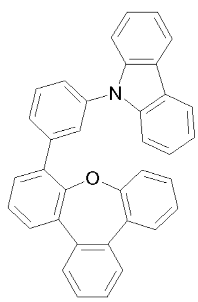 | 79% |

(fortgesetzt)

| Edukt 1 | Edukt 2 | Produkt | Ausbeute |
|---|---|---|---|
| 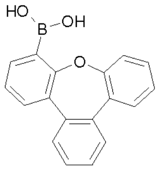 | 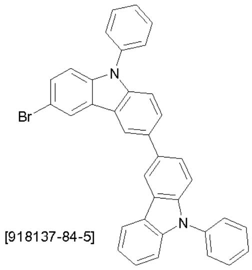 [918137-84-5] | 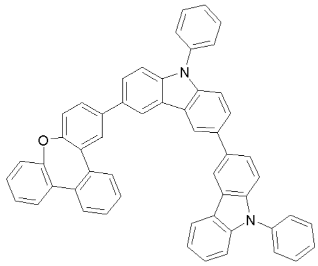 | 88% |
| 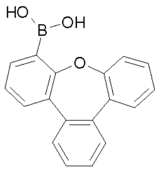 | 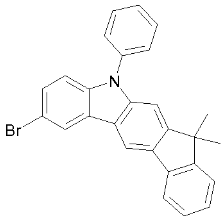 | 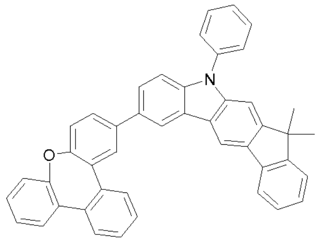 | 83% |
| 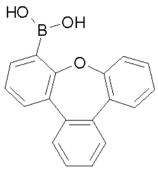 | 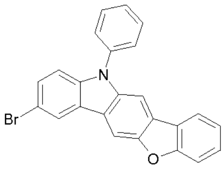 [1377576-51-6] | 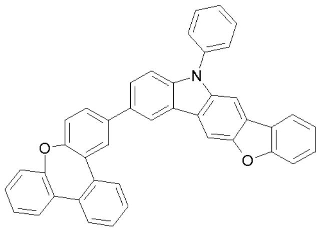 | 84% |
| 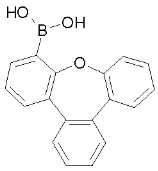 | 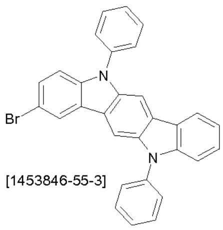 [1453846-55-3] | 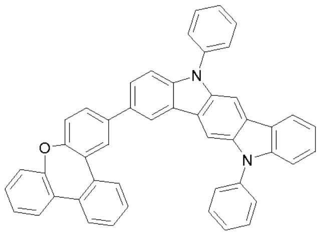 | 86% |
| 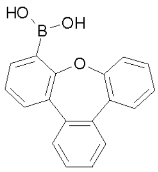 | 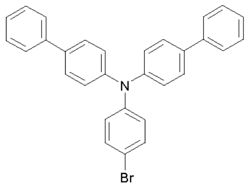 [499128-71-1] | 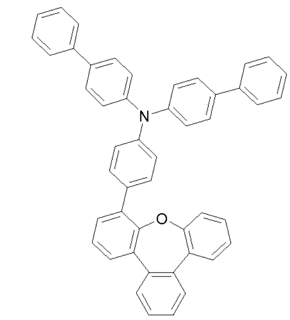 | 82% |
| 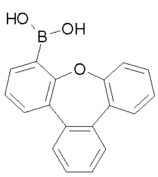 | 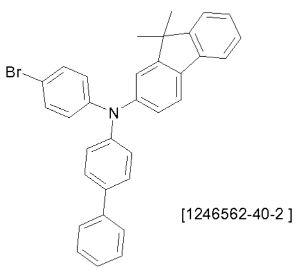 [1246562-40-2 ] | 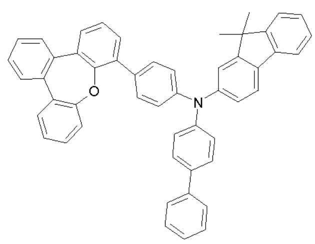 | 80% |

(fortgesetzt)

| Edukt 1 | Edukt 2 | Produkt | Ausbeute |
|---|---|---|---|
| 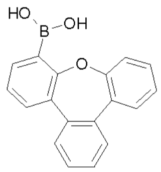 | 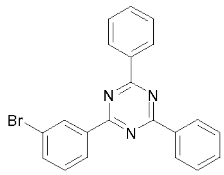 [864377-31-1] | 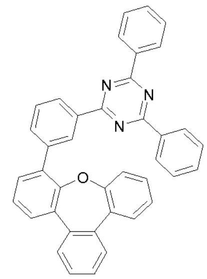 | 85% |
| 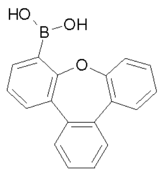 | 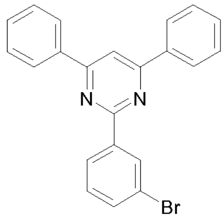 [864377-22-0] | 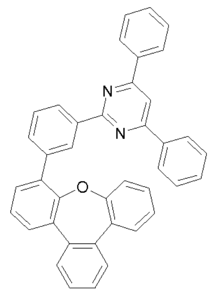 | 87% |
| 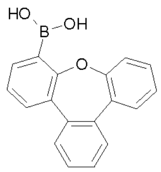 | 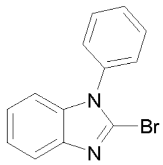 [1418123-78-0] | 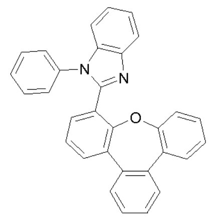 | 82% |
| 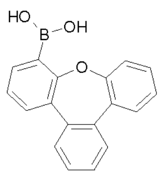 | 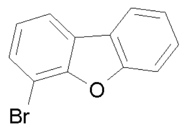 | 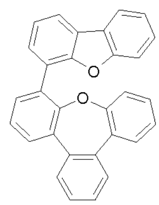 | 91% |
| 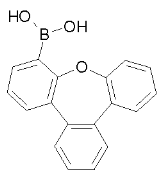 | 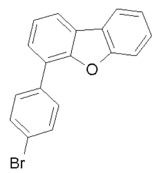 | 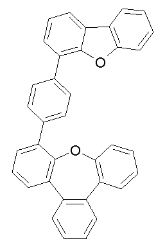 | 90% |
| 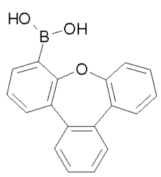 | 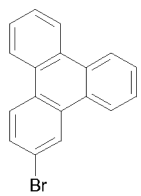 | 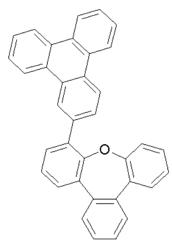 | 89% |

(fortgesetzt)

| Edukt 1 | Edukt 2 | Produkt | Ausbeute |
|---|---|---|---|
| 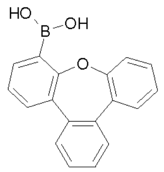 | 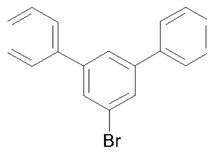 [103068-20-8] | 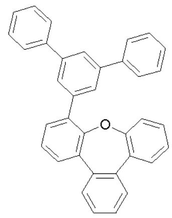 | 93% |
| 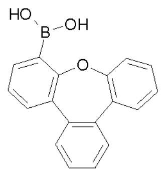 | 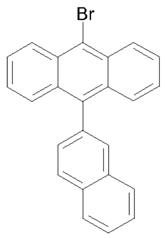 [474688-73-8] | 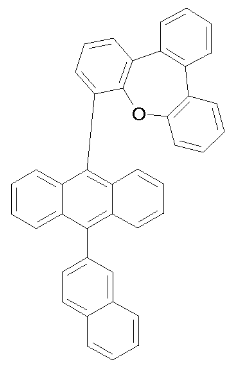 | 79% |

Analog können folgende Verbindungen mit 0,5 eq. entsprechende Boronsäure erhalten werden:

| Edukt 1 | Edukt 2 | Produkt | Ausbeute |
|---|---|---|---|
| 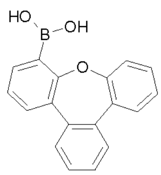 | 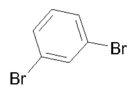 | 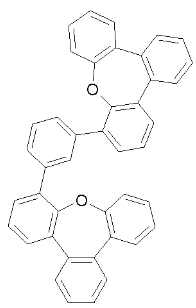 | 79% |
| 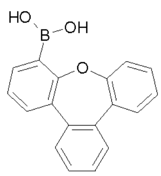 | 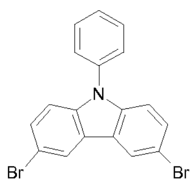 [57103-20-5] | 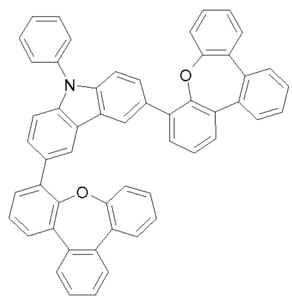 | 75% |
| 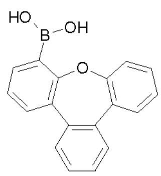 | 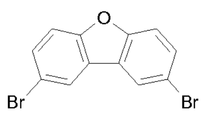 [10016-52-1] | 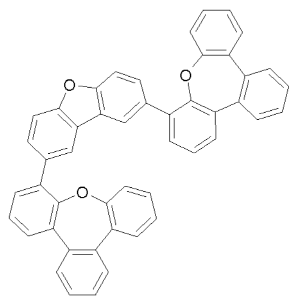 | 76% |

(fortgesetzt)

| Edukt 1 | Edukt 2 | Produkt | Ausbeute |
|---|---|---|---|
| 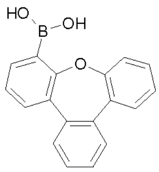 | 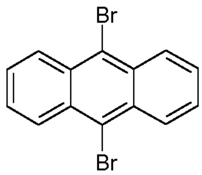 [523-27-3] | 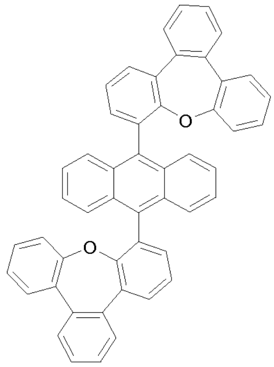 | 79% |

**Beispiel 15**

**Synthese von 3-(Tribenz[a,c,e]oxepin-6-yl)-9-phenyl-9H-carbazol**

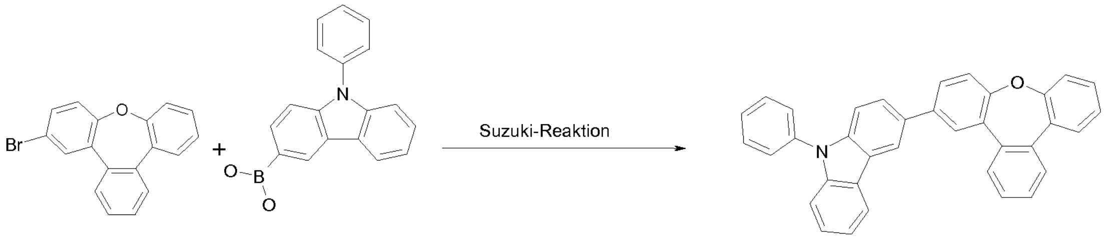


6-Brom-tribenz[a,c,e]oxepin **(Int-5)** (132,3 mg; 0,41 mmol) und N-Phenyl-carbazol-3-boronsäure (129,3 mg; 0,45 mmol; 1,1 mol%) werden zusammen mit Kaliumcarbonat (124,5 mg; 0,90 mmol; 2,2 mol%) in einer Mischung aus 3,5 mL Ethylenglycoldimethylether, 3,5 mL Toluol und 2,5 mL VE-Wasser vorgelegt. Durch die Mischung wird 30 min Argon geleitet. Danach werden zu der Mischung Tri(o-tolyl)-phosphin (3,37 mg; 0,011 mmol; 4 mol%) und $Pd(OAc)_2$ (1,24 mg; 0,006 mmol; 2 mol%) geben. Die Reaktionsmischung wird über Nacht auf 85°C erhitzt. Nach dem Abkühlen wird die organische Phase abgetrennt und die wässrige Phase mit 50 mL $CH_2Cl_2$ extrahiert. Die vereinigten organischen Phasen werden mit 50 mL Wasser extrahiert und mit $MgSO_4$ getrocknet. Das Lösungsmittel wird im Vakuum entfernt und der ölige Rückstand säulenchromatographisch an Kieselgel mit Heptan/DCM (2/1) als Eluent gereinigt. Das Zielprodukt wird als leicht gelblicher Feststoff in 72% Ausbeute (100,5 mg, 0,31 mmol) erhalten.

Analog können folgende Verbindungen erhalten werden:

| Edukt 1 | Edukt 2 | Produkt | Ausbeute |
|---|---|---|---|
| 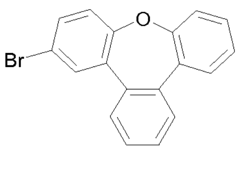 | 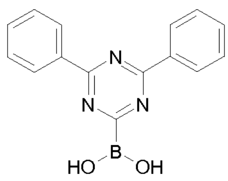 [1251825-65-6] | 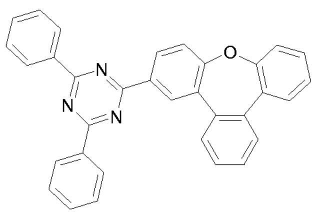 | 56% |

(fortgesetzt)

| Edukt 1 | Edukt 2 | Produkt | Ausbeute |
|---|---|---|---|
| 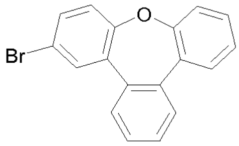 | 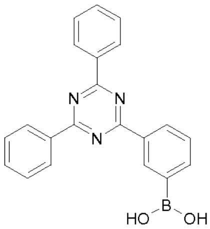 [1269508-31-7] | 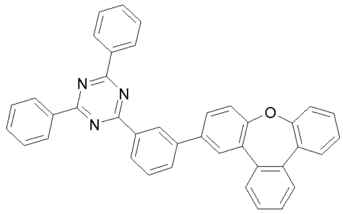 | 92% |
| 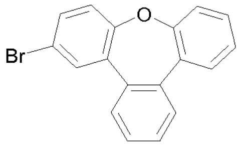 | 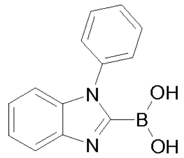 [1214723-25-7] | 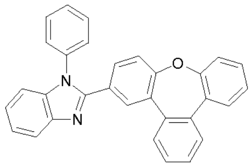 | 75% |
| 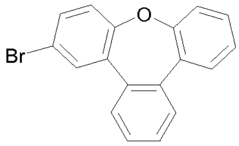 | 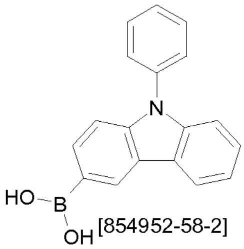 [854952-58-2] | 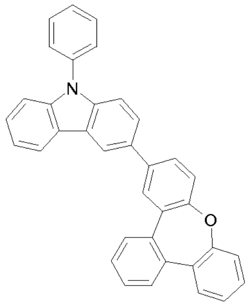 | 76% |
| 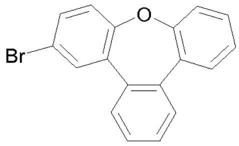 | 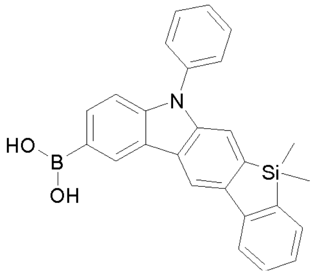 [1377576-48-1] | 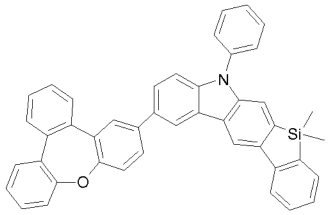 | 84% |
| 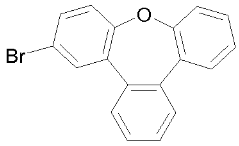 | 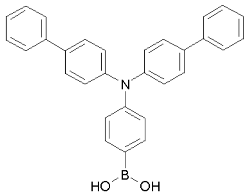 [943836-24-6] | 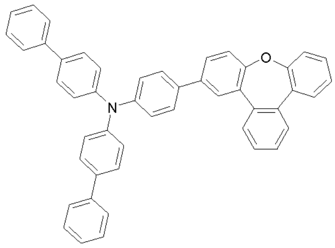 | 89% |

Analog können folgende Verbindungen erhalte werden, wenn 0,5 eq. der Boronsäure eingesetzt werden.

| Edukt 1 | Edukt 2 | Produkt | Ausbeute |
|---|---|---|---|
| 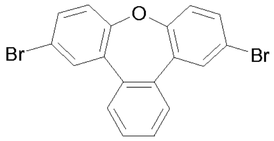 | 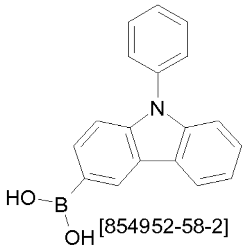 [854952-58-2] | 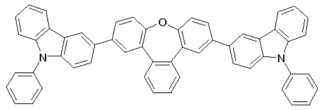 | 72% |
| 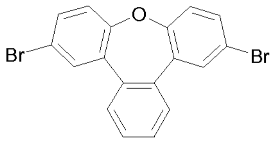 | 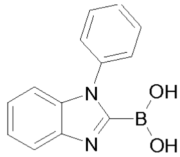 [1214723-25-7] | 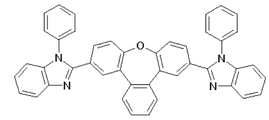 | 74% |
| 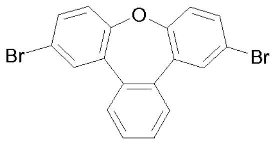 | 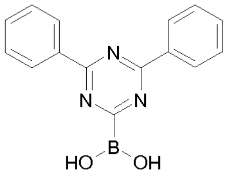 [1251825-65-6] | 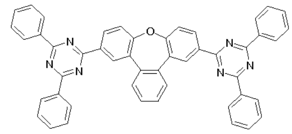 | 63% |
| 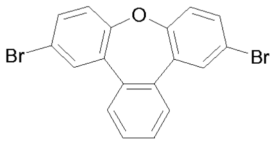 | 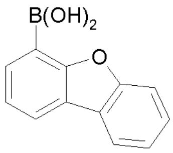 [100124-06-9] | 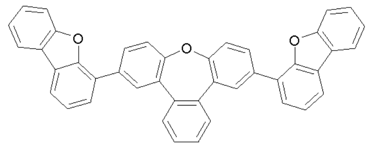 | 72% |
| 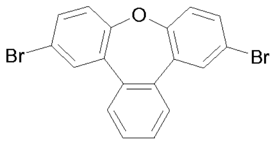 | 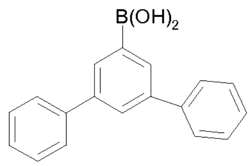 [128388-54-5] | 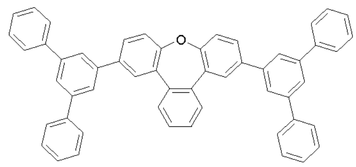 | 76% |

## Beispiel 16

### Synthese von 2-(12-Bromo-tribenz[a,c,e]oxepin-8-yl)-4,6-diphenyl-[1,3,5]triazine

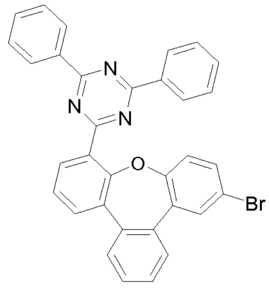

5 g(10,5 mmol) 2-(Tribenz[a,c,e]oxepin-8-yl)-4,6-diphenyl-[1,3,5]triazin werden mit 2,06 g N-Bromsuccinimid (11,6 mmol; 110 mol%) in 100 mL trockenem Dimethylformamid (DMF) vorgelegt. Die Reaktionsmischung wird 48 h auf 60°C erhitzt und danach das Lösungsmittel im Vakuum entfernt. Der Rückstand wird säulenchromatographisch an

Kieselgel mit Heptan/DCM (2/1) als Eluent gereinigt. Das Bromid wird als farbloser Feststoff in 74% Ausbeute erhalten (4,31 g; 7,78 mmol) erhalten.

Analog können folgende mit 2 eq. NBS entsprechende Dibromide erhalten werden:

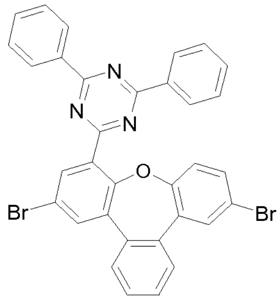

## Beispiel 17

**Synthese von 3-[10-(4,6-Diphenyl-[1,3,5]triazine-2-yl)-tribenz[a,c,e]oxepin-6-yl]-9-phenyl-9H-carbazol**

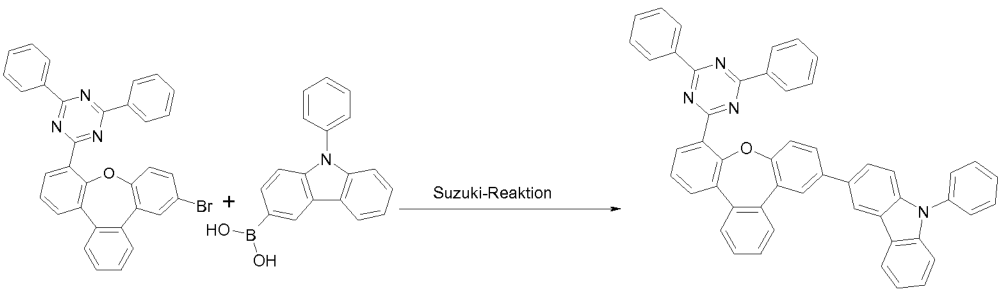


5 g(9 mmol) 2-(12-Bromo-tribenz[a,c,e]oxepin-8-yl)-4,6-diphenyl-[1,3,5]triazin und N-Phenyl-carbazol-3-boronsäure (2,85 g; 9,9 mmol; 110 mol%) werden zusammen mit Kaliumcarbonat (2,74 g; 19,8 mmol; 220 mol%) in einer Mischung aus 200 mL Ethylenglycoldimethylether, 200 mL Toluol und 150 mL VE-Wasser vorgelegt. Durch die Mischung wird 30 min Argon geleitet. Danach werden zu der Mischung Tri(o-tolyl)-phosphin (274 mg; 0,90 mmol; 10 mol%) und $Pd(OAc)_2$ (101 mg; 0,45 mmol; 5 mol%) gegeben. Die Reaktionsmischung wird über Nacht auf 85°C erhitzt. Nach dem Abkühlen wird die organische Phase abgetrennt und die wässrige Phase mit 50 mL $CH_2Cl_2$ extrahiert. Die vereinigten organischen Phasen werden mit 50 mL Wasser extrahiert und mit $MgSO_4$ getrocknet. Das Lösungsmittel wird im Vakuum entfernt und der ölige Rückstand säulenchromatographisch an Kieselgel mit Heptan/DCM (2/1) als Eluent gereinigt. Der Rückstand wird aus Toluol umkristallisiert und abschließend im Hochvakuum (p = 5 x $10^{-6}$ mbar) sublimiert. Ausbeute: 5,62 g (7,85 mmol), 87%; Reinheit: 99,9% n. HPLC.

Analog können folgende Verbindungen erhalten werden:

| Edukt 1 | Edukt 2 | Produkt | Ausbeute |
|---|---|---|---|
| 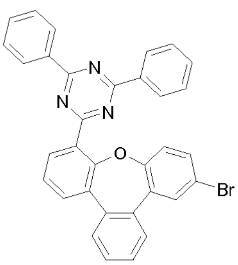 | 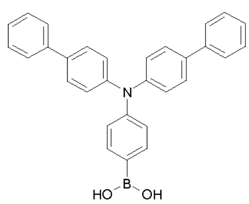 [943836-24-6] | 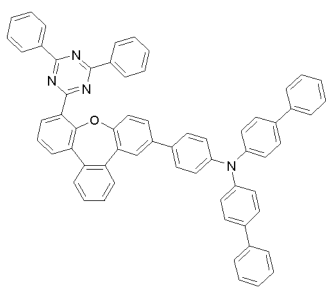 | 75% |

(fortgesetzt)

| Edukt 1 | Edukt 2 | Produkt | Ausbeute |
|---|---|---|---|
| 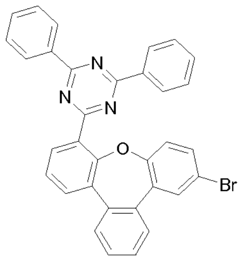 | 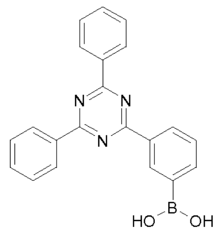 [1269508-31-7] | 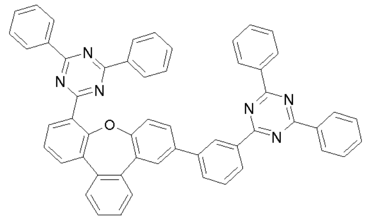 | 69% |
| 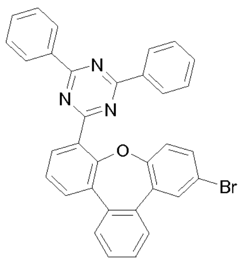 | 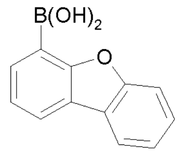 [100124-06-9] | 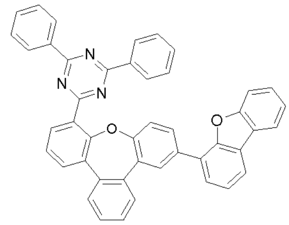 | 76% |
| 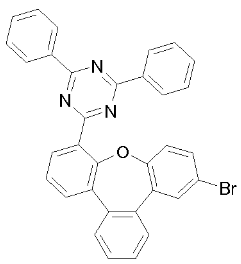 | 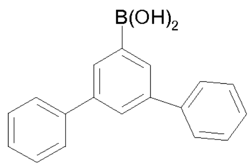 [128388-54-5] | 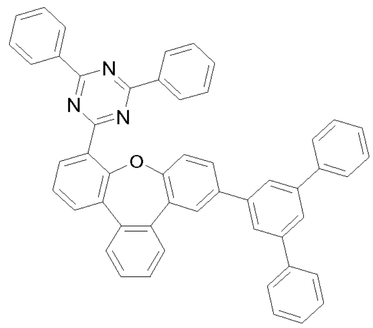 | 79% |
| 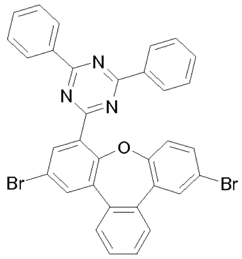 | 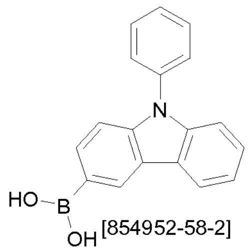 [854952-58-2] | 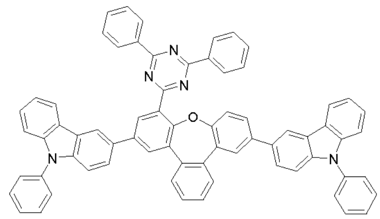 | 62% |

**Beispiel 18**

**Synthese von Biphenyl-4-yl-(9,9-dimethyl-9H-fluoren-2-yl)-(9-oxa-tribenzo[a,c,e]cyclohepten-6-yl)-amin**

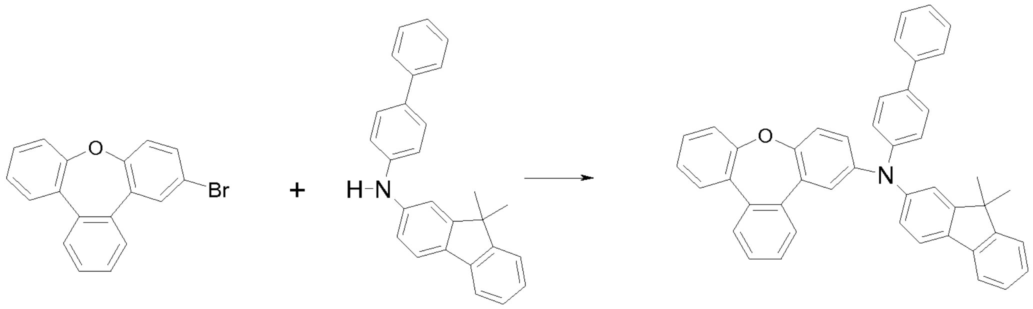

Ein Gemisch aus 16 g (50 mmol) 6-Brom-tribenz[a,c,e]oxepin, 21,7 g (60 mmol) 4-Biphenyl-2-(9,9'-dimethylfluorenyl)-amin, [897671-69-1], 7,7 g (80 mmol) Natrium-tert-butylat, 1,4 g (5 mmol) Tricyclohexylamin, 561 mg (2,5 mmol) Palladium(II)acetat und 300 mL Mesitylen wird 24 h unter Rückfluss erhitzt. Nach dem Erkalten versetzt man mit 200 mL Wasser, rührt 30 min. nach, trennt die org. Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. $10^{-6}$ mbar, T = 330 - 340°C). Ausbeute:21,7 g (34 mmol), 72%; Reinheit: 99,9% n. HPLC.

Analog können folgende Verbindungen erhalten werden:

| Edukt 1 | Edukt 2 | Produkt | Ausbeute |
|---|---|---|---|
| 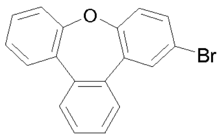 | 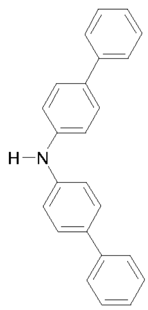 102113-98-4 | 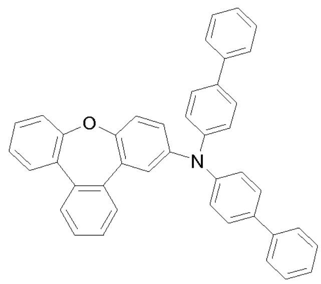 | 43 % |
| 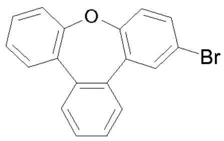 | 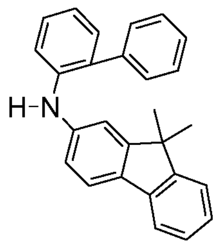 1198395-24-2 | 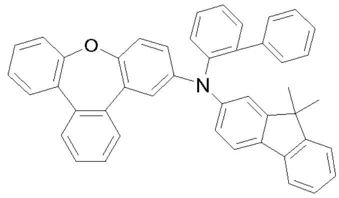 | 72 % |
| 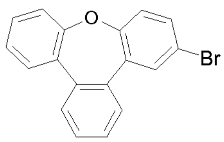 | 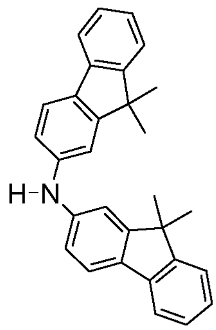 500717-23-7 | 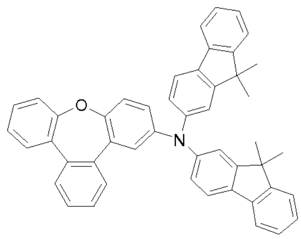 | 61 % |
| 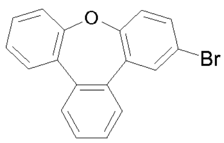 | 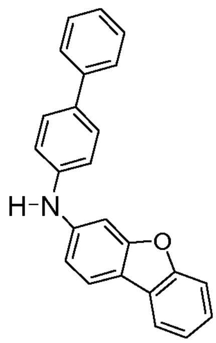 1290039-85-8 | 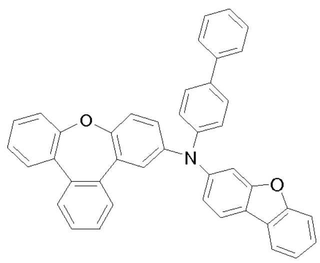 | 73 % |

(fortgesetzt)

| Edukt 1 | Edukt 2 | Produkt | Ausbeute |
|---|---|---|---|
| 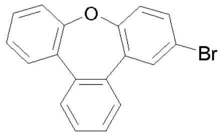 | 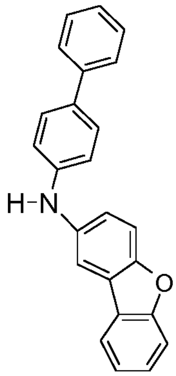 1300028-94-7 | 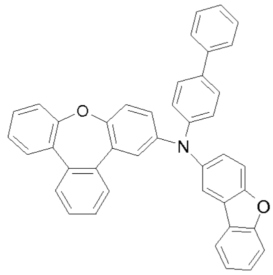 | 67 % |
| 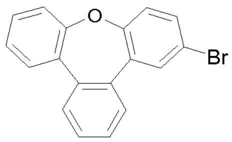 | 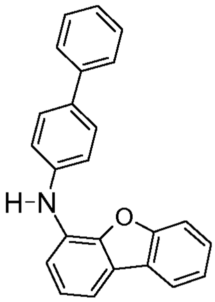 1318338-47-4 | 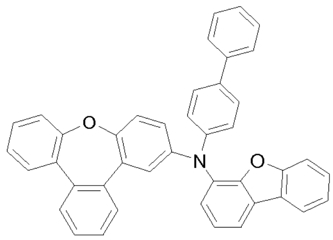 | 60 % |
| 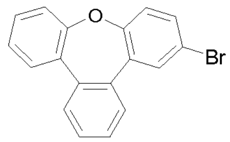 | 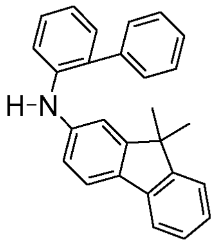 1198395-24-2 | 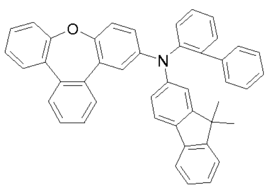 | 64 % |
| 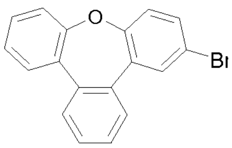 | 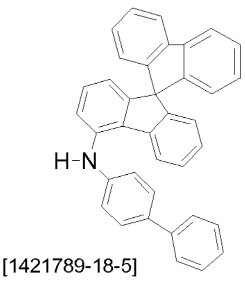 [1421789-18-5] | 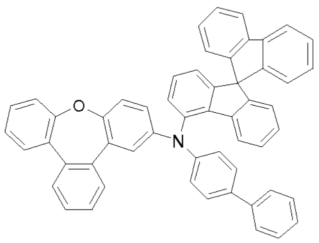 | 72% |

Analog können mit 0,5 eq. Dibromiden folgende Verbindungen erhalten werden:

| Edukt 1 | Edukt 2 | Produkt | Ausbeute |
|---|---|---|---|
| 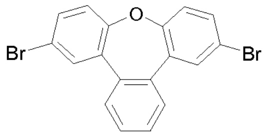 | 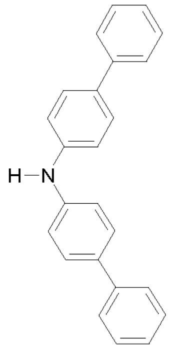 102113-98-4 | 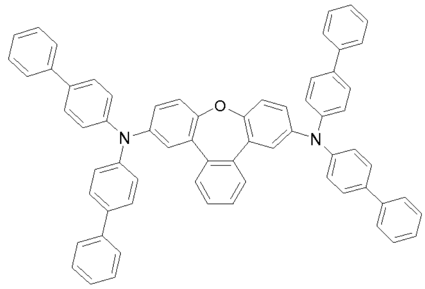 | 58% |
| 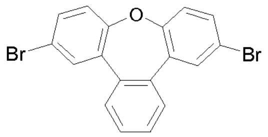 | 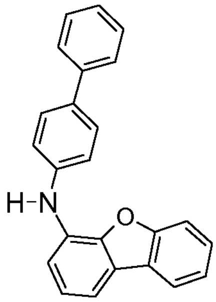 1318338-47-4 | 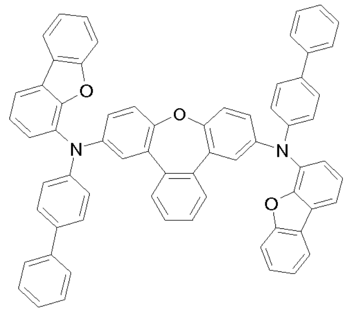 | 69% |

**Beispiel 19**

**Synthese von 9-(9-Oxa-tribenzo[a,c,e]cyclohepten-6-yl)-9'-phenyl-9H,9'H-[3,3']bicarbazolyl**

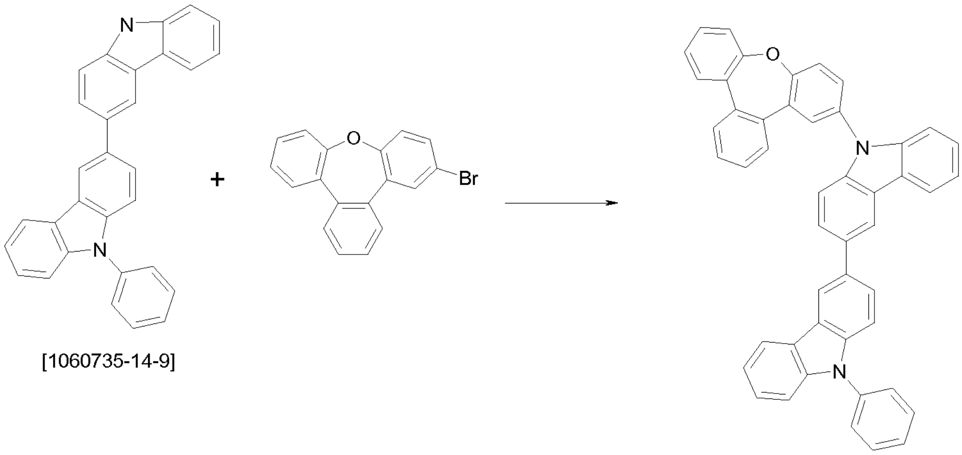

[1060735-14-9]

18,7 g (46 mmol) 9-Phenyl-3,3'-bicarbazol und 14,8 g (46 mmol) 6-Bromtribenz[a,c,e]oxepin werden in 450 mL Toluol gelöst und mittels Schutzgaseinleitung entgast. Anschließend wird mit 7 mL (7 mmol, 1 M Lösung in Toluol) Tri-tert-butylphosphin, 633,8 mg (2,82 mmol) $Pd(OAc)_2$ und 7 g (76 mmol) NaOtBu versetzt. Die Feststoffe werden zuvor entgast, die Reaktionsmischung wird nachentgast und anschließend unter Rückfluss für 3 h gerührt. Die warme Reaktionslösung wird über Alox B (Aktivitätsstufe 1) filtriert, mit Wasser gewaschen, getrocknet und eingeengt. Der Rückstand wird aus Toluol umkristallisiert und abschließend im Hochvakuum (p = 5 x $10^{-6}$ mbar) sublimiert. Ausbeute:24 g (38 mmol), 83%; Reinheit: 99.9% n. HPLC.

Analog können folgende Verbindungen erhalten werden:

| Edukt 1 | Edukt 2 | Produkt | Ausbeute |
|---|---|---|---|
| 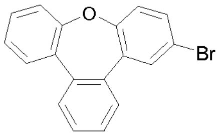 | 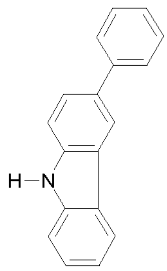 [103012-26-6] | 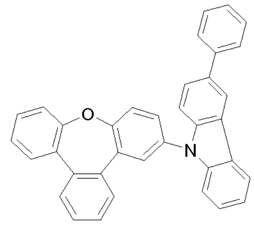 | 68% |
| 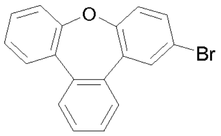 | 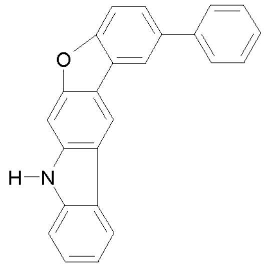 [1439927-96-4] | 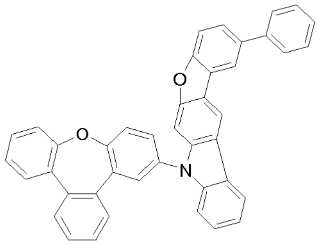 | 65% |
| 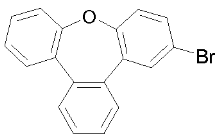 | 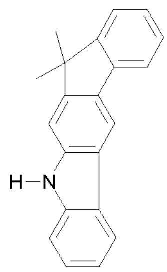 [1257220-47-5]] | 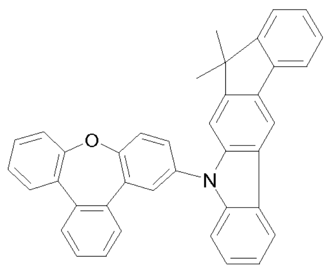 | 64% |
| 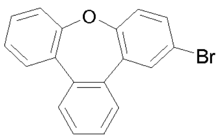 | 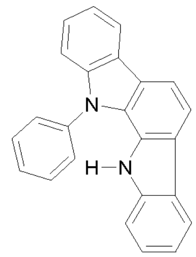 [1024598-06-8] | 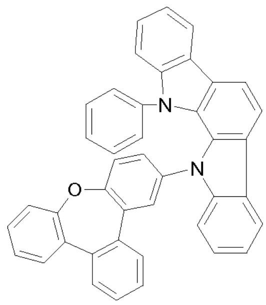 | 61% |

Analog kann mit 0,5 eq. der entsprechenden Dibromide die folgende Verbindung erhalten werden.

| Edukt 1 | Edukt 2 | Produkt | Ausbeute |
|---|---|---|---|
| 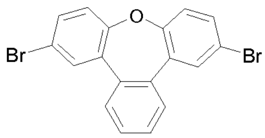 | 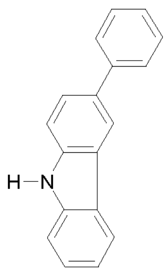 | 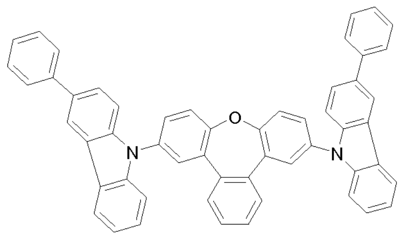 | 68% |

(fortgesetzt)

| Edukt 1 | Edukt 2 | Produkt | Ausbeute |
|---|---|---|---|
| | [103012-26-6] | | |
| | 102113-98-4 | | |

**Beispiel 20**

**Synthese von (2-Chloro-phenyl)-(9-oxa-tribenzo[a,c,e]cyclohepten-6-yl)-amin**

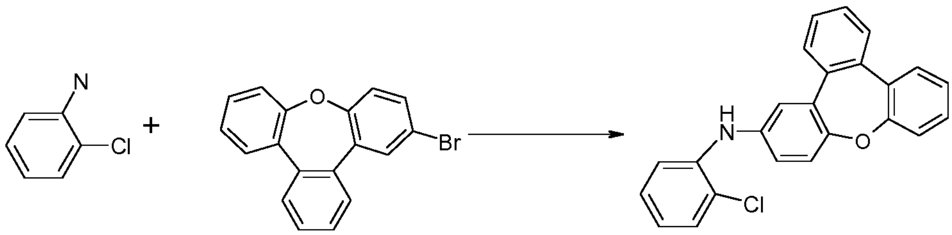

25,5 g (79 mmol) 6-Brom-tribenz[a,c,e]oxepin, 10 mL (95 mmol) 2-Chloranilin, 36,3 g (111 mmol) Casiumcarbonat, 0,89 g (3,9 mmol) Palladium(II)acetat und 3,9 g (6 mmol) 2,2'-Bis-diphenylphosphanyl-[1,1']binapthaleny werden in 500 mL Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 22 g (61 mmol) 78% der Theorie.

**Beispiel 21**

**Cyclisierung**

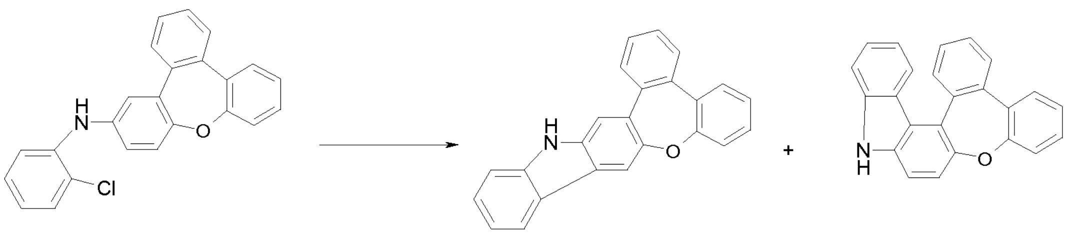

**21a** 51%

**21b** 40%

36,7 g (102 mmol) (2-Chloro-phenyl)-(9-oxa-tribenzo[a,c,e]cyclohepten-6-yl)-amin, 32 g (268 mmol) Kaliumcarbonat, 0.6 g (2,7 mmol) Palladium(II)-acetat und 4,2 mL (4,2 mmol) Tri-tert-butylphosphan werden in 350 mL Dimethylacetamid suspendiert und 6 h unter Rückfluss gerührt. Nach dem Erkalten wird die Reaktionmischung mit 300 mL Wasser und 400 mL Ethylacetat versetzt. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Die Isomeren werden chromatogrphisch getrennt. Ausbeute: 30 g (91 mmol) 92% der Theorie.

**Beispiel 22**

**Arylierung**

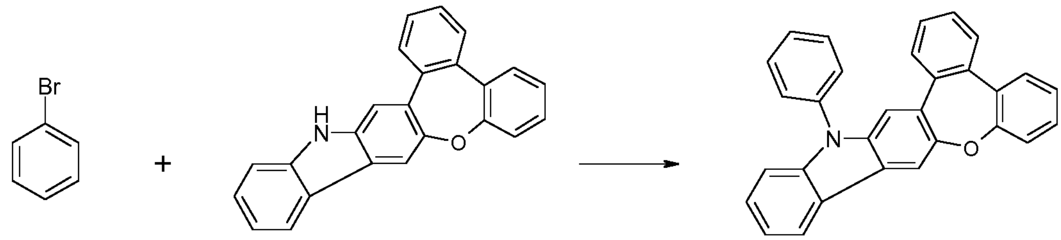

35 g (106 mmol) der Verbindung 21a, 17,9 g (114 mmol) Brombenzol und 30,5 g NaOtBu werden in 1,5 L p-Xylol suspendiert. Zu dieser Suspension werden 0,5 g (2,11 mmol) $Pd(OAc)_2$ und 6 mL einer 1 M Tri-tert-butylphosphin (1M Lösung in Toluol) gegeben. Die Reaktionsmischung wird 16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert, aus Toluol umkristallisiert und abschließend im Hochvakuum sublimiert, Reinheit beträgt 99,9% bei einer Ausbeute von 29 g (73 mmol; 69%).

Analog können die folgenden Verbindung hergestellt werden.

| Edukt 1 | Edukt 2 | Produkt | Ausbeute |
|---|---|---|---|
| 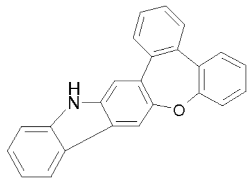 | 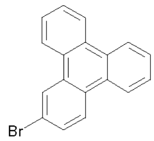 [ 19111-87-6] | 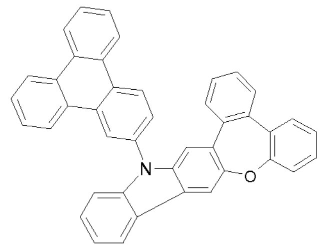 | 78% |
| 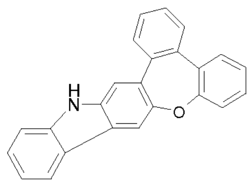 | 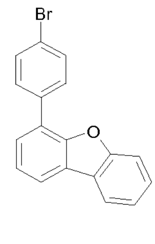 [955959-84-9] | 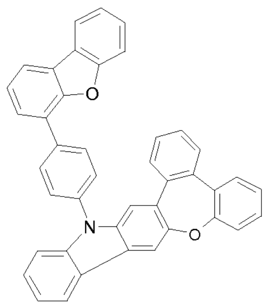 | 79% |
| 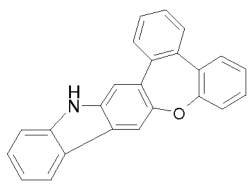 | 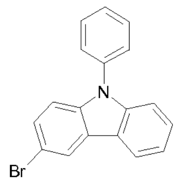 [1153-85-1] | 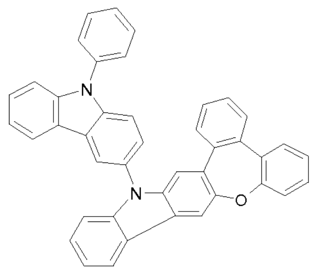 | 88% |

(fortgesetzt)

| Edukt 1 | Edukt 2 | Produkt | Ausbeute |
|---|---|---|---|
| 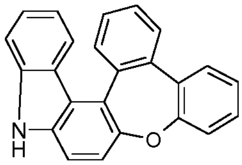 | 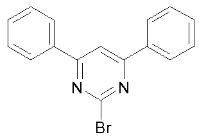 [56181-49-8] | 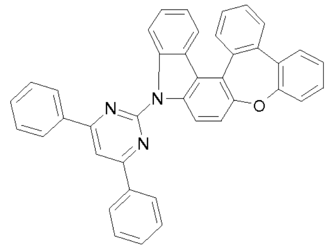 | 83% |

**Beispiel 23**

**Herstellung von OLEDs**

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1 und 2) werden die Daten verschiedener OLEDs vorgestellt. Als Substrate werden Glasplättchen verwendet, die mit strukturiertem ITO (Indium Zinn Oxid) der Dicke 50 nm beschichtet sind.

Die OLEDs haben prinzipiell folgenden Schichtaufbau: Substrat / Interlayer (IL) / Lochinjektionsschicht (HIL) / Elektronenblockierschicht (EBL) / Emissionsschicht (EML) / Elektronentransportschicht (ETL) / Elektroneninjektionsschicht (EIL) und abschließend eine Kathode. Die EIL wird durch Aufdampfen einer 2 nm dicken Schicht bestehend aus Liq erhalten. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:D1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 $cd/m^2$ bestimmt und daraus die CIE 1931 x und y Farbkoordinaten berechnet. Die Angabe EQE @ 1000 $cd/m^2$ bezeichnet die externe Quanteneffizienz bei einer Betriebsleuchtdichte von 1000 $cd/m^2$. LD80 @ 100 $mA/cm^2$ bzw. 60 $mA/cm^2$ ist die Lebensdauer bis das OLED auf 80% der Anfangsintensität abgefallen ist, wenn das OLED bei einem Strom von 100 $mA/cm^2$ bzw. 60 $mA/cm^2$ betrieben wird. Für grün emittierende OLEDs wird eine Starthelligkeit von 100 $mA/cm^2$ bzw. für blau emittierende OLEDs eine Starthelligkeit von 60 $mA/cm^2$ gewählt. Die Daten der verschiedenen OLEDs sind in Tabelle 2 zusammengefasst.

**Verwendung von erfindungsgemäßen Verbindungen als Matrixmaterialien, als Emitter und als Elektronentransportmaterial in grün bzw. blau fluoreszierenden OLEDs**

Insbesondere eignen sich erfindungsgemäße Verbindungen als Matrixmaterial, Dotand oder auch als Elektronentransportmaterial in OLEDs. Sie eignen sich als Einzelschicht, aber auch als Mixed Komponente als innerhalb der EML oder ETL.

Verglichen mit Referenz Bauteilen (V1 oder V4) zeigen alle Proben mit den erfindungsgemäßen Verbindungen höhere Effizienzen, niedrigere Betriebsspannung, bzw. deutlich verbesserte Lebensdauern in grün bzw. blau bzw. grün fluoreszierenden OLEDs. Bauteil V5 zeigt mit der erfindungsgemäßen Verbindung INV-3 eine tiefere Farbe als das Referenzbauteil V4.

| Tabelle 1: Aufbau der OLEDs | | | | | |
|---|---|---|---|---|---|
| ***Bsp.*** | ***IL*** | ***HIL*** | ***EBL*** | ***EML*** | ***ETL*** |
| | *Dicke / nm* | *Dicke / nm* | *Dicke / nm* | *Dicke / nm* | *Dicke / nm* |
| *V1* | *HIL1 5 nm* | *HIL2 140 nm* | *NPB 20 nm* | *H1(95%):G1(5%) 20 nm* | *ETM1(50%):LiQ(50%) 30 nm* |
| *V2* | *HIL1 5 nm* | *HIL2 140 nm* | *NPB 20 nm* | *INV-1(95%):G1(5%) 20 nm* | *ETM1(50%):LiQ(50%) 30 nm* |
| *V3* | *HIL1 5 nm* | *HIL2 140 nm* | *NPB 20 nm* | *INV-2(95%):G1(5%) 20 nm* | *ETM1(50%):LiQ(50%) 30 nm* |
| *V4* | *HIL1 5 nm* | *HIL2 140 nm* | *NPB 20 nm* | *H1(95%):B1(5%) 20 nm* | *ETM1(50%):LiQ(50%) 30 nm* |
| *V5* | *HIL1 5 nm* | *HIL2 140 nm* | *NPB 20 nm* | *H1(95%):INV-3(5%) 20 nm* | *ETM1(50%):LiQ(50%) 30 nm* |
| *V6* | *HIL1 5 nm* | *HIL2 140 nm* | *NPB 20 nm* | *H1(95%):B1(5%) 20 nm* | *INV-6(50%):LiQ(50%) 30 nm* |

| | Tabelle 2: Daten der OLEDs | | | | |
|---|---|---|---|---|---|
| ***Bsp.*** | ***EQE @ 1000 cd/m2*** | ***U @ 1000 cd/m2*** | ***LD80 @ 100 mA/cm$^2$ bzw. 60 mA/cm$^2$*** | ***CIE*** | |
| | *%* | *V* | *[h]* | *x* | y |
| *V1* | *7.0* | *4.2* | *190* | 0.27 | 0.66 |
| *V2* | *7.5* | *4.4* | *190* | 0.27 | 0.67 |
| *V3* | *6.8* | *4.0* | *270* | 0.27 | 0.67 |
| *V4* | *6.3* | *5.0* | *210* | 0.14 | 0.16 |
| *V5* | *6.7* | *4.7* | *200* | 0.13 | 0.14 |
| *V6* | *6.3* | *4.8* | *250* | 0.14 | 0.16 |

| Tabelle 3: Verwendete Materialien | | |
|---|---|---|
| 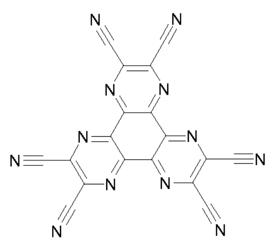 | 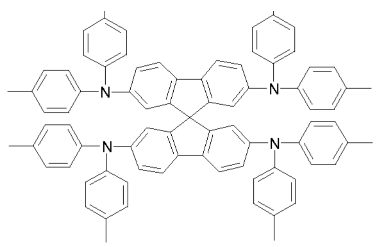 | 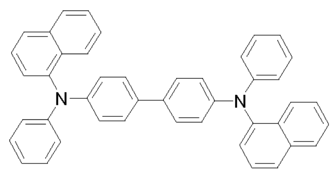 |
| HIL1 | HIL2 | NPB |
| 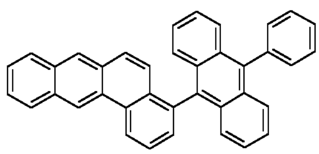 | 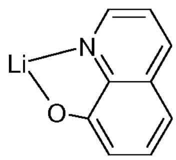 | 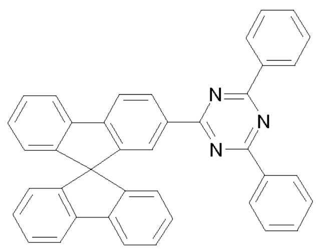 |
| H1 | LiQ | ETM1 |

(fortgesetzt)

| Tabelle 3: Verwendete Materialien | | |
|---|---|---|
| 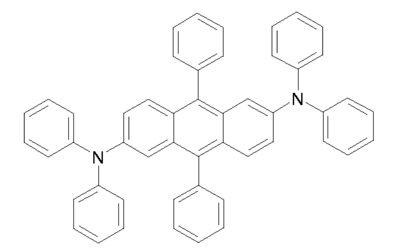 | 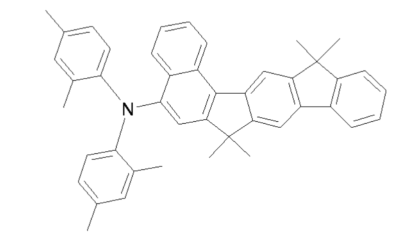 | 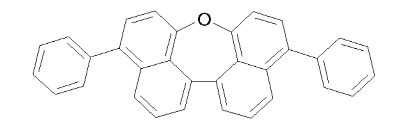 |
| G1 | B1 | INV-1 |
| 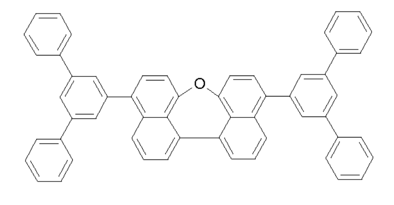 | 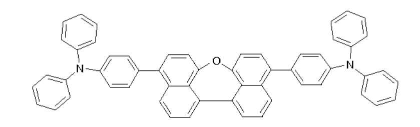 | 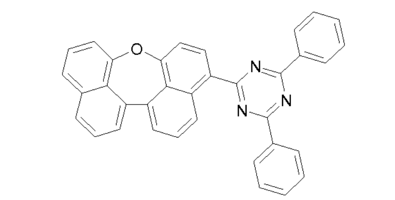 |
| INV-2 | INV-3 | INV-4 |

**Beispiel 24**

**Herstellung von OLEDs**

In den folgenden Beispielen E1 bis E27 (Tabellen 4 und 5) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufge¬schleudert) und bei 180°C für 10 min. ausgeheizt. Diese beschichteten Glasplättchen bilden die Substrate, auf welche die OLEDs aufgebracht werden.

Die OLEDs haben prinzipiell folgenden Schichtaufbau: Substrat / Loch-transport¬schicht (HTL) / Zwischenschicht (IL) / Elektronenblockierschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 4 zu entnehmen. Eine Bezeichnung wie "INV-10" in der Tabelle bezieht sich auf die erfindungsgemäßen Materialien, deren Struktur in der Tabellle 6 gezeigt sind.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:INV-7:TEG2 (59%:29%:12%) bedeutet hierbei, dass das Material IC1 in einem Volumenanteil von 59%, INV-7 in einem Anteil von 29% und TEG2 in einem Anteil von 12% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungs¬¬effizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 5 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$. Die Daten der verschiedenen OLEDs sind in Tabelle 5 zusammengefasst. Man sieht, dass sich mit erfindungsgemäßen Materialien hervorragende Leistungsdaten bei Verwendung als Lochtransportmaterial, als Matrixmaterial für phosphoreszierende Emitter und bei Verwendung als Elektronentransportmaterial erzielen lassen.

Tabelle 4: Aufbau der OLEDs

| Bsp. | HTL Dicke | IL Dicke | EBL Dicke | EML Dicke | HBL Dicke | ETL Dicke | EIL Dicke |
|---|---|---|---|---|---|---|---|
| E1 | HATCN 5nm | SpMA1 50nm | SpMA2 10nm | INV-5:TEG2 (83%:17%) 30nm | ST2 10nm | ST2:LiQ (50%:50%) 30nm | --- |
| E2 | HATCN 5nm | SpMA1 50nm | SpMA2 10nm | IC1:INV-6:TEG2 (44%:44%:12%) 30nm | ST2 10nm | ST2:LiQ (50%:50%) 30nm | --- |
| E3 | HATCN 5nm | SpMA1 50nm | SpMA2 10nm | IC1:INV-7:TEG2 (59%:29%:12%) 30nm | ST2 10nm | ST2:LiQ (50%:50%) 30nm | --- |
| E4 | HATCN 5nm | SpMA1 50nm | SpMA2 10nm | INV-5:INV-8:TEG2 (44%:44%:12%) 30nm | ST2 10nm | ST2:LiQ (50%:50%) 30nm | --- |
| E5 | HATCN 5nm | SpMA1 50nm | SpMA2 10nm | IC3:INV-9:TEG2 (55%:35%:10%) 30nm | ST2 10nm | ST2:LiQ (50%:50%) 30nm | --- |
| E6 | HATCN 5nm | SpMA1 50nm | INV-10 10nm | IC3:TEG2 (83%:17%) 30nm | ST2 10nm | ST2:LiQ (50%:50%) 30nm | --- |
| E7 | HATCN 5nm | --- | SpMA1 60nm | INV-11:TEG1 (83%:17%) 30nm | ST2 10nm | ST2:LiQ (50%:50%) 30nm | --- |
| E8 | HATCN 5nm | SpMA1 50nm | SpMA2 10nm | ST1:INV-12:TEG2 (28%:55%:17%) 30nm | ST2 10nm | ST2:LiQ (50%:50%) 30nm | --- |
| E9 | HATCN 5nm | SpMA1 50nm | SpMA2 10nm | L1:INV-13:TEG2 (33%:50%:17%) 30nm | ST2 10nm | ST2:LiQ (50%:50%) 30nm | --- |
| E10 | HATCN 5nm | SpMA1 50nm | SpMA2 10nm | IC1:INV-14:TEG2 (44%:44%:12%) 30nm | ST2 10nm | ST2:LiQ (50%:50%) 30nm | --- |
| E11 | HATCN 5nm | SpMA1 50nm | SpMA2 10nm | L2:INV-15:TEG2 (42%:41%:17%) 30nm | --- | ST2:LiQ (50%:50%) 40nm | --- |
| E12 | HATCN 5nm | SpMA1 50nm | SpMA2 10nm | IC1:INV-16:TEG2 (44%:44%:12%) 30nm | ST2 10nm | ST2:LiQ (50%:50%) 30nm | --- |
| E13 | HATCN 5nm | SpMA1 50nm | SpMA2 10nm | IC1:TEG2 (83%:17%) 30nm | ST2 10nm | INV-17 30nm | LiQ 3nm |
| E14 | HATCN 5nm | SpMA1 50nm | SpMA2 10nm | IC1:INV-18:TEG2 (44%:44%:12%) 30nm | ST2 10nm | ST2:LiQ (50%:50%) 30nm | --- |
| E15 | HATCN 5nm | SpMA1 50nm | SpMA2 10nm | INV-19:TEG2 (83%:17%) 30nm | --- | ST2:LiQ (50%:50%) 40nm | --- |
| E16 | HATCN 5nm | SpMA1 50nm | SpMA2 10nm | INV-20:TEG2 (83%:17%) 30nm | ST2 10nm | ST2:LiQ (50%:50%) 30nm | --- |
| E17 | HATCN 5nm | SpMA1 50nm | SpMA2 10nm | IC1:TEG2 (83%:17%) 30nm | ST2 10nm | INV-20:LiQ (50%:50%) 30nm | --- |
| E18 | SpA1 90nm | HATCN 5nm | SpMA1 130nm | 9a:IC2:TER3 (40%:50%:10%) 40nm | IC3 5nm | ST2:LiQ (50%:50%) 35nm | --- |
| E19 | HATCN 5nm | SpMA1 50nm | INV-21 10nm | IC3:TEG1 (90%:10%) 30nm | ST2 10nm | ST2:LiQ (50%:50%) 30nm | --- |
| E20 | HATCN 5nm | SpMA1 f50nm | INV-22 10nm | IC1:TEG2 (83%:17%) 30nm | ST2 10nm | ST2:LiQ (50%:50%) 30nm | --- |
| E21 | HATCN 5nm | SpMA1 50nm | INV-23 10nm | IC1:TEG2 (83%:17%) 30nm | ST2 10nm | ST2:LiQ (50%:50%) 30nm | --- |
| E22 | HATCN | INV-24 | SpMA1 | IC3:TEG1 | IC3 | ST2:LiQ | --- |

(fortgesetzt)

| Bsp. | HTL Dicke | IL Dicke | EBL Dicke | EML Dicke | HBL Dicke | ETL Dicke | EIL Dicke |
|---|---|---|---|---|---|---|---|
| | 5nm | 50nm | 10nm | (90%:10%) 30nm | 10nm | (50%:50%) 30nm | |
| E23 | HATCN 5nm | SpMA1 50nm | SpMA2 10nm | L1:INV-25:TEG2 (29%:59%:12%) 30nm | ST2 10nm | ST2:LiQ (50%:50%) 30nm | --- |
| E24 | HATCN 5nm | SpMA1 50nm | SpMA2 10nm | L1:INV-26:TEG2 (29%:59%:12%) 30nm | ST2 10nm | ST2:LiQ (50%:50%) 30nm | --- |
| E25 | HATCN 5nm | SpMA1 50nm | SpMA2 10nm | IC1:INV-27:TEG2 (44%:44%:12%) 30nm | ST2 10nm | ST2:LiQ (50%:50%) 30nm | --- |
| E26 | HATCN 5nm | SpMA1 50nm | SpMA2 10nm | IC1:INV-28:TEG2 (40%:48%:12%) 30nm | ST2 10nm | ST2:LiQ (50%:50%) 30nm | --- |
| E27 | HATCN 5nm | SpMA1 50nm | SpMA2 10nm | INV-29:TEG2 (83%:17%) 30nm | ST2 10nm | ST2:LiQ (50%:50%) 30nm | --- |

Tabelle 5: Daten der OLEDs

| Bsp. | U1000 (V) | SE1000 (cd/A) | LE1000 (lm/W) | EQE 1000 | CIE x/y bei 1000 cd/$m^2$ |
|---|---|---|---|---|---|
| E1 | 3.4 | 62 | 58 | 16.6% | 0.34/0.62 |
| E2 | 3.2 | 67 | 70 | 18.1% | 0.34/0.62 |
| E3 | 3.4 | 57 | 52 | 15.4% | 0.34/0.62 |
| E4 | 3.4 | 68 | 63 | 18.4% | 0.34/0.62 |
| E5 | 3.2 | 75 | 73 | 20.2% | 0.34/0.62 |
| E6 | 3.2 | 76 | 75 | 20.7% | 0.34/0.62 |
| E7 | 3.5 | 74 | 67 | 20.0% | 0.33/0.63 |
| E8 | 3.3 | 68 | 64 | 18.3% | 0.34/0.62 |
| E9 | 3.1 | 72 | 72 | 19.5% | 0.34/0.62 |
| E10 | 3.3 | 62 | 59 | 16.8% | 0.34/0.62 |
| E11 | 3.1 | 72 | 73 | 19.4% | 0.33/0.63 |
| E12 | 3.2 | 72 | 71 | 19.3% | 0.34/0.62 |
| E13 | 3.5 | 65 | 59 | 17.5% | 0.35/0.62 |
| E14 | 3.2 | 68 | 68 | 18.6% | 0.35/0.62 |
| E15 | 3.1 | 77 | 79 | 20.8% | 0.34/0.62 |
| E16 | 3.8 | 62 | 52 | 16.7% | 0.33/0.63 |
| E17 | 4.4 | 65 | 47 | 17.7% | 0.35/0.62 |
| E18 | 4.6 | 11.2 | 8.2 | 12.2% | 0.67/0.33 |
| E19 | 3.3 | 72 | 69 | 19.8% | 0.36/0.61 |
| E20 | 3.2 | 62 | 62 | 17.0% | 0.35/0.61 |
| E21 | 3.2 | 64 | 63 | 17.3% | 0.35/0.62 |
| E22 | 3.4 | 73 | 67 | 20.0% | 0.34/0.62 |
| E23 | 3.3 | 59 | 56 | 16.0% | 0.35/0.61 |
| E24 | 3.2 | 61 | 60 | 16.7% | 0.35/0.62 |
| E25 | 3.2 | 71 | 70 | 19.1% | 0.33/0.63 |

(fortgesetzt)

| Bsp. | U1000 (V) | SE1000 (cd/A) | LE1000 (lm/W) | EQE 1000 | CIE x/y bei 1000 cd/m$^2$ |
|---|---|---|---|---|---|
| E26 | 3.5 | 63 | 58 | 17.2% | 0.36/0.61 |
| E27 | 4.1 | 72 | 55 | 19.4% | 0.35/0.62 |

Tabelle 6: Strukturformeln der Materialien für die OLEDs

| | |
|---|---|
| 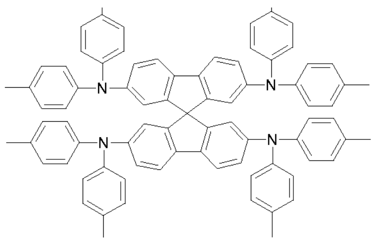 | 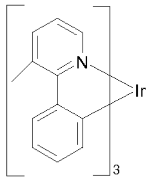 |
| SpA1 | TEG1 |
| 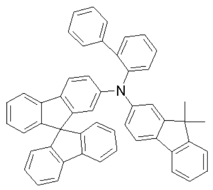 | 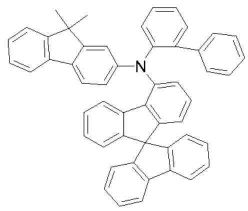 |
| SpMA1 | SpMA2 |
| 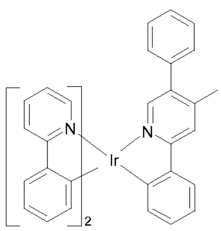 | 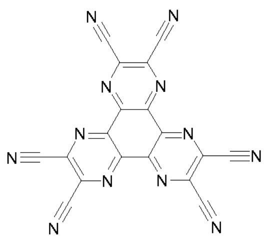 |
| TEG2 | HATCN |
| 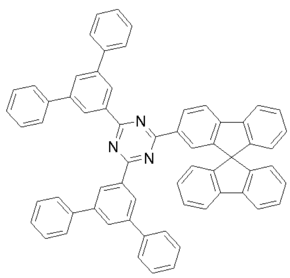 | 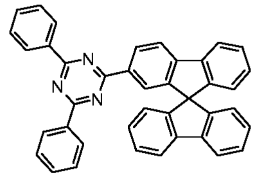 |
| ST2 | ST1 |
| 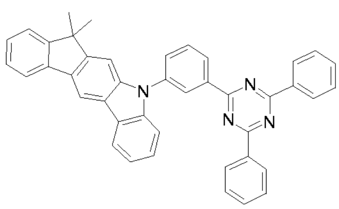 | 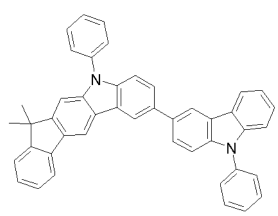 |
| IC1 | IC2 |

(fortgesetzt)

| | |
|---|---|
| 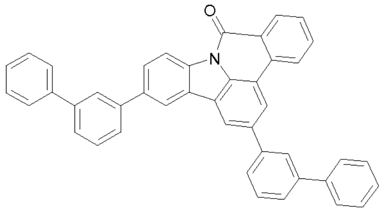 | 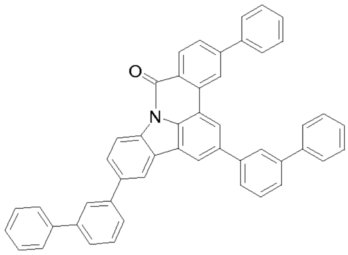 |
| L1 | L2 |
| 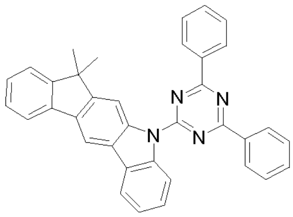 | 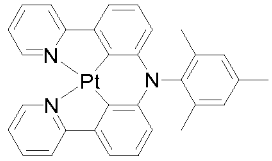 |
| IC3 | TER3 |
| 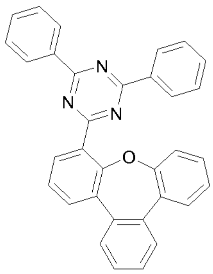 | 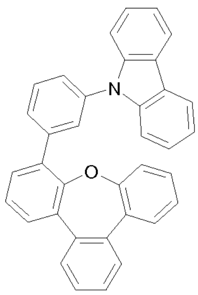 |
| INV-5 | INV-6 |
| 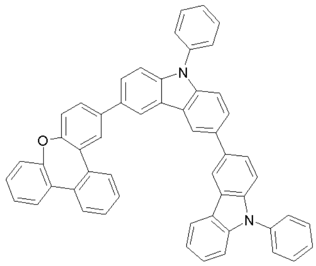 | 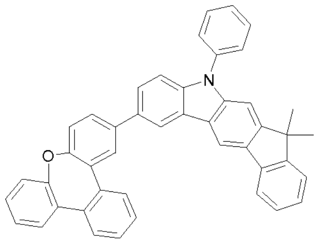 |
| INV-7 | INV-8 |
| 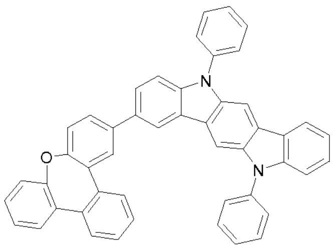 | 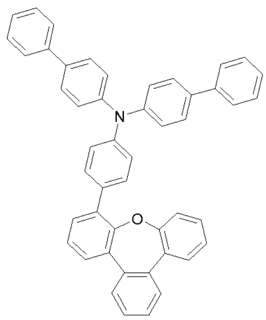 |
| INV-9 | INV-10 |

(fortgesetzt)

| | |
|---|---|
| 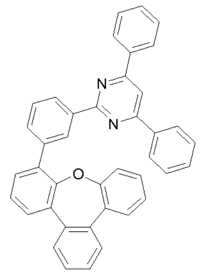 | 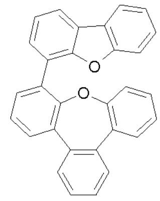 |
| INV-11 | INV-12 |
| 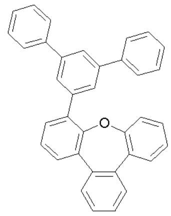 | 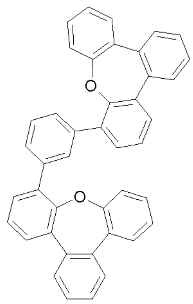 |
| INV-13 | INV-14 |
| 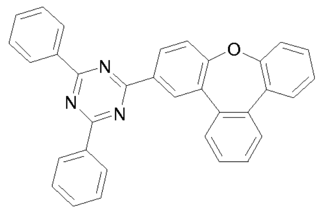 | 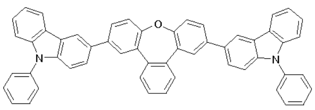 |
| INV-15 | INV-16 |
| 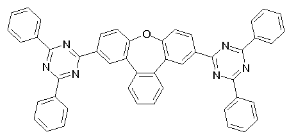 | 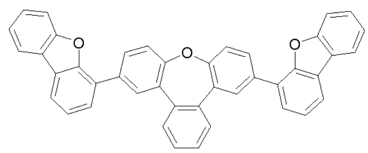 |
| INV-17 | INV-18 |
| 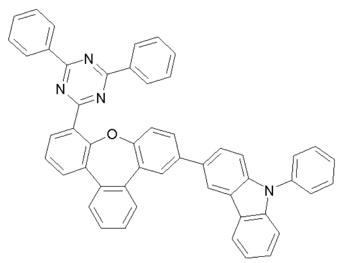 | 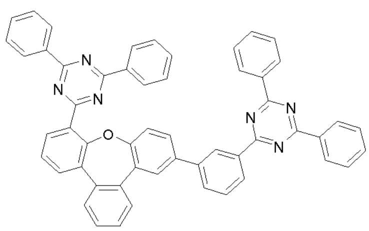 |
| INV-19 | INV-20 |

(fortgesetzt)

| | |
|---|---|
| 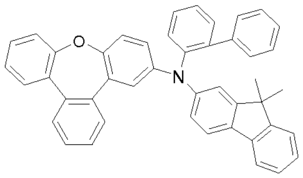 | 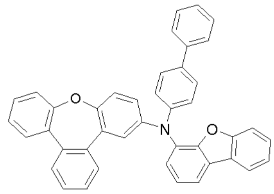 |
| INV-21 | INV-22 |
| 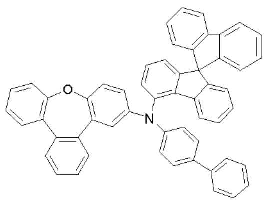 | 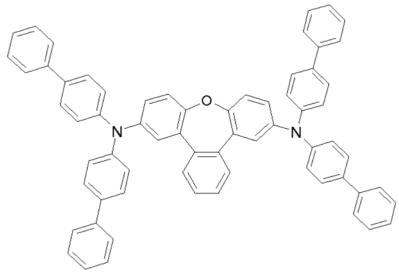 |
| INV-23 | INV-24 |
| 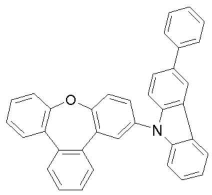 | 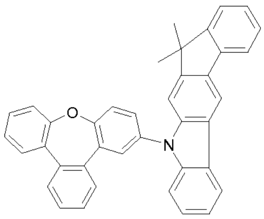 |
| INV-25 | INV-26 |
| 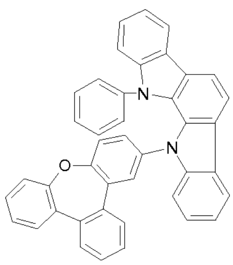 | 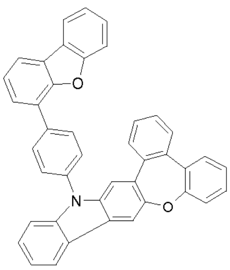 |
| INV-27 | INV-28 |
| 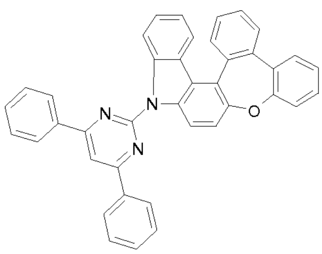 | |
| INV-29 | |

**Patentansprüche**

1. Elektronische Vorrichtung enthaltend wenigstens eine Verbindung der allgemeinen Formel (5) oder (7),

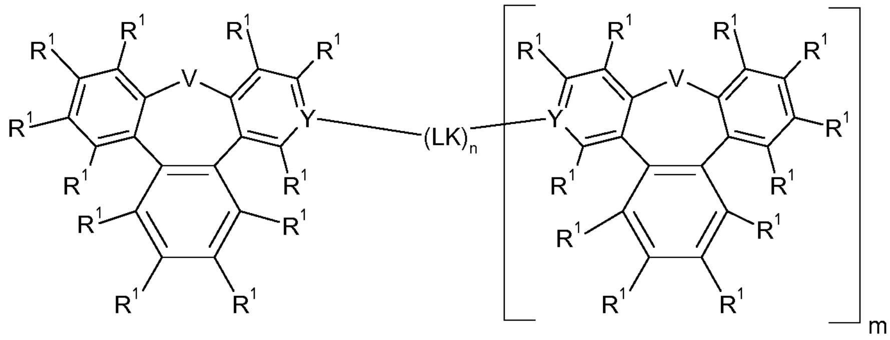

Formel (5)

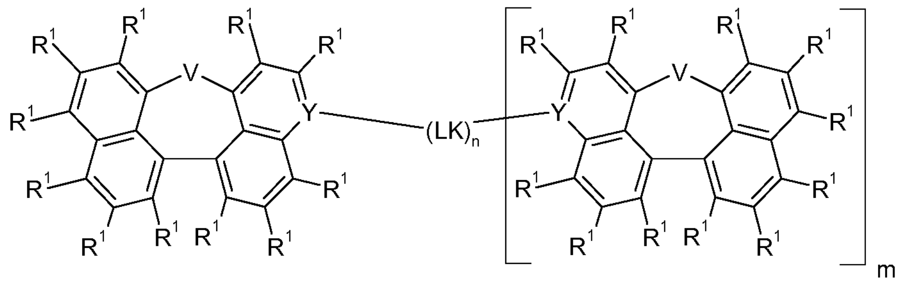

Formel (7),

wobei für die Symbole und Indizes gilt:

V ist entweder O oder S und ganz bevorzugt O;
X ist gleich oder verschieden bei jedem Auftreten N oder $CR^1$, bevorzugt $CR^1$;
m ist 0 (Monomer), 1 (Dimer) oder 2 (Trimer);
n ist 0 oder 1, wobei gilt:

n = 0 für m = 0 und
n = 1 für m = 1 oder für m = 2;

Y ist gleich ein $sp^2$-hybridisiertes Kohlenstoffatom für m ungleich 0 oder gleich X für m = 0;
LK ist für den Fall m=1 eine Einfachbindung oder ein bifunktioneller Linker; LK kann mit einem oder mehreren Resten $R^1$ substituiert sein, wobei die Reste $R^1$ bei jedem Auftreten gleich oder verschieden sein können;
ist für m = 2 ein trifunktioneller Linker, wobei der Linker mit einem oder mehreren Resten $R^1$ substituiert sein kann, wobei die Reste $R^1$ bei jedem Auftreten gleich oder verschieden sein können;
ist, wenn n = 0 ist, nicht vorhanden, so dass ein Monomer vorliegt;
$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkyl- alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches oder heteroaromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 40 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches oder heteroaromatisches Ringsystem bilden;

mit der Maßgabe, dass wenn m = 0 ist, wenigstens einer der Reste $R^1$ ungleich H ist.

**2.** Elektronische Vorrichtung gemäß Anspruch 1 enthaltend die Verbindungen der Formel (5) oder (7) in einer Emissionsschicht (EML), Elektronentransportschicht (ETL) oder in einer Lochblockierschicht (HBL), bevorzugt in einer EML oder ETL und besonders bevorzugt in einer EML.

**3.** Elektronische Vorrichtung gemäß Anspruch 1, **dadurch gekennzeichnet, dass** sie gewählt ist aus organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren, organischen Photorezeptoren, bevorzugt aus einer organische Elektrolumineszenzvorrichtung.

**4.** Elektronische Vorrichtung gemäß Anspruch 1 oder 3, **dadurch gekennzeichnet, dass** sie eine organische Elektrolumineszenzvorrichtung ist, die ausgewählt ist auch der Gruppe bestehend aus organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

**5.** Verfahren zur Herstellung einer elektronischen Vorrichtung gemäß einem oder mehreren der Ansprüche 1 bis 4, **dadurch gekennzeichnet, dass** mindestens eine organische Schicht durch Gasphasenabscheidung oder aus Lösung aufgebracht wird.

**6.** Elektronische Vorrichtung gemäß Anspruch 4, zur Verwendung in der Medizin zur Phototherapie, bevorzugt zur Verwendung zur Phototherapie der Haut, ganz bevorzugt zur Verwendung zur Behandlung oder Vorbeugung von Psoriasis, atopischer Dermatitis, Ikterus, Neugeborenenikterus, Vitiligo, Inflammation, Schwerzen und zur Verwendung zur Wundheilung.

**7.** Verwendung der Vorrichtung gemäß Anspruch 4 in der Kosmetik, bevorzugt zur Reduktion oder Vorbeugung von Hautalterung (Skin Ageing), Hautfalten, Krähenfüsse, Akne, Komedonen und Zellulithe.

**8.** Verwendung der Vorrichtung gemäß Anspruch 4 in Displays oder zur Beleuchtung.

**9.** Zusammensetzung enthaltend wenigstens eine Verbindung der allgemeinen Formel (5) oder (7),

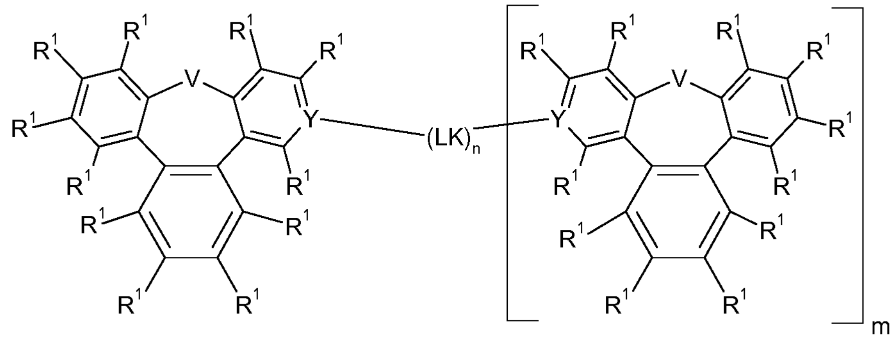

Formel (5)

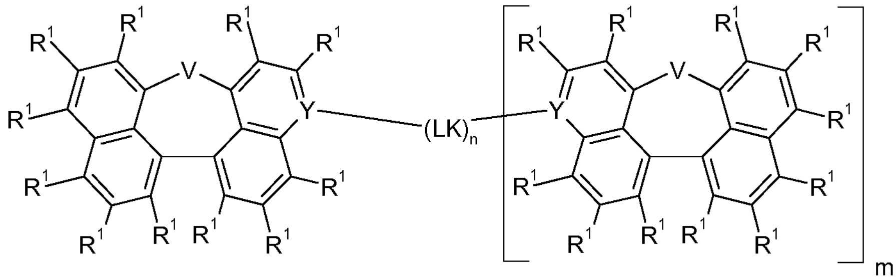

Formel (7),

wobei für die Symbole und Indizes gilt:

V ist entweder O oder S und ganz bevorzugt O;
X ist gleich oder verschieden bei jedem Auftreten N oder $CR^1$, bevorzugt $CR^1$;
m ist 0 (Monomer), 1 (Dimer) oder 2 (Trimer);
n ist 0 oder 1, wobei gilt:

n = 0 für m = 0 und
n = 1 für m = 1 oder für m = 2;

Y ist gleich ein $sp^2$-hybridisiertes Kohlenstoffatom für m ungleich 0 oder gleich X für m = 0;
LK ist für den Fall m=1 eine Einfachbindung oder ein bifunktioneller Linker; LK kann mit einem oder mehreren Resten $R^1$ substituiert sein, wobei die Reste $R^1$ bei jedem Auftreten gleich oder verschieden sein können;
ist für m = 2 ein trifunktioneller Linker, wobei der Linker mit einem oder mehreren Resten $R^1$ substituiert sein kann, wobei die Reste $R^1$ bei jedem Auftreten gleich oder verschieden sein können;
ist, wenn n = 0 ist, nicht vorhanden, so dass ein Monomer vorliegt;
$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkyl- alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches oder heteroaromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 40 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches oder heteroaromatisches Ringsystem bilden;

mit der Maßgabe, dass wenn m = 0 ist, wenigstens einer der Reste $R^1$ ungleich H ist,
sowie wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Loch-blockiermaterialien, n-Dotanden oder p-Dotanden.

**10.** Zusammensetzung gemäß Anspruch 9, dadurch charakterisiert, dass die zusätzliche Verbindung ein phosphoreszierender Emitter ist.

**11.** Zusammensetzung gemäß Anspruch 9 oder 10, dadurch charakterisiert, dass die zusätzliche Verbindung ein Host- oder Matrixmaterial ist.

**12.** Zusammensetzung gemäß einem oder mehreren der Ansprüche 9 bis 11, dadurch charakterisiert, dass die zusätzliche Verbindung eine Bandlücke (band gap) von 2.5 eV oder mehr, bevorzugt 3.0 eV oder mehr, ganz bevorzugt von 3.5 eV oder mehr aufweist.

**13.** Verbindung der allgemeinen Formel (5)

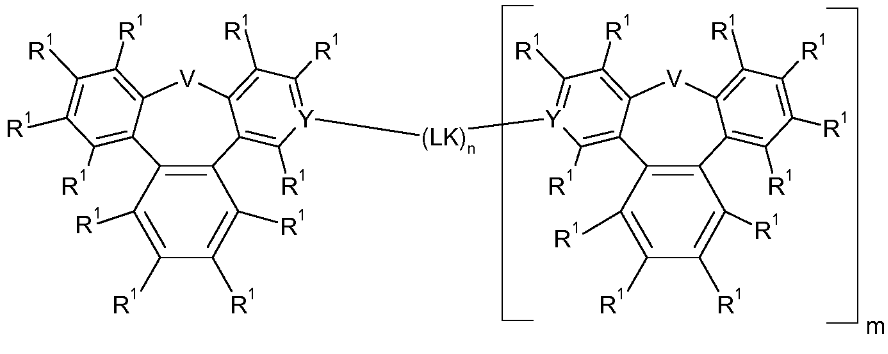

Formel (5),

wobei für die Symbole und Indizes gilt:

V ist O;
m ist 0 (Monomer), 1 (Dimer) oder 2 (Trimer);
n ist 0 oder 1, wobei gilt:

n = 0 für m = 0 und
n = 1 für m = 1 oder für m = 2;

Y ist gleich ein $sp^2$-hybridisiertes Kohlenstoffatom für m ungleich 0 oder gleich X für m = 0;
X ist gleich oder verschieden bei jedem Auftreten $CR^1$,
LK ist für den Fall m=1 eine Einfachbindung oder ein bifunktioneller Linker; LK kann mit einem oder mehreren Resten $R^1$ substituiert sein, wobei die Reste $R^1$ bei jedem Auftreten gleich oder verschieden sein können; ist für m = 2 ein trifunktioneller Linker, wobei der Linker mit einem oder mehreren Resten $R^1$ substituiert sein kann, wobei die Reste $R^1$ bei jedem Auftreten gleich oder verschieden sein können;
ist, wenn n = 0 ist, nicht vorhanden, so dass ein Monomer vorliegt;
$R^1$ ist gleich oder verschieden bei jedem Auftreten H, D, F, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkoxy-gruppe mit 1 bis 40 C-Atomen oder eine geradkettige Alkenyl- oder Alkinylgruppe mit 2 bis 40 C-Atomen
oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O oder $CONR^2$ ersetzt sein können und wobei ein oder mehrere H-Atome durch D, F, I, CN oder $NO_2$ ersetzt sein können,
oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen; dabei können zwei oder mehrere benachbarte Reste $R^1$ miteinander ein mono- oder polycyclisches, aliphatisches oder heteroaromatisches Ringsystem bilden, das mit einem oder mehreren Resten $R^2$ substituiert sein kann;
$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkyl- alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches oder heteroaromatisches Ringsystem bilden;
$R^3$ ist gleich oder verschieden bei jedem Auftreten H, D, F oder ein aliphatischer, aromatischer und/oder heteroaromatischer Kohlenwasserstoffrest mit 1 bis 40 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches oder heteroaromatisches Ringsystem bilden;

mit der Maßgabe, dass wenn m = 0 ist, wenigstens einer der Reste $R^1$ ungleich H ist

**14.** Verbindung gemäß Anspruch 13, **dadurch gekennzeichnet, dass** sie die folgende Formel (10a), (10b), (10c) oder (10d) aufweist

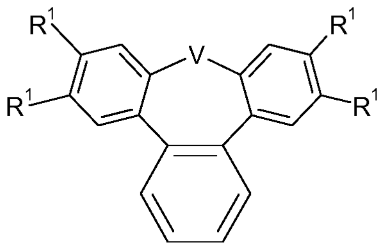

Formel (10a)

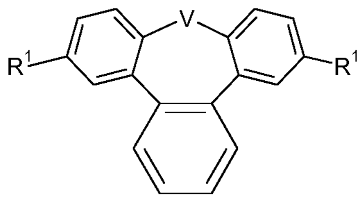

Formel (10b)

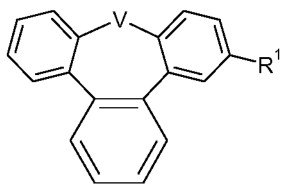

Formel (10c)

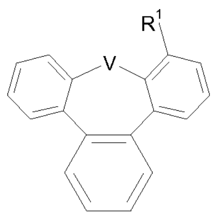

Formel (10d).

**15.** Verbindung der allgemeinen Formel (7)

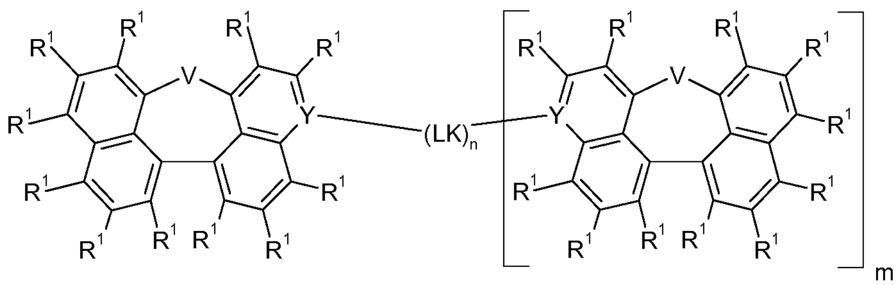

Formel (7),

wobei für die Symbole und Indizes gilt:

V ist O;

X ist gleich oder verschieden bei jedem Auftreten $CR^1$;
m ist 0 (Monomer), 1 (Dimer) oder 2 (Trimer);
n ist 0 oder 1, wobei gilt:

n = 0 für m = 0 und
n = 1 für m = 1 oder für m = 2;

Y ist gleich ein $sp^2$-hybridisiertes Kohlenstoffatom für m ungleich 0 oder gleich X für m = 0;
LK ist für den Fall m=1 eine Einfachbindung oder ein bifunktioneller Linker; LK kann mit einem oder mehreren Resten $R^1$ substituiert sein, wobei die Reste $R^1$ bei jedem Auftreten gleich oder verschieden sein können;
ist für m = 2 ein trifunktioneller Linker, wobei der Linker mit einem oder mehreren Resten $R^1$ substituiert sein kann, wobei die Reste $R^1$ bei jedem Auftreten gleich oder verschieden sein können;
ist, wenn n = 0 ist, nicht vorhanden, so dass ein Monomer vorliegt;
$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können,
oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q; dabei können zwei oder mehrere benachbarte Reste $R^1$ miteinander ein mono- oder polycyclisches, aliphatisches oder aromatisches oder heteroaromatisches Ringsystem bilden, das mit einem oder mehreren Resten $R^2$ substituiert sein kann;
$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkyl- alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, $C\equiv C$, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches oder heteroaromatisches Ringsystem bilden;
$R^3$ ist gleich oder verschieden bei jedem Auftreten H, D, F oder ein aliphatischer, aromatischer und/oder heteroaromatischer Kohlenwasserstoffrest mit 1 bis 40 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches oder heteroaromatisches Ringsystem bilden;

mit der Maßgabe, dass wenn m = 0 ist, wenigstens einer der Reste $R^1$ ungleich H ist.

**16.** Verbindung gemäß Anspruch 15, **dadurch gekennzeichnet, dass** sie die folgende Formel (14), (15), (16), (17), (18) oder (19) aufweist

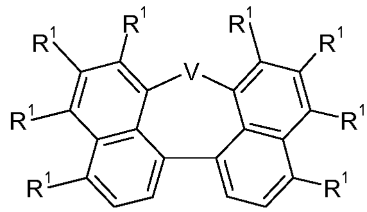

Formel (14)

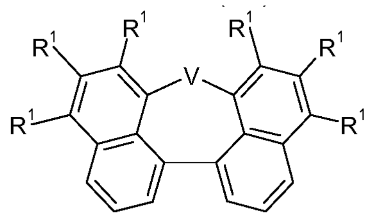

Formel (15)

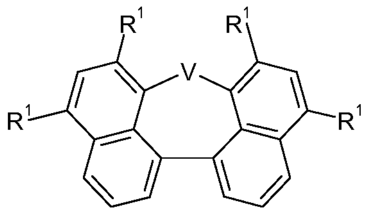

Formel (16)

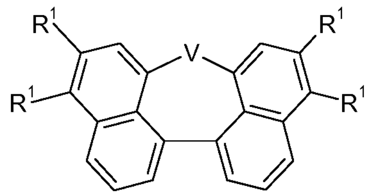

Formel (17)

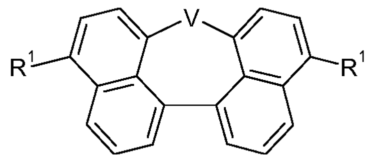

Formel (18)

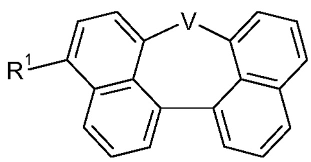

Formel (19).

**17.** Verbindungen gemäß einem der Ansprüche 13 bis 16, ausgewählt aus

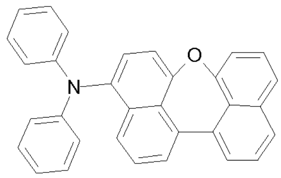

Formel (A-1)

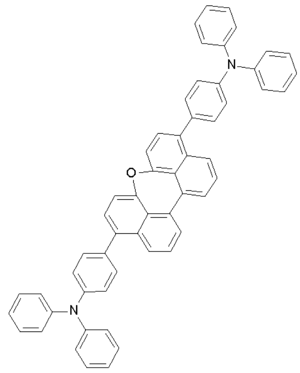

Formel (A-2)

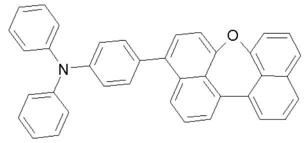

Formel (A-3)

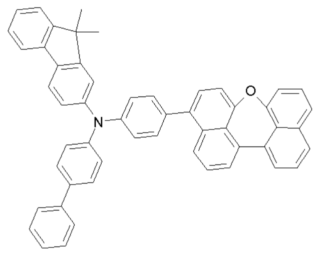

Formel (A-4)

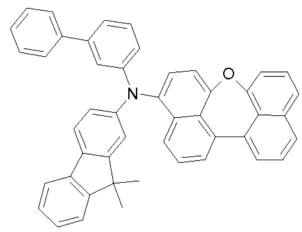

Formel (A-5)

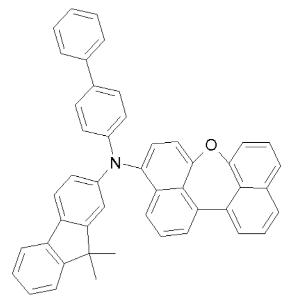

Formel (A-6)

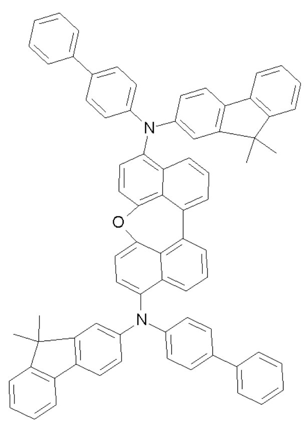

Formel (A-7)

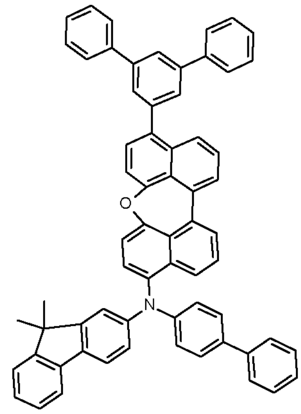

Formel (A-8)

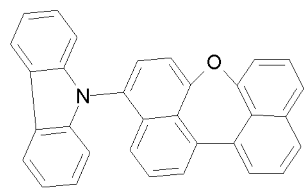

Formel (A-9)

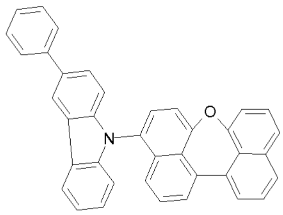

Formel (A-10)

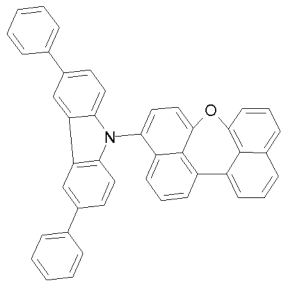

Formel (A-11)

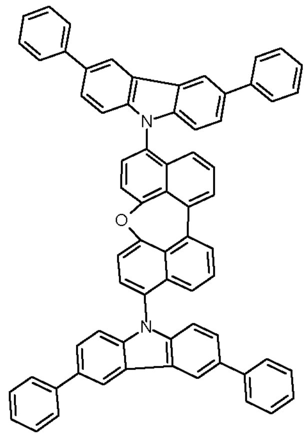

Formel (A-12)

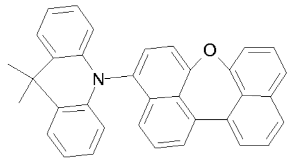

Formel (A-13)

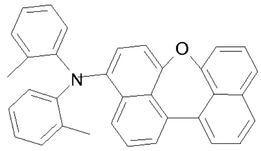

Formel (A-14)

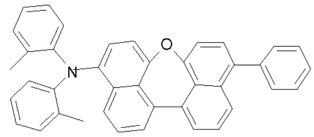

Formel (A-15)

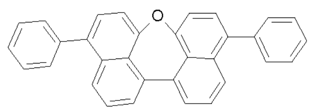

Formel (A-17)

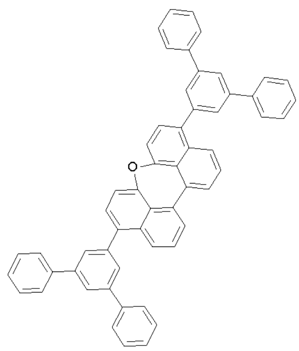

Formel (A-17)

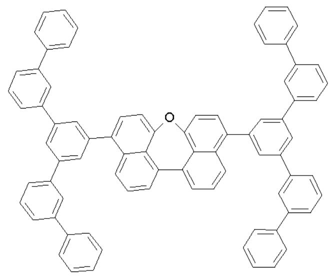

Formel (A-18)

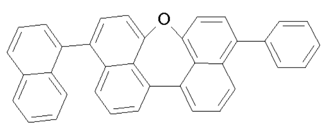

Formel (A-19)

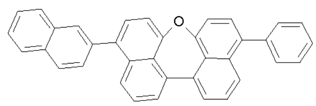

Formel (A-20)

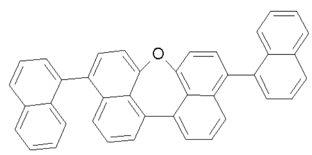

Formel (A-21)

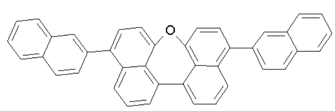

Formel (A-22)

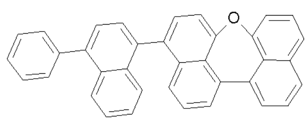

Formel (A-23)

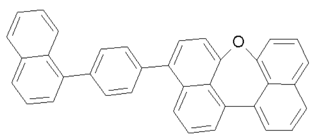

Formel (A-24)

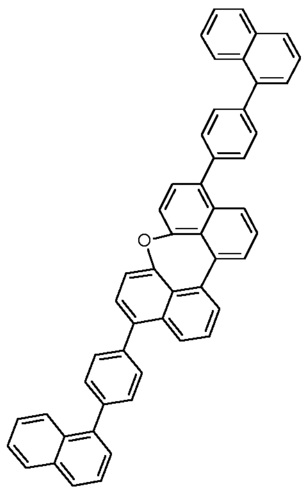

Formel (A-25)

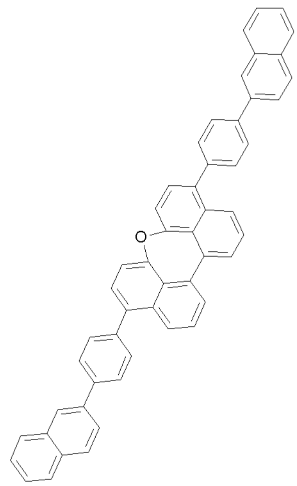

Formel (A-26)

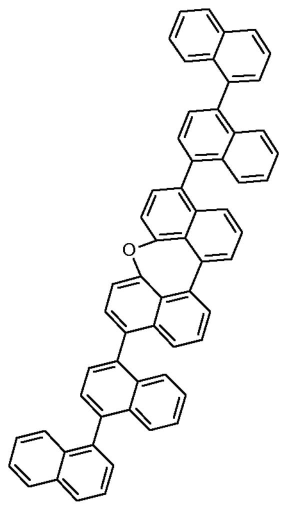

Formel (A-27)

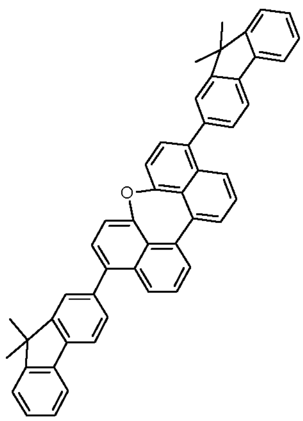

Formel (A-28)

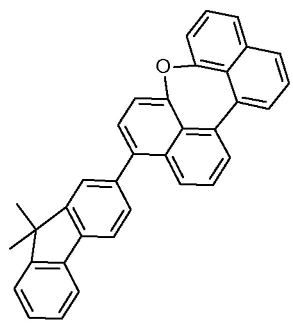

Formel (A-29)

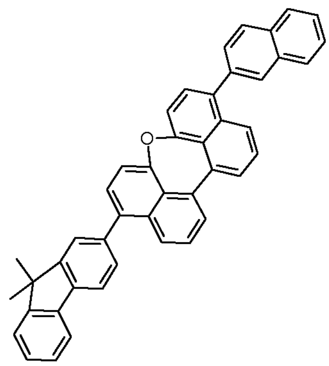

Formel (A-30)

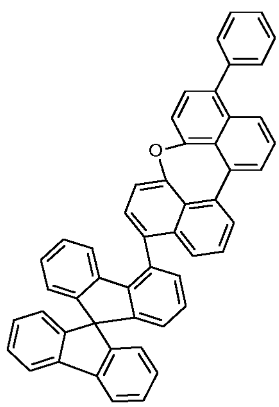

Formel (A-31)

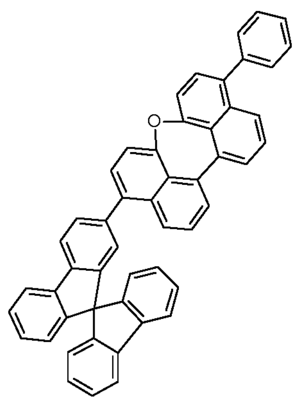

Formel (A-32)

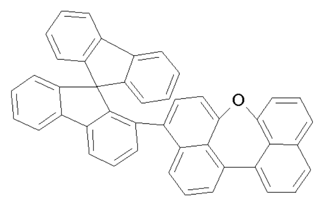

Formel (A-33)

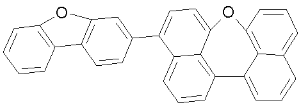
Formel (A-34)

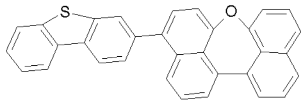
Formel (A-36)

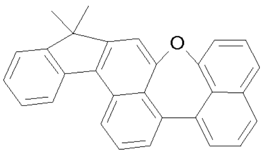
Formel (A-40)

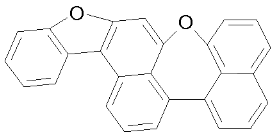
Formel (A-41)

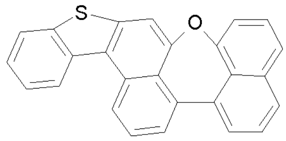
Formel (A-42)

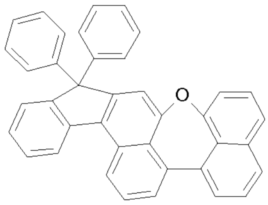
Formel (A-43)

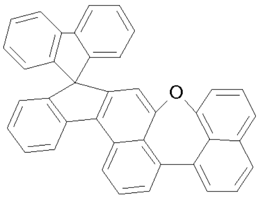
Formel (A-44)

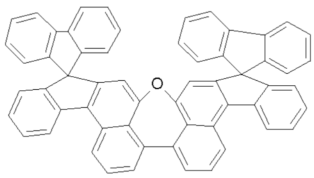
Formel (A-45)

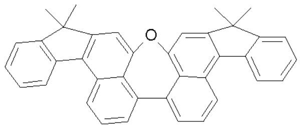
Formel (A-46)

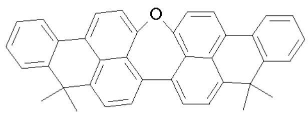
Formel (A-49)

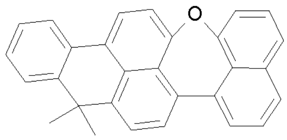
Formel (A-50)

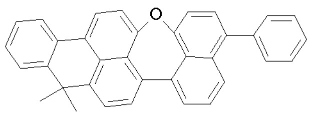
Formel (A-51)

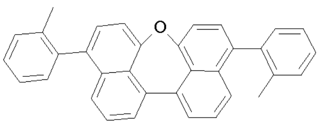
Formel (A-52)

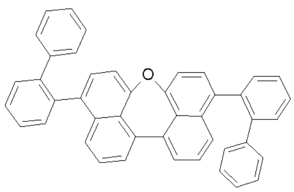
Formel (A-53)

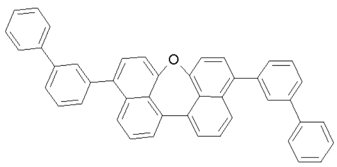
Formel (A-54)

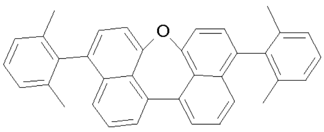
Formel (A-55)

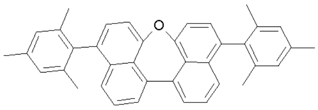
Formel (A-56)

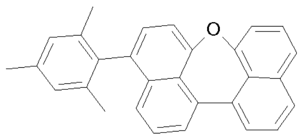
Formel (A-57)

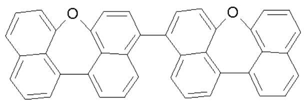

Formel (A-58)

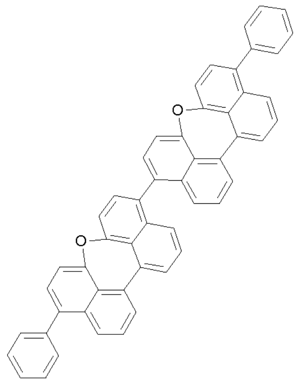

Formel (A-59)

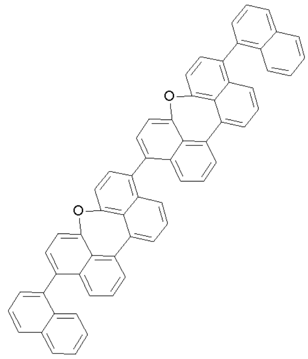

Formel (A-60)

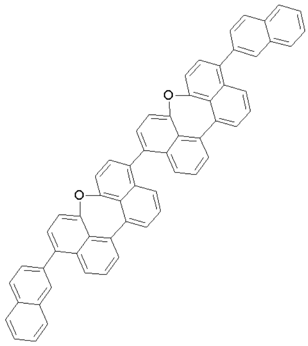

Formel (A-61)

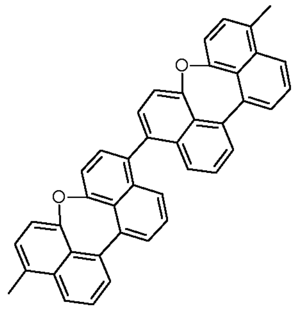

Formel (A-62)

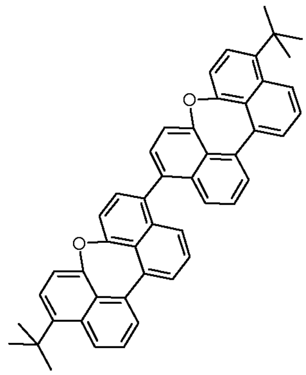

Formel (A-63)

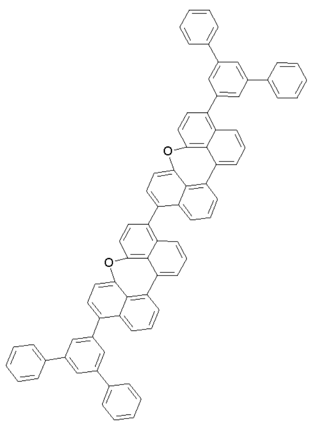

Formel (A-64)

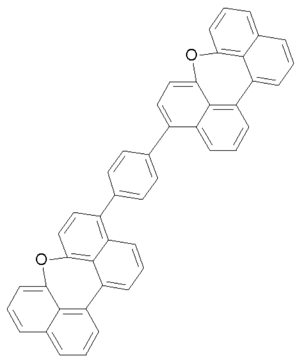

Formel (A-65)

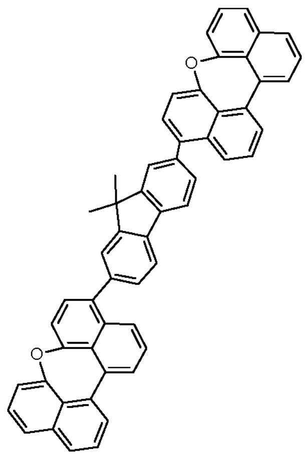

Formel (A-66)

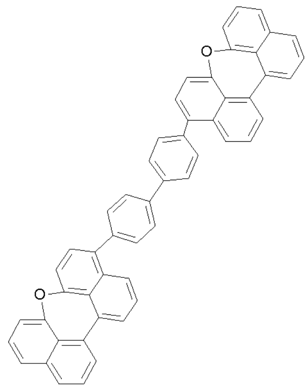

Formel (A-67)

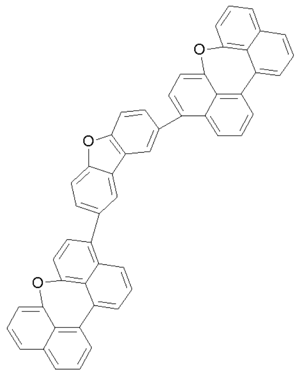

Formel (A-68)

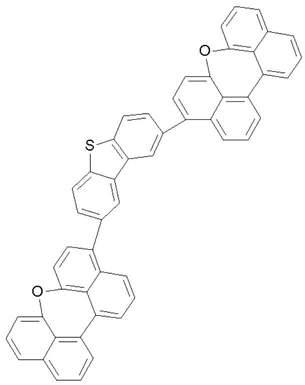

Formel (A-69)

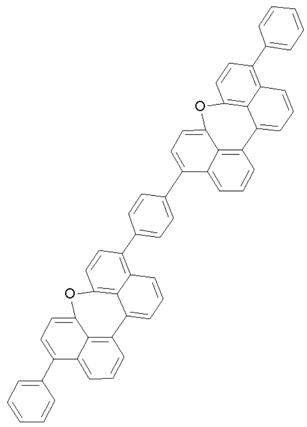

Formel (A-69a)

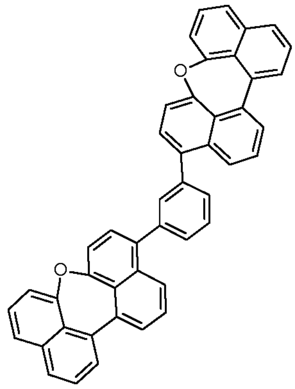

Formel (A-69b)

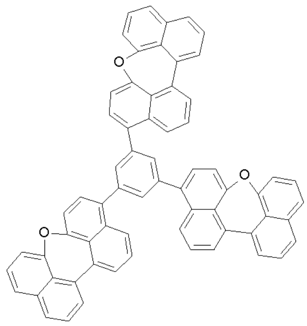

Formel (A-69c)

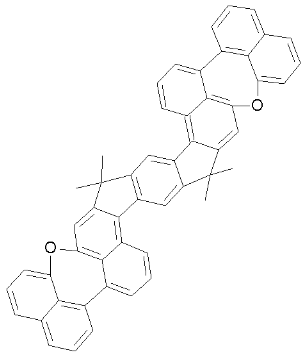

Formel (A-69d)

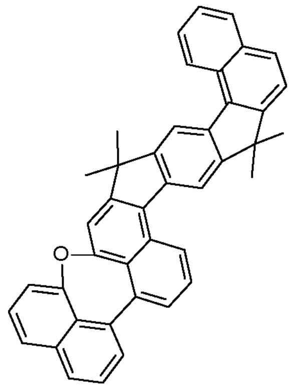

Formel (A-69e)

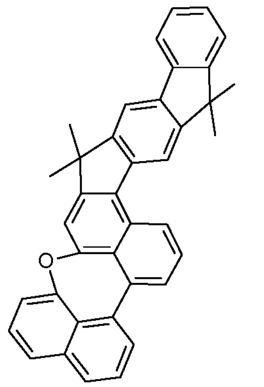

Formel (A-69f)

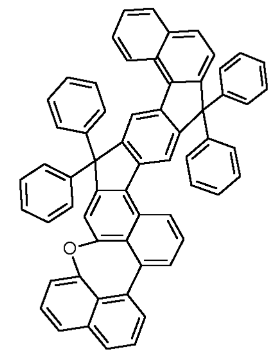

Formel (A-69g)

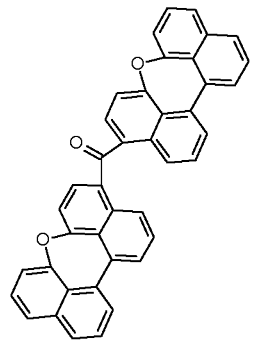

Formel (A-69h)

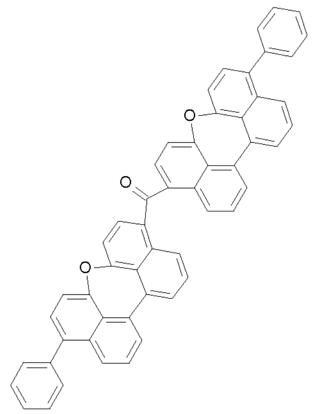

Formel (A-69i)

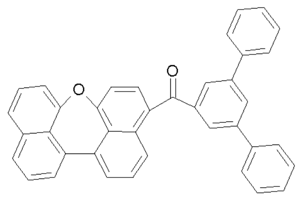

Formel (A-69j)

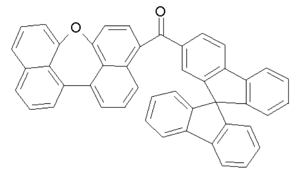

Formel (A-69k)

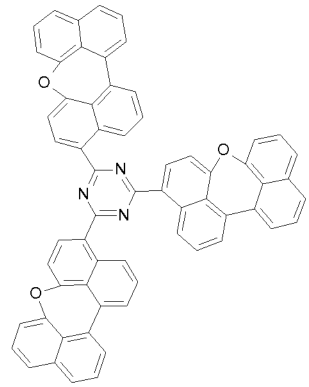

Formel (A-69l)

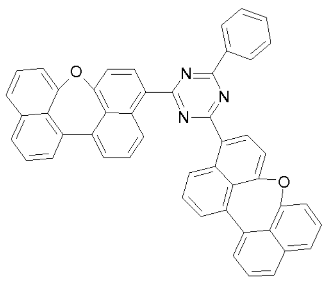

Formel (A-69m)

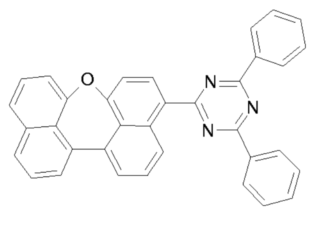

Formel (A-69n)

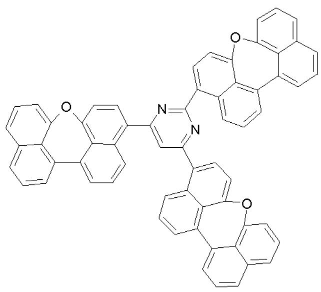

Formel (A-69o)

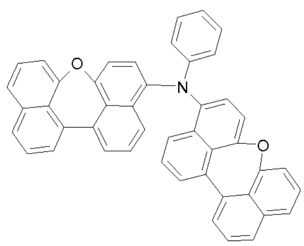

Formel (A-69q)

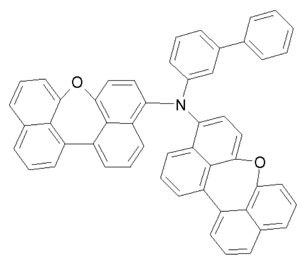

Formel (A-69r)

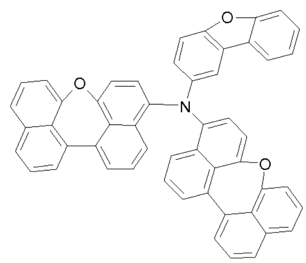

Formel (A-69s)

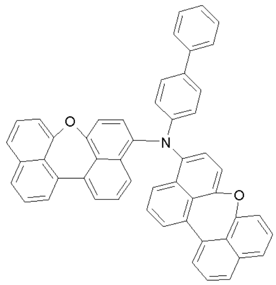
Formel (A-69t)

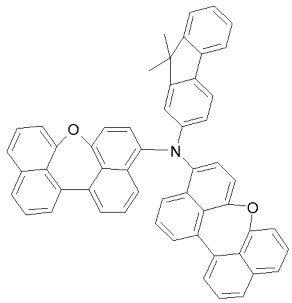
Formel (A-69u)

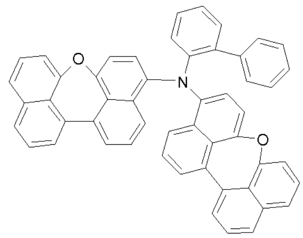
Formel (A-70)

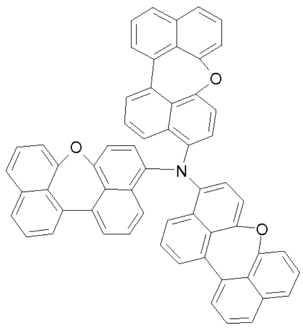
Formel (A-71)

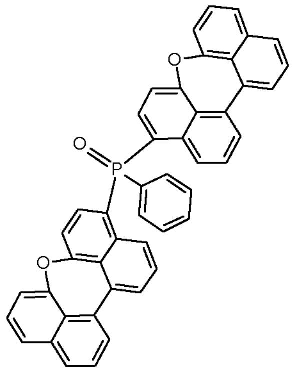
Formel (A-72)

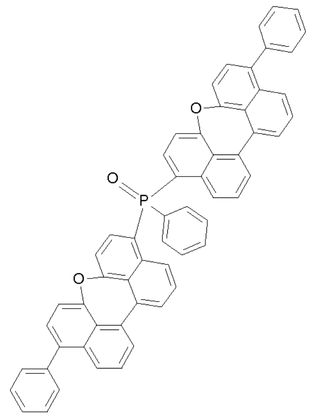
Formel (A-73)

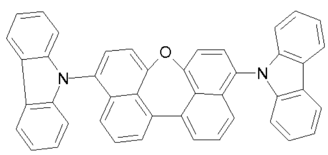
Formel (A-74)

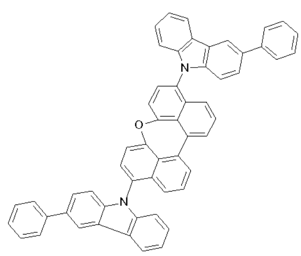
Formel (A-75)

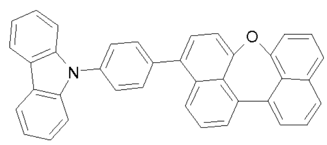
Formel (A-76)

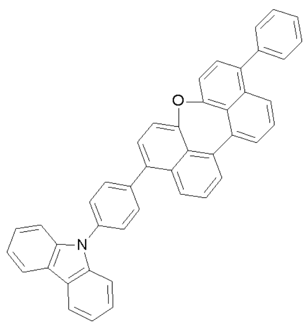
Formel (A-77)

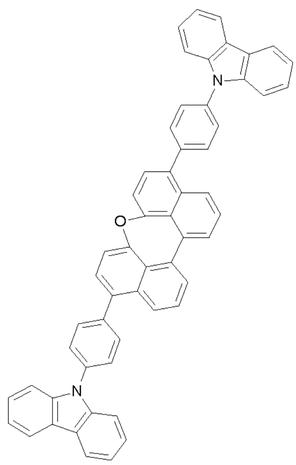
Formel (A-78)

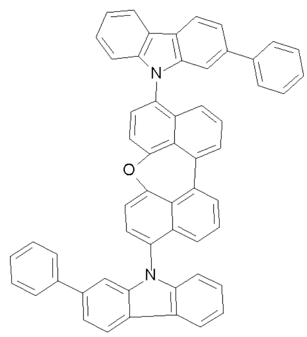
Formel (A-79)

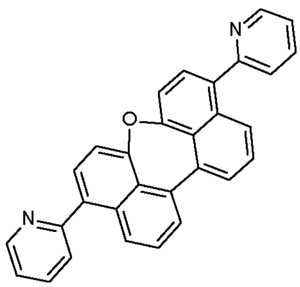
Formel (A-80)

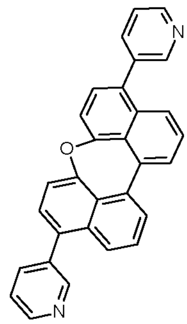
Formel (A-81)

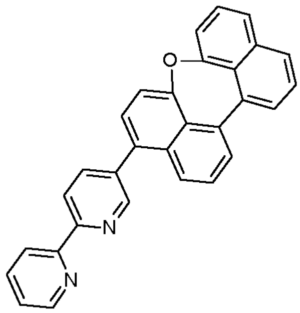
Formel (A-82)

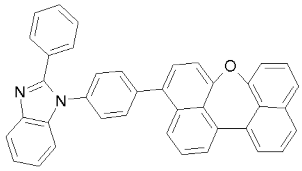
Formel (A-83)

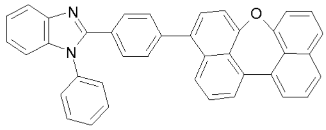
Formel (A-84)

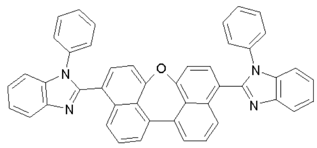
Formel (A-85)

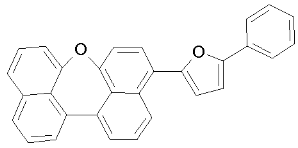
Formel (A-86)

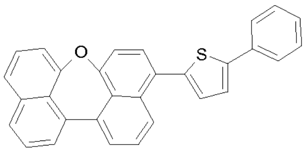
Formel (A-87)

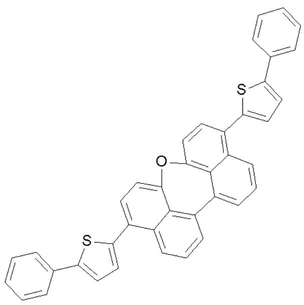
Formel (A-88)

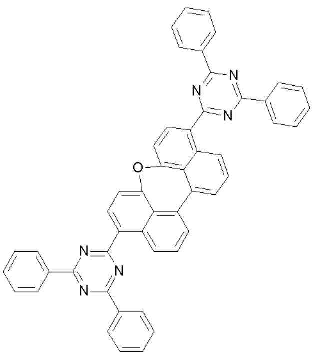
Formel (A-89)

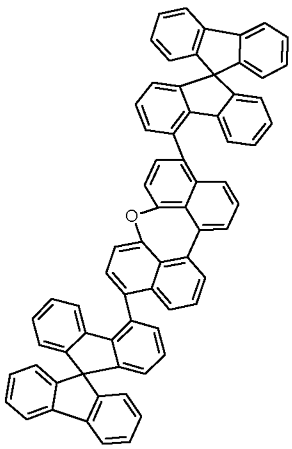
Formel (A-90)

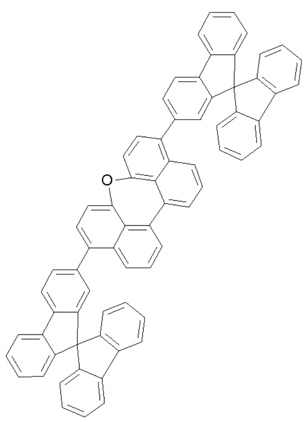
Formel (A-91)

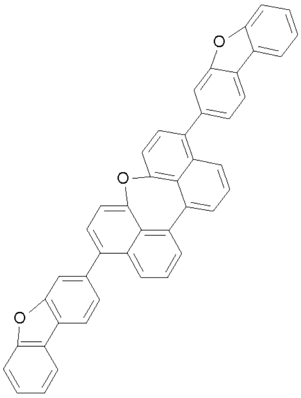

Formel (A-92)

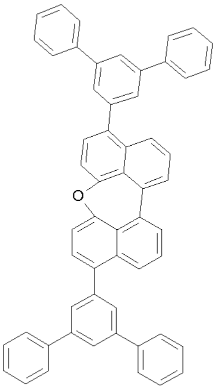

Formel (A-93)

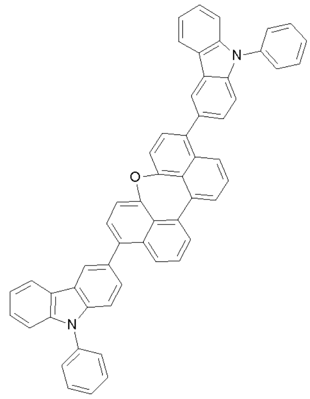

Formel (A-94)

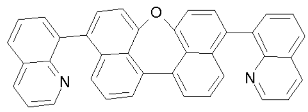

Formel (A-95)

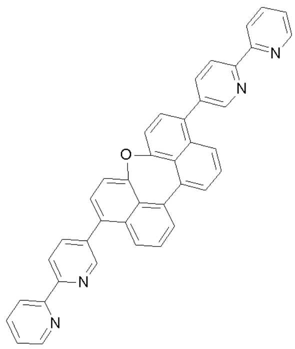

Formel (A-96)

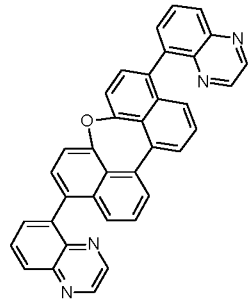

Formel (A-97)

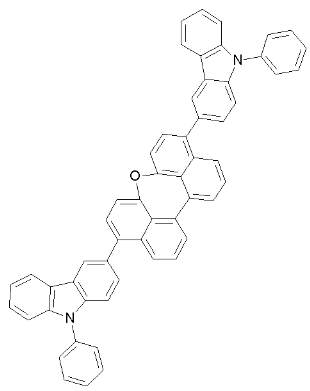

Formel (A-98)

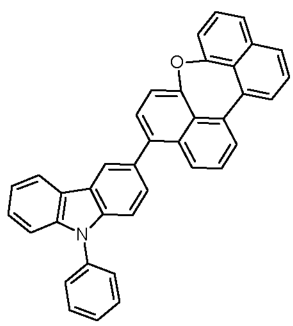

Formel (A-99)

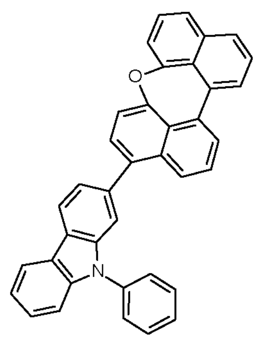

Formel (A-100)

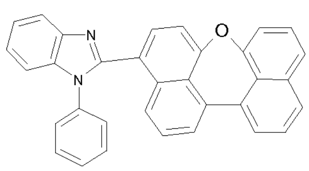

Formel (A-101)

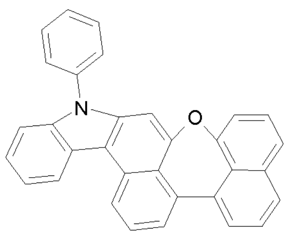

Formel (A-102)

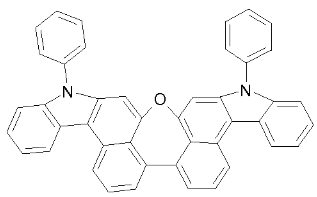

Formel (A-103)

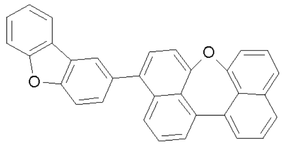

Formel (A-104)

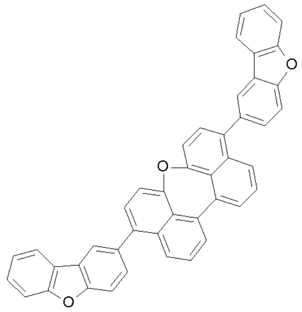

Formel (A-105)

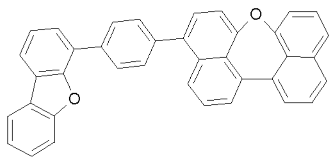

Formel (A-106)

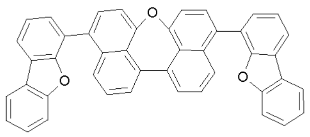

Formel (A-107)

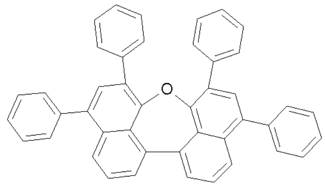

Formel (A-108)

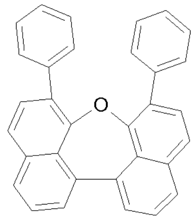

Formel (A-109)

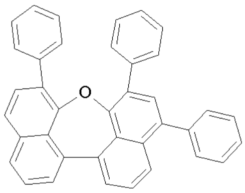

Formel (A-110)

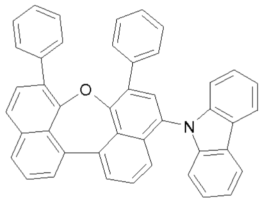

Formel (A-111)

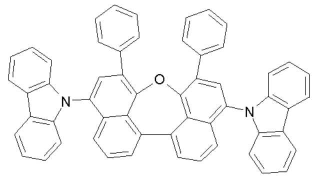

Formel (A-112)

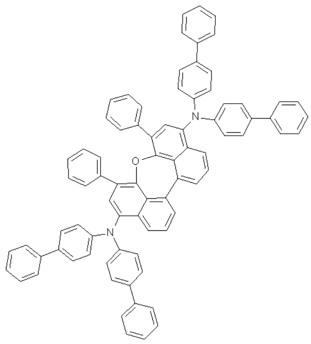

Formel (A-113)

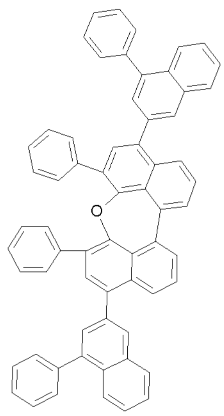

Formel (A-114)

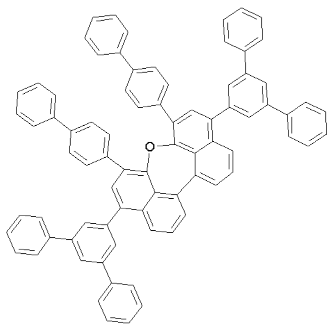

Formel (A-115)

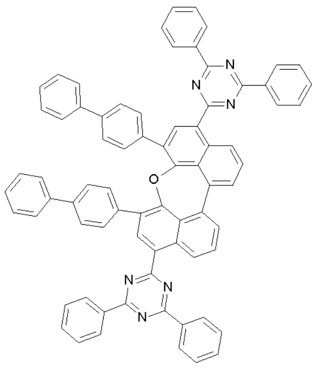

Formel (A-116)

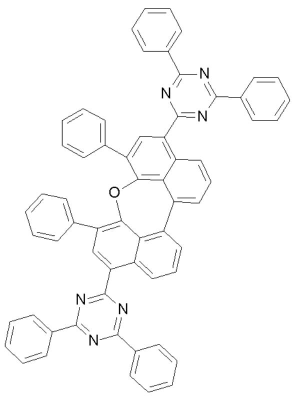

Formel (A-117)

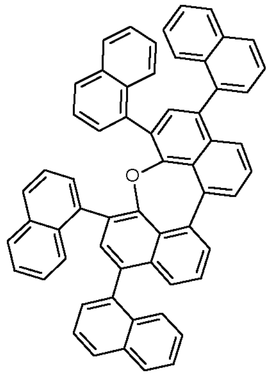

Formel (A-118)

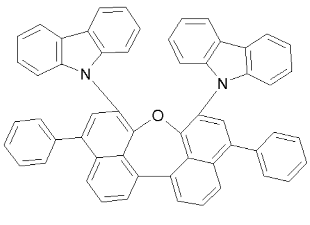

Formel (A-119)

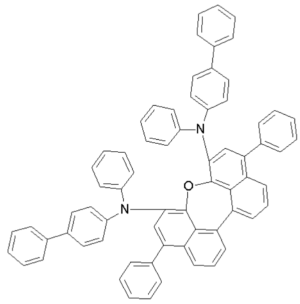

Formel (A-120)

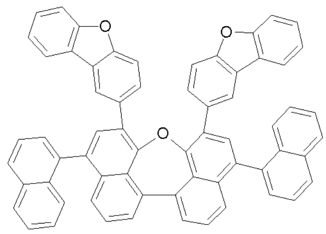

Formel (A-121)

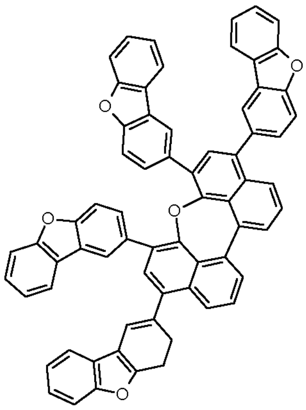

Formel (A-122)

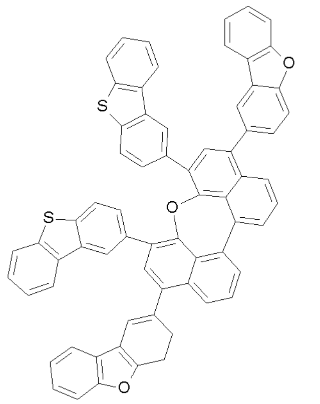

Formel (A-123)

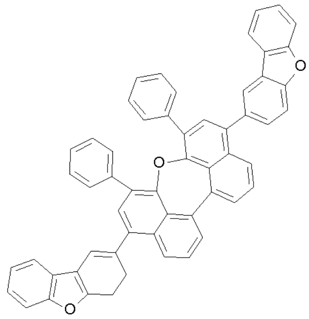

Formel (A-124)

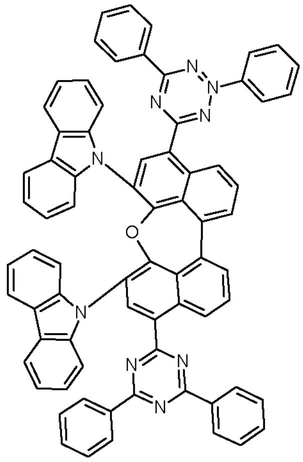
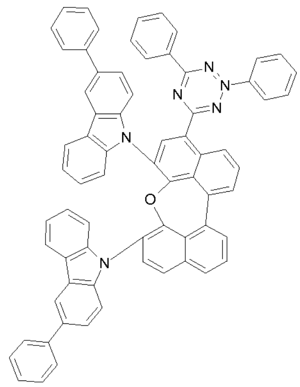
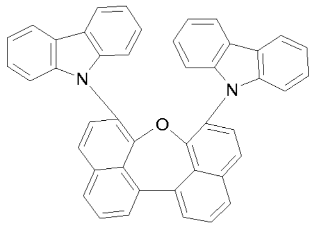

Formel (A-125) Formel (A-126) Formel (A-127)

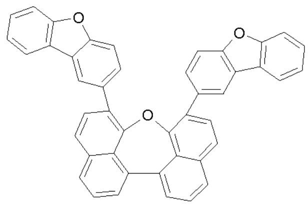
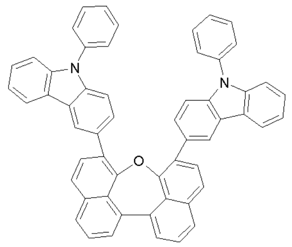
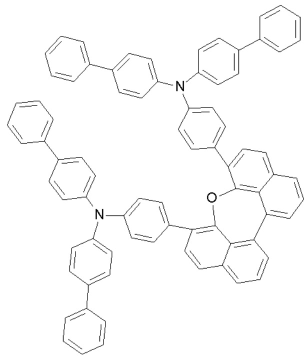

Formel (A-128) Formel (A-129) Formel (A-130)

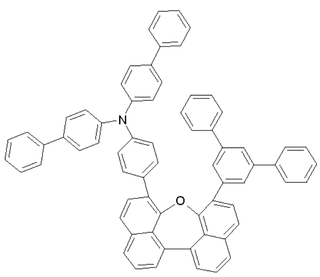
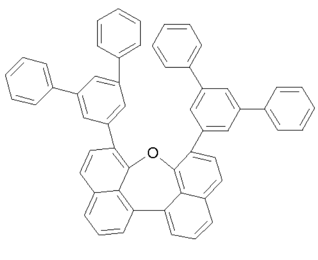
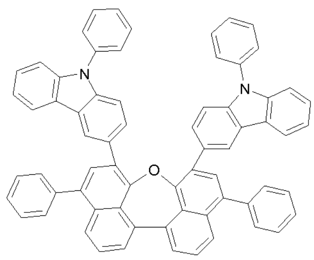

Formel (A-131) Formel (A-132) Formel (A-133)

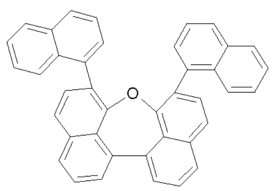
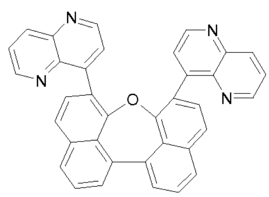
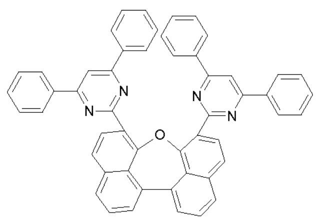

Formel (A-134) Formel (A-135) Formel (A-136)

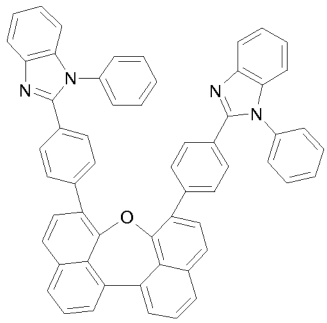

Formel (A-137)

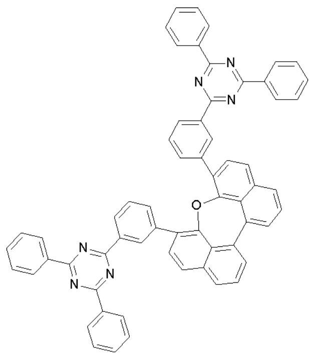

Formel (A-138)

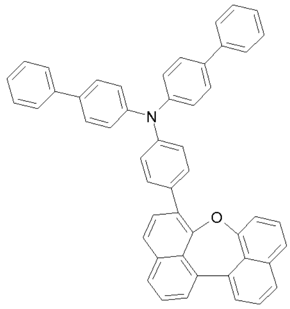

Formel (A-139)

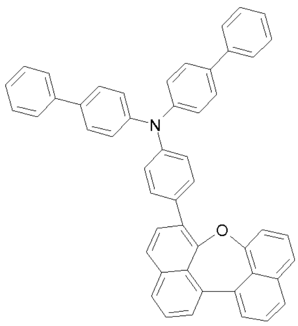

Formel (A-140)

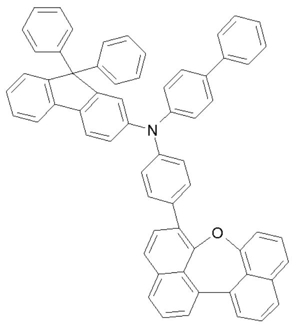

Formel (A-141)

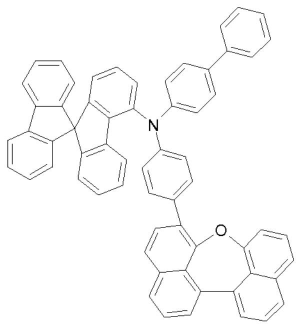

Formel (A-142)

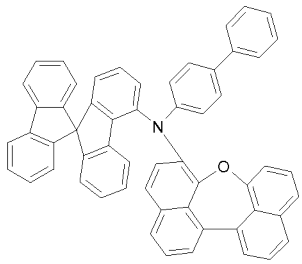

Formel (A-143)

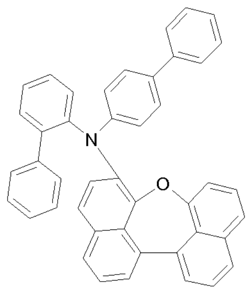

Formel (A-144)

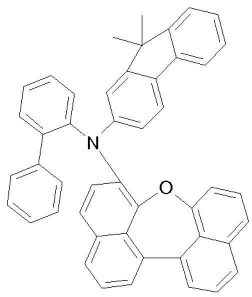

Formel (A-145)

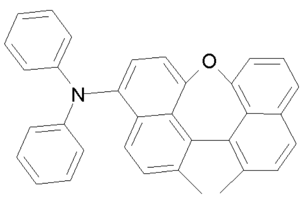

Formel (A-146)

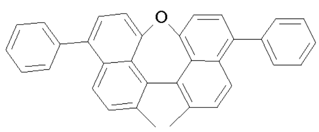

Formel (A-147)

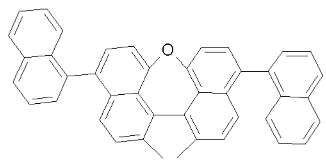

Formel (A-148)

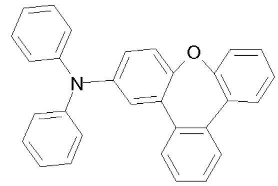
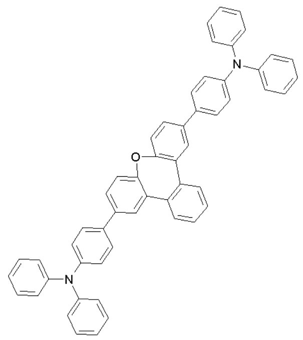
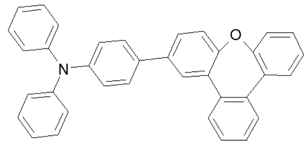

Formel (A-149) Formel (A-149a) Formel (A-149b)

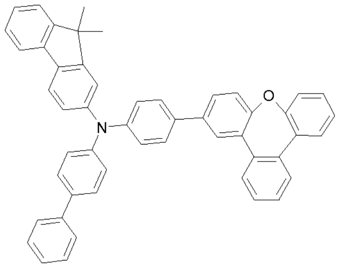
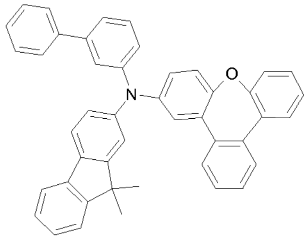
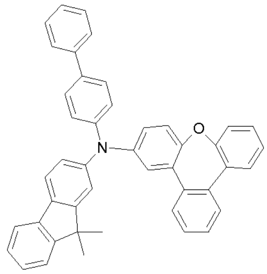

Formel (A-150) Formel (A-151) Formel (A-152)

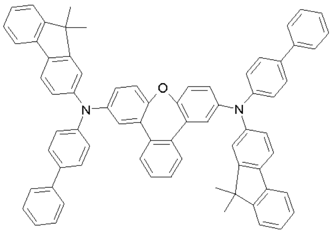
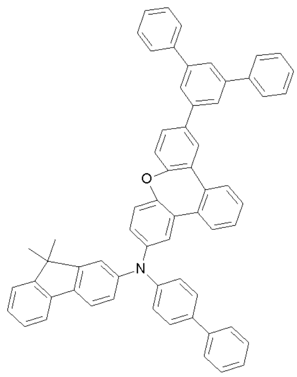
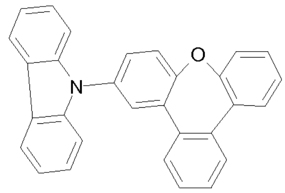

Formel (A-153) Formel (A-154) Formel (A-155)

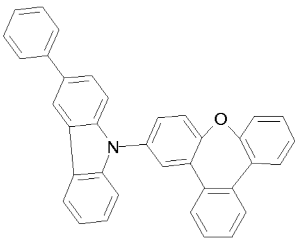
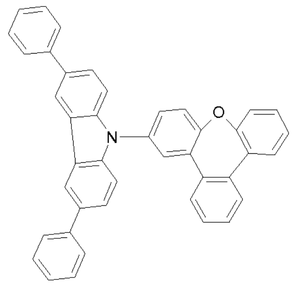
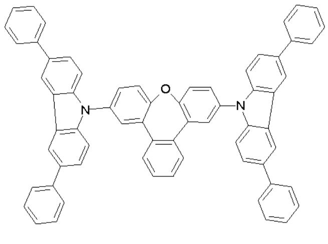

Formel (A-156) Formel (A-157) Formel (A-158)

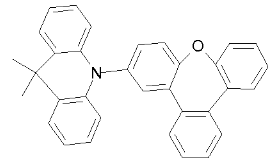

Formel (A-159)

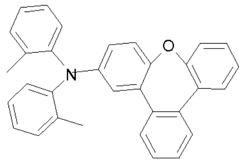

Formel (A-160)

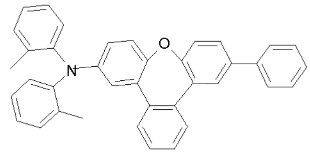

Formel (A-161)

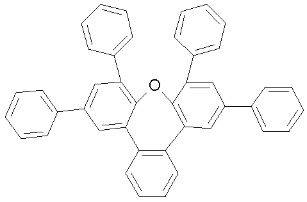

Formel (A-162)

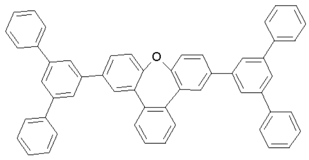

Formel (A-163)

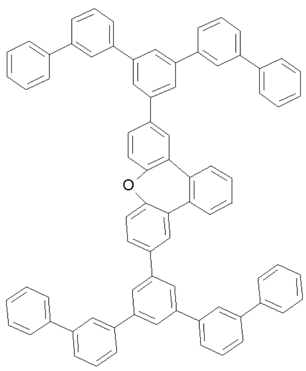

Formel (A-164)

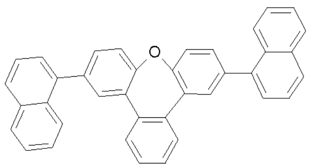

Formel (A-165)

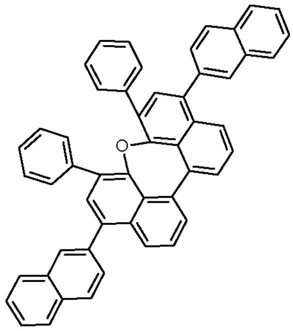

Formel (A-166)

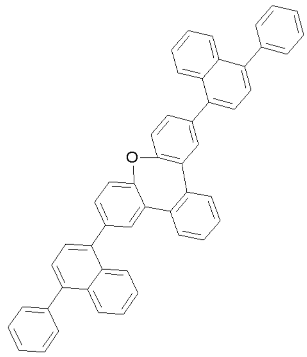

Formel (A-167)

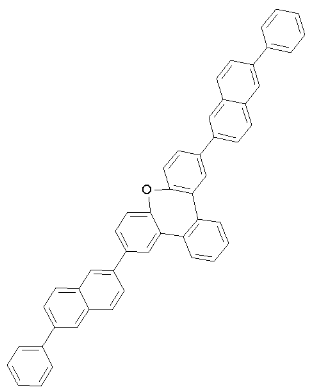

Formel (A-168)

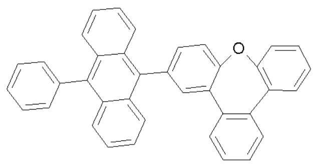

Formel (A-169)

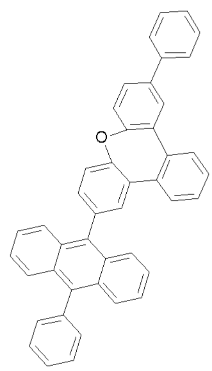

Formel (A-170)

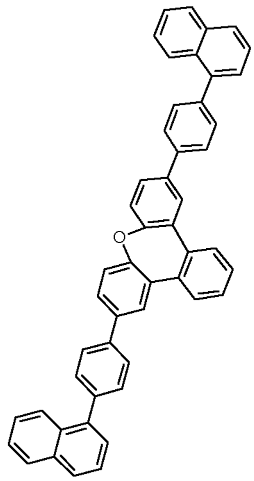

Formel (A-171)

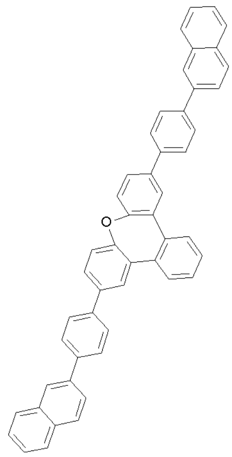

Formel (A-172)

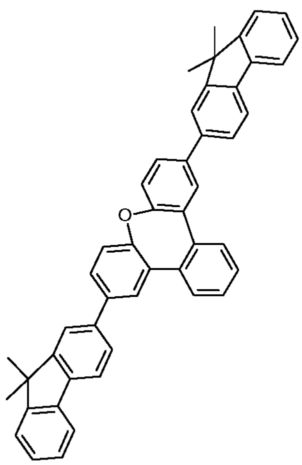

Formel (A-174)

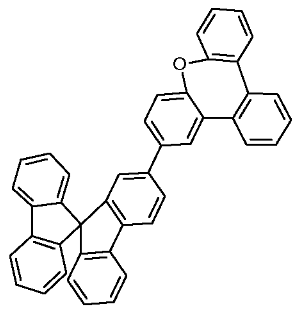

Formel (A-175)

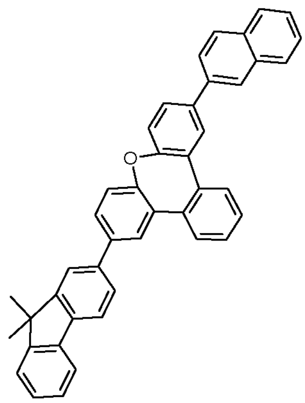

Formel (A-176)

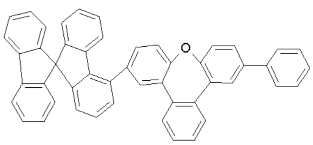

Formel (A-177)

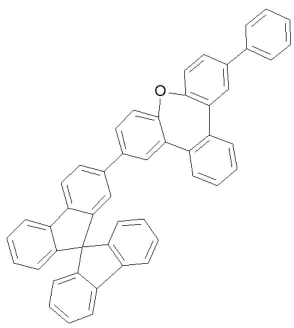

Formel (A-178)

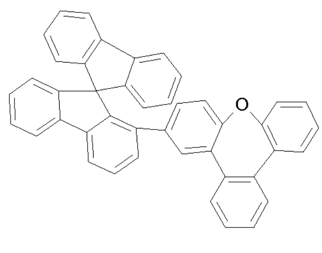

Formel (A-179)

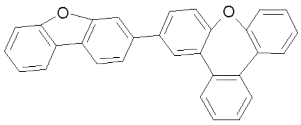
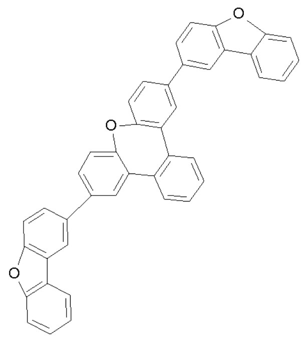
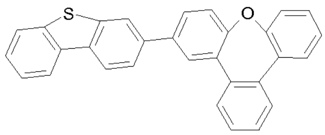

Formel (A-180) Formel (A-181) Formel (A-182)

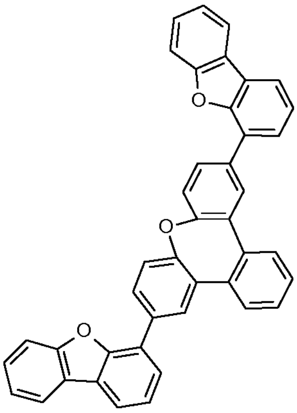
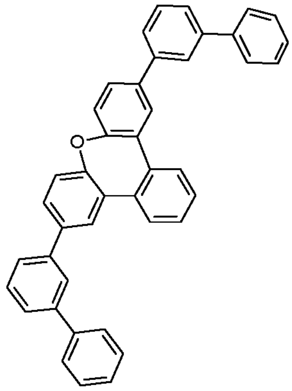
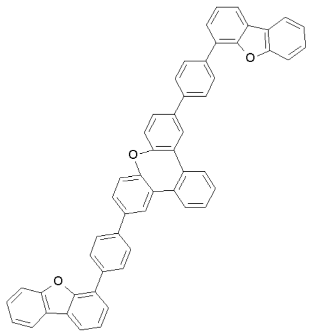

Formel (A-183) Formel (A-184) Formel (A-185)

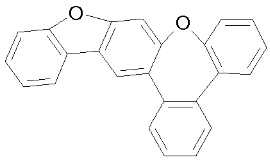
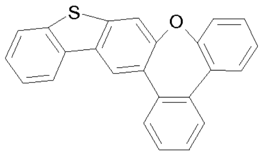

Formel (A-187) Formel (A-188)

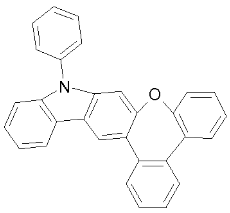

Formel (A-190)

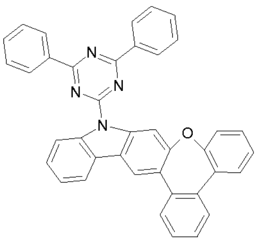

Formel (A-193)

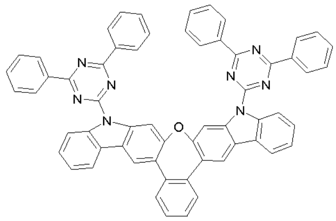

Formel (A-194)

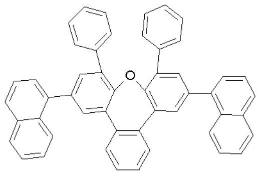

Formel (A-195)

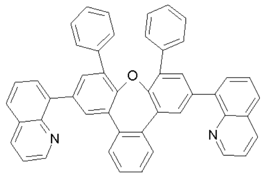

Formel (A-196)

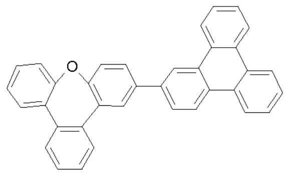

Formel (A-197)

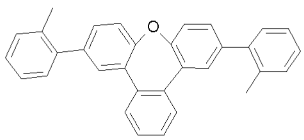

Formel (A-198)

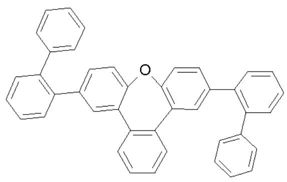

Formel (A-199)

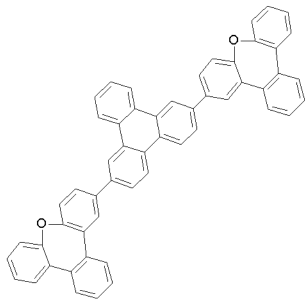

Formel (A-200)

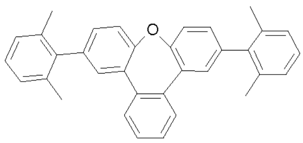

Formel (A-201)

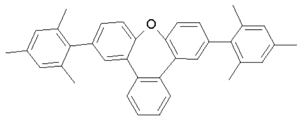

Formel (A-202)

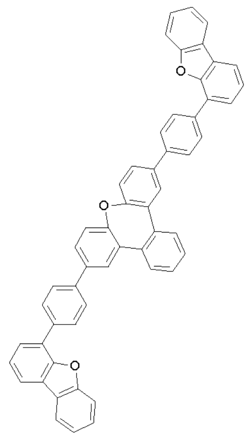

Formel (A-203)

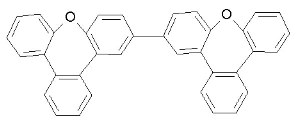

Formel (A-204)

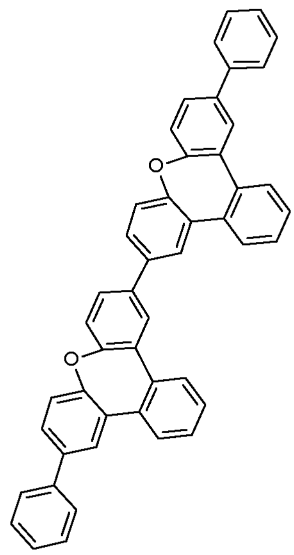

Formel (A-205)

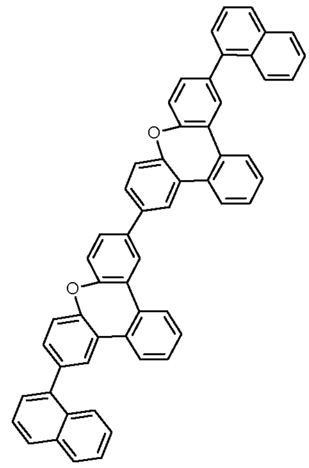

Formel (A-206)

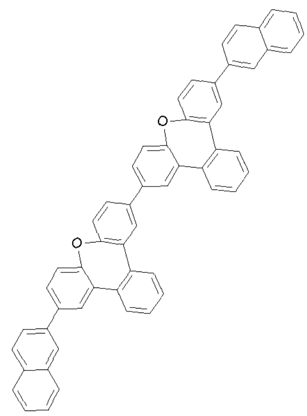

Formel (A-207)

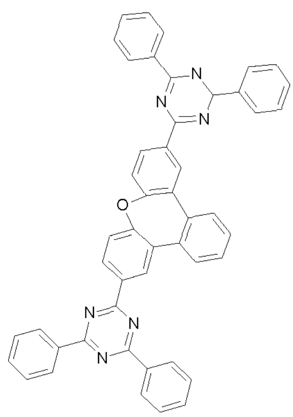

Formel (A-208)

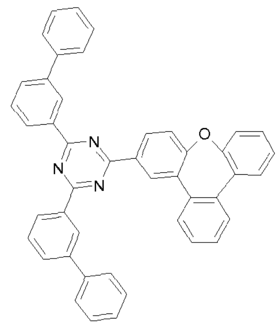

Formel (A-209)

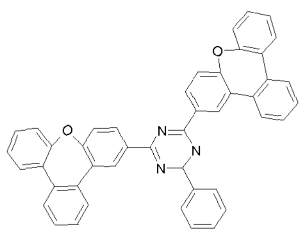

Formel (A-210)

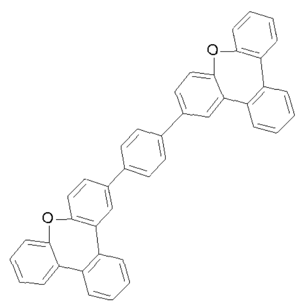

Formel (A-211)

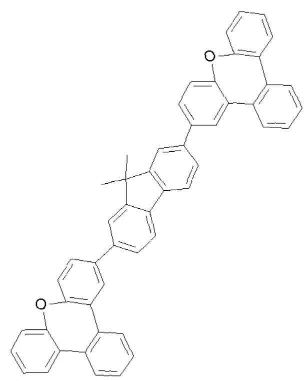

Formel (A-212)

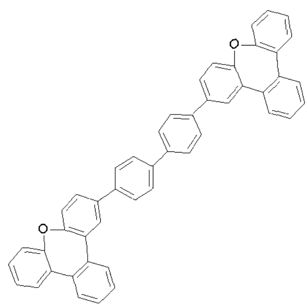

Formel (A-213)

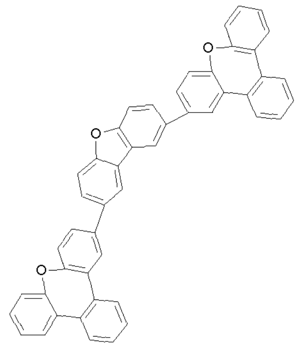

Formel (A-214)

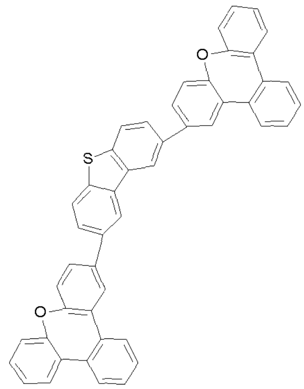

Formel (A-215)

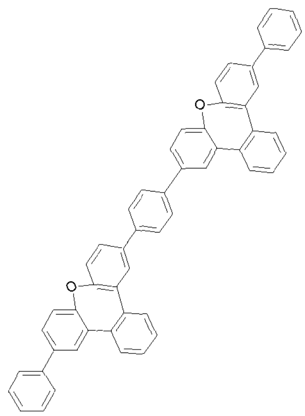

Formel (A-216)

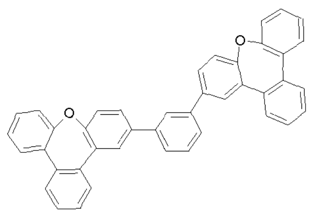

Formel (A-217)

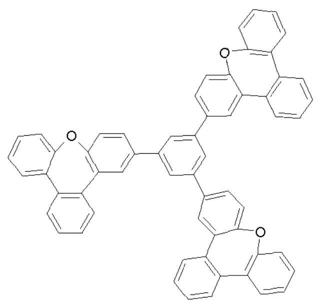

Formel (A-218)

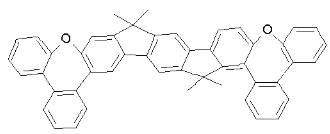

Formel (A-219)

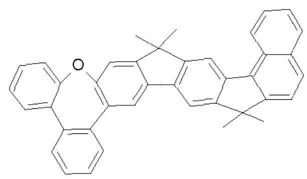

Formel (A-220)

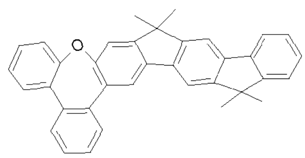

Formel (A-221)

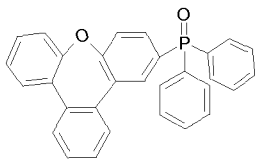

Formel (A-222)

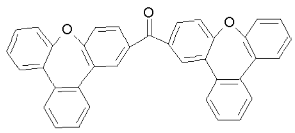

Formel (A-223)

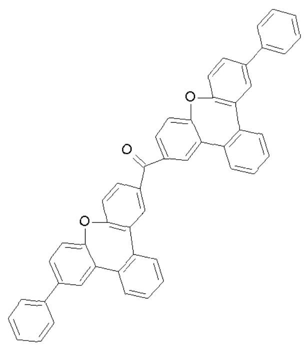

Formel (A-224)

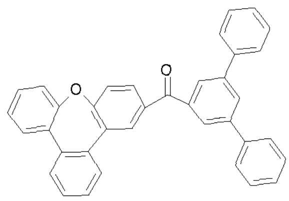

Formel (A-225)

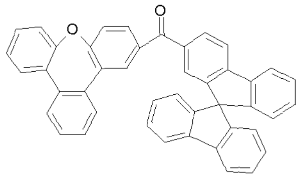

Formel (A-226)

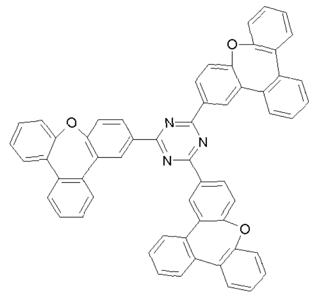

Formel (A-227)

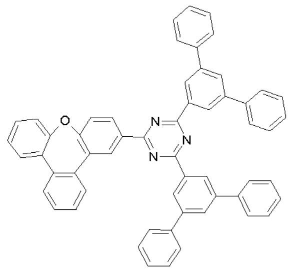

Formel (A-228)

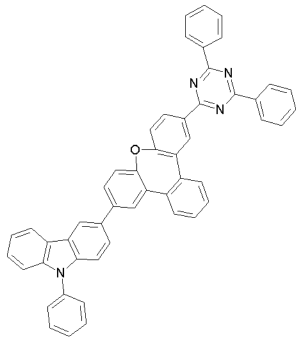

Formel (A-229)

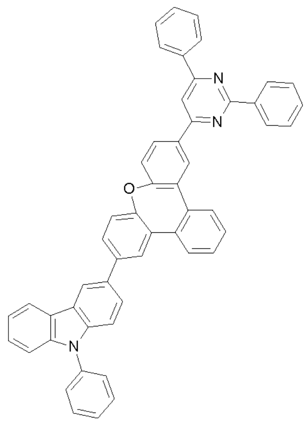

Formel (A-230)

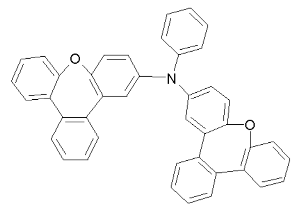

Formel (A-231)

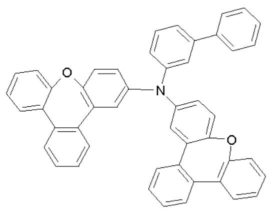

Formel (A-232)

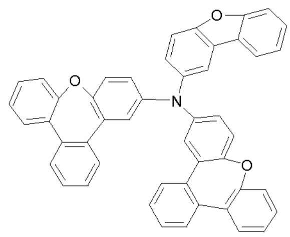

Formel (A-233)

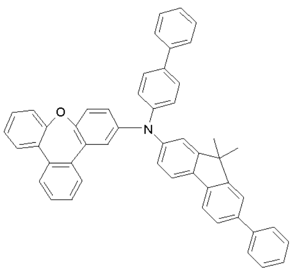

Formel (A-234)

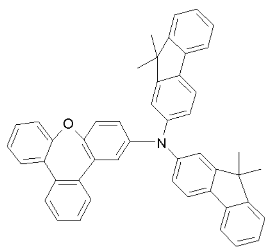

Formel (A-235)

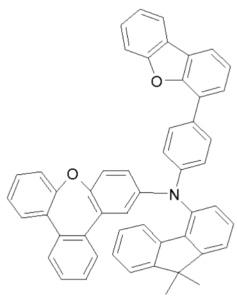

Formel (A-236)

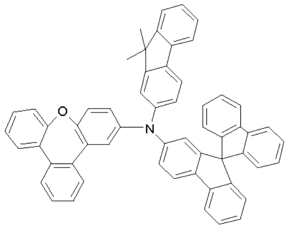
Formel (A-237)

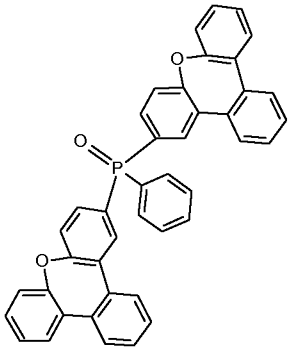
Formel (A-238)

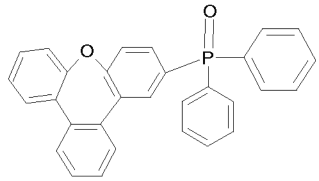
Formel (A-239)

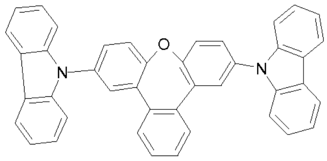
Formel (A-240)

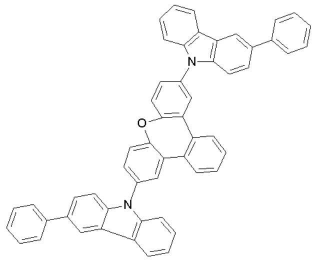
Formel (A-241)

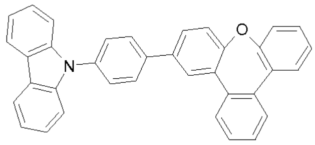
Formel (A-242)

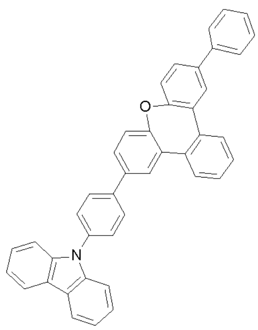
Formel (A-243)

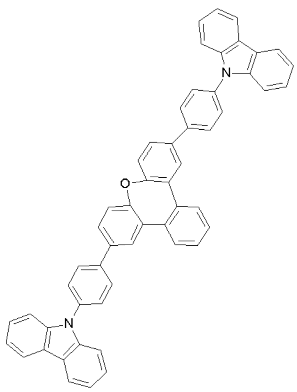
Formel (A-244)

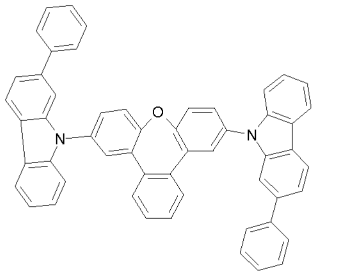
Formel (A-245)

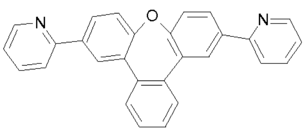
Formel (A-246)

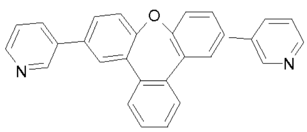
Formel (A-247)

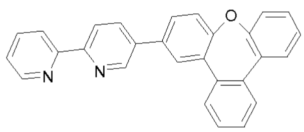
Formel (A-248)

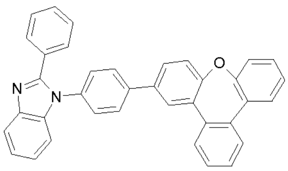
Formel (A-249)

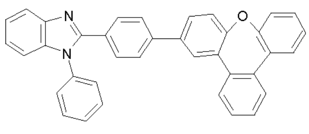
Formel (A-250)

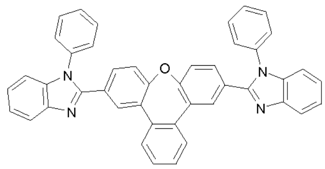
Formel (A-251)

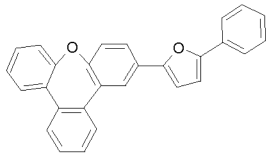
Formel (A-252)

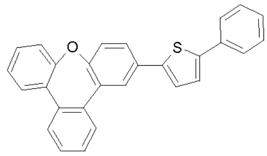
Formel (A-253)

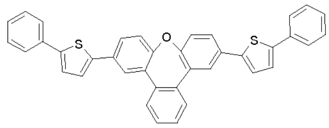
Formel (A-254)

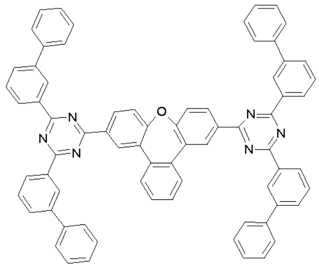
Formel (A-255)

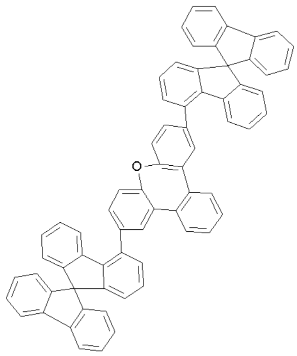
Formel (A-256)

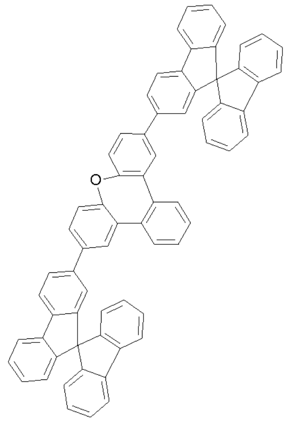
Formel (A-257)

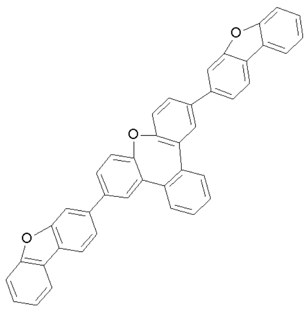
Formel (A-258)

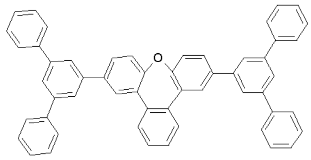
Formel (A-259)

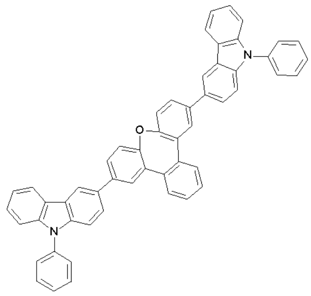
Formel (A-260)

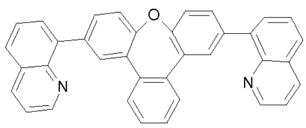
Formel (A-261)

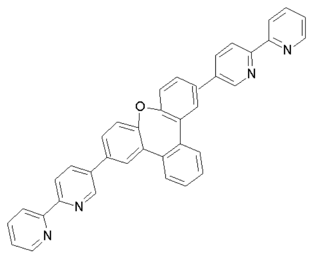
Formel (A-262)

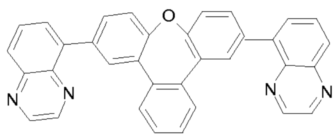
Formel (A-263)

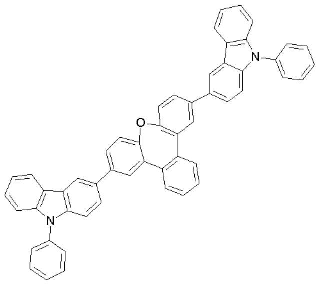
Formel (A-264)

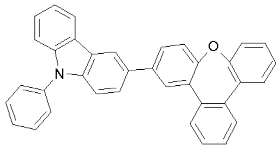
Formel (A-265)

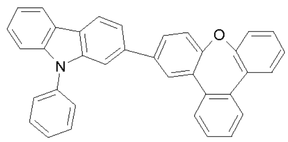
Formel (A-266)

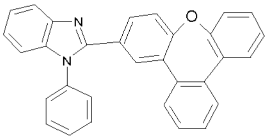
Formel (A-267)

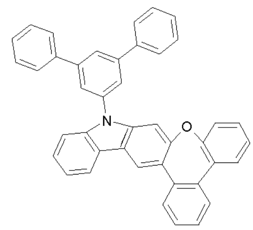
Formel (A-268)

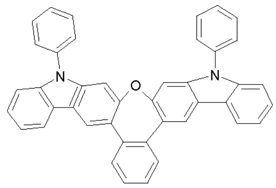
Formel (A-269)

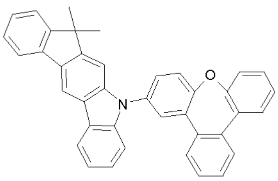
Formel (A-270)

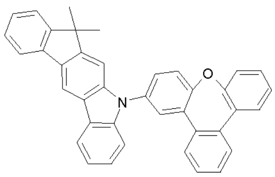
Formel (A-271)

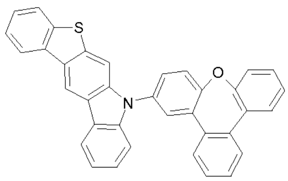
Formel (A-272)

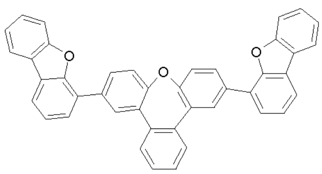
Formel (A-273)

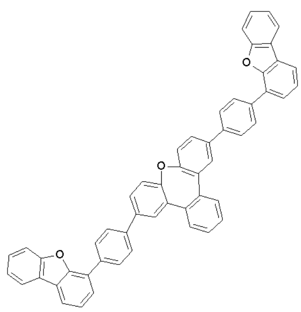
Formel (A-274)

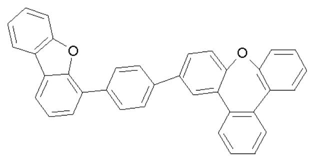
Formel (A-275)

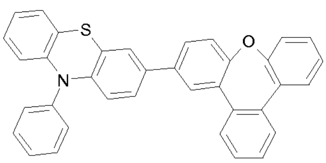
Formel (A-276)

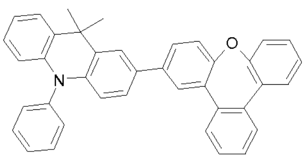
Formel (A-277)

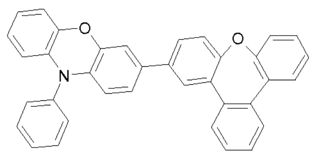
Formel (A-278)

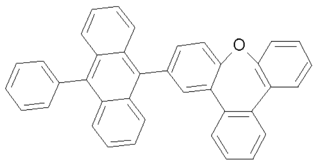

Formel (A-279)

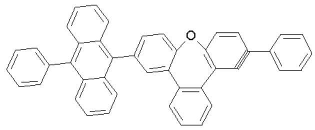

Formel (A-280)

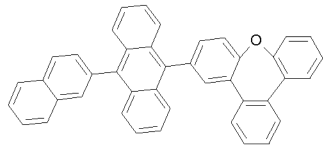

Formel (A-281)

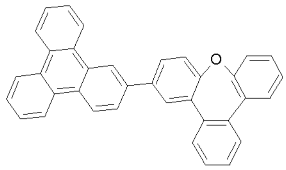

Formel (A-282)

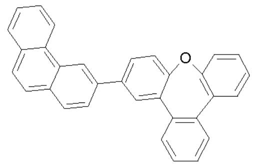

Formel (A-283)

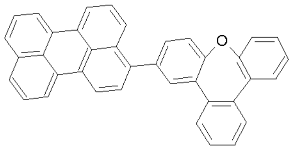

Formel (A-284)

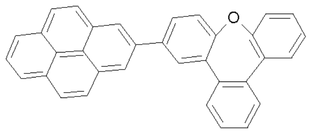

Formel (A-285)

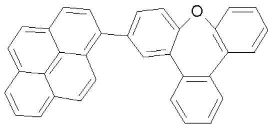

Formel (A-286)

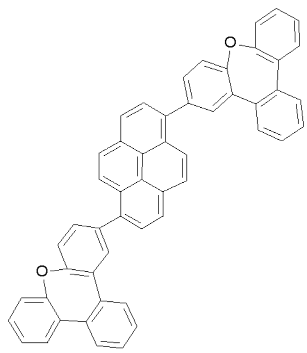

Formel (A-287)

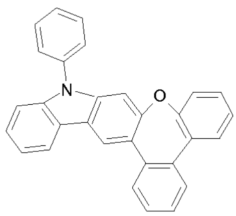

Formel (A-288)

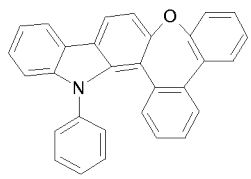

Formel (A-289)

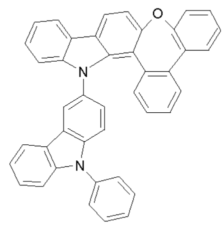

Formel (A-290)

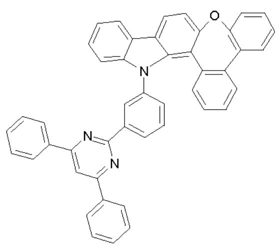

Formel (A-291)

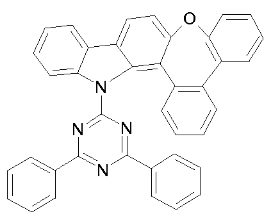

Formel (A-292)

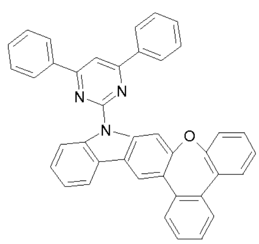

Formel (A-293)

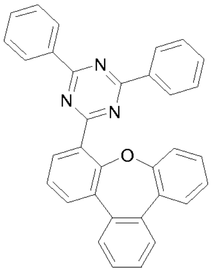
INV-5

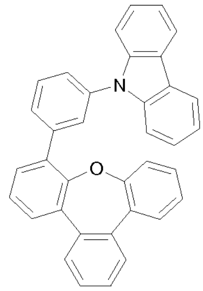
INV-6

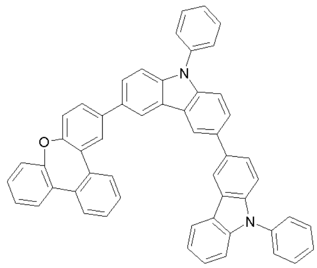
INV-7

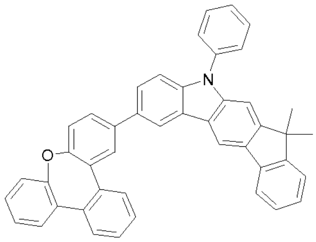
INV-8

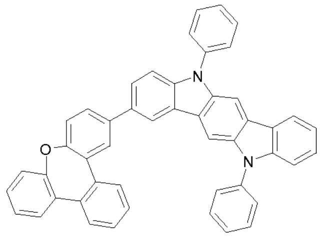
INV-9

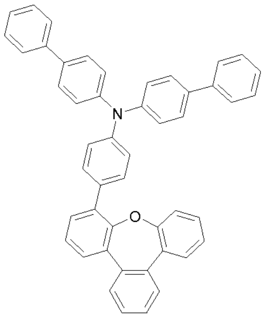
INV-10

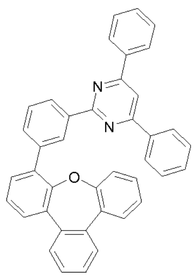
INV-11

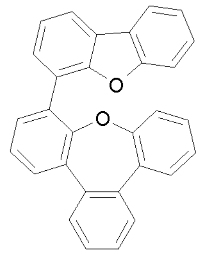
INV-12

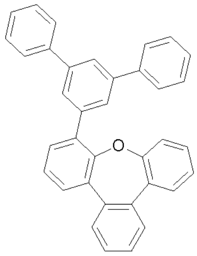
INV-13

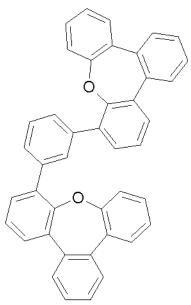
INV-14

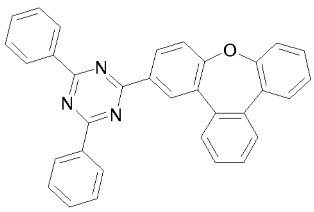
INV-15

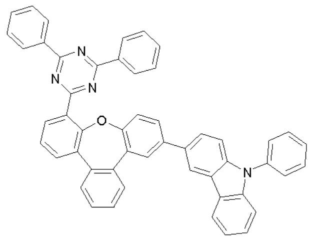
INV-19

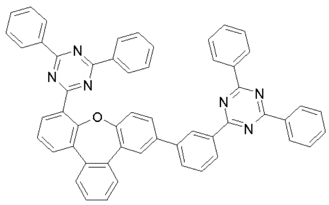
INV-20

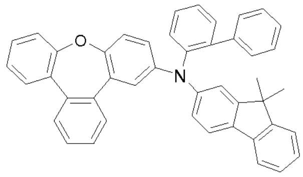
INV-21

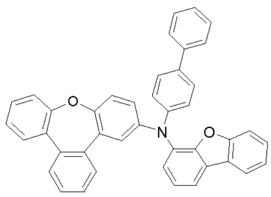
INV-22

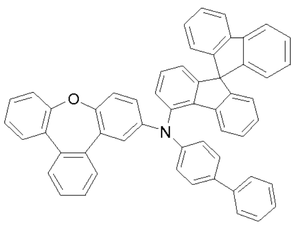
INV-23

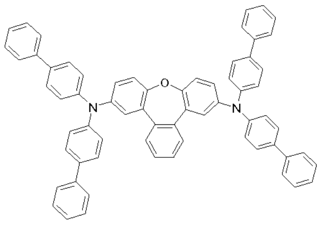
INV-24

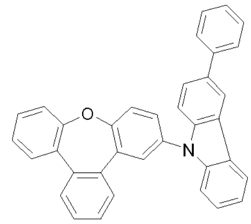
INV-25

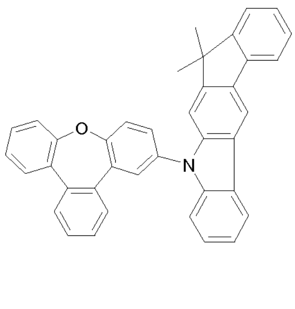
INV-26

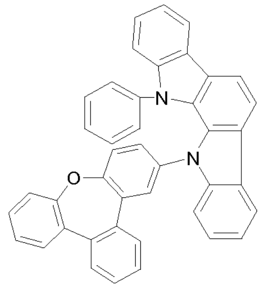
INV-27

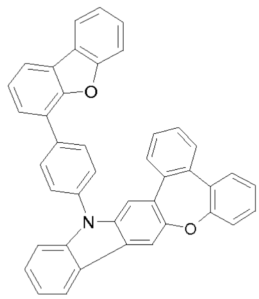
INV-28.

**18.** Verfahren zur Herstellung der Verbindung gemäß einem oder mehreren der Ansprüche 13 bis 17 mit Hilfe der Suzuki-Kupplung.

**19.** Verfahren zur Herstellung der Verbindung gemäß einem oder mehreren der Ansprüche 13 bis 17 mit Hilfe der Buchwald- oder Ullmann-Kupplung.

## Claims

**1.** Electronic device comprising at least one compound of the general formula (5) or (7),

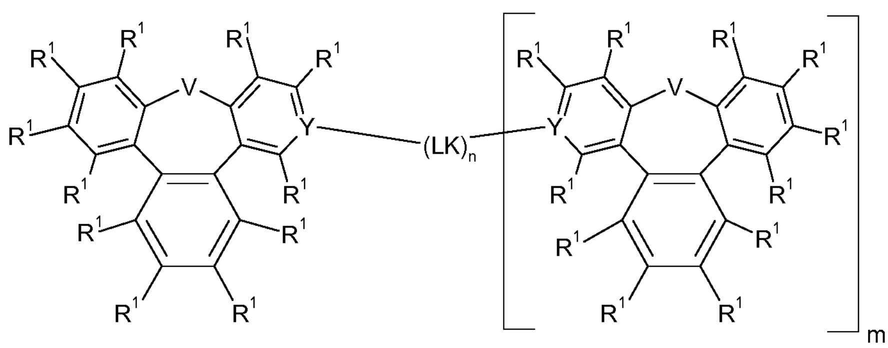
formula (5)

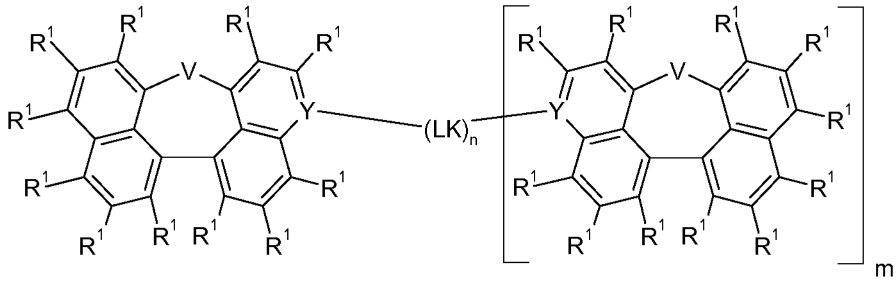

formula (7)

where the following applies to the symbols and indices:

V is either O or S and very preferably O;
X is, identically or differently on each occurrence, N or $CR^1$, preferably $CR^1$;
m is 0 (monomer), 1 (dimer) or 2 (trimer);
n is 0 or 1, where:

n = 0 for m = 0 and
n = 1 for m = 1 or for m = 2;

Y is equal to an $sp^2$-hydridised carbon atom for m not equal to 0 or the same as X for m = 0;
LK is, in the case where m = 1, a single bond or a difunctional linker; LK may be substituted by one or more radicals $R^1$, where the radicals $R^1$ may be identical or different on each occurence;
is, for m = 2, a trifunctional linker, where the linker may be substituted by one or more radicals $R^1$, where the radicals $R^1$ may be identical or different on each occurence;
is, if n = 0, not present, so that a monomer is present;
$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylheteroarylamino group having 10 to 40 aromatic ring atoms, which may be substituted by one or more radicals $R^2$, or a combination of two or more of these groups or a crosslinkable group Q; two or more adjacent radicals $R^1$ may form a mono- or polycyclic, aliphatic or aromatic or heteroaromatic ring system, which may be substituted by one or more radicals $R^2$, with one another;
$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic or heteroaromatic ring system with one another;
$R^3$ is, identically or differently on each occurrence, H, D, F or an aliphatic, aromatic and/or heteroaromatic hydrocarbon radical having 1 to 40 C atoms, in which, in addition, one or more H atoms may be replaced by F; two or more substituents $R^3$ may also form a mono- or polycyclic, aliphatic or aromatic or heteroaromatic ring

system with one another;

with the proviso that, if m = 0, at least one of the radicals $R^1$ is not equal to H.

2. Electronic device according to Claim 1, comprising the compounds of the formula (5) or (7) in an emission layer (EML), electron-transport layer (ETL) or in a hole-blocking layer (HBL), preferably in an EML or ETL and particularly preferably in an EML.

3. Electronic device according to Claim 1, **characterised in that** it is selected from organic integrated circuits (OICs), organic field-effect transistors (OFETs), organic thin-film transistors (OTFTs), organic electroluminescent devices, organic solar cells (OSCs), organic optical detectors, organic photoreceptors, preferably from an organic electroluminescent device.

4. Electronic device accoding to Claim 1 or 3, **characterised in that** it is an organic electroluminescent device which is also selected from the group consisting of organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

5. Process for the production of an electronic device according to one or more of Claims 1 to 4, **characterised in that** at least one organic layer is applied by gas-phase deposition or from solution.

6. Electronic device according to Claim 4 for use in medicine for phototherapy, preferably for use for phototherapy of the skin, very preferably for use for the treatment or prevention of psoriasis, atopic dermatitis, jaundice, jaundice of the newborn, vitiligo, inflammation, pain and for use for wound healing.

7. Use of the device according to Claim 4 in the cosmetics field, preferably for reduction or prevention of skin aging, skin wrinkles, crows feet, acne, comedones and cellulite.

8. Use of the device according to Claim 4 in displays or for lighting.

9. Composition comprising at least one compound of the general formula (5) or (7),

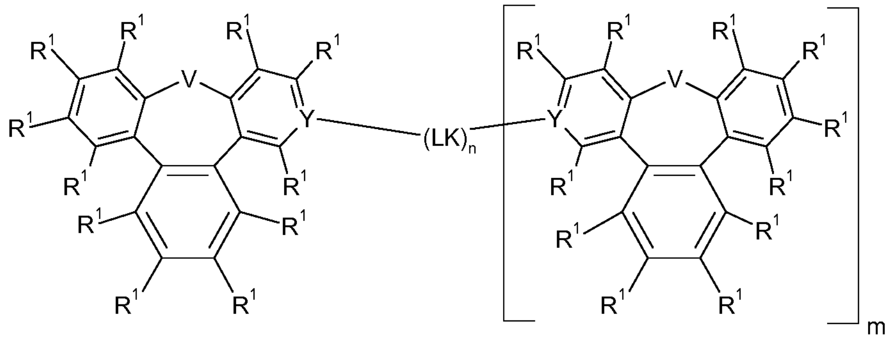

formula (5)

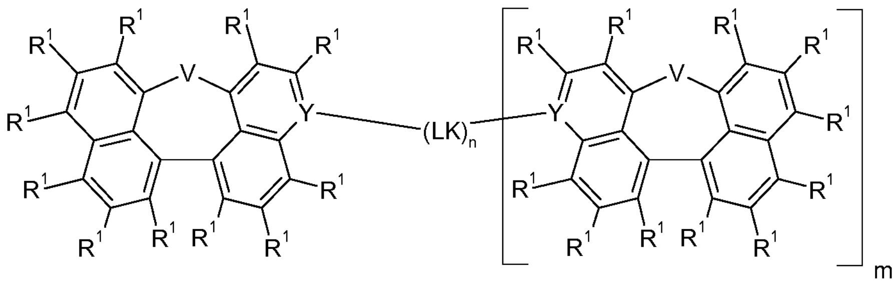

formula (7)

where the following applies to the symbols and indices:

V is either O or S and very preferably O;
X is identically or differently on each occurrence, N or $CR^1$, preferably $CR^1$;
m is 0 (monomer), 1 (dimer) or 2 (trimer);
n is 0 or 1, where:

n = 0 for m = 0 and
n = 1 for m = 1 or for m = 2;

Y is equal to an $sp^2$-hydridised carbon atom for m not equal to 0 or the same as X for m = 0;
LK is, in the case where m = 1, a single bond or a difunctional linker; LK may be substituted by one or more radicals $R^1$, where the radicals $R^1$ may be identical or different on each occurence;
is, for m = 2, a trifunctional linker, where the linker may be substituted by one or more radicals $R^1$, where the radicals $R^1$ may be identical or different on each occurence;
is, if n = 0, not present, so that a monomer is present;
$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylheteroarylamino group having 10 to 40 aromatic ring atoms, which may be substituted by one or more radicals $R^2$, or a combination of two or more of these groups or a crosslinkable group Q; two or more adjacent radicals $R^1$ may form a mono- or polycyclic, aliphatic or aromatic or heteroaromatic ring system, which may be substituted by one or more radicals $R^2$, with one another;
$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic or heteroaromatic ring system with one another;
$R^3$ is, identically or differently on each occurrence, H, D, F or an aliphatic, aromatic and/or heteroaromatic hydrocarbon radical having 1 to 40 C atoms, in which, in addition, one or more H atoms may be replaced by F; two or more substituents $R^3$ may also form a mono- or polycyclic, aliphatic or aromatic or heteroaromatic ring system with one another;

with the proviso that, if m = 0, at least one of the radicals $R^1$ is not equal to H,
and at least one further compound selected from the group consisting of fluorescent emitters, phosphorescent emitters, host materials, matrix materials, electron-transport materials, electron-injection materials, hole-conductor materials, hole-injection materials, electronblocking materials and hole-blocking materials, n-dopants or p-dopants.

**10.** Composition according to Claim 9, **characterised in that** the additional compound is a phosphorescent emitter

**11.** Composition according to Claim 9 or 10, **characterised in that** the additional compound is a host or matrix material.

**12.** Composition according to one or more of Claims 9 to 11, **characterised in that** the additional compound has a

band gap of 2.5 eV or more, preferably 3.0 eV or more, very preferably 3.5 eV or more.

**13.** Compound of the general formula (5)

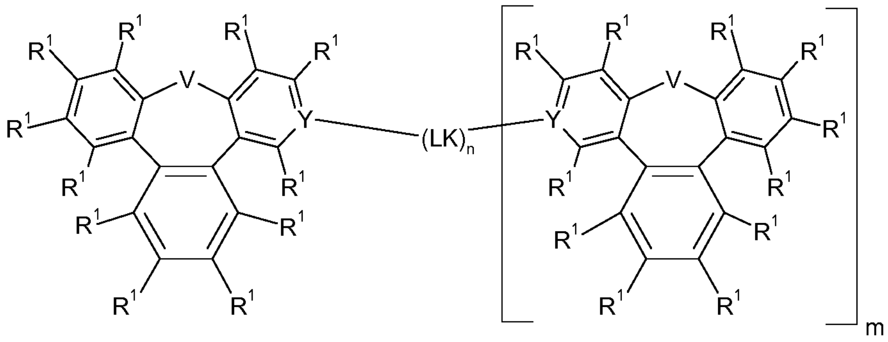

formula (5)

where the following applies to the symbols and indices:

V is O;
m is 0 (monomer), 1 (dimer) or 2 (trimer);
n is 0 or 1, where:

n = 0 form = 0 and
n = 1 for m = 1 or for m = 2;

Y is equal to an $sp^2$-hydridised carbon atom for m not equal to 0 or the same as X for m = 0;
X is, identically or differently on each occurrence, $CR^1$,
LK is, in the case where m = 1, a single bond or a difunctional linker; LK may be substituted by one or more radicals $R^1$, where the radicals $R^1$ may be identical or different on each occurence;
is, for m = 2, a trifunctional linker, where the linker may be substituted by one or more radicals $R^1$, where the radicals $R^1$ may be identical or different on each occurence;
is, if n = 0, not present, so that a monomer is present;
$R^1$ is, identically or differently on each occurrence, H, D, F, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkoxy group having 1 to 40 C atoms or a straight-chain alkenyl or alkynyl group having 2 to 40 C atoms
or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O or $CONR^2$ and where one or more H atoms may be replaced by D, F, I, CN or $NO_2$,
or an aromatic or heteroaromatic ring system having 5 to 60 aromatic ring atoms, which may in each case be substituted by one or more radicals $R^2$,
or an aryloxy, arylalkoxy or heteroaryloxy group having 5 to 60 aromatic ring atoms, which may be substituted by one or more radicals $R^2$, or a diarylamino group, diheteroarylamino group or arylheteroarylamino group having 10 to 40 aromatic ring atoms, which may be substituted by one or more radicals $R^2$, or a combination of two or more of these groups; two or more adjacent radicals $R^1$ may form a mono- or polycyclic, aliphatic or heteroaromatic ring system, which may be substituted by one or more radicals $R^2$, with one another;
$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group

having 10 to 40 aromatic ring atoms, which may be substituted by one or more radicals $R^3$, or a combination of two or more of these groups; two or more adjacent radicals $R^2$ may form a mono- or polycyclic, aliphatic or aromatic or heteroaromatic ring system with one another;

$R^3$ is, identically or differently on each occurrence, H, D, F or an aliphatic, aromatic and/or heteroaromatic hydrocarbon radical having 1 to 40 C atoms, in which, in addition, one or more H atoms may be replaced by F; two or more substituents $R^3$ may also form a mono- or polycyclic, aliphatic or aromatic or heteroaromatic ring system with one another;

with the proviso that, if m = 0, at least one of the radicals $R^1$ is not equal to H.

**14.** Compound according to Claim 13, **characterised in that** it has the following formula (10a), (10b), (10c) or (10d)

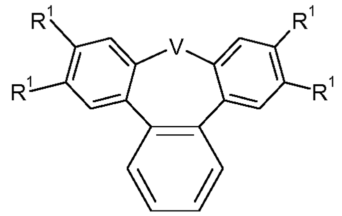

formula (10a)

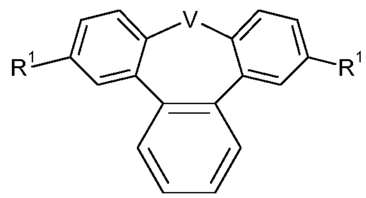

formula (10b)

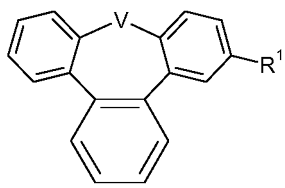

formula (10c)

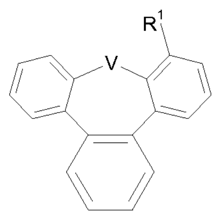

formula (10d).

**15.** Compound of the general formula (7)

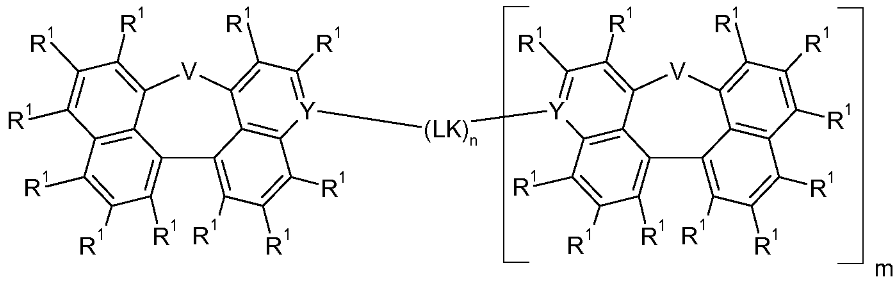

formula (7)

where the following applies to the symbols and indices:

V is O;
X is, identically or differently on each occurrence, $CR^1$,
m is 0 (monomer), 1 (dimer) or 2 (trimer);
n is 0 or 1, where:

n = 0 for m = 0 and
n = 1 for m = 1 or for m = 2;

Y is equal to an $sp^2$-hydridised carbon atom for m not equal to 0 or the same as X for m = 0;
LK is, in the case where m = 1, a single bond or a difunctional linker; LK may be substituted by one or more radicals $R^1$, where the radicals $R^1$ may be identical or different on each occurence;
is, for m = 2, a trifunctional linker, where the linker may be substituted by one or more radicals $R^1$, where the radicals $R^1$ may be identical or different on each occurence;
is, if n = 0, not present, so that a monomer is present;
$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$,
or an aromatic or heteroaromatic ring system having 5 to 60 aromatic ring atoms, which may in each case be substituted by one or more radicals $R^2$,
or an aryloxy, arylalkoxy or heteroaryloxy group having 5 to 60 aromatic ring atoms, which may be substituted by one or more radicals $R^2$,
or a diarylamino group, diheteroarylamino group or arylheteroarylamino group having 10 to 40 aromatic ring atoms, which may be substituted by one or more radicals $R^2$, or a combination of two or more of these groups or a crosslinkable group Q; two or more adjacent radicals $R^1$ may form a mono- or polycyclic, aliphatic or aromatic or heteroaromatic ring system, which may be substituted by one or more radicals $R^2$, with one another;
$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, $C\equiv C$, $Si(R^3)_2$, $Ge(R^3)_2$, $Sn(R^3)_2$, C=O, C=S, C=Se, $C=NR^3$, $P(=O)(_R{}^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic or heteroaromatic ring system with one another;
$R^3$ is, identically or differently on each occurrence, H, D, F or an aliphatic, aromatic and/or heteroaromatic hydrocarbon radical having 1 to 40 C atoms, in which, in addition, one or more H atoms may be replaced by F;

two or more substituents $R^3$ may also form a mono- or polycyclic, aliphatic or aromatic or heteroaromatic ring system with one another;

with the proviso that, if m = 0, at least one of the radicals $R^1$ is not equal to H.

**16.** Compound according to Claim 15, **characterised in that** it has the following formula (14), (15), (16), (17), (18) or (19)

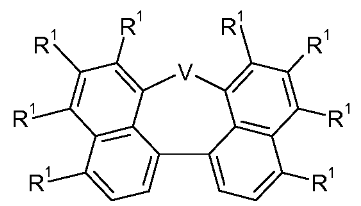

formula (14)

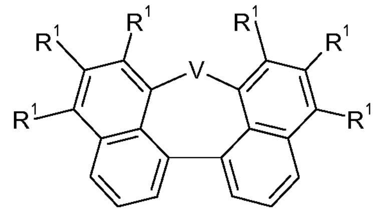

formula (15)

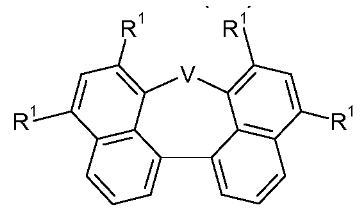

formula (16)

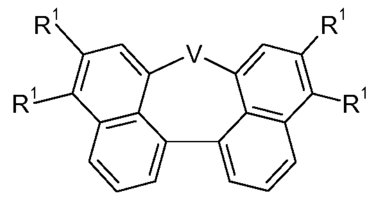

formula (17)

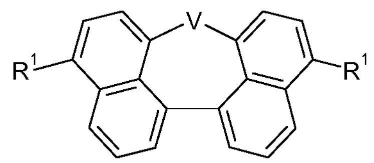

formula (18)

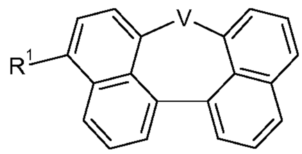

formula (19).

**17.** Compounds according to one of Claims 13 to 16, selected from

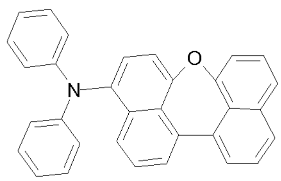

formula (A-1)

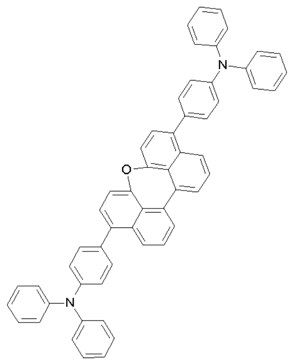

formula (A-2)

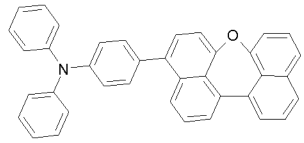

formula (A-3)

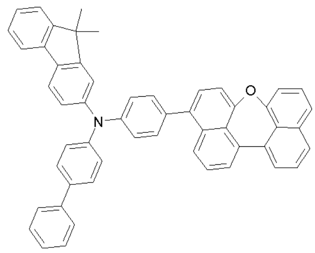

formula (A-4)

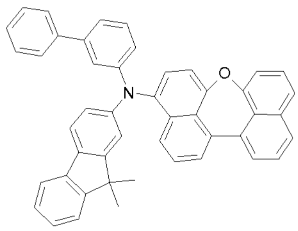

formula (A-5)

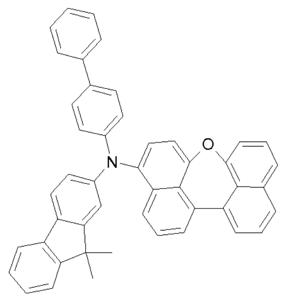

formula (A-6)

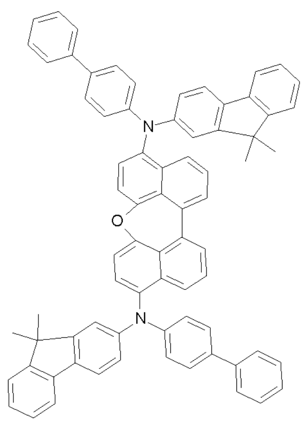

formula (A-7)

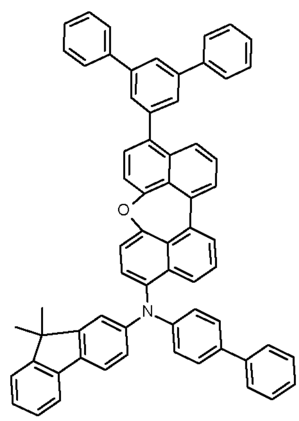

formula (A-8)

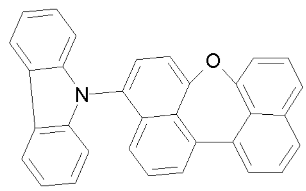

formula (A-9)

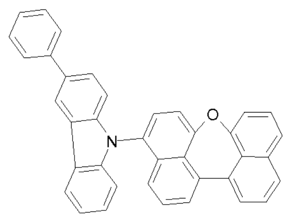

formula (A-10)

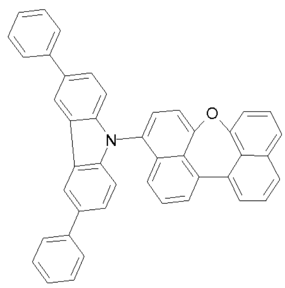

formula (A-11)

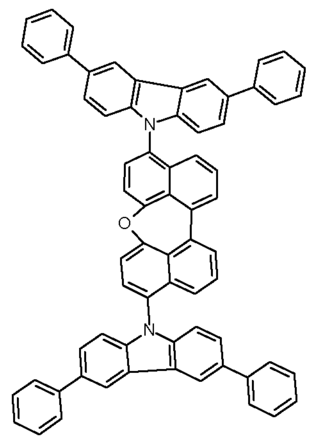

formula (A-12)

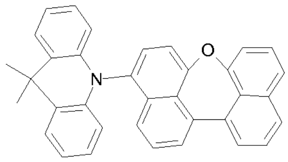

formula (A-13)

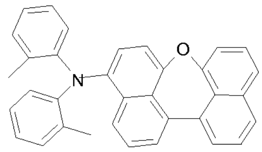

formula (A-14)

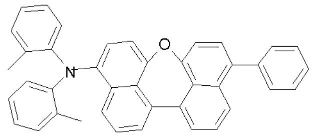

formula (A-15)

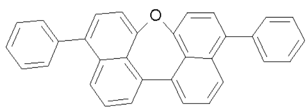

formula (A-17)

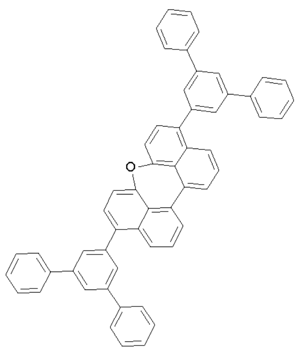

formula (A-17)

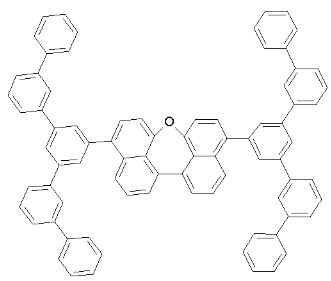

formula (A-18)

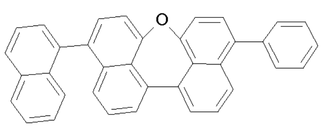

formula (A-19)

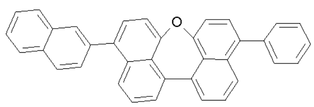

formula (A-20)

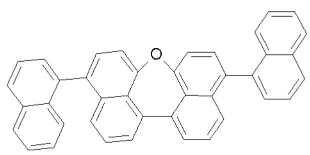

formula (A-21)

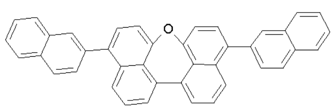

formula (A-22)

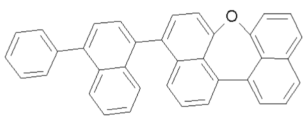

formula (A-23)

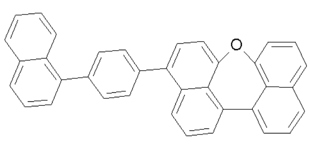

formula (A-24)

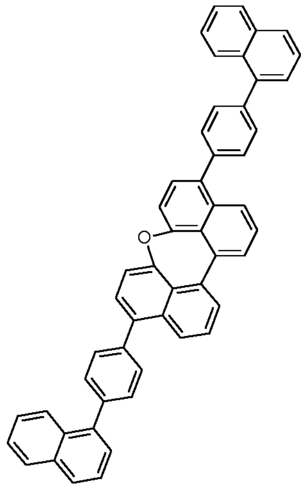

formula (A-25)

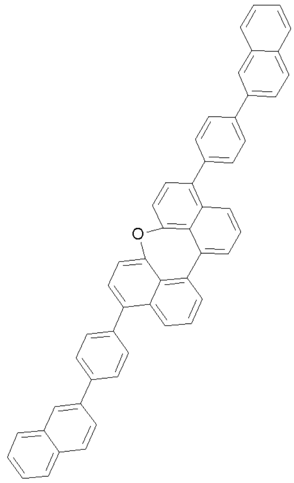

formula (A-26)

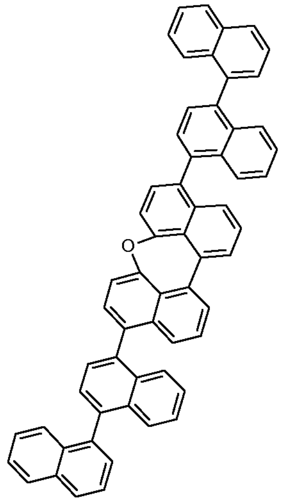

formula (A-27)

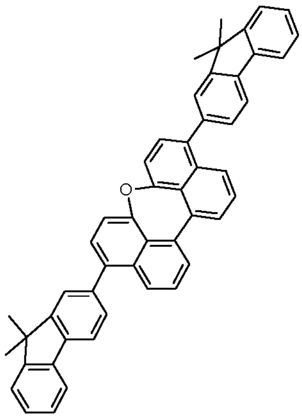

formula (A-28)

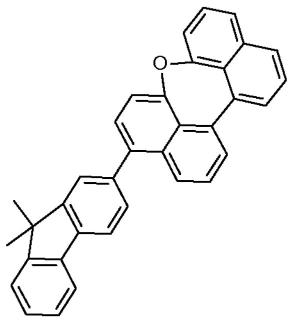

formula (A-29)

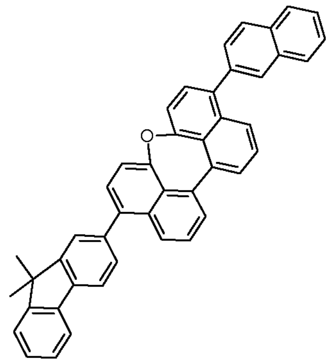

formula (A-30)

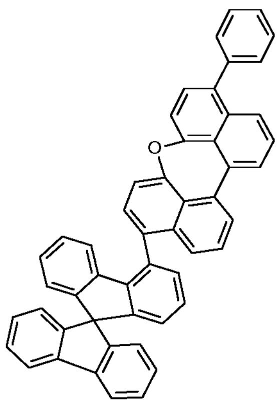

formula (A-31)

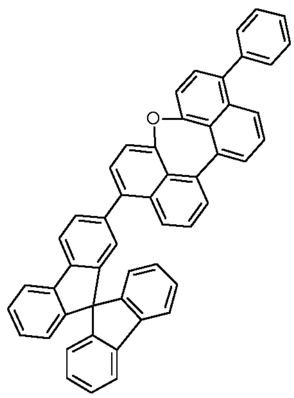

formula (A-32)

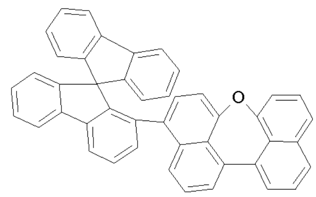

formula (A-33)

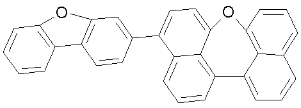

formula (A-34)

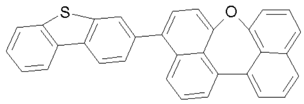

formula (A-36)

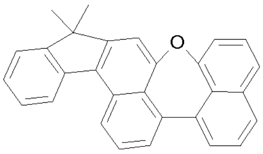

formula (A-40)

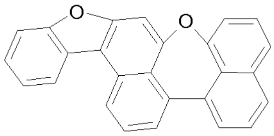

formula (A-41)

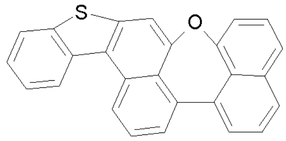

formula (A-42)

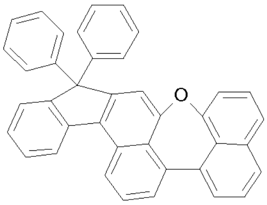

formula (A-43)

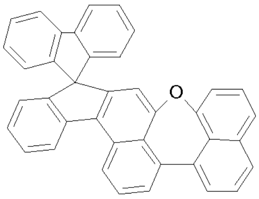

formula (A-44)

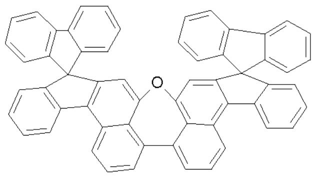

formula (A-45)

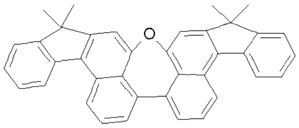

formula (A-46)

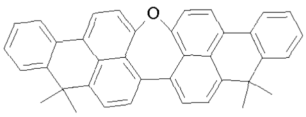

formula (A-49)

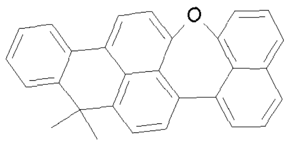

formula (A-50)

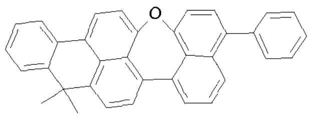

formula (A-51)

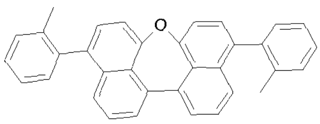

formula (A-52)

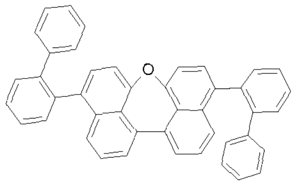

formula (A-53)

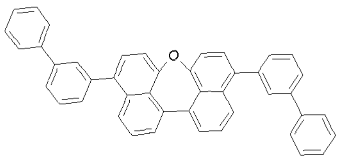

formula (A-54)

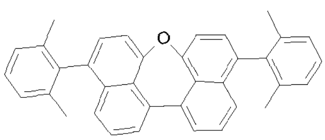

formula (A-55)

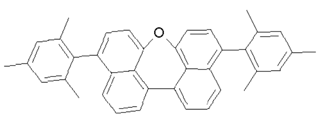

formula (A-56)

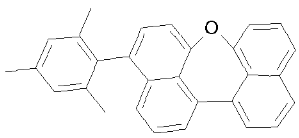

formula (A-57)

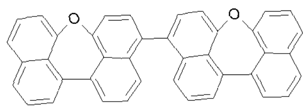

formula (A-58)

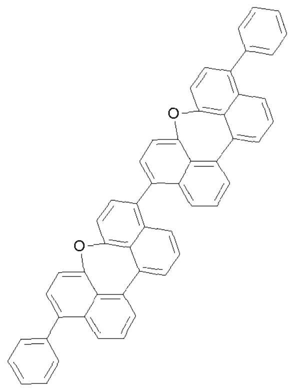

formula (A-59)

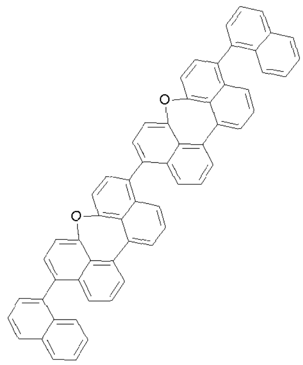

formula (A-60)

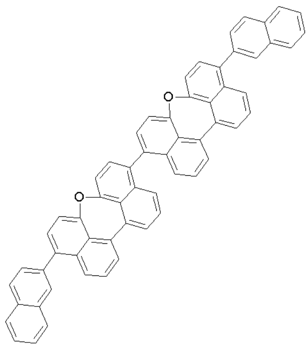

formula (A-61)

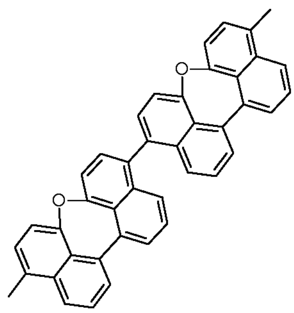

formula (A-62)

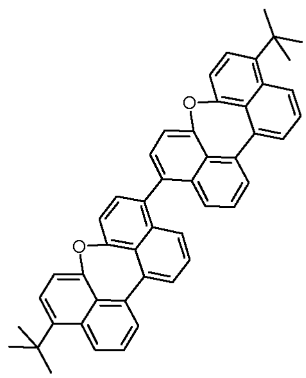

formula (A-63)

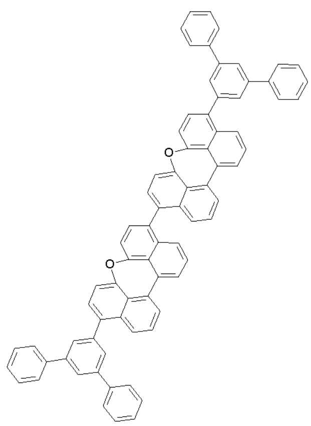

formula (A-64)

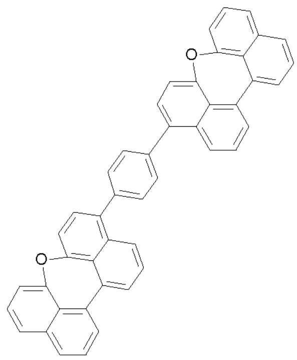

formula (A-65)

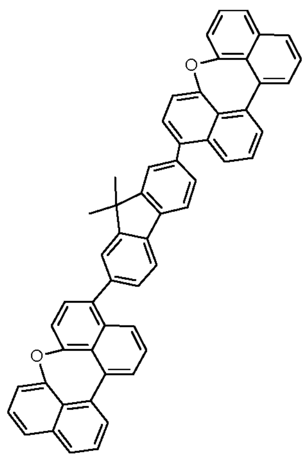

formula (A-66)

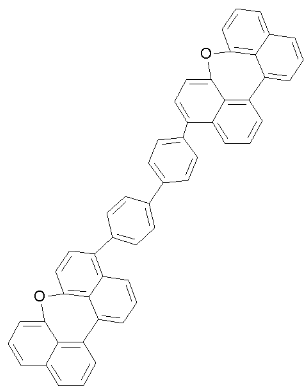

formula (A-67)

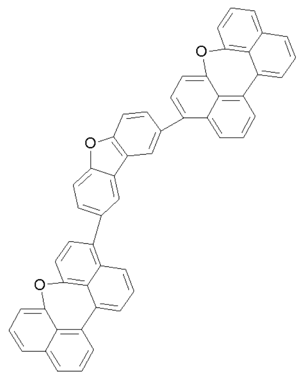

formula (A-68)

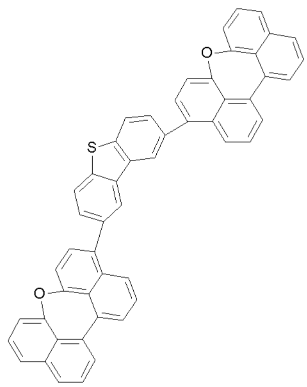

formula (A-69)

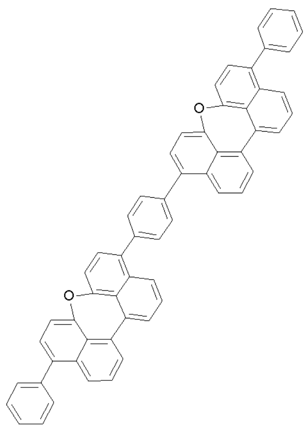

formula (A-69a)

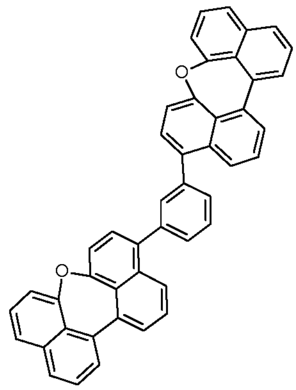

formula (A-69b)

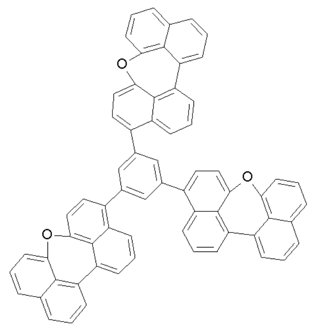

formula (A-69c)

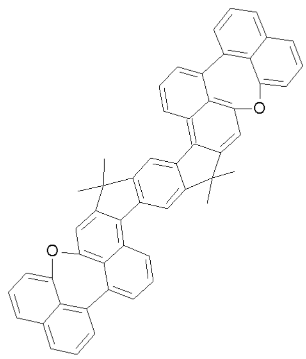

formula (A-69d)

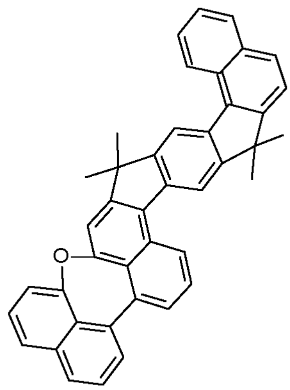

formula (A-69e)

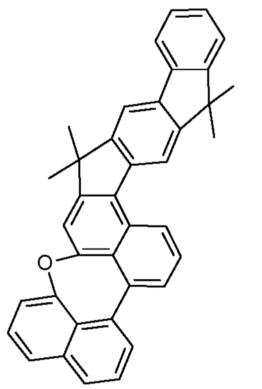

formula (A-69f)

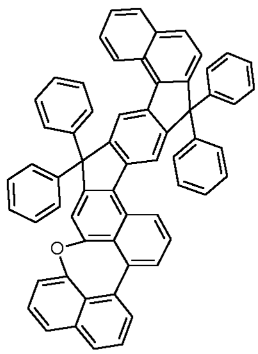

formula (A-69g)

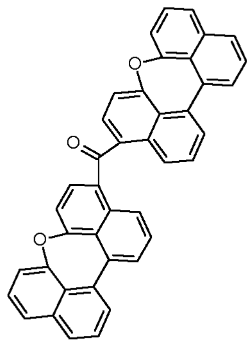

formula (A-69h)

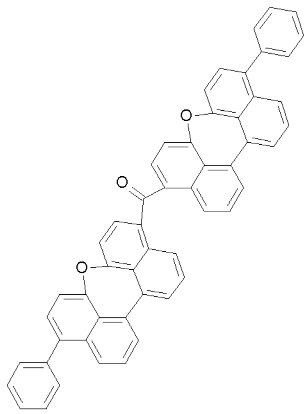

formula (A-69i)

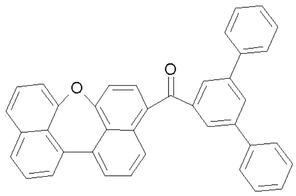

formula (A-69j)

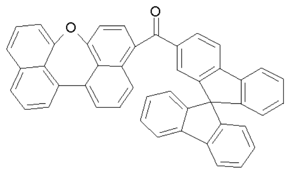

formula (A-69k)

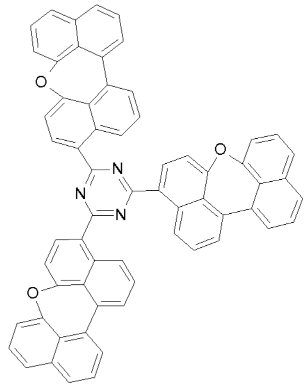

formula (A-69l)

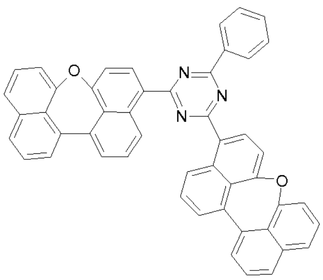

formula (A-69m)

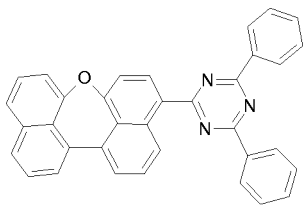

formula (A-69n)

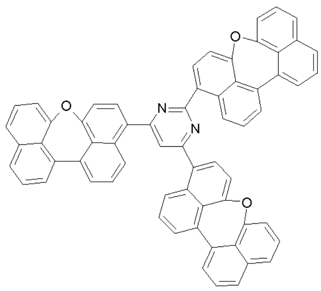

formula (A-69o)

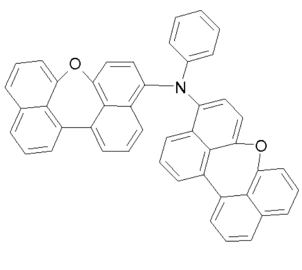

formula (A-69q)

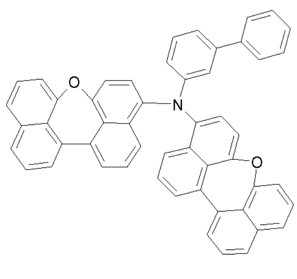

formula (A-69r)

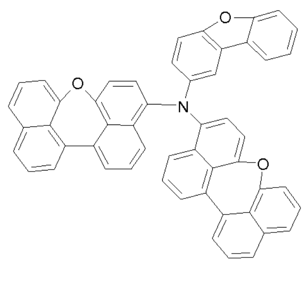

formula (A-69s)

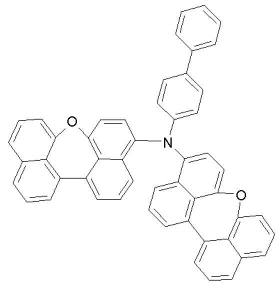

formula (A-69t)

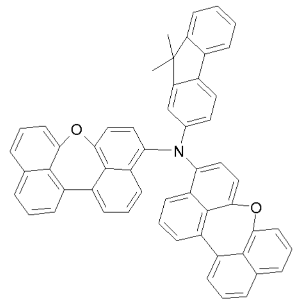

formula (A-69u)

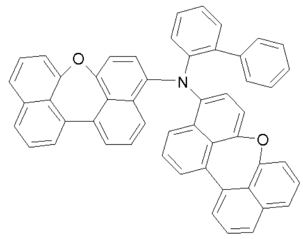

formula (A-70)

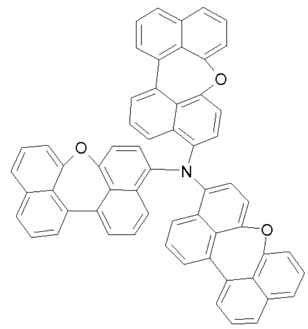

formula (A-71)

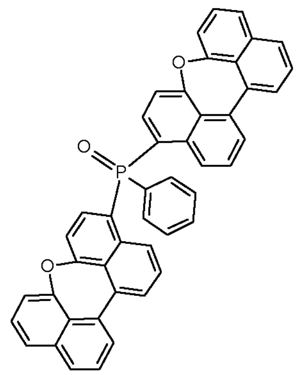

formula (A-72)

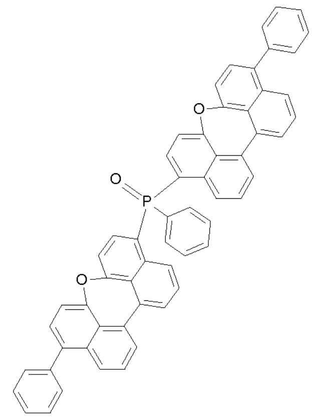

formula (A-73)

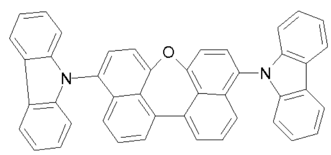

formula (A-74)

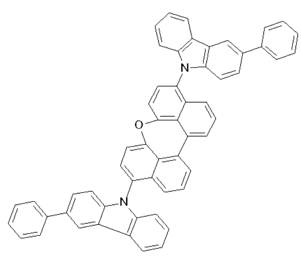

formula (A-75)

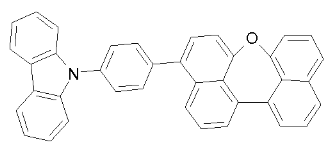

formula (A-76)

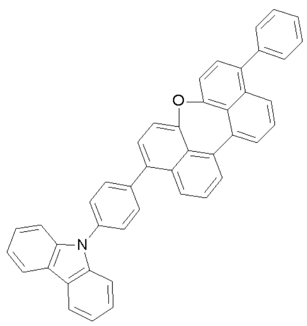

formula (A-77)

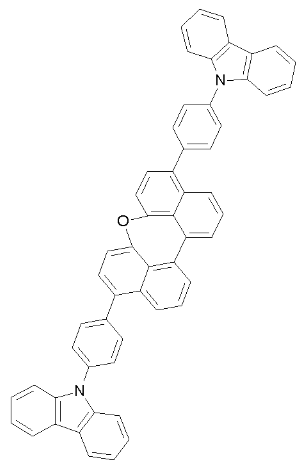

formula (A-78)

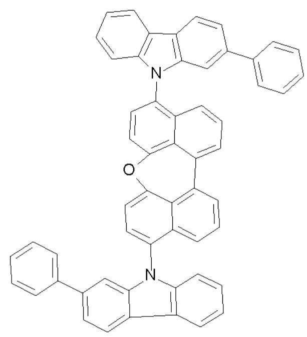

formula (A-79)

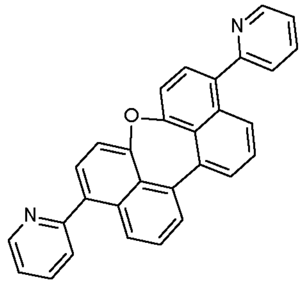

formula (A-80)

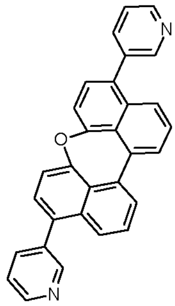

formula (A-81)

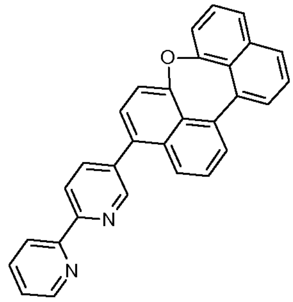

formula (A-82)

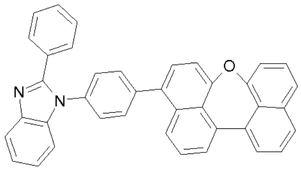

formula (A-83)

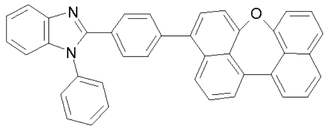

formula (A-84)

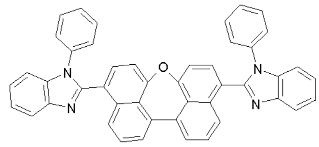

formula (A-85)

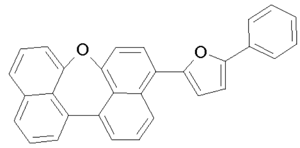

formula (A-86)

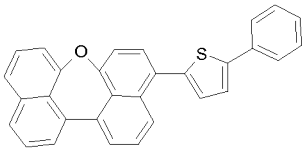

formula (A-87)

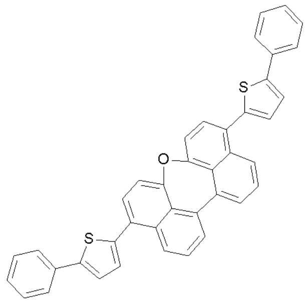

formula (A-88)

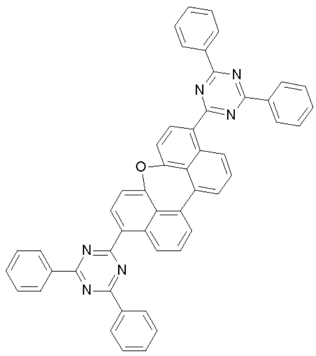

formula (A-89)

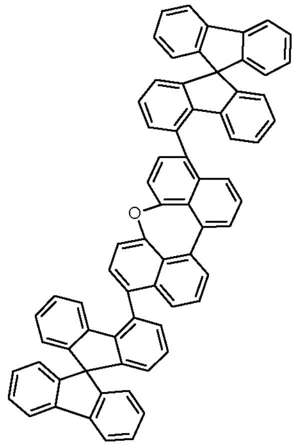

formula (A-90)

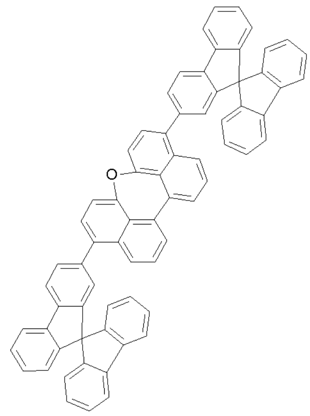

formula (A-91)

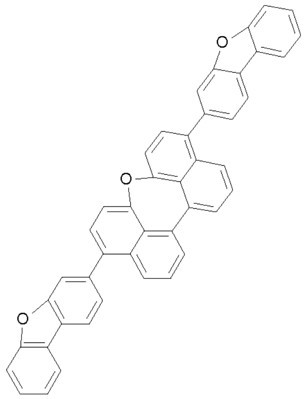

formula (A-92)

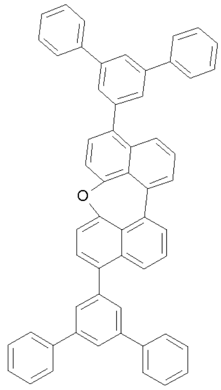

formula (A-93)

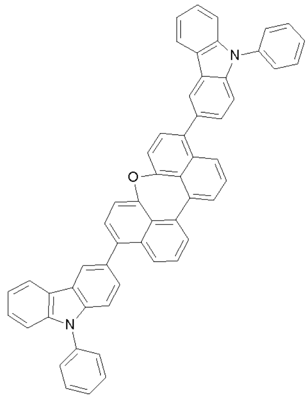

formula (A-94)

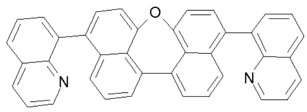

formula (A-95)

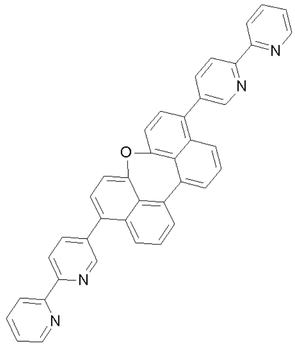

formula (A-96)

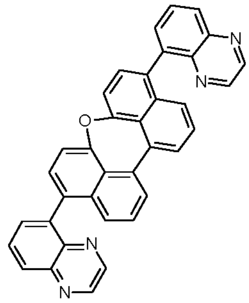

formula (A-97)

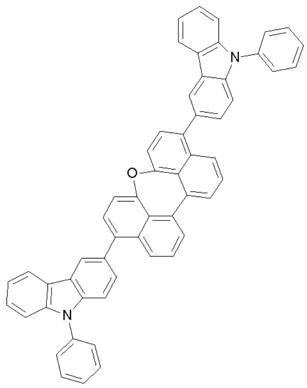

formula (A-98)

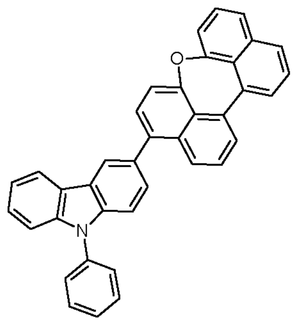

formula (A-99)

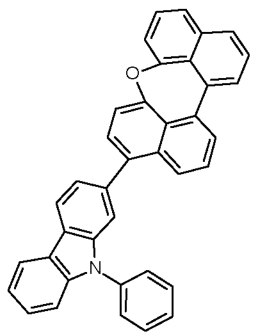

formula (A-100)

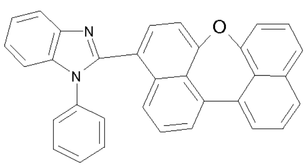

formula (A-101)

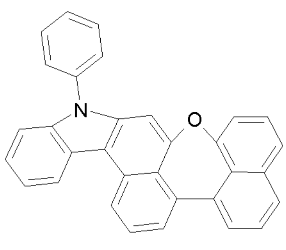

formula (A-102)

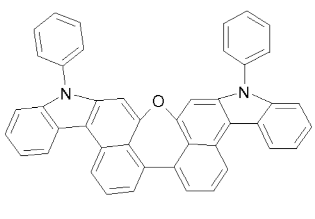

formula (A-103)

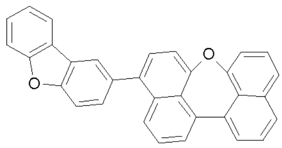

formula (A-104)

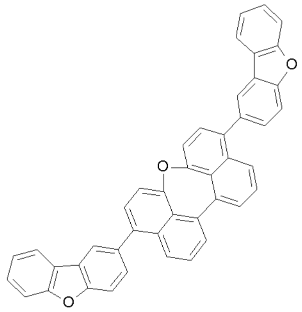

formula (A-105)

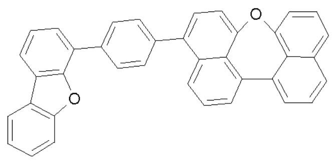

formula (A-106)

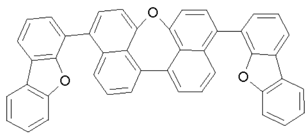

formula (A-107)

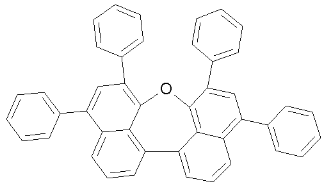

formula (A-108)

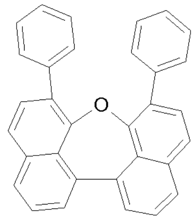

formula (A-109)

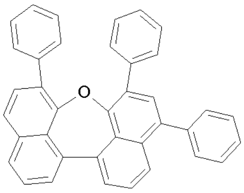

formula (A-110)

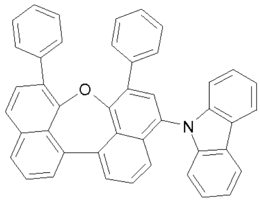

formula (A-111)

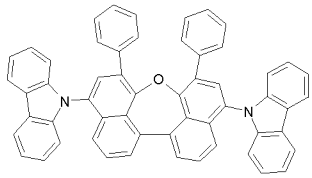

formula (A-112)

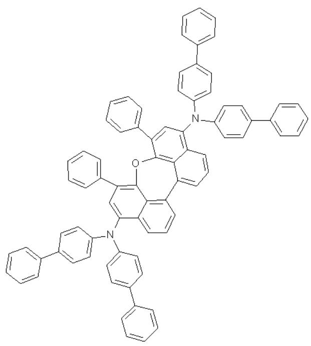

formula (A-113)

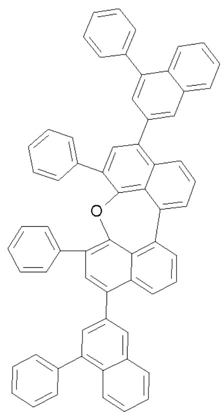

formula (A-114)

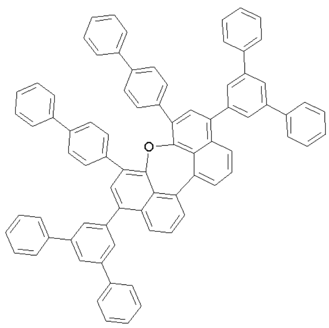

formula (A-115)

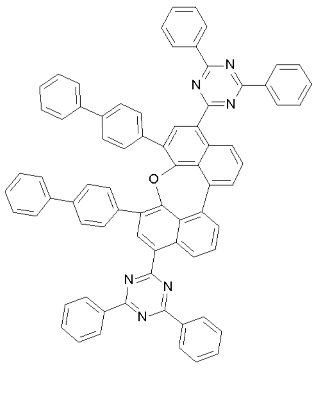

formula (A-116)

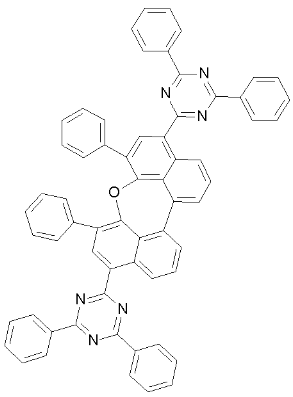

formula (A-117)

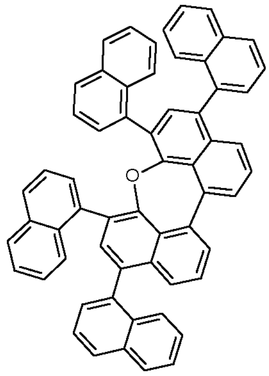

formula (A-118)

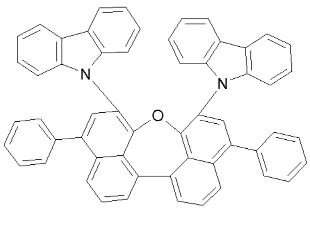

formula (A-119)

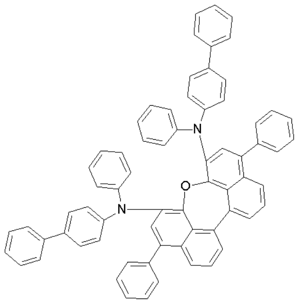

formula (A-120)

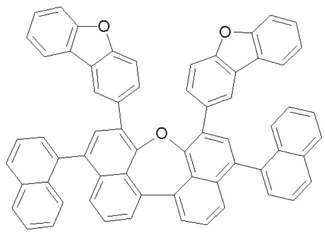

formula (A-121)

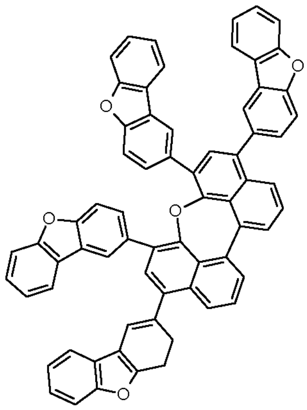

formula (A-122)

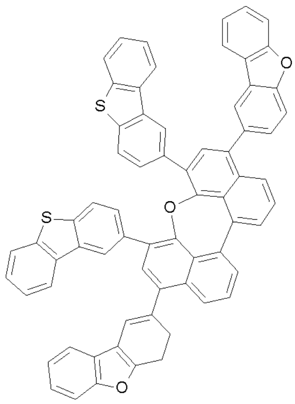

formula (A-123)

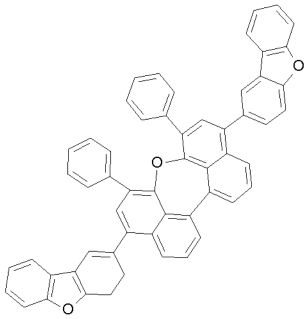

formula (A-124)

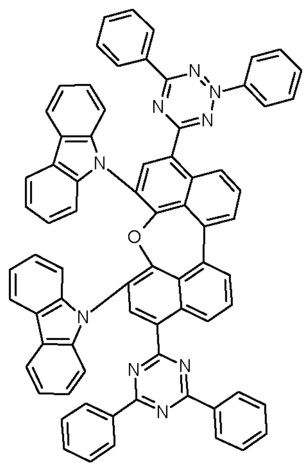

formula (A-125)

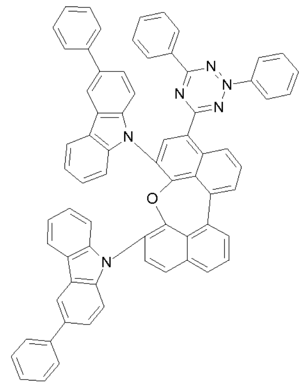

formula (A-126)

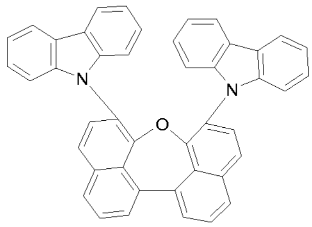

formula (A-127)

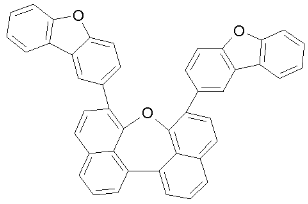

formula (A-128)

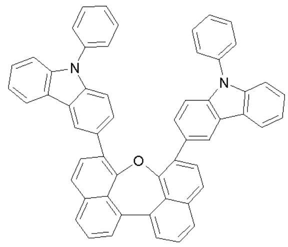

formula (A-129)

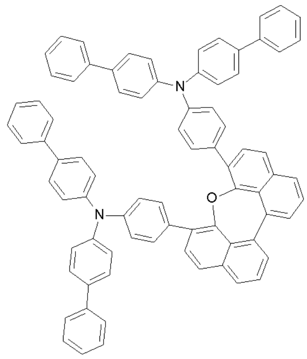

formula (A-130)

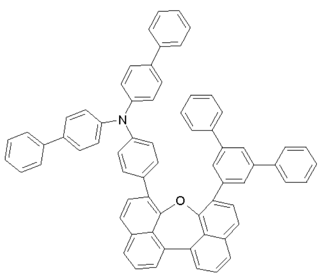

formula (A-131)

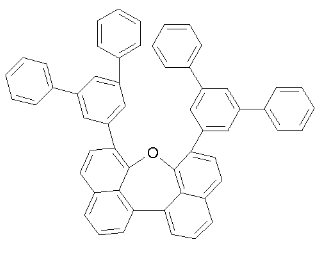

formula (A-132)

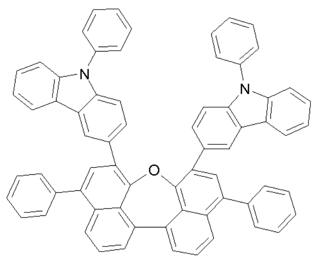

formula (A-133)

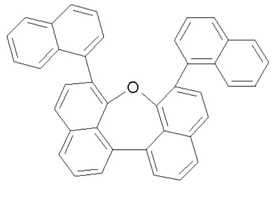

formula (A-134)

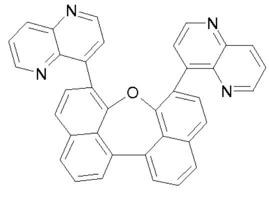

formula (A-135)

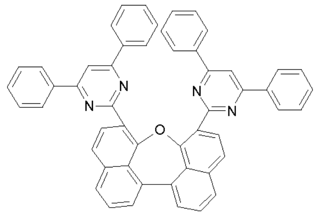

formula (A-136)

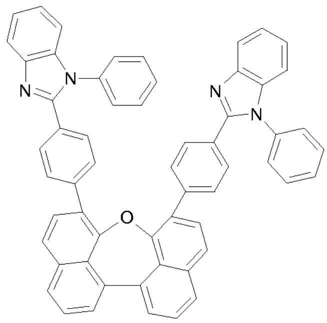

formula (A-137)

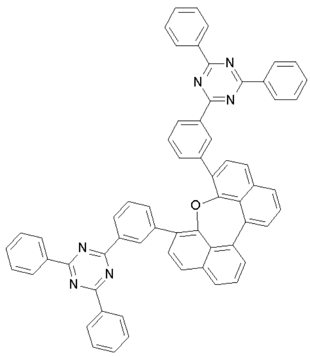

formula (A-138)

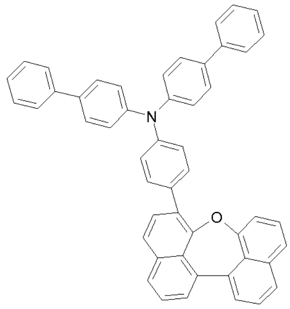

formula (A-139)

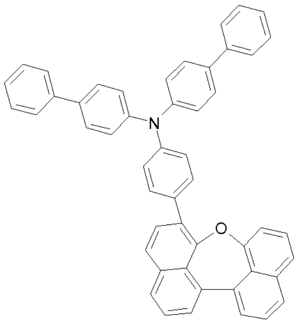

formula (A-140)

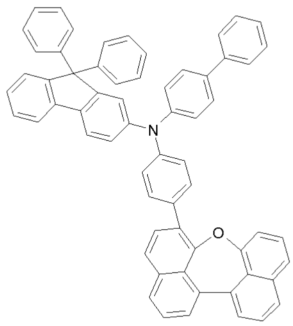

formula (A-141)

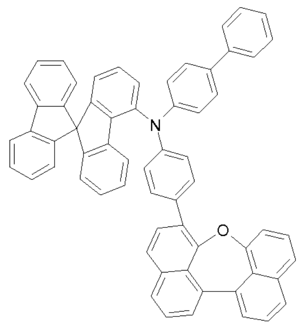

formula (A-142)

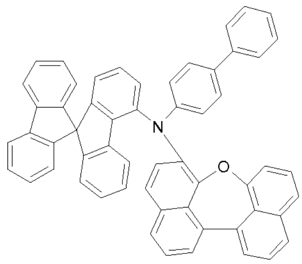

formula (A-143)

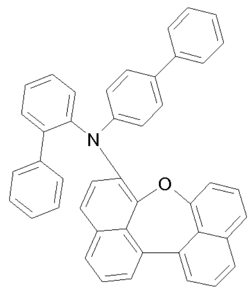

formula (A-144)

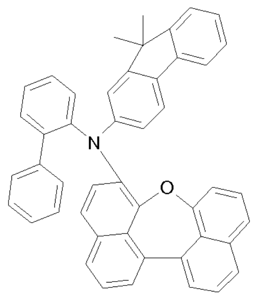

formula (A-145)

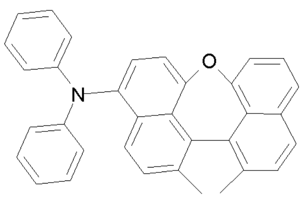

formula (A-146)

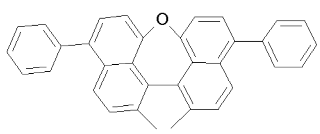

formula (A-147)

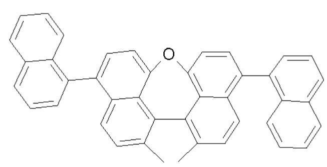

formula (A-148)

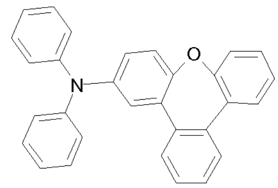

formula (A-149)

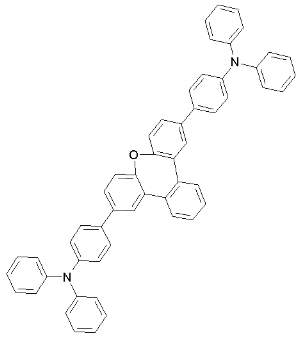

formula (A-149a)

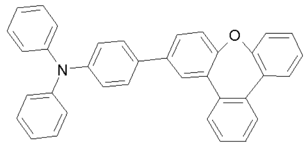

formula (A-149b)

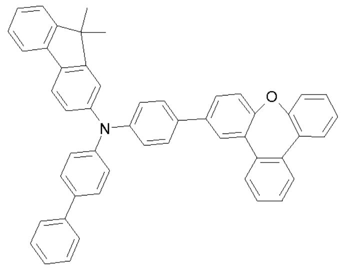

formula (A-150)

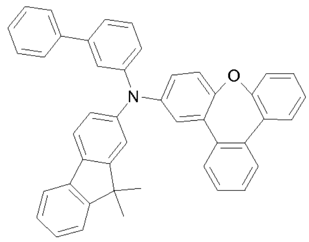

formula (A-151)

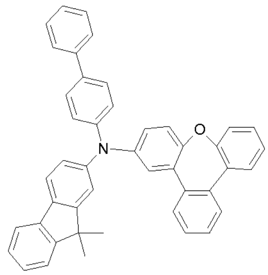

formula (A-152)

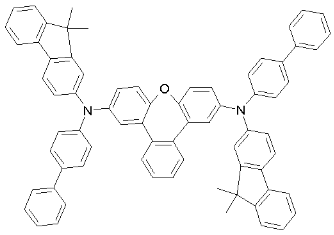

formula (A-153)

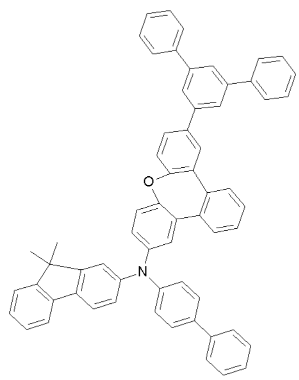

formula (A-154)

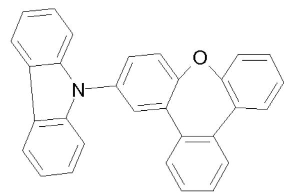

formula (A-155)

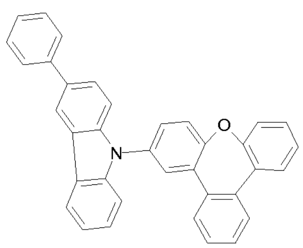

formula (A-156)

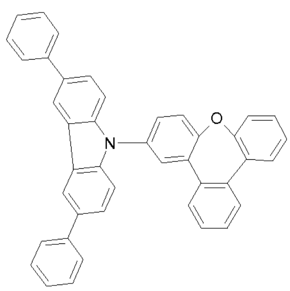

formula (A-157)

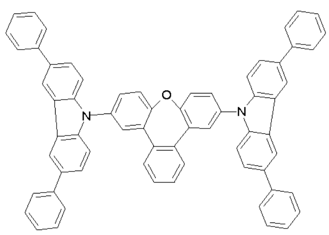

formula (A-158)

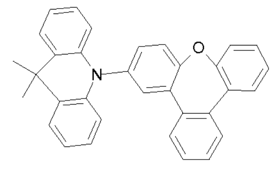

formula (A-159)

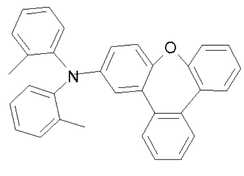

formula (A-160)

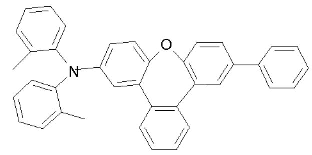

formula (A-161)

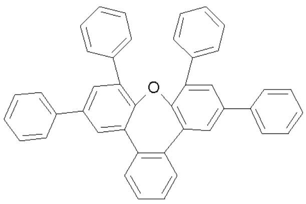

formula (A-162)

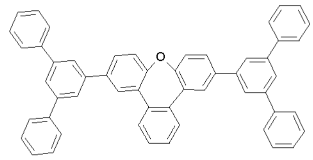

formula (A-163)

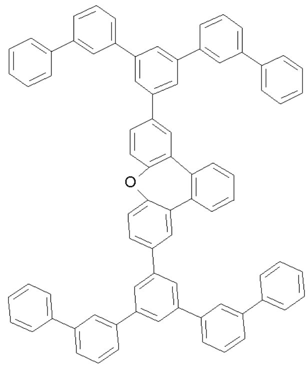

formula (A-164)

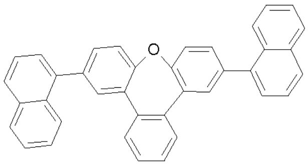

formula (A-165)

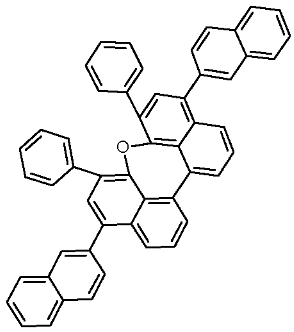

formula (A-166)

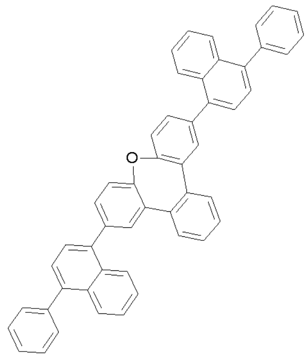

formula (A-167)

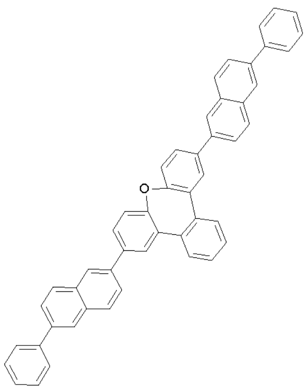

formula (A-168)

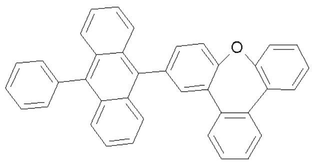

formula (A-169)

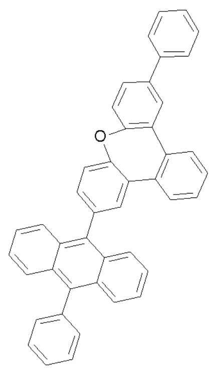

formula (A-170)

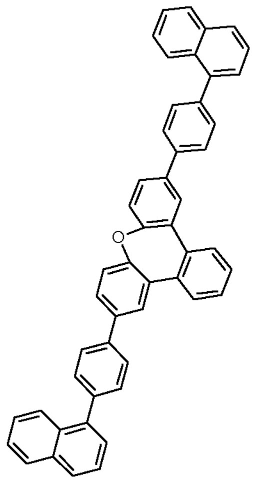

formula (A-171)

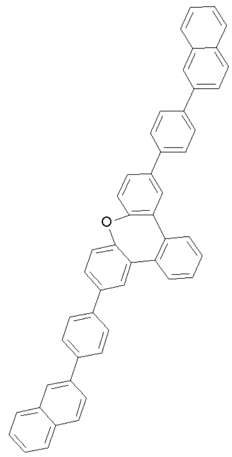

formula (A-172)

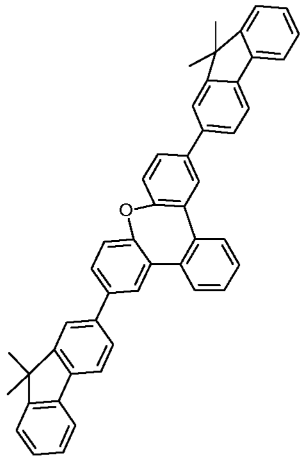

formula (A-174)

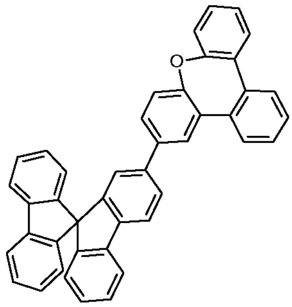

formula (A-175)

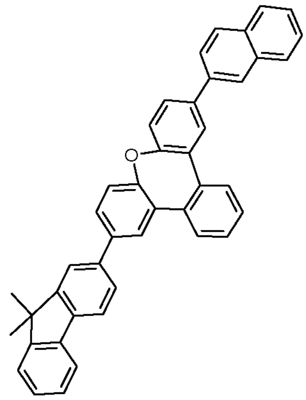

formula (A-176)

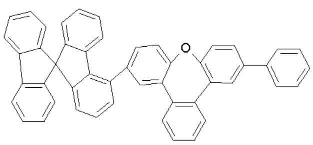

formula (A-177)

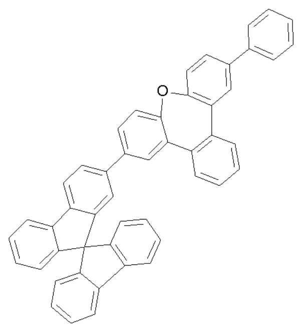

formula (A-178)

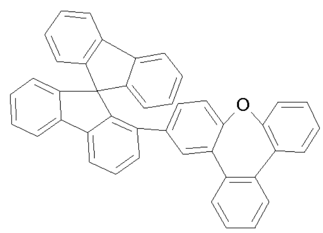

formula (A-179)

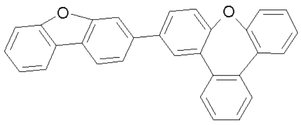
formula (A-180)

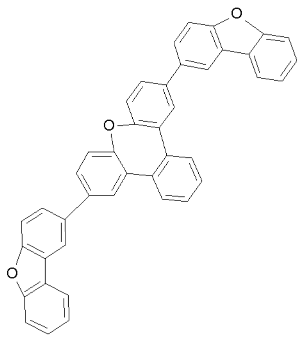
formula (A-181)

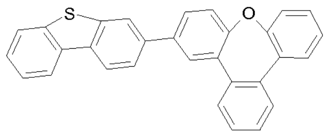
formula (A-182)

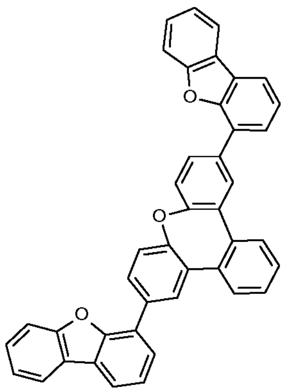
formula (A-183)

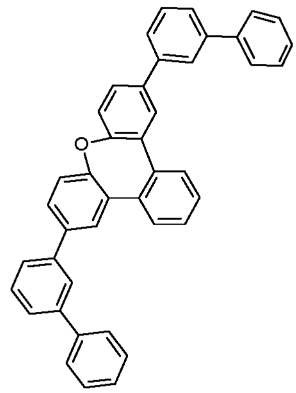
formula (A-184)

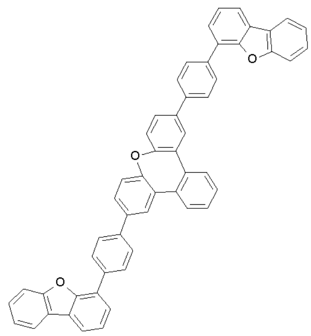
formula (A-185)

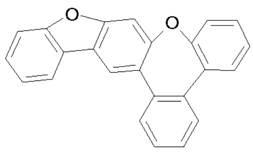
formula (A-187)

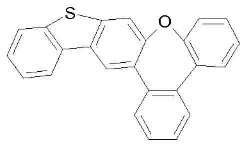
formula (A-188)

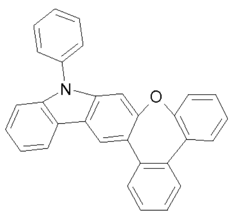
formula (A-190)

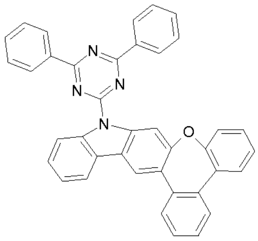
formula (A-193)

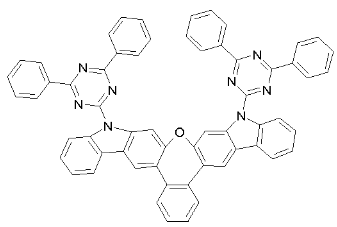
formula (A-194)

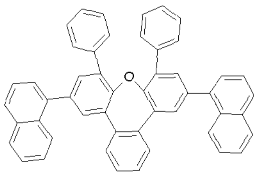

formula (A-195)

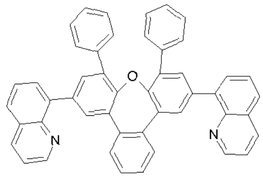

formula (A-196)

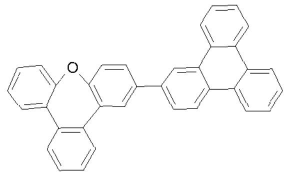

formula (A-197)

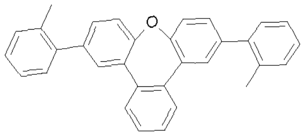

formula (A-198)

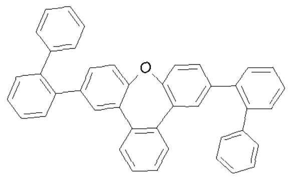

formula (A-199)

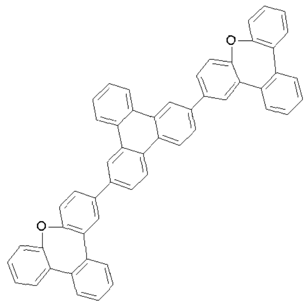

formula (A-200)

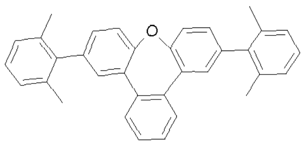

formula (A-201)

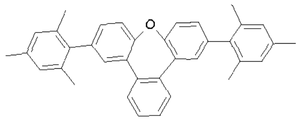

formula (A-202)

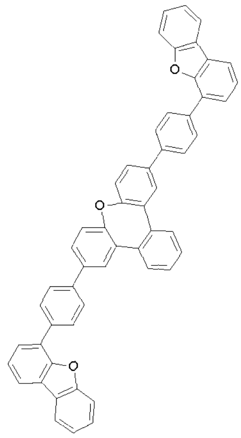

formula (A-203)

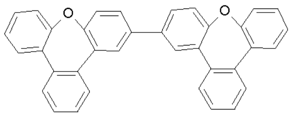

formula (A-204)

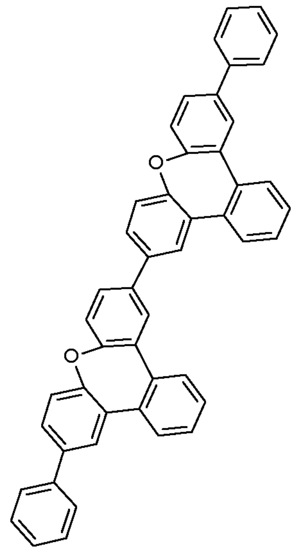

formula (A-205)

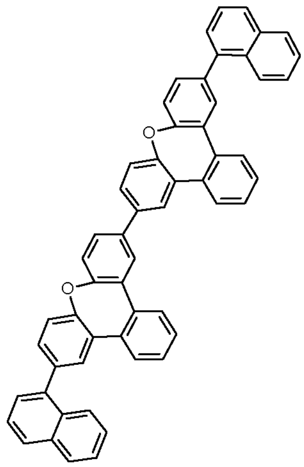

formula (A-206)

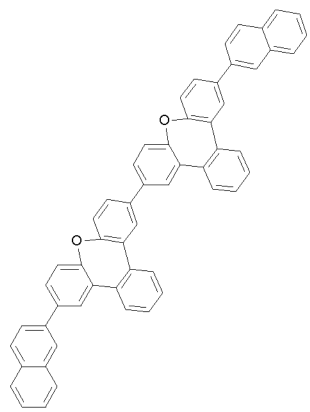

formula (A-207)

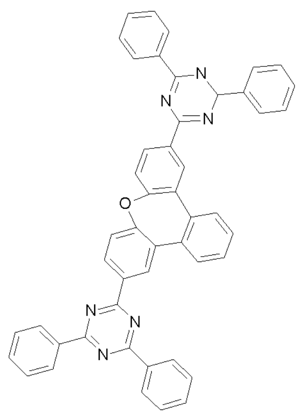

formula (A-208)

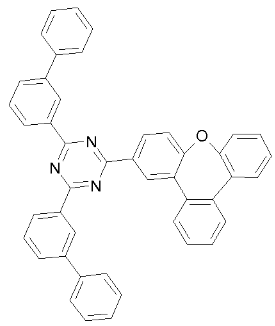

formula (A-209)

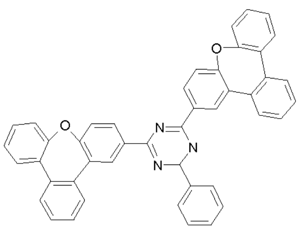

formula (A-210)

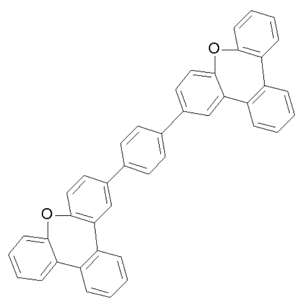

formula (A-211)

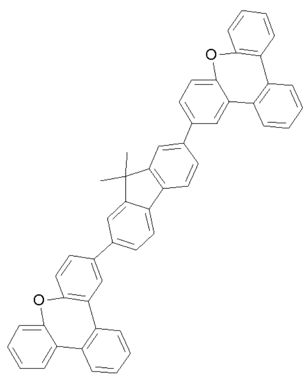

formula (A-212)

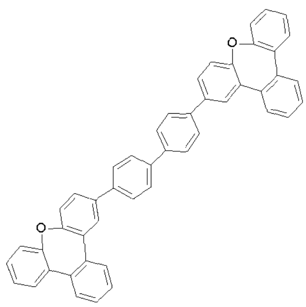

formula (A-213)

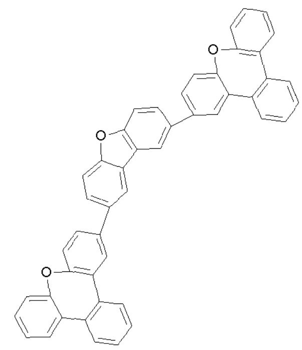

formula (A-214)

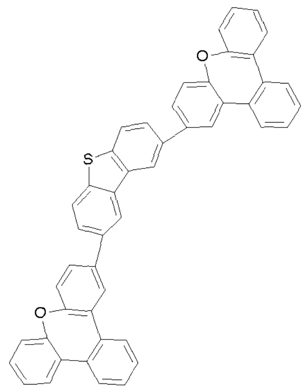

formula (A-215)

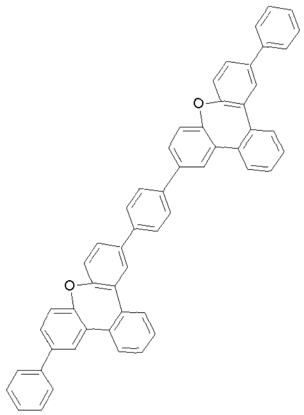

formula (A-216)

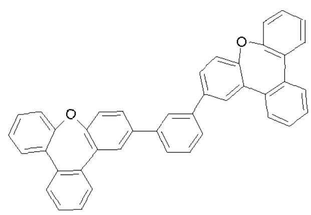

formula (A-217)

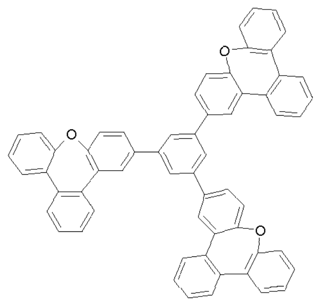

formula (A-218)

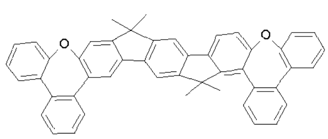

formula (A-219)

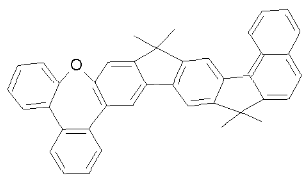

formula (A-220)

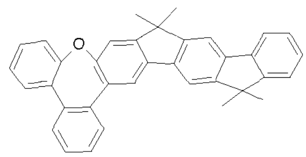

formula (A-221)

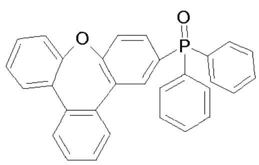

formula (A-222)

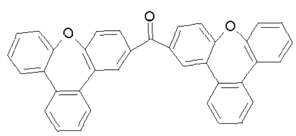

formula (A-223)

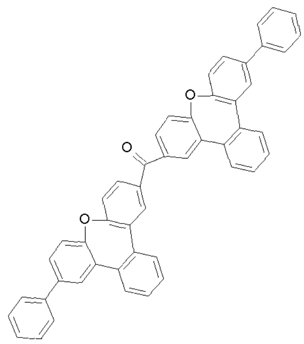

formula (A-224)

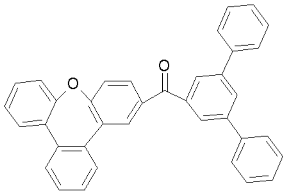
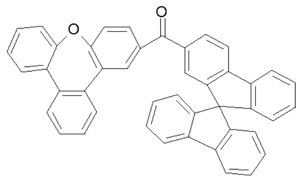
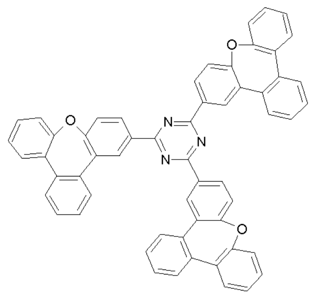

formula (A-225) formula (A-226) formula (A-227)

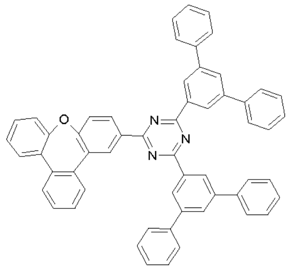
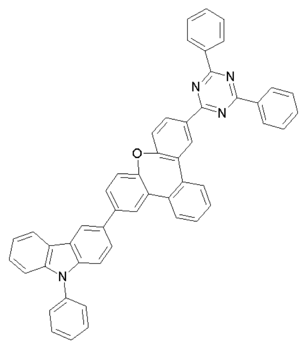
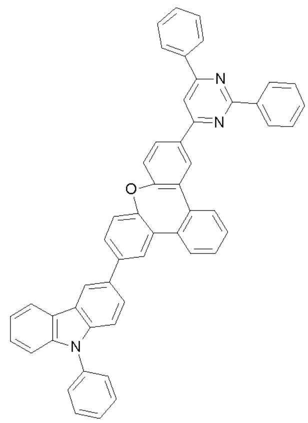

formula (A-228) formula (A-229) formula (A-230)

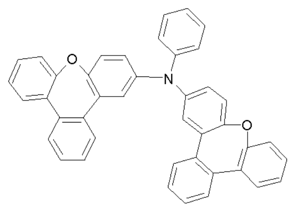
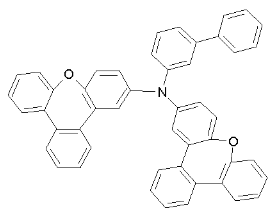
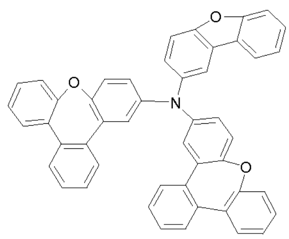

formula (A-231) formula (A-232) formula (A-233)

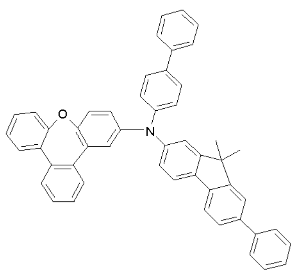
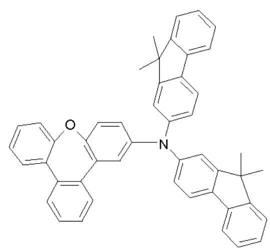
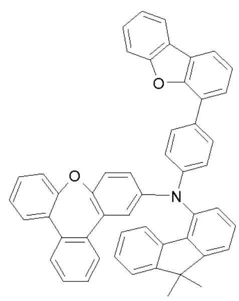

formula (A-234) formula (A-235) formula (A-236)

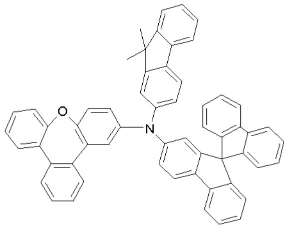
formula (A-237)

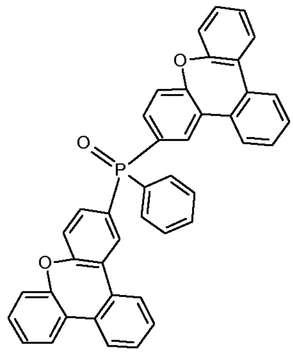
formula (A-238)

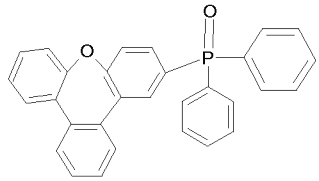
formula (A-239)

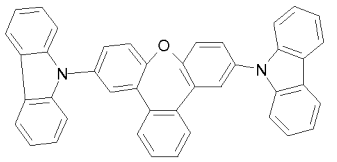
formula (A-240)

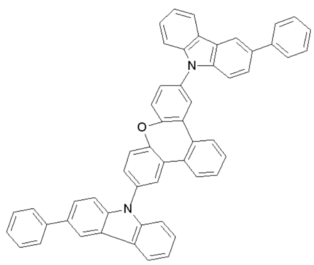
formula (A-241)

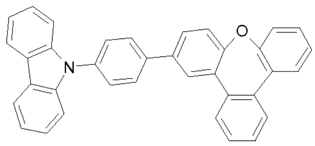
formula (A-242)

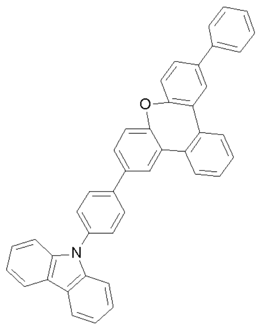
formula (A-243)

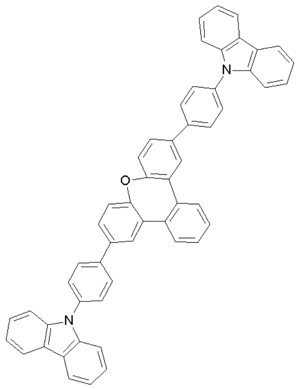
formula (A-244)

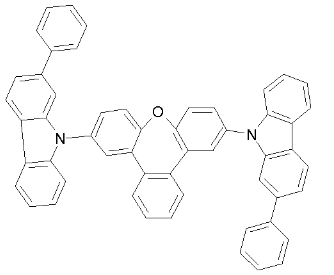
formula (A-245)

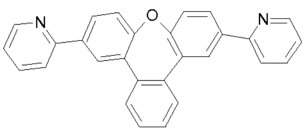
formula (A-246)

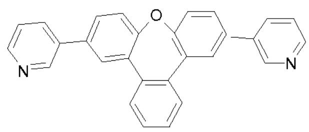
formula (A-247)

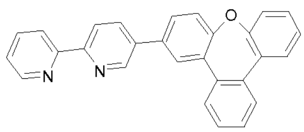
formula (A-248)

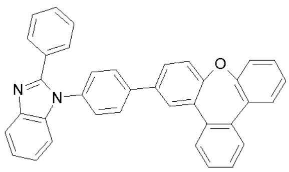
formula (A-249)

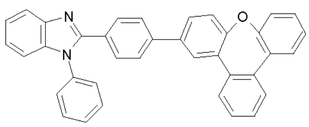
formula (A-250)

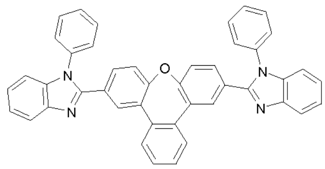
formula (A-251)

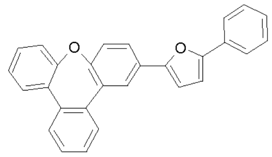

formula (A-252)

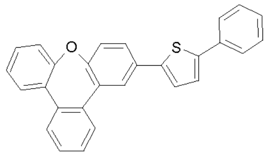

formula (A-253)

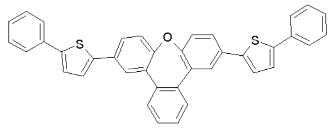

formula (A-254)

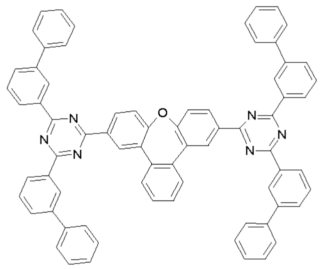

formula (A-255)

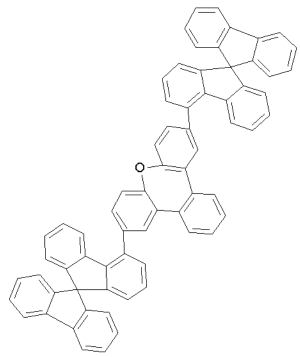

formula (A-256)

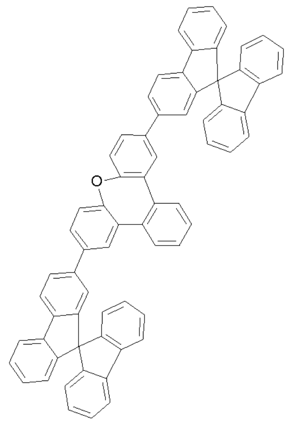

formula (A-257)

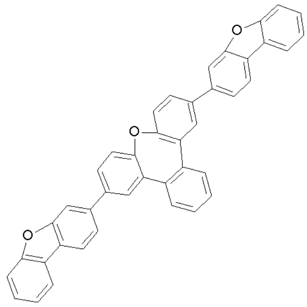

formula (A-258)

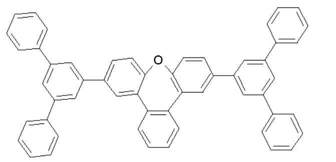

formula (A-259)

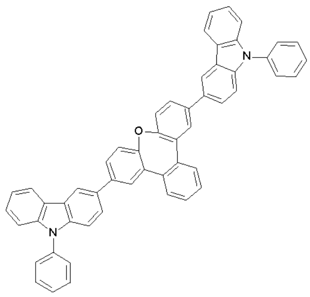

formula (A-260)

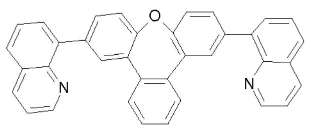

formula (A-261)

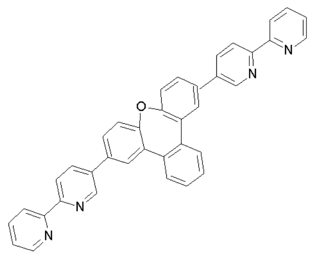

formula (A-262)

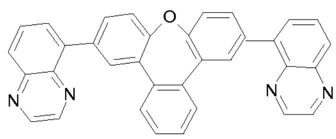

formula (A-263)

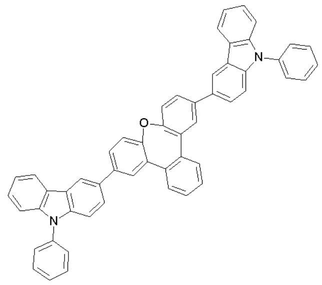
formula (A-264)

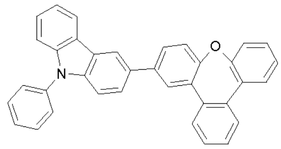
formula (A-265)

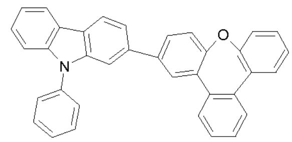
formula (A-266)

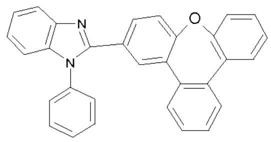
formula (A-267)

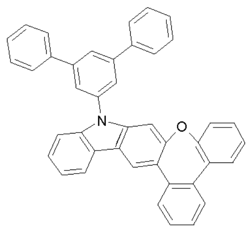
formula (A-268)

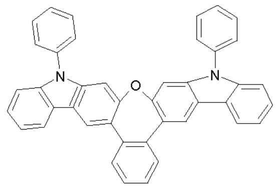
formula (A-269)

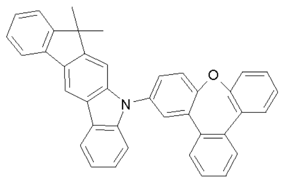
formula (A-270)

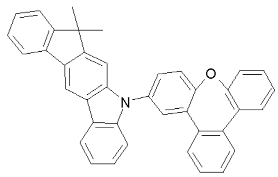
formula (A-271)

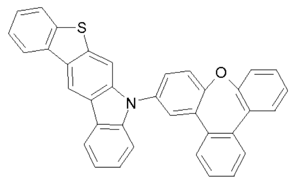
formula (A-272)

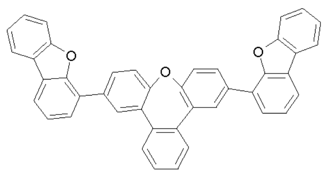
formula (A-273)

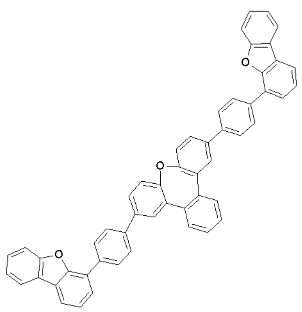
formula (A-274)

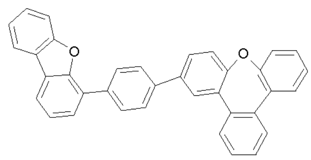
formula (A-275)

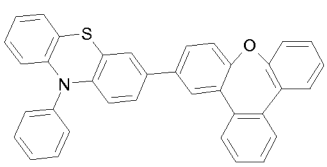
formula (A-276)

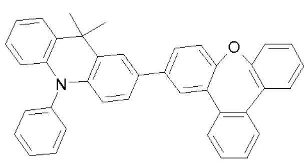
formula (A-277)

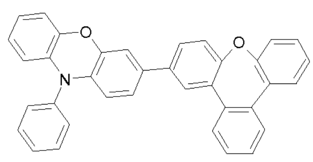
formula (A-278)

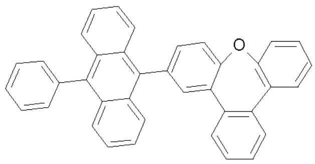

formula (A-279)

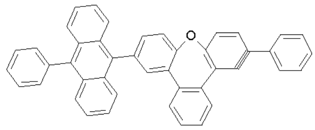

formula (A-280)

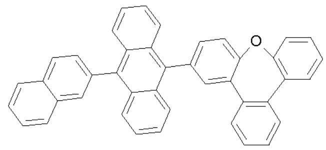

formula (A-281)

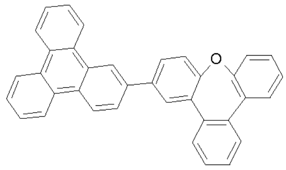

formula (A-282)

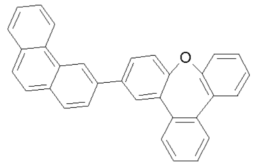

formula (A-283)

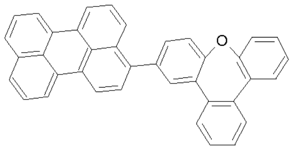

formula (A-284)

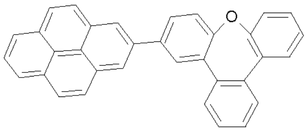

formula (A-285)

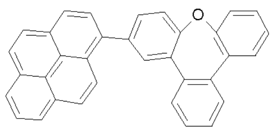

formula (A-286)

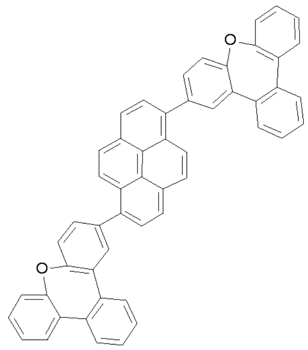

formula (A-287)

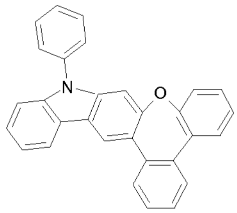

formula (A-288)

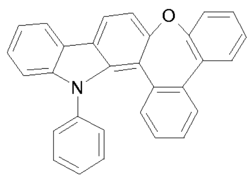

formula (A-289)

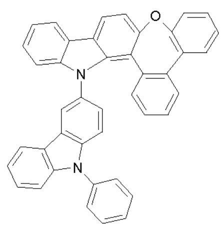

formula (A-290)

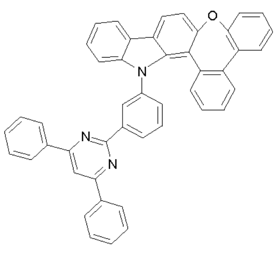

formula (A-291)

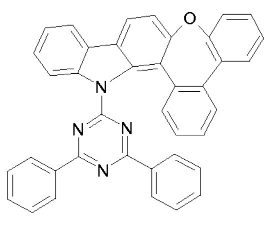

formula (A-292)

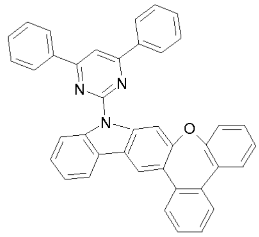

formula (A-293)

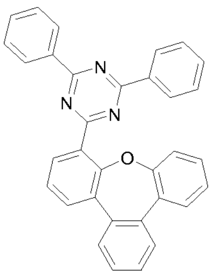

INV-5

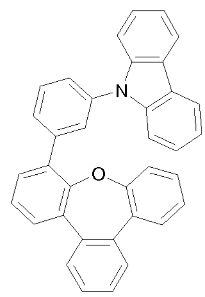

INV-6

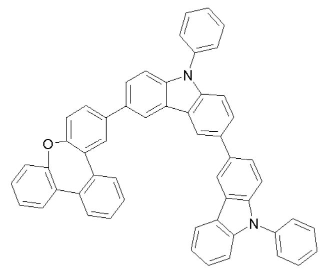

INV-7

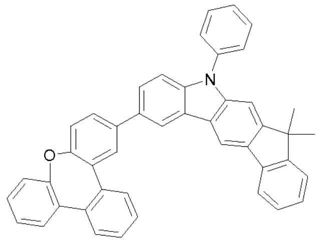

INV-8

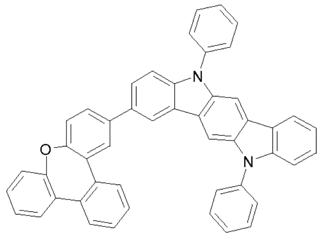

INV-9

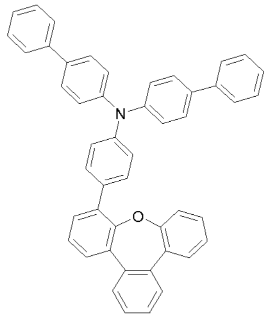

INV-10

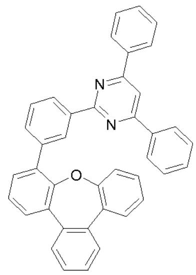

INV-11

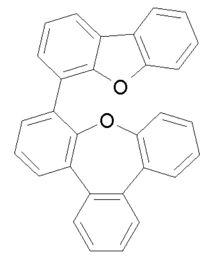

INV-12

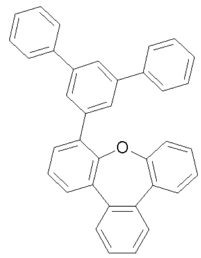

INV-13

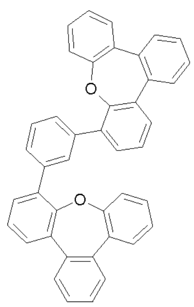

INV-14

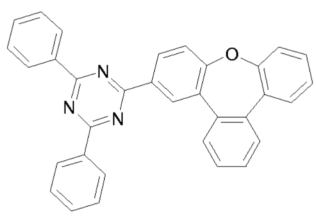

INV-15

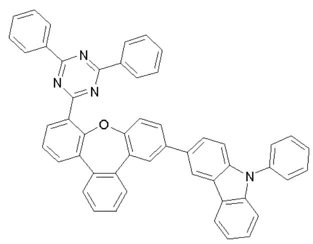

INV-19

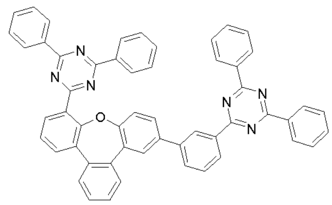

INV-20

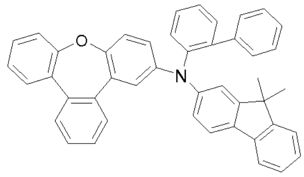

INV-21

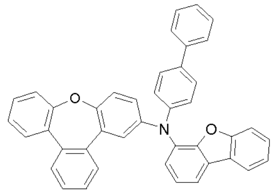

INV-22

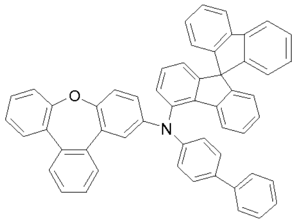

INV-23

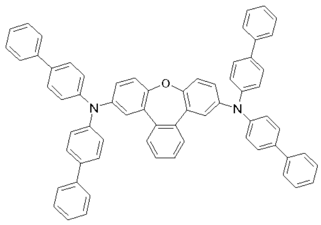

INV-24

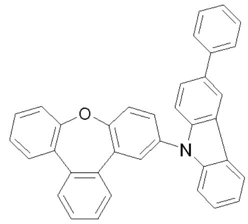

INV-25

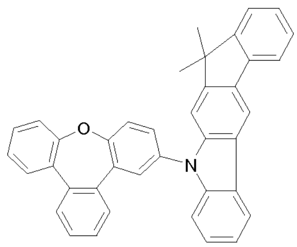

INV-26

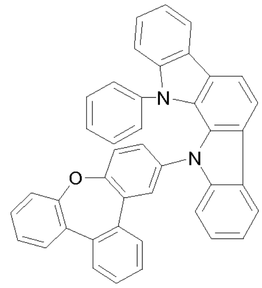

INV-27

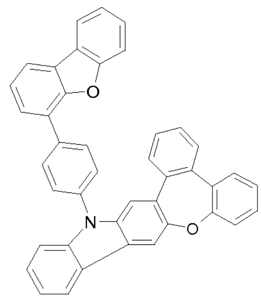

INV-28.

18. Process for the preparation of the compound according to one or more of Claims 13 to 17 with the aid of Suzuki coupling.

19. Process for the preparation of the compound according to one or more of Claims 13 to 17 with the aid of Buchwald or Ullmann coupling.

## Revendications

1. Dispositif électronique comprenant au moins un composé de la formule générale (5) ou (7) :

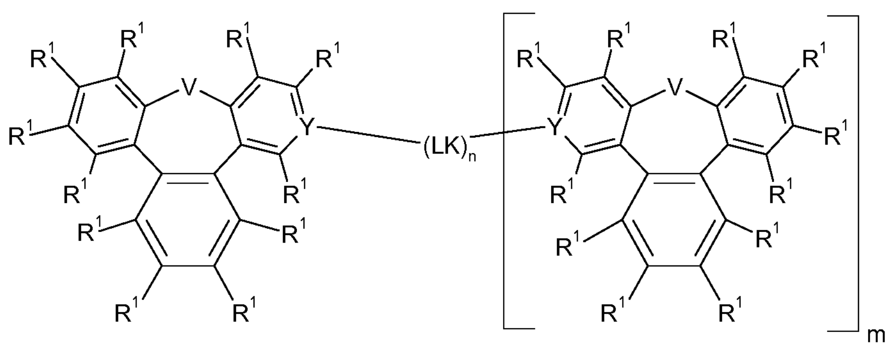

formule (5)

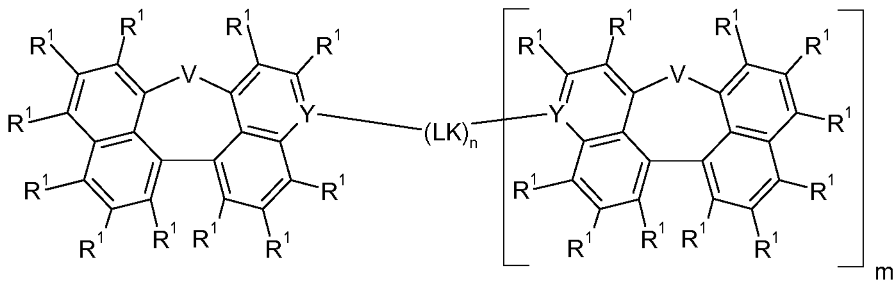

formule (7)

dans lesquelles ce qui suit s'applique aux symboles et aux indices :

V est soit O, soit S et de façon très préférable O ;
X est, de manière identique ou différente pour chaque occurrence, N ou $CR^1$, de préférence $CR^1$ ;
m est 0 (monomère), 1 (dimère) ou 2 (trimère) ;
n est 0 ou 1, où :

n = 0 pour m = 0 et
n = 1 pour m = 1 ou pour m = 2 ;

Y est égal à un atome de carbone $sp^2$-hydridé pour m non égal à 0 ou la même chose que X pour m = 0 ;
LK est, dans le cas dans lequel m = 1, une liaison simple ou un coupleur bifonctionnel ; LK peut être substitué par un radical ou par plusieurs radicaux $R^1$, où les radicaux $R^1$ peuvent être identiques ou différents pour chaque occurrence ;
est, pour m = 2, un coupleur trifonctionnel, où le coupleur peut être substitué par un radical ou par plusieurs radicaux $R^1$, où les radicaux $R^1$ peuvent être identiques ou différents pour chaque occurrence ;
est, si n = 0, non présent, de sorte qu'un monomère est présent ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux de ces groupes ou plus ou un groupe réticulable Q ; deux radicaux $R^1$ adjacents ou plus peuvent former un système de cycle aliphatique ou aromatique ou hétéroaromatique mono- ou polycyclique l'un avec l'autre ou les uns avec les autres, lequel peut être substitué par un radical ou par plusieurs radicaux $R^2$ ;
$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(_{R^3})$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalkoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe

arylhétéroarylamino qui comporte de 10 à 40 atomes de cycle aromatique, lequel peut être substitué par un radical ou par plusieurs radicaux $R^3$, ou une combinaison de deux de ces groupes ou plus ; deux radicaux $R^2$ adjacents ou plus peuvent former un système de cycle aliphatique ou aromatique ou hétéro-aromatique mono- ou polycyclique l'un avec l'autre ou les uns avec les autres ;

$R^3$ est, de manière identique ou différente pour chaque occurrence, H, D, F ou un radical hydrocarbone aliphatique, aromatique et/ou hétéroaromatique qui comporte de 1 à 40 atome(s) de C, où, en outre, un ou plusieurs atome(s) de H peut/peuvent être remplacé(s) par F ; deux substituants $R^3$ ou plus peuvent également former un système de cycle aliphatique ou aromatique ou hétéro-aromatique mono- ou polycyclique l'un avec l'autre ou les uns avec les autres ;

étant entendu que, si m = 0, au moins l'un des radicaux $R^1$ n'est pas égal à H.

**2.** Dispositif électronique selon la revendication 1, comprenant les composés de la formule (5) ou (7) dans une couche d'émission (EML), dans une couche de transport d'électrons (ETL) ou dans une couche de blocage de trous (HBL), de préférence dans une EML ou dans une ETL est de façon particulièrement préférable, dans une EML.

**3.** Dispositif électronique selon la revendication 1, **caractérisé en ce qu'**il est sélectionné parmi les circuits intégrés organiques (OIC), les transistors à effet de champ organiques (OFET), les transistors à film mince organiques (OTFT), les dispositifs électroluminescents organiques, les cellules solaires organiques (OSC), les détecteurs optiques organiques, les photorécepteurs organiques, de préférence parmi un dispositif électroluminescent organique.

**4.** Dispositif électronique selon la revendication 1 ou 3, **caractérisé en ce qu'**il s'agit d'un dispositif électroluminescent organique qui est également sélectionné parmi le groupe qui est constitué par les transistors à émission de lumière organiques (OLET), les dispositifs à extinction de champ organiques (OFQD), les cellules électrochimiques à émission de lumière organiques (OLEC, LEC, LEEC), les diodes laser organiques (O-laser) et les diodes à émission de lumière organiques (OLED), de préférence les OLEC et les OLED, de façon très préférable les OLED.

**5.** Procédé pour la fabrication d'un dispositif électronique selon une ou plusieurs des revendications 1 à 4, **caractérisés en ce qu'**au moins une couche organique est appliquée au moyen d'un dépôt en phase gazeuse ou à partir d'une solution.

**6.** Dispositif électronique selon la revendication 4 pour une utilisation en médecine pour la photothérapie, de préférence pour une utilisation pour la photothérapie de la peau, de façon très préférable pour une utilisation pour le traitement ou la prévention du psoriasis, de la dermatite atopique, de la jaunisse, de la jaunisse du nouveau-né, du vitiligo, de l'inflammation, de la douleur et pour une utilisation pour la cicatrisation des plaies.

**7.** Utilisation du dispositif selon la revendication 4 dans le domaine des cosmétiques, de préférence pour la réduction ou la prévention du vieillissement de la peau, des rides de la peau, des pattes d'oie, de l'acné, des comédons et de la cellulite.

**8.** Utilisation du dispositif selon la revendication 4 dans des affichages ou pour l'éclairage.

**9.** Composition comprenant au moins un composé de la formule générale (5) ou (7) :

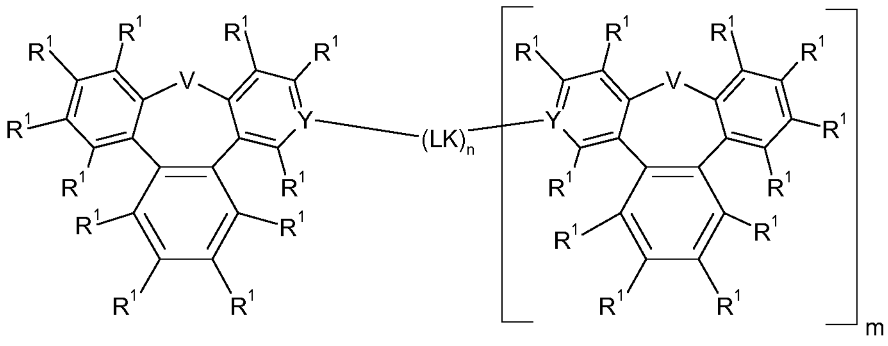

formule (5)

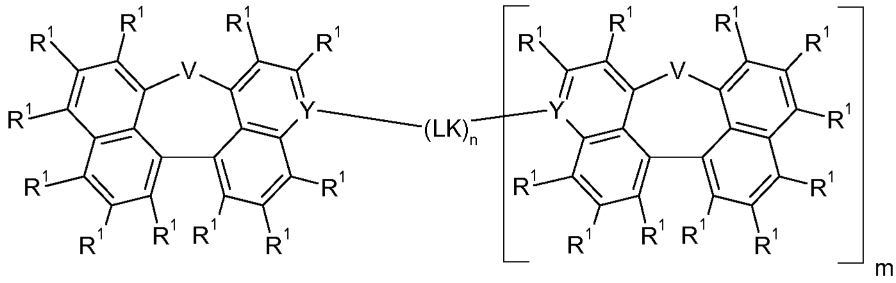

formule (7)

dans lesquelles ce qui suit s'applique aux symboles et aux indices :

V est soit O, soit S et de façon très préférable O ;
X est, de manière identique ou différente pour chaque occurrence, N ou $CR^1$, de préférence $CR^1$ ;
m est 0 (monomère), 1 (dimère) ou 2 (trimère) ;
n est 0 ou 1, où :

n = 0 pour m = 0 et
n = 1 pour m = 1 ou pour m = 2 ;

Y est égal à un atome de carbone $sp^2$-hydridisé pour m non égal à 0 ou la même chose que X pour m = 0 ;
LK est, dans le cas dans lequel m = 1, une liaison simple ou un coupleur bifonctionnel ; LK peut être substitué par un radical ou par plusieurs radicaux $R^1$, où les radicaux $R^1$ peuvent être identiques ou différents pour chaque occurrence ;
est, pour m = 2, un coupleur trifonctionnel, où le coupleur peut être substitué par un radical ou par plusieurs radicaux $R^1$, où les radicaux $R^1$ peuvent être identiques ou différents pour chaque occurrence ;
est, si n = 0, non présent, de sorte qu'un monomère est présent ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux de ces groupes ou plus ou un groupe réticulable Q ; deux radicaux adjacents $R^1$ ou plus peuvent former un système de cycle aliphatique ou aromatique ou hétéroaromatique mono- ou polycyclique l'un avec l'autre ou les uns avec les autres, lequel peut être substitué par un radical ou par plusieurs radicaux $R^2$ ;
$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(_{R}{}^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino, un groupe

arylhétéroarylamino qui comporte de 10 à 40 atomes de cycle aromatique, lequel peut être substitué par un radical ou par plusieurs radicaux $R^3$, ou une combinaison de deux de ces groupes ou plus ; deux radicaux $R^2$ adjacents ou plus peuvent former un système de cycle aliphatique ou aromatique ou hétéroaromatique mono- ou polycyclique l'un avec l'autre ou les uns avec les autres ;

$R^3$ est, de manière identique ou différente pour chaque occurrence, H, D, F ou un radical hydrocarbone aliphatique, aromatique et/ou hétéroaromatique qui comporte de 1 à 40 atome(s) de C, où, en outre, un ou plusieurs atome(s) de H peut/peuvent être remplacé(s) par F ; deux substituants $R^3$ ou plus peuvent également former un système de cycle aliphatique ou aromatique ou hétéroaromatique mono- ou polycyclique l'un avec l'autre ou les uns avec les autres ;

étant entendu que, si m = 0, au moins l'un des radicaux $R^1$ n'est pas égal à H,
et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous, les dopants n ou les dopants p.

10. Composition selon la revendication 9, **caractérisée en ce que** le composé additionnel est un émetteur phosphorescent.

11. Composition selon la revendication 9 ou 10, **caractérisée en ce que** le composé additionnel est un matériau hôte ou un matériau de matrice.

12. Composition selon une ou plusieurs des revendications 9 à 11, **caractérisée en ce que** le composé additionnel présente une bande interdite de 2,5 eV ou plus, de préférence de 3,0 eV ou plus, de façon très préférable de 3,5 eV ou plus.

13. Composé de la formule générale (5) :

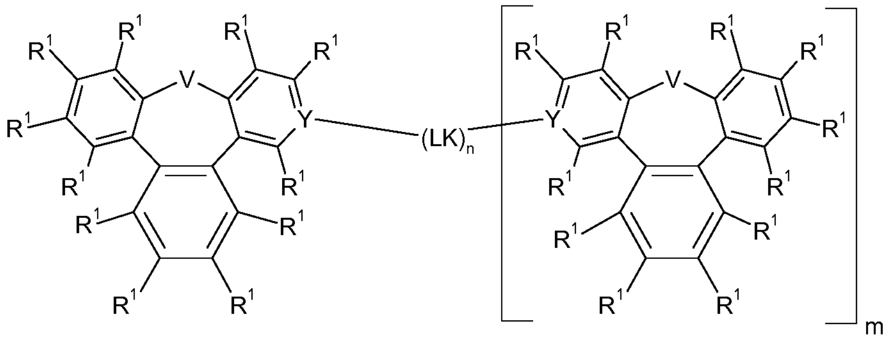

formule (5)

dans laquelle ce qui suit s'applique aux symboles et aux indices :

V est O ;
m est 0 (monomère), 1 (dimère) ou 2 (trimère) ;
n est 0 ou 1, où
n = 0 pour m = 0 et
n = 1 pour m = 1 ou pour m = 2 ;
Y est égal à un atome de carbone $sp^2$-hydridisé pour m non égal à 0 ou la même chose que X pour m = 0 ;
X est, de manière identique ou différente pour chaque occurrence, $CR^1$ ;
LK est, dans le cas dans lequel m = 1, une liaison simple ou un coupleur bifonctionnel ; LK peut être substitué par un radical ou par plusieurs radicaux $R^1$, où les radicaux peuvent être identiques ou différents pour chaque occurrence ;
est, pour m = 2, un coupleur trifonctionnel, où le coupleur peut être substitué par un radical ou par plusieurs radicaux $R^1$, où les radicaux $R^1$ peuvent être identiques ou différents pour chaque occurrence ;
est, si n = 0, non présent, de sorte qu'un monomère est présent ;
$R^1$ est, de manière identique ou différente pour chaque occurrence, H, D, F, I, $N(R^2)_2$, CN, $NO_2$, $Si(R^2)_3$, $B(OR^2)_2$,

$C(=O)R^2$, $P(=O)(R^2)_2$, $S(=O)R^2$, $S(=O)_2R^2$, $OSO_2R^2$, un groupe alcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupes $CH_2$ non adjacents peut/peuvent être remplacé(s) par $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O ou $CONR^2$ et où un ou plusieurs atome(s) de H peut/ peuvent être remplacé(s) par D, F, I, CN ou $NO_2$,
ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalkoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux ou plus de ces groupes ; deux radicaux $R^1$ adjacents ou plus peuvent former un système de cycle aliphatique ou aromatique ou hétéroaromatique mono- ou polycyclique l'un avec l'autre ou les uns avec les autres, lequel peut être substitué par un radical ou par plusieurs radicaux, $R^2$ ;
$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, $C\equiv C$, $Si(R^3)_2$, $Ge(R^3)_2$, $Sn(R^3)_2$, C=O, C=S, C=Se, $C=NR^3$, $P(=O)(_{R}{}^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un systèmes de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux ou plus de ces groupes ; deux radicaux $R^2$ adjacents ou plus peuvent former un système de cycle aliphatique ou aromatique ou hétéroaromatique mono- ou polycyclique l'un avec l'autre ou les uns avec les autres ;
$R^3$ est, de manière identique ou différente pour chaque occurrence, H, D, F ou un radical hydrocarbone aliphatique, aromatique et/ou hétéroaromatique qui comporte de 1 à 40 atome(s) de C, où, en outre, un ou plusieurs atome(s) de H peut/peuvent être remplacé(s) par F ; deux substituants $R^3$ ou plus peuvent également former l'un avec l'autre ou les uns avec les autres un système de cycle aliphatique ou aromatique ou hétéroaromatique mono- ou polycyclique ;

étant entendu que, si m = 0, au moins l'un des radicaux $R^1$ n'est pas égal à H.

14. Composé selon la revendication 13, **caractérisé en ce qu'**il présente la formule qui suit (10a), (10b), (10c) ou (10d) :

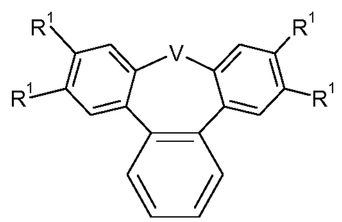

formule (10a)

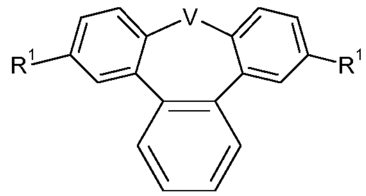

formule (10b)

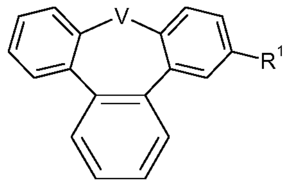

Formule (10c)

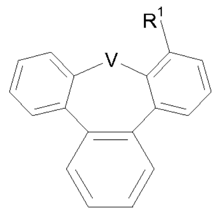

formule (10d).

**15.** Composé de la formule générale (7) :

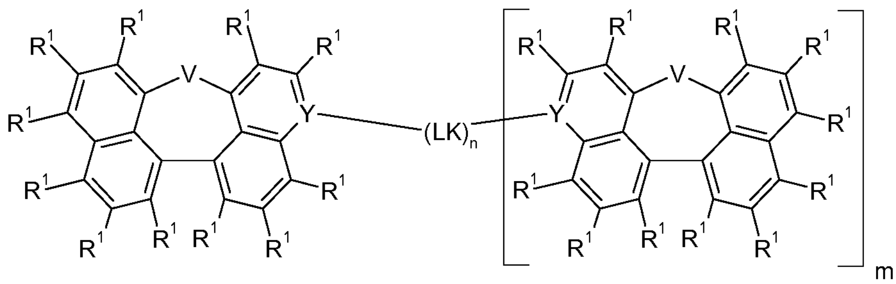

formule (7)

dans laquelle ce qui suit s'applique aux symboles et aux indices :

V est O ;
X est, de manière identique ou différente pour chaque occurrence, $CR^1$ ;
m est 0 (monomère), 1 (dimère) ou 2 (trimère) ;
n est 0 ou 1, où :

n = 0 pour m = 0 et
n = 1 pour m = 1 ou pour m = 2;

Y est égal à un atome de carbone $sp^2$-hydridisé pour m non égal à 0 ou la même chose que X pour m = 0 ;
LK est, dans le cas dans lequel m = 1, une liaison simple ou un coupleur bifonctionnel ; LK peut être substitué par un radical ou par plusieurs radicaux $R^1$, où les radicaux $R^1$ peuvent être identiques ou différents pour chaque occurrence ;
est, pour m = 2, un coupleur trifonctionnel, où le coupleur peut être substitué par un radical ou par plusieurs

radicaux $R^1$, où les radicaux $R^1$ peuvent être identiques ou différents pour chaque occurrence ;
est, si n = 0, non présent, de sorte qu'un monomère est présent ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux de ces groupes ou plus ou un groupe réticulable Q ; deux radicaux $R^1$ adjacents ou plus peuvent former l'un avec l'autre ou les uns avec les autres un système de cycle aliphatique ou aromatique ou hétéroaromatique mono- ou polycyclique, lequel peut être substitué par un radical ou par plusieurs radicaux $R^2$ ;
$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO2R^3$, un groupe alkyle, alcoxy ou thioalcoxy en chaîne droite qui comporte de 1 à 40 atome(s) de C ou un groupe alkényle ou alkynyle qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalkoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux de ces groupes ou plus ; deux radicaux $R^2$ adjacents ou plus peuvent former l'un avec l'autre ou les uns avec les autres un système de cycle aliphatique ou aromatique ou hétéroaromatique mono- ou polycyclique ;
$R^3$ est, de manière identique ou différente pour chaque occurrence, H, D, F ou un radical hydrocarbone aliphatique, aromatique et/ou hétéroaromatique qui comporte de 1 à 40 atome(s) de C, où, en outre, un ou plusieurs atome(s) de H peut/peuvent être remplacé(s) par F ; deux substituants $R^3$ ou plus peuvent également former l'un avec l'autre ou les uns avec les autres un système de cycle aliphatique ou aromatique ou hétéroaromatique mono- ou polycyclique ;

étant entendu que, si m = 0, au moins l'un des radicaux $R^1$ n'est pas égal à H.

**16.** Composé selon la revendication 15, **caractérisé en ce qu'**il présente la formule qui suit (14), (15), (16), (17), (18) ou (19) :

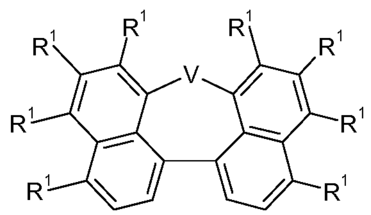

formule (14)

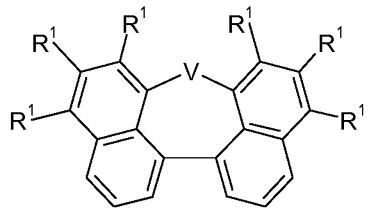

formule (15)

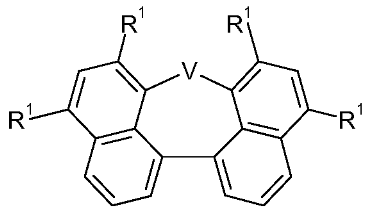

formule (16)

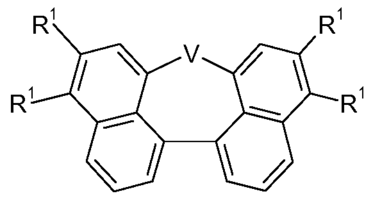

formule (17)

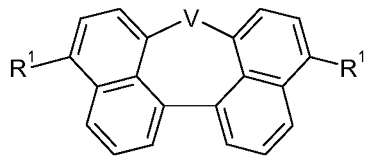

formule (18)

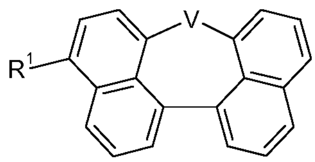

formule (19).

**17.** Composés selon l'une des revendications 13 à 16, sélectionnés parmi :

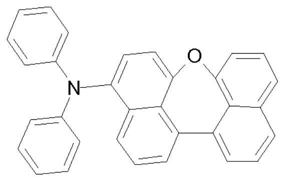

formule (A-1)

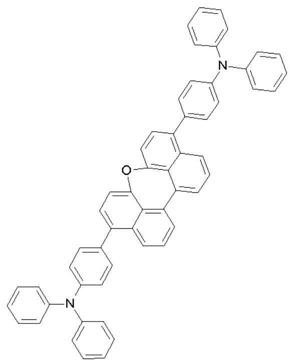

formule (A-2)

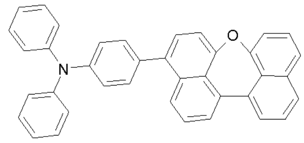

formule (A-3)

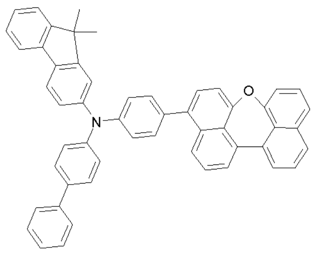

formule (A-4)

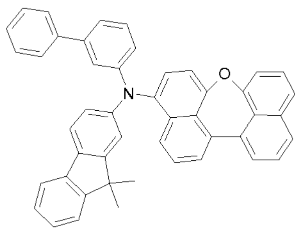

formule (A-5)

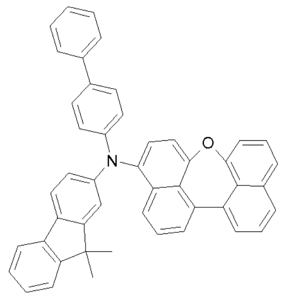

formule (A-6)

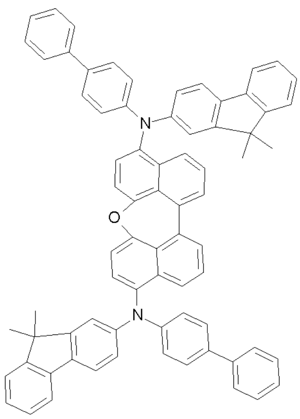

formule (A-7)

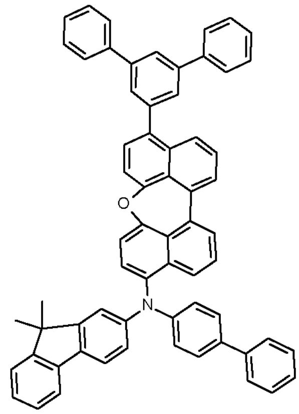

formule (A-8)

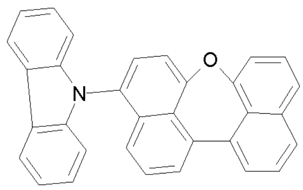

formule (A-9)

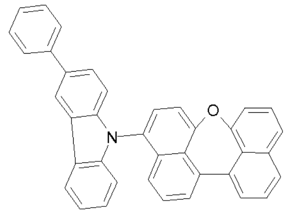

formule (A-10)

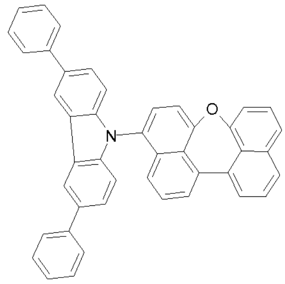

formule (A-11)

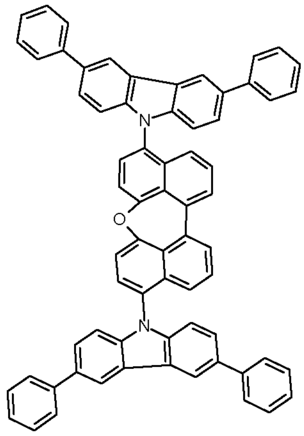

formule (A-12)

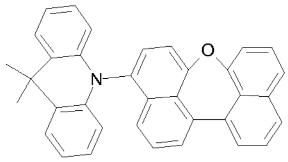

formule (A-13)

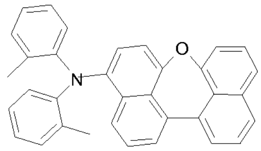

formule (A-14)

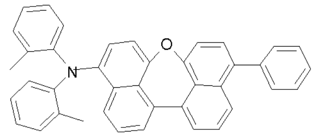

formule (A-15)

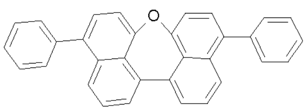

formule (A-17)

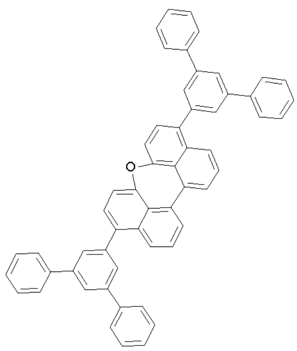

formule (A-17)

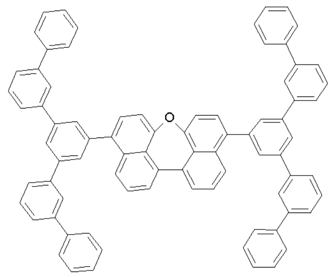

formule (A-18)

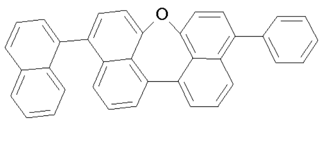

formule (A-19)

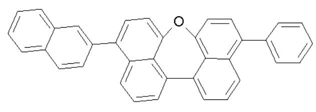

formule (A-20)

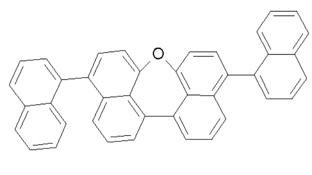

formule (A-21)

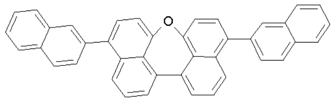

formule (A-22)

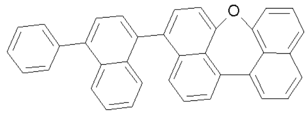

formule (A-23)

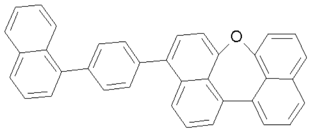

formule (A-24)

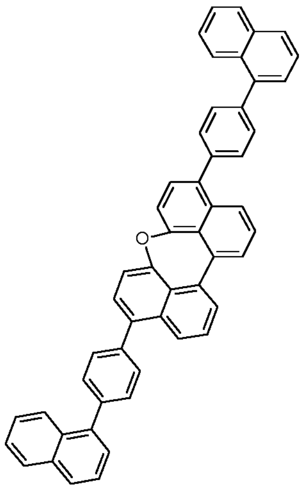

formule (A-25)

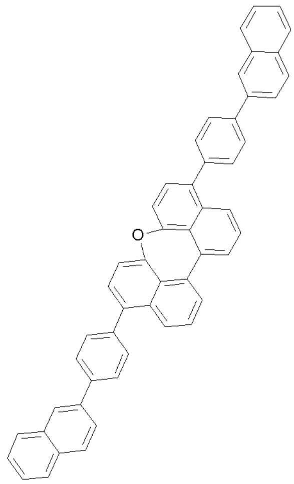

formule (A-26)

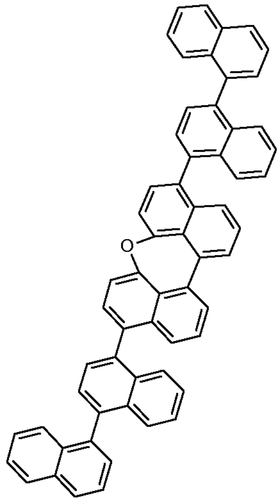

formule (A-27)

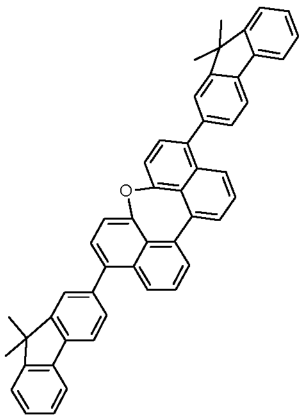

formule (A-28)

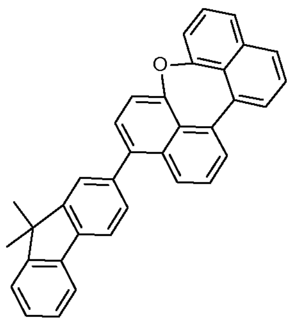

formule (A-29)

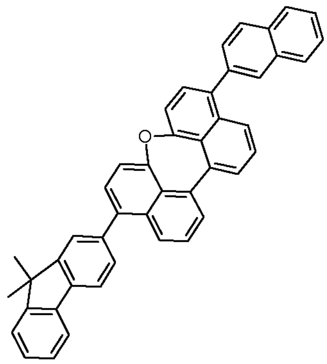

formule (A-30)

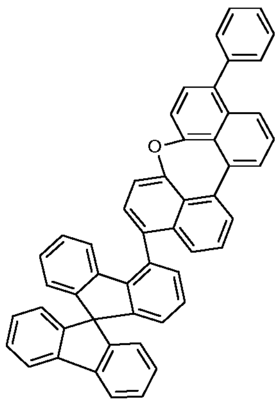

formule (A-31)

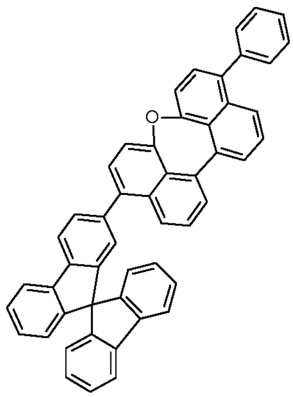

formule (A-32)

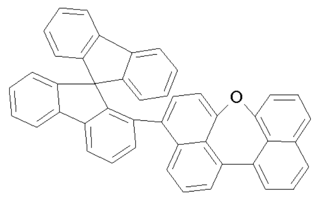

formule (A-33)

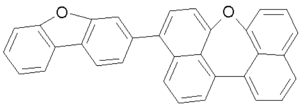

formule (A-34)

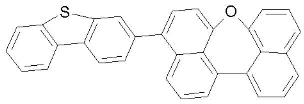

formule (A-36)

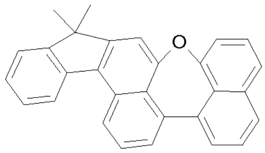

formule (A-40)

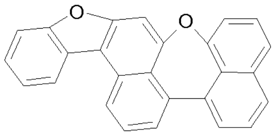

formule (A-41)

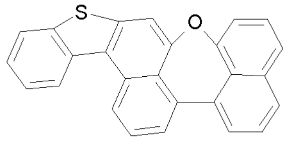

formule (A-42)

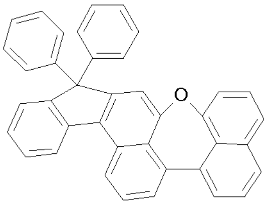

formule (A-43)

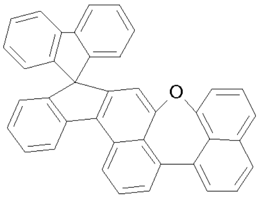

formule (A-44)

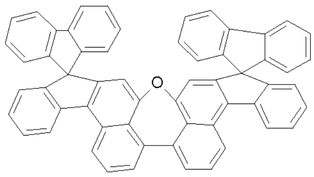

formule (A-45)

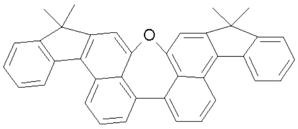

formule (A-46)

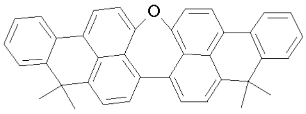

formule (A-49)

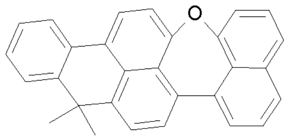

formule (A-50)

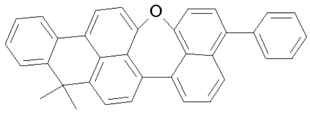

formule (A-51)

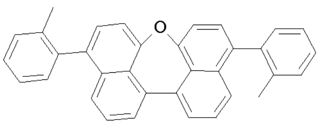

formule (A-52)

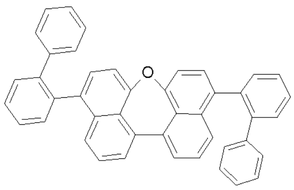

formule (A-53)

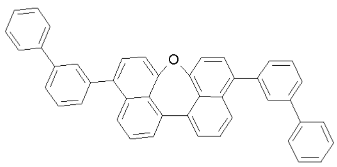

formule (A-54)

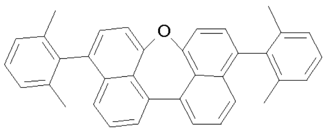

formule (A-55)

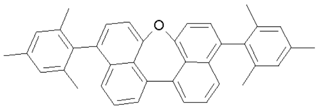

formule (A-56)

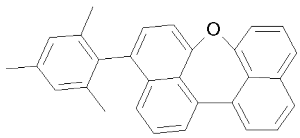

formule (A-57)

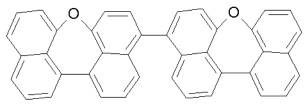

formule (A-58)

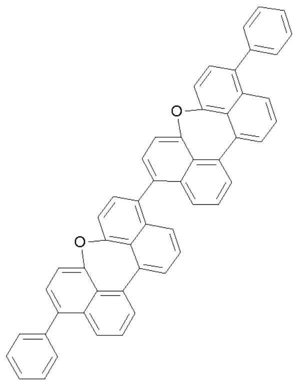

formule (A-59)

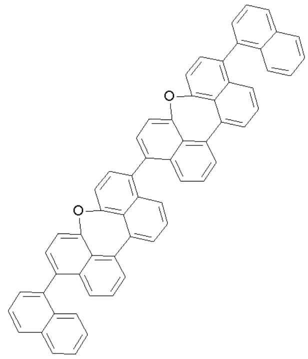

formule (A-60)

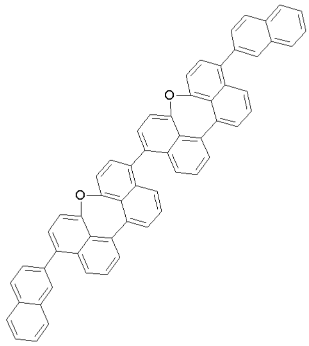

formule (A-61)

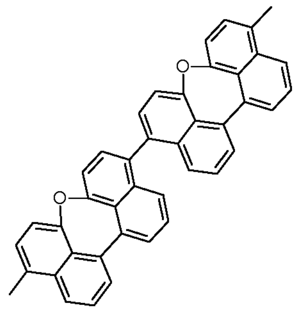

formule (A-62)

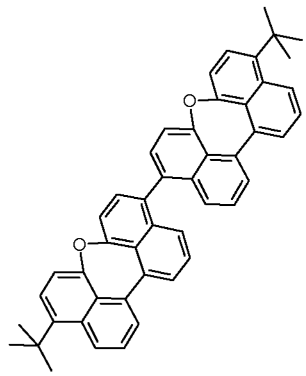

formule (A-63)

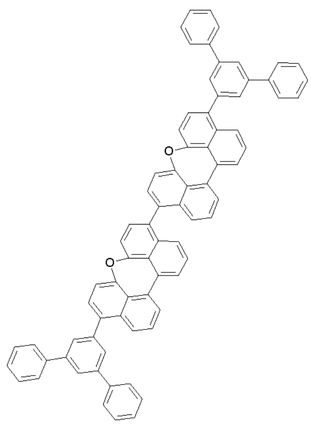

formule (A-64)

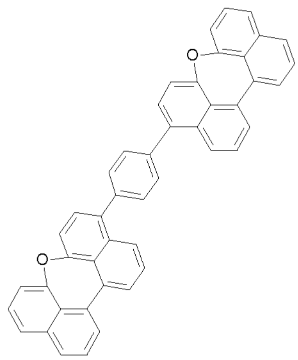

formule (A-65)

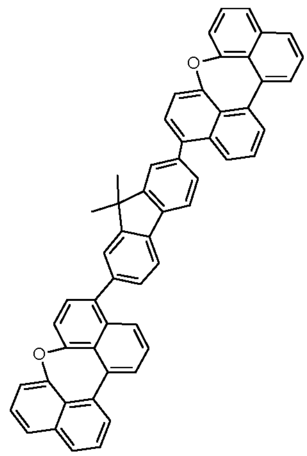

formule (A-66)

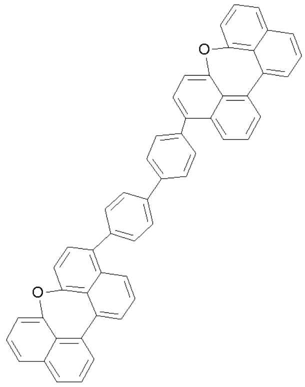
formule (A-67)

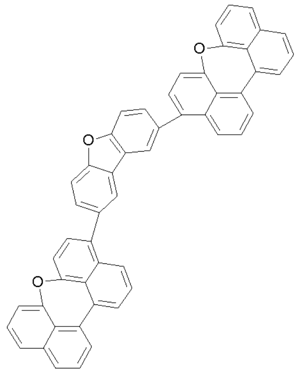
formule (A-68)

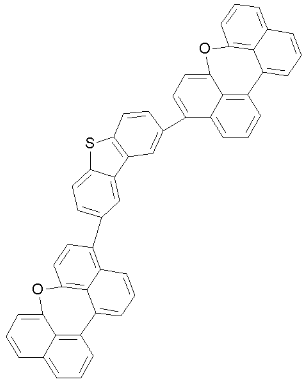
formule (A-69)

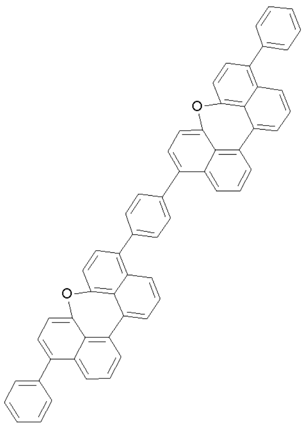
formule (A-69a)

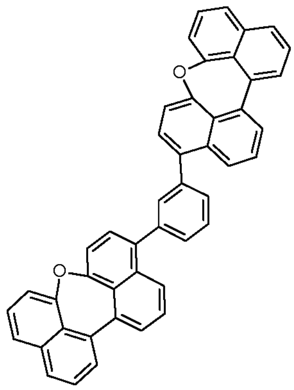
formule (A-69b)

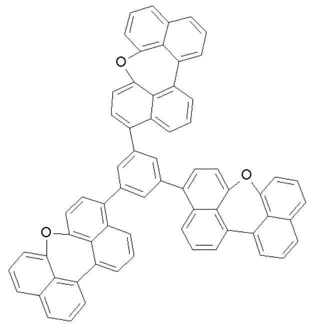
formule (A-69c)

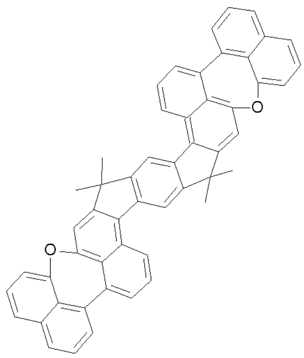
formule (A-69d)

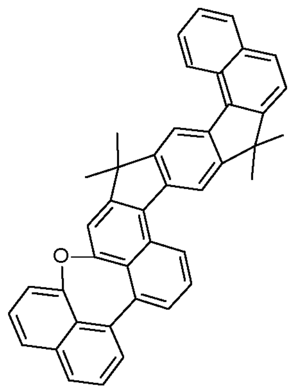
formule (A-69e)

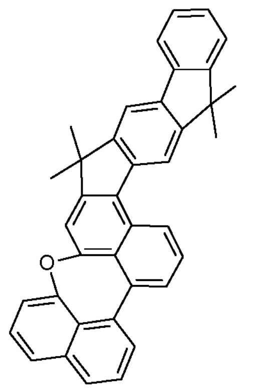
formule (A-69f)

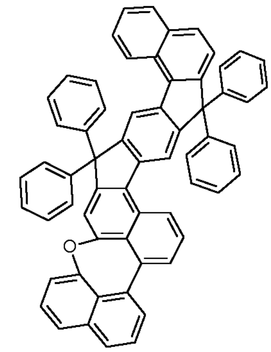

formule (A-69g)

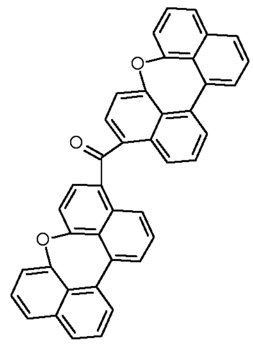

formule (A-69h)

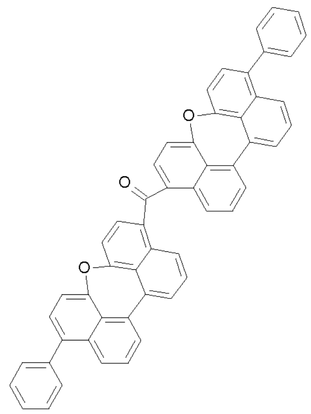

formule (A-69i)

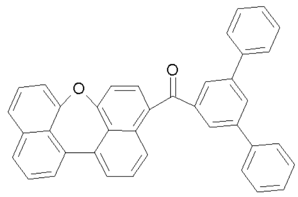

formule (A-69j)

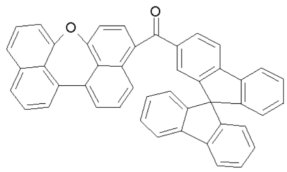

formule (A-69k)

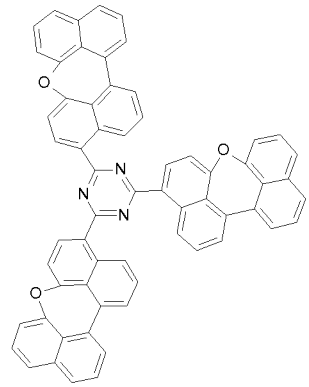

formule (A-69l)

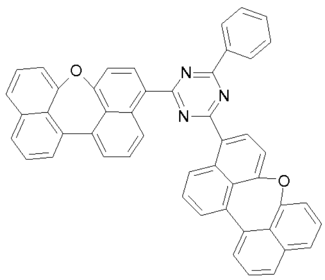

formule (A-69m)

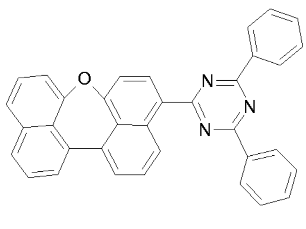

formule (A-69n)

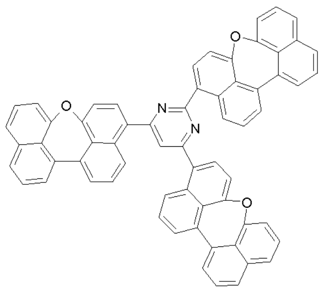

formule (A-69o)

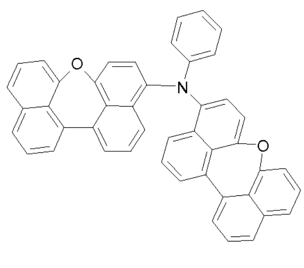

formule (A-69q)

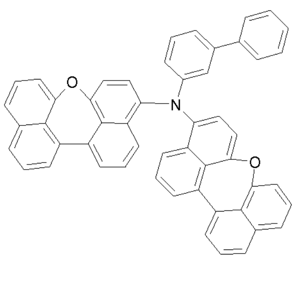

formule (A-69r)

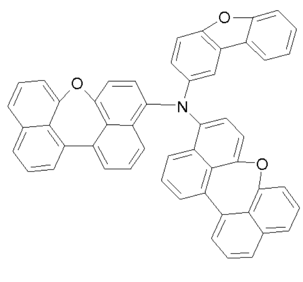

formule (A-69s)

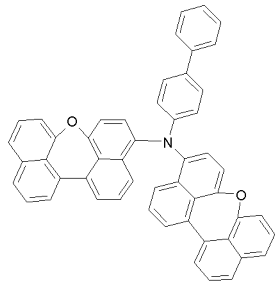
formule (A-69t)

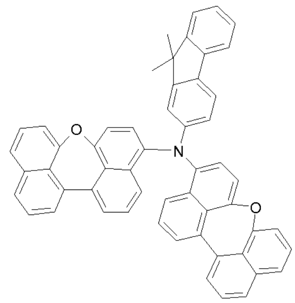
formule (A-69u)

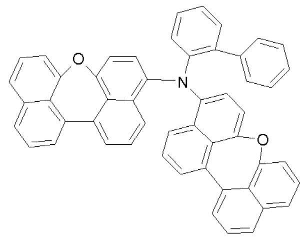
formule (A-70)

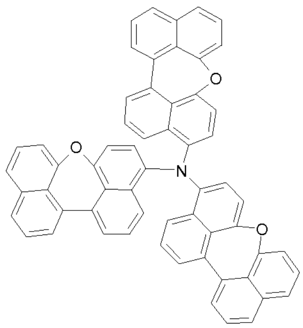
formule (A-71)

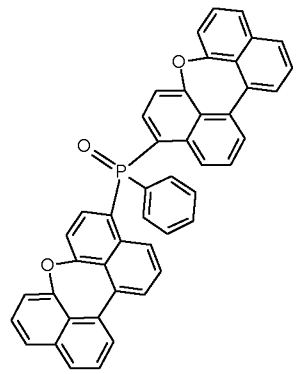
formule (A-72)

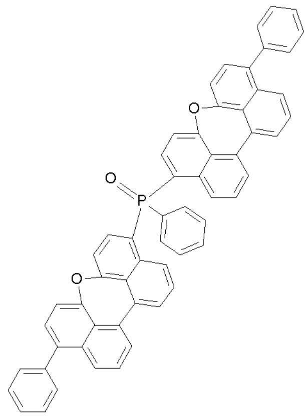
formule (A-73)

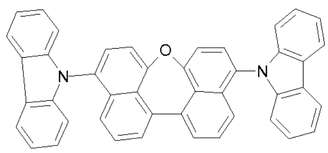
formule (A-74)

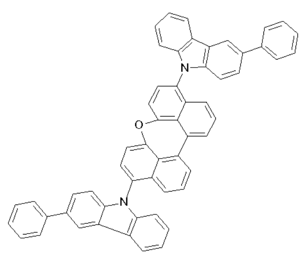
formule (A-75)

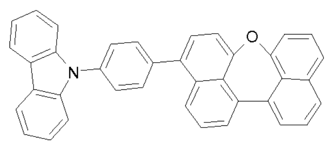
formule (A-76)

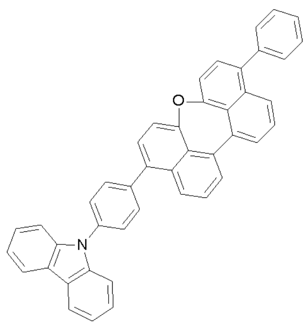
formule (A-77)

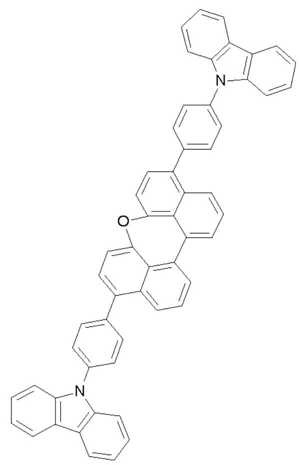
formule (A-78)

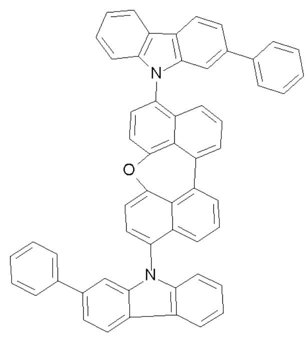
formule (A-79)

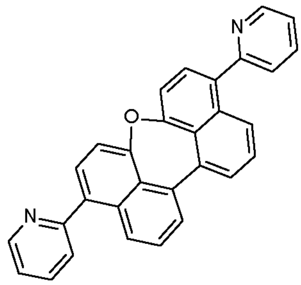
formule (A-80)

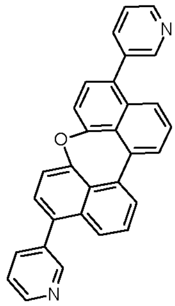
formule (A-81)

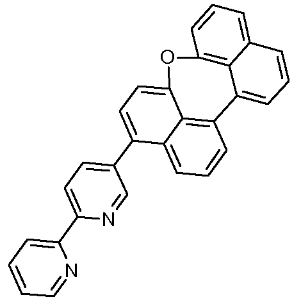
formule (A-82)

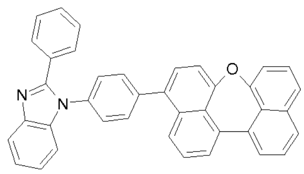
formule (A-83)

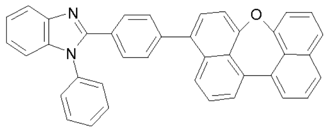
formule (A-84)

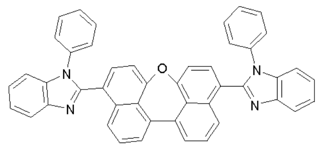
formule (A-85)

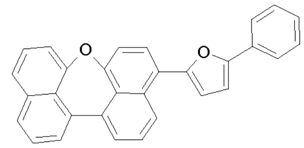
formule (A-86)

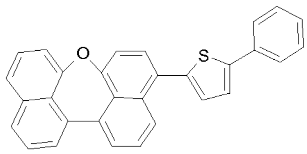
formule (A-87)

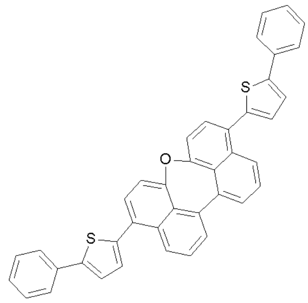
formule (A-88)

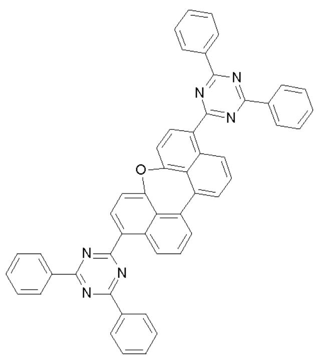
formule (A-89)

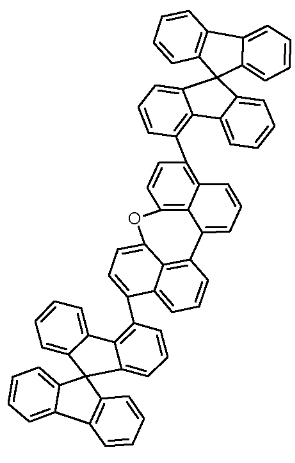
formule (A-90)

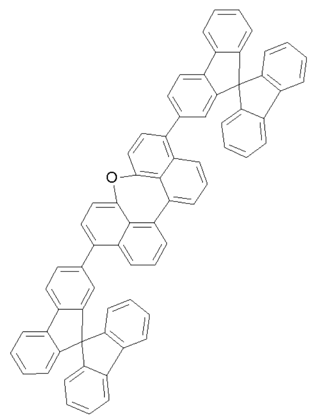
formule (A-91)

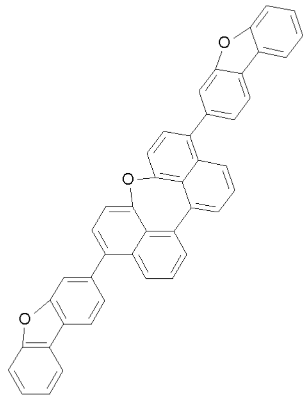
formule (A-92)

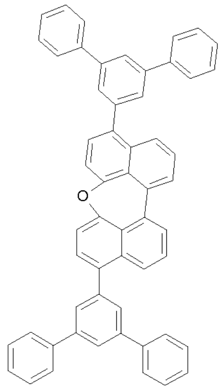
formule (A-93)

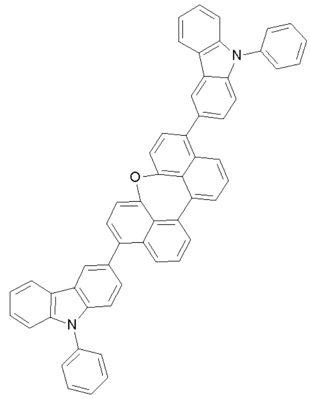
formule (A-94)

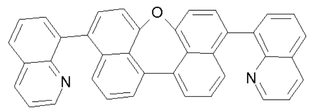
formule (A-95)

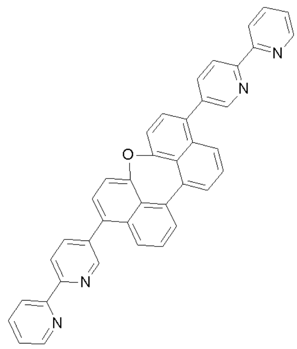
formule (A-96)

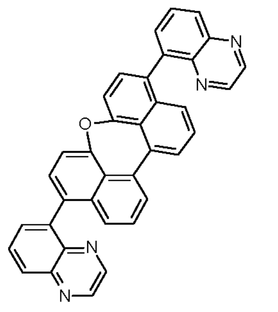
formule (A-97)

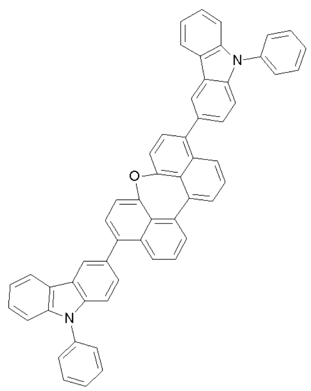
formule (A-98)

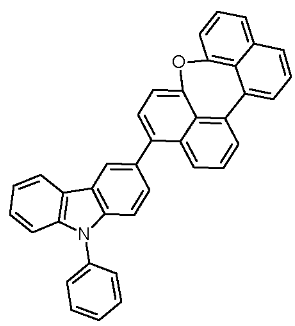
formule (A-99)

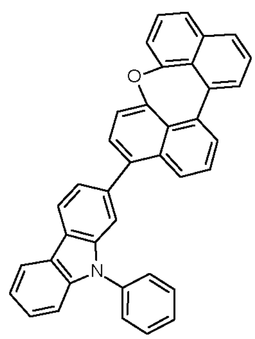
formule (A-100)

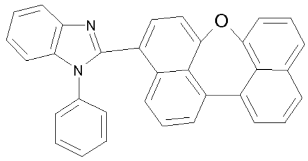
formule (A-101)

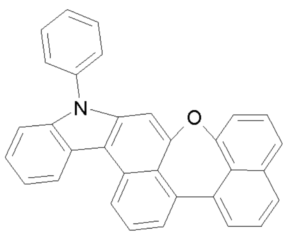
formule (A-102)

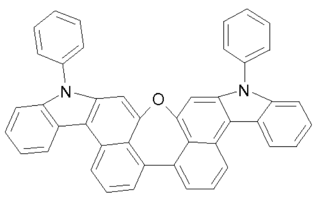
formule (A-103)

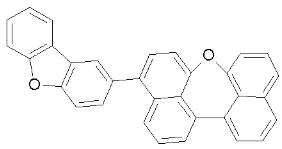 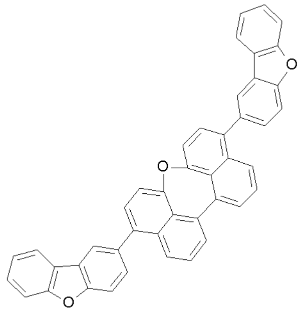 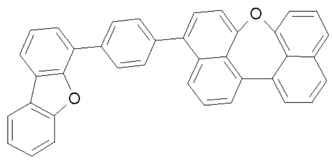

formule (A-104) formule (A-105) formule (A-106)

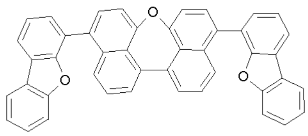 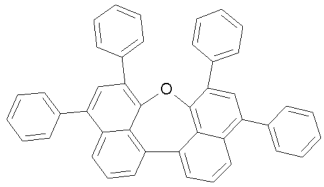 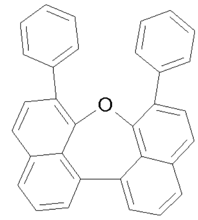

formule (A-107) formule (A-108) formule (A-109)

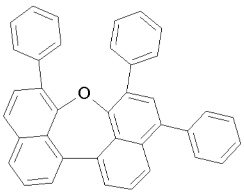 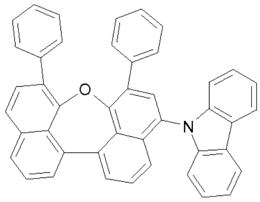 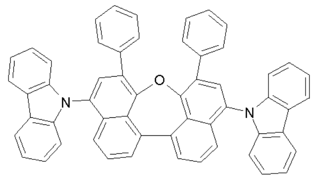

formule (A-110) formule (A-111) formule (A-112)

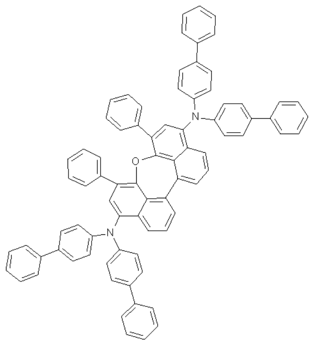 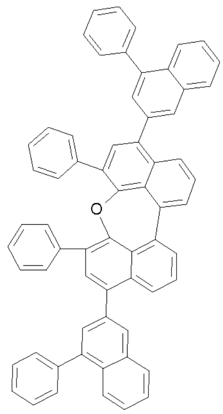 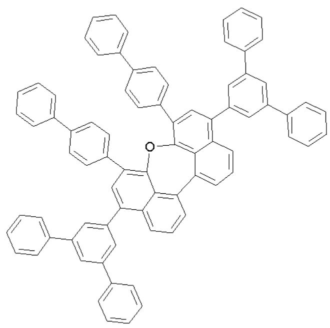

formule (A-113) formule (A-114) formule (A-115)

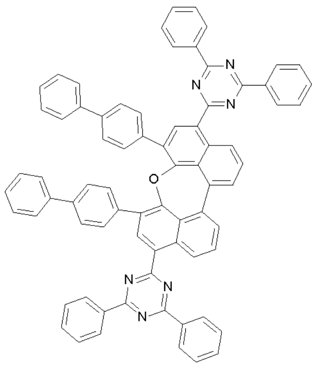

formule (A-116)

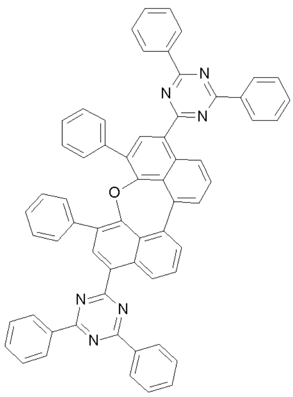

formule (A-117)

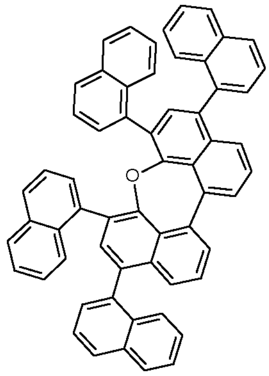

formule (A-118)

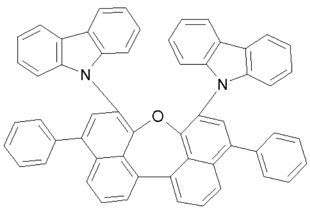

formule (A-119)

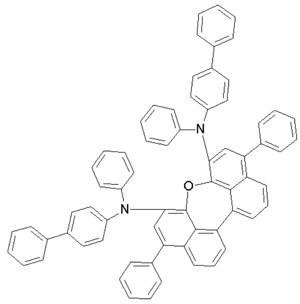

formule (A-120)

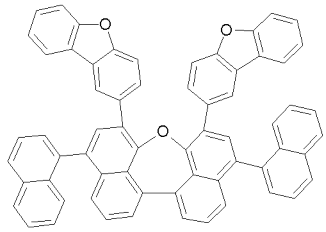

formule (A-121)

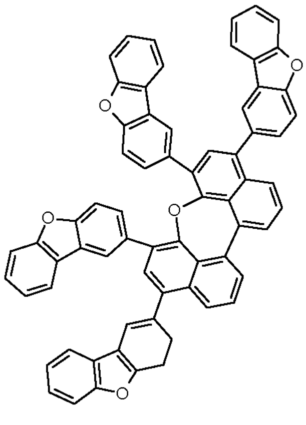

formule (A-122)

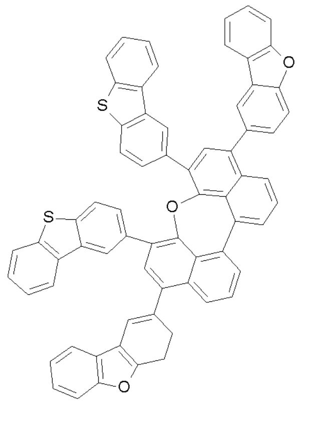

formule (A-123)

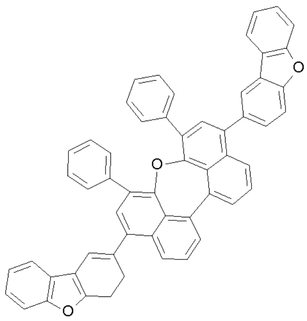

formule (A-124)

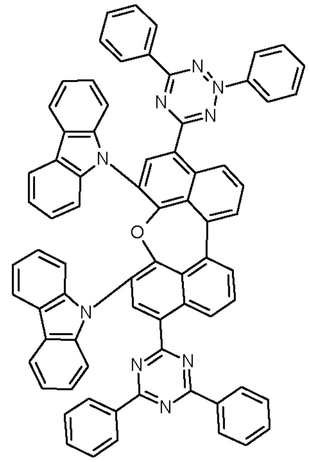

formule (A-125)

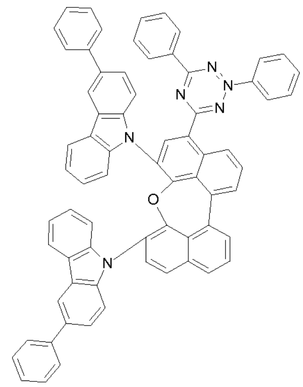

formule (A-126)

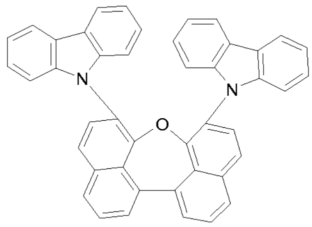

formule (A-127)

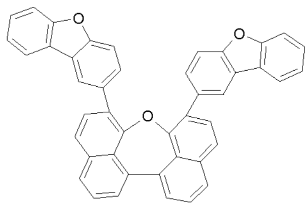

formule (A-128)

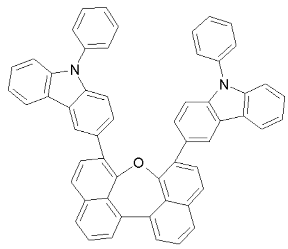

formule (A-129)

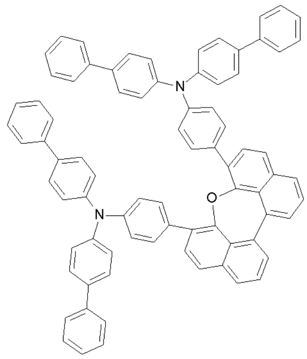

formule (A-130)

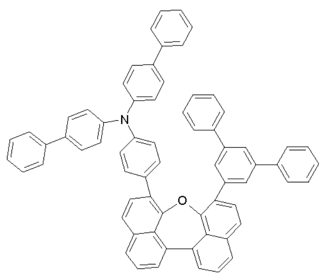

formule (A-131)

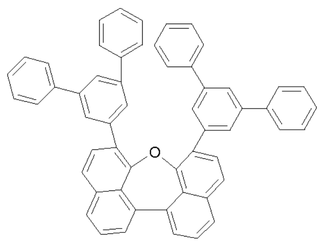

formule (A-132)

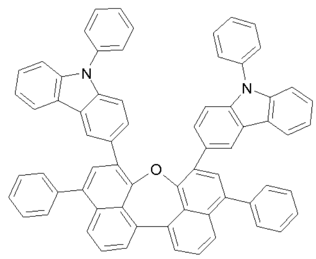

formule (A-133)

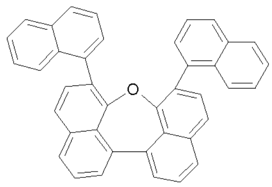

formule (A-134)

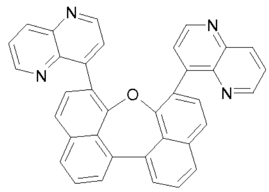

formule (A-135)

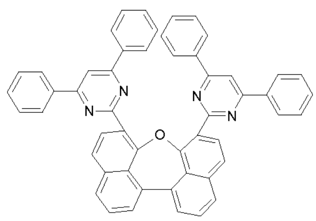

formule (A-136)

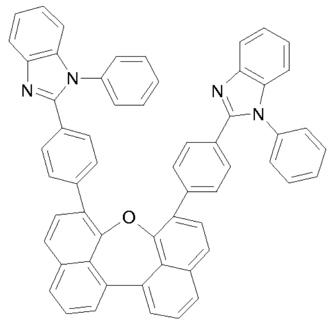

formule (A-137)

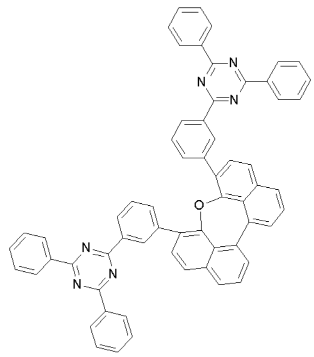

formule (A-138)

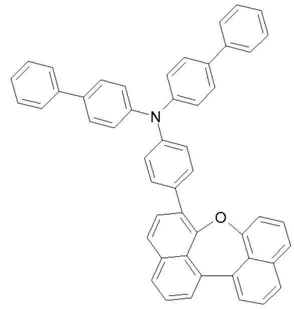

formule (A-139)

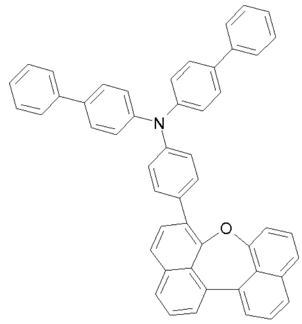

formule (A-140)

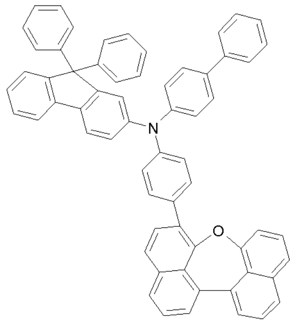

formule (A-141)

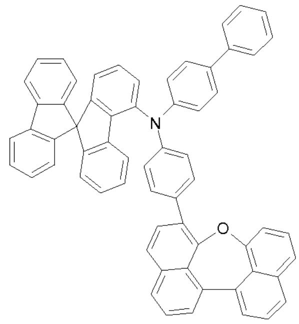

formule (A-142)

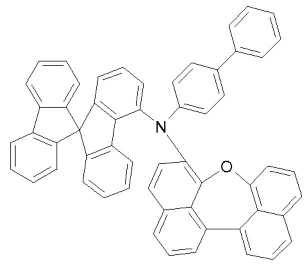

formule (A-143)

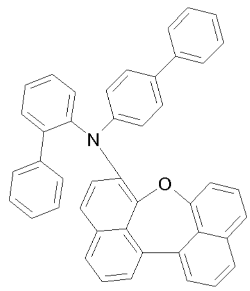

formule (A-144)

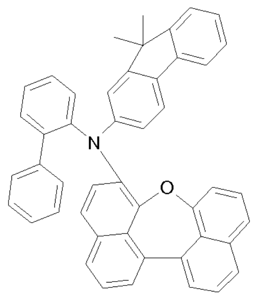

formule (A-145)

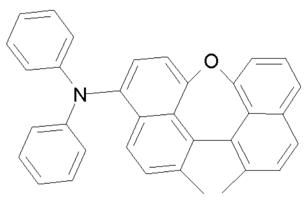

formule (A-146)

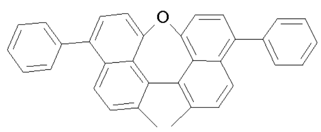

formule (A-147)

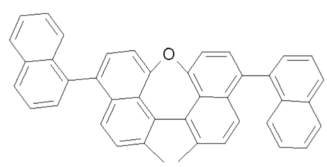

formule (A-148)

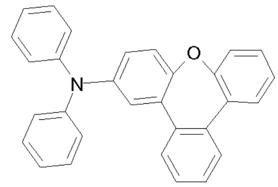
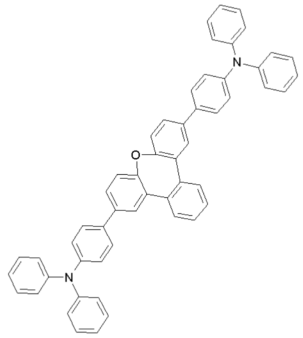
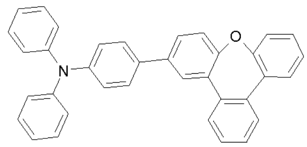

formule (A-149) formule (A-149a) formule (A-149b)

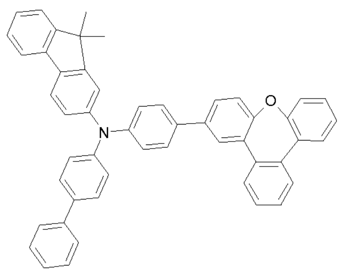
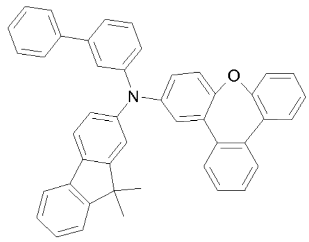
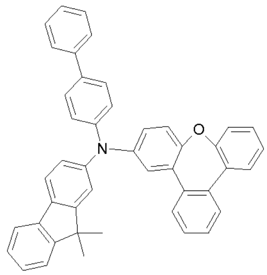

formule (A-150) formule (A-151) formule (A-152)

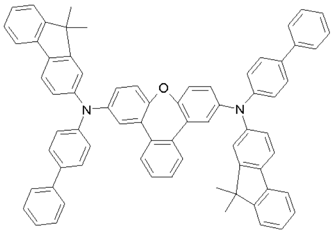
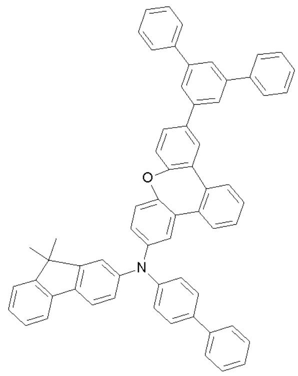
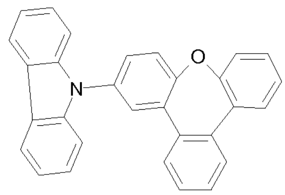

formule (A-153) formule (A-154) formule (A-155)

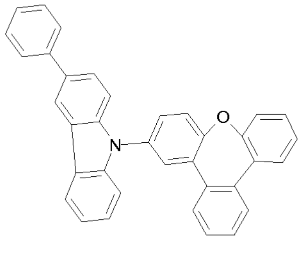
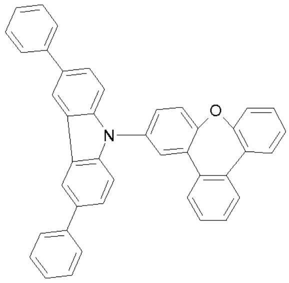
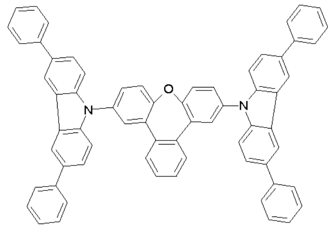

formule (A-156) formule (A-157) formule (A-158)

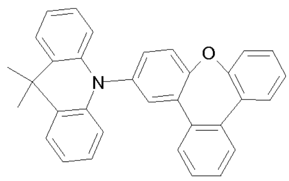 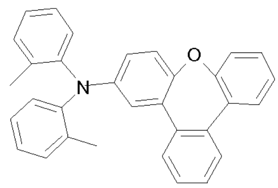 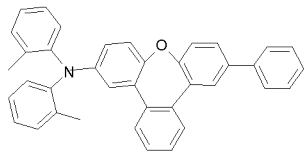

formule (A-159) formule (A-160) formule (A-161)

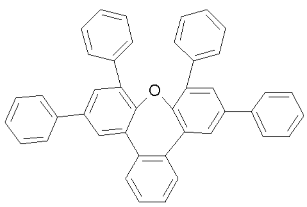 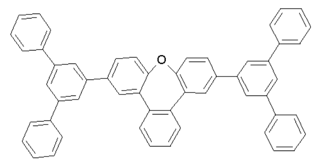 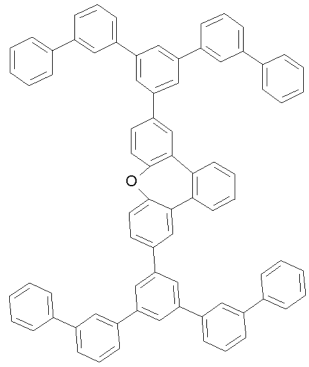

formule (A-162) formule (A-163) formule (A-164)

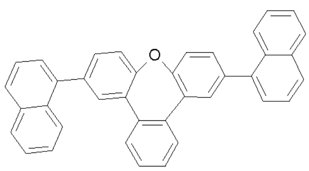 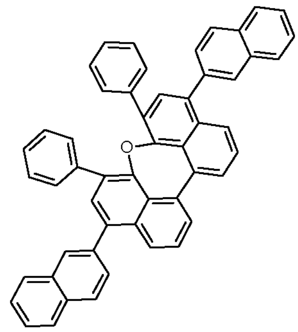 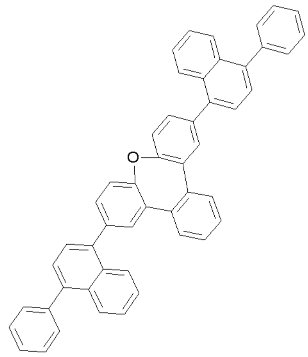

formule (A-165) formule (A-166) formule (A-167)

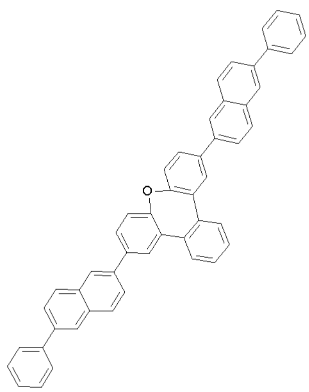 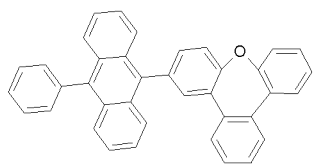 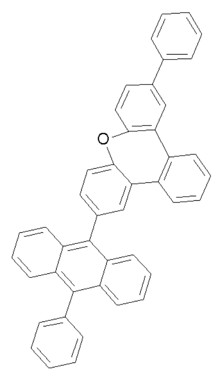

formule (A-168) formule (A-169) formule (A-170)

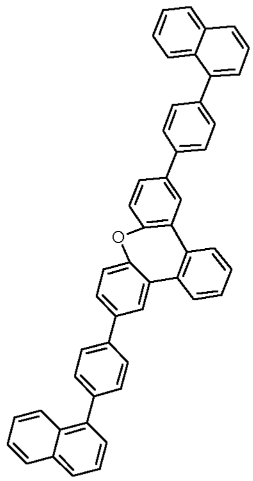

formule (A-171)

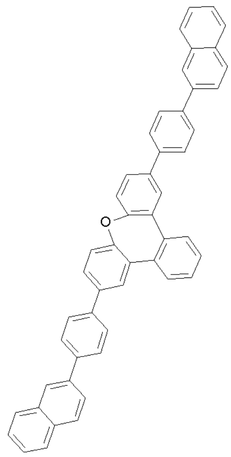

formule (A-172)

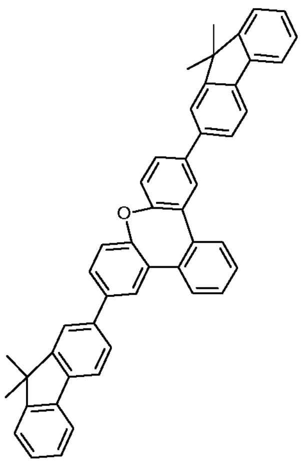

formule (A-174)

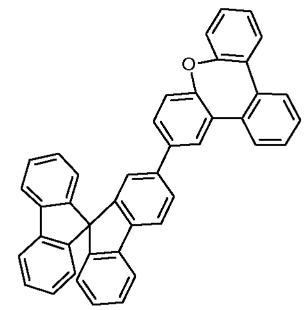

formule (A-175)

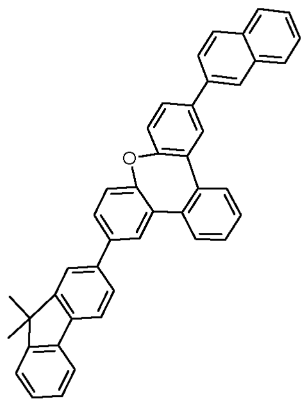

formule (A-176)

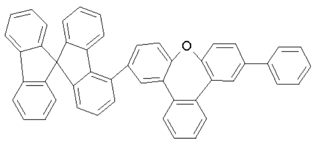

formule (A-177)

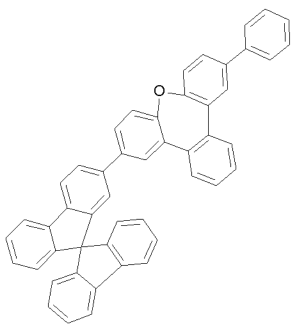

formule (A-178)

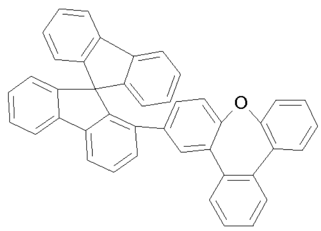

formule (A-179)

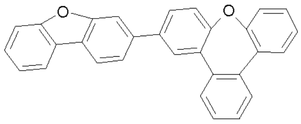

formule (A-180)

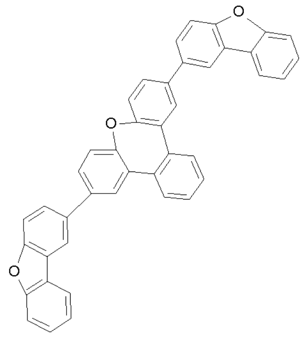

formule (A-181)

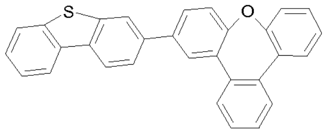

formule (A-182)

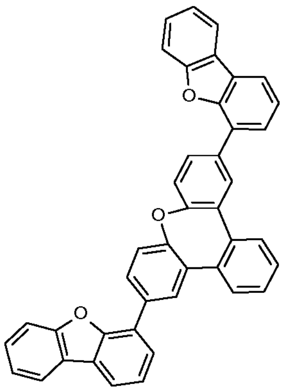

formule (A-183)

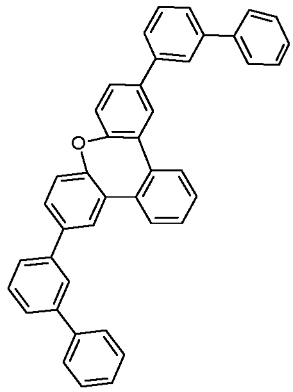

formule (A-184)

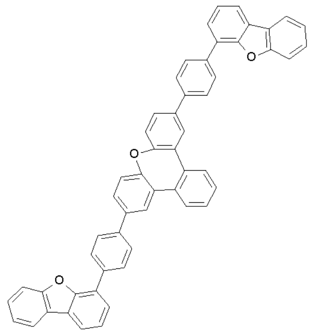

formule (A-185)

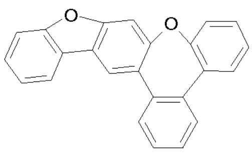

formule (A-187)

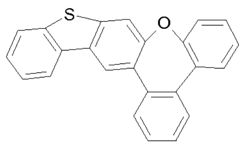

formule (A-188)

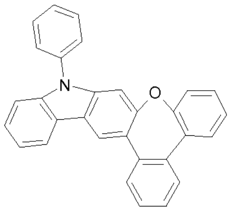

formule (A-190)

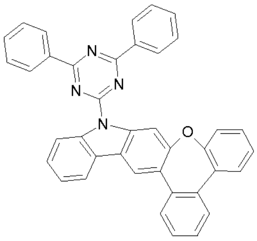

formule (A-193)

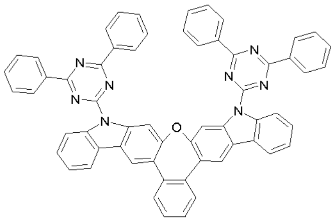

formule (A-194)

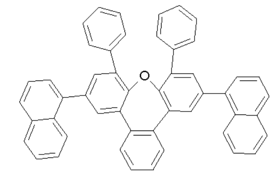

formule (A-195)

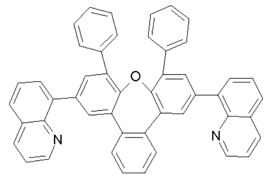

formule (A-196)

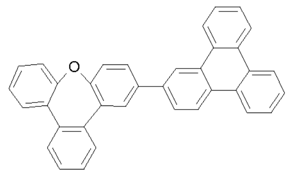

formule (A-197)

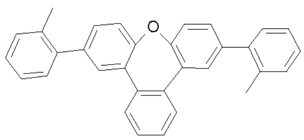

formule (A-198)

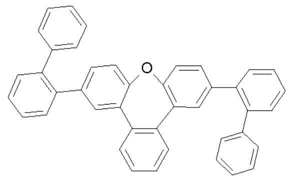

formule (A-199)

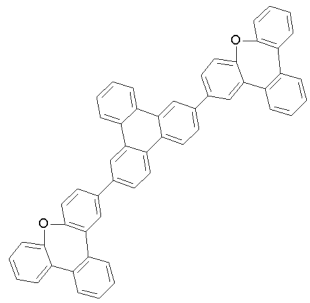

formule (A-200)

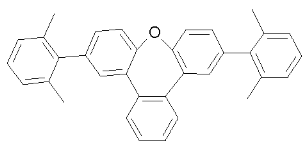

formule (A-201)

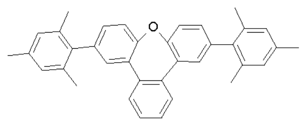

formule (A-202)

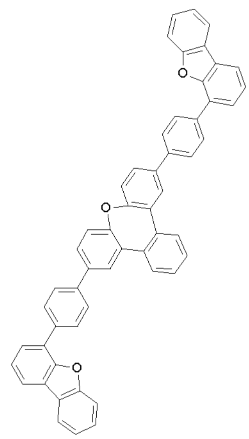

formule (A-203)

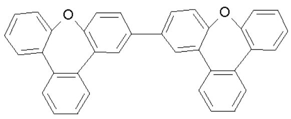

formule (A-204)

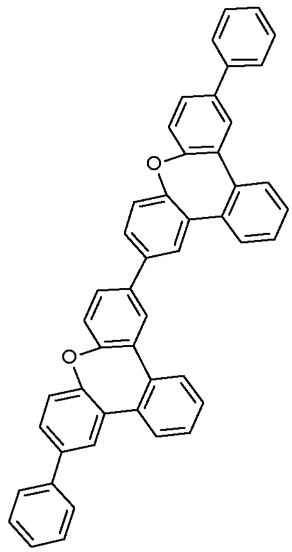

formule (A-205)

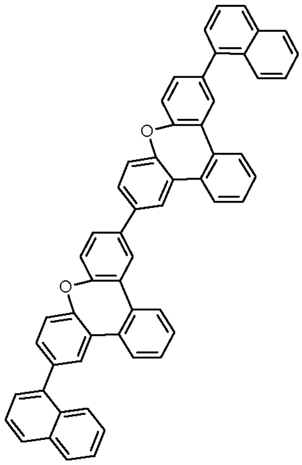

formule (A-206)

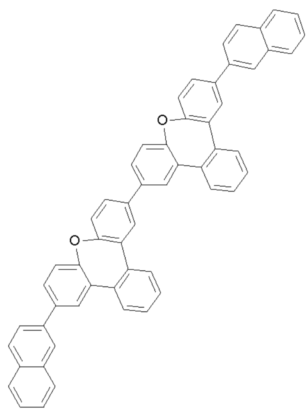

formule (A-207)

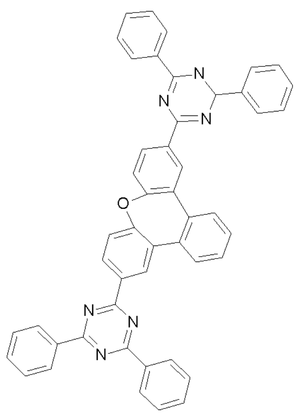

formule (A-208)

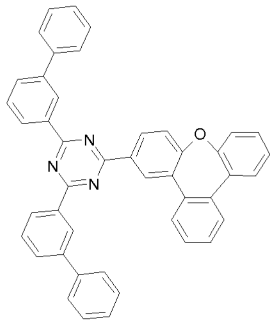

formule (A-209)

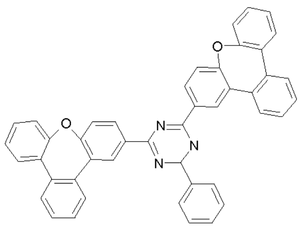

formule (A-210)

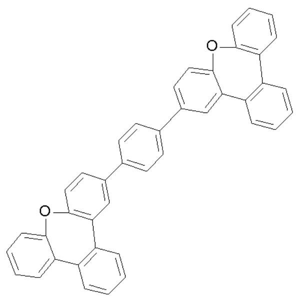

formule (A-211)

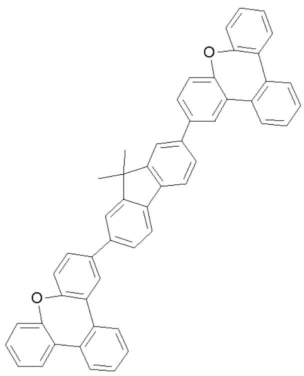

formule (A-212)

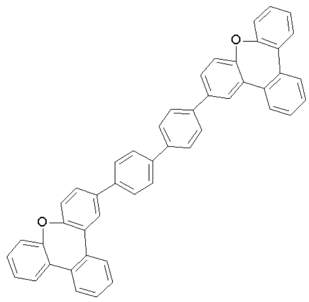

formule (A-213)

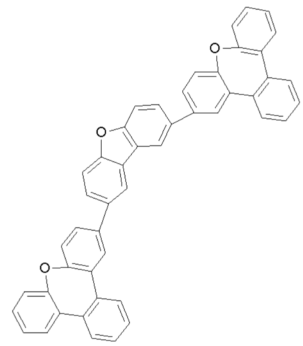

formule (A-214)

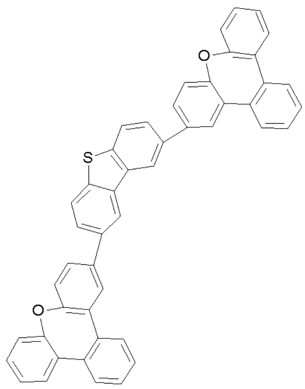

formule (A-215)

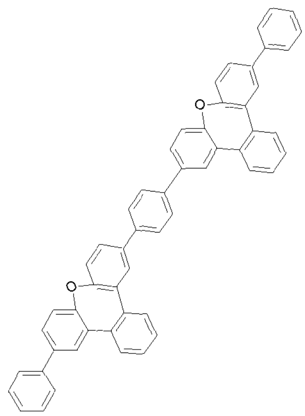

formule (A-216)

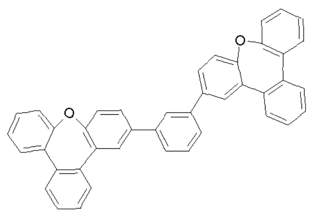

formule (A-217)

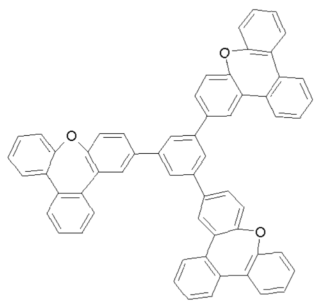

formule (A-218)

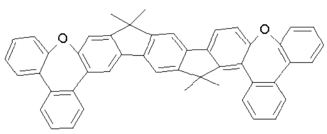

formule (A-219)

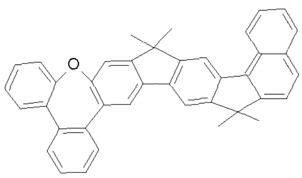

formule (A-220)

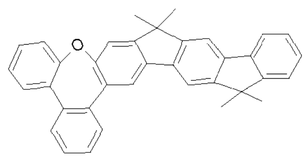

formule (A-221)

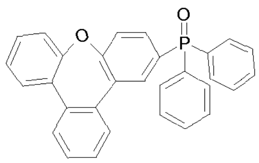

formule (A-222)

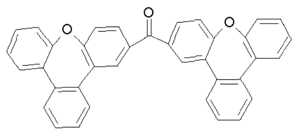

formule (A-223)

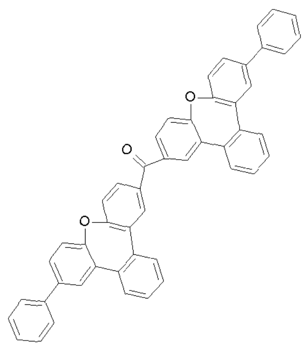

formule (A-224)

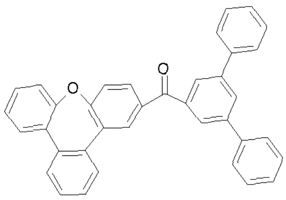
formule (A-225)

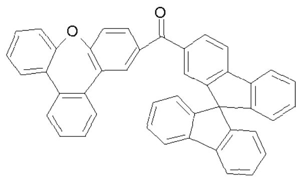
formule (A-226)

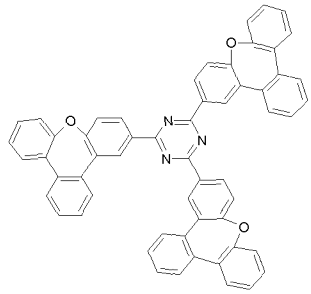
formule (A-227)

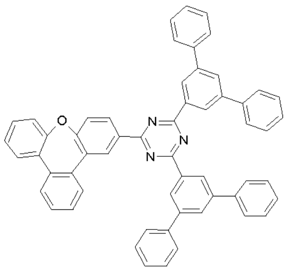
formule (A-228)

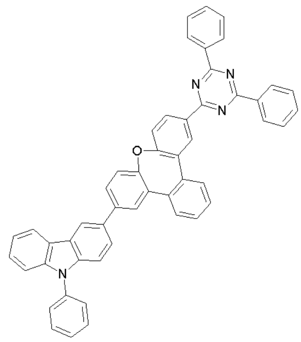
formule (A-229)

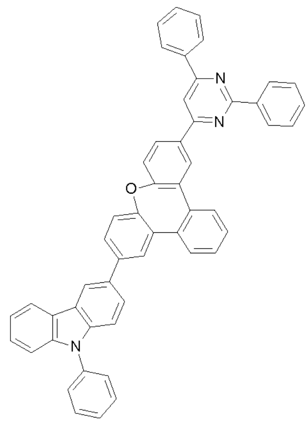
formule (A-230)

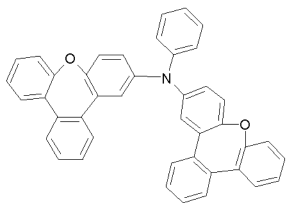
formule (A-231)

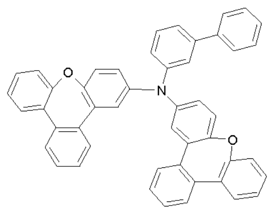
formule (A-232)

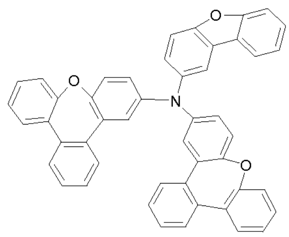
formule (A-233)

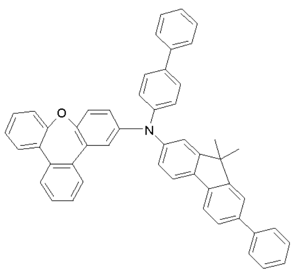
formule (A-234)

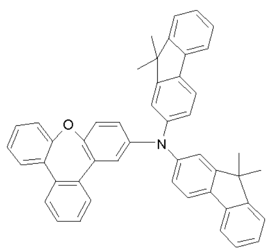
formule (A-235)

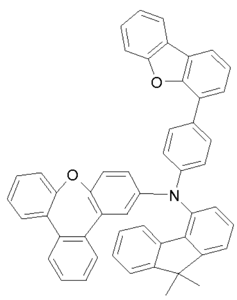
formule (A-236)

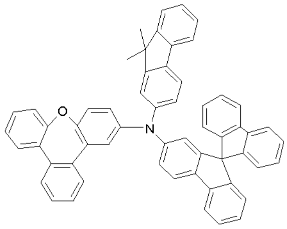
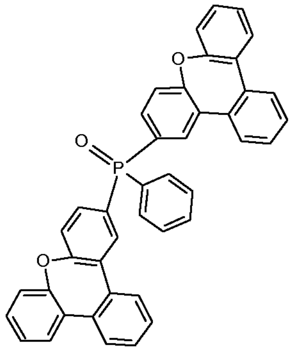
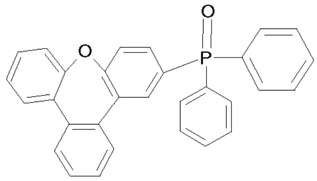

formule (A-237) formule (A-238) formule (A-239)

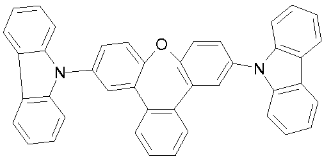
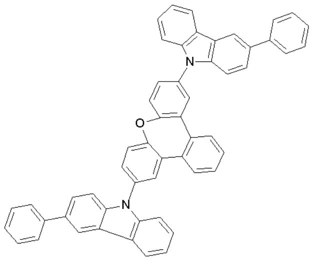
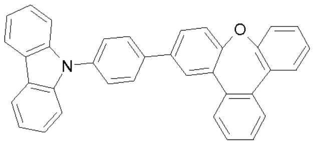

formule (A-240) formule (A-241) formule (A-242)

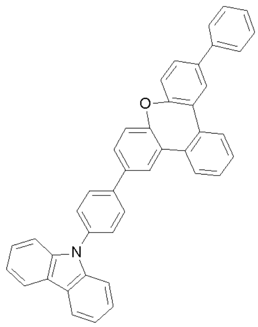
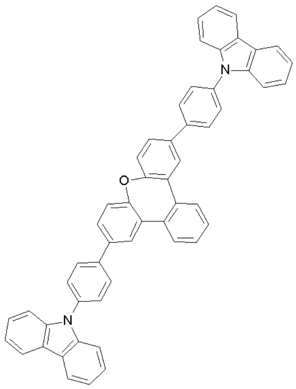
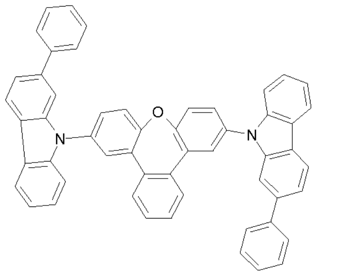

formule (A-243) formule (A-244) formule (A-245)

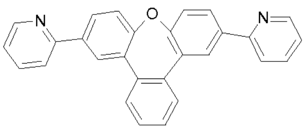
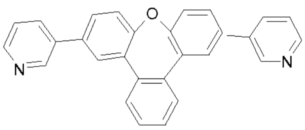
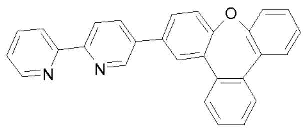

formule (A-246) formule (A-247) formule (A-248)

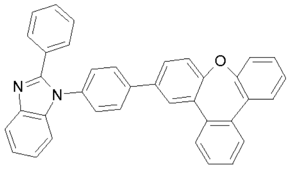
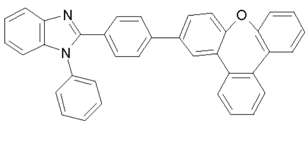
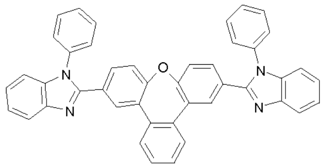

formule (A-249) formule (A-250) formule (A-251)

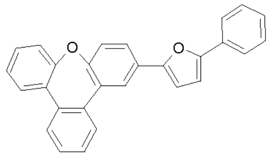
formule (A-252)

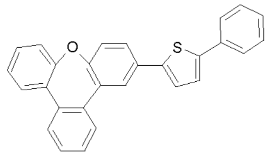
formule (A-253)

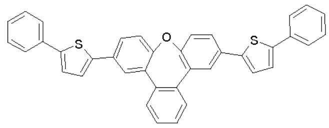
formule (A-254)

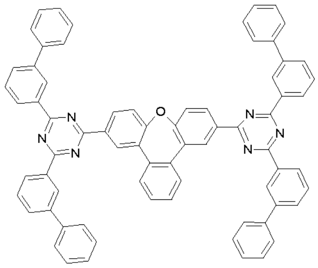
formule (A-255)

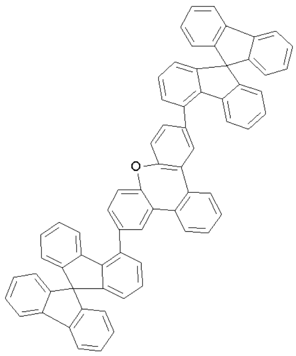
formule (A-256)

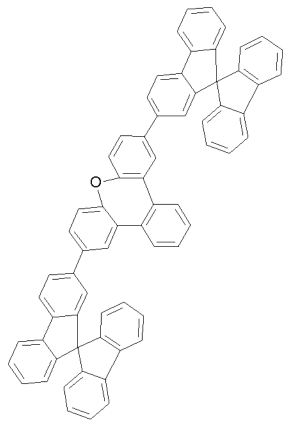
formule (A-257)

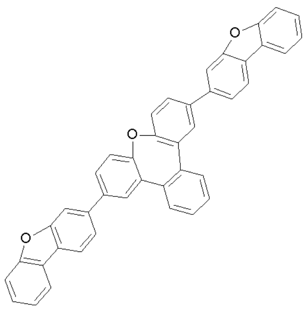
formule (A-258)

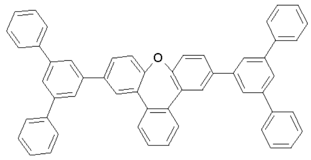
formule (A-259)

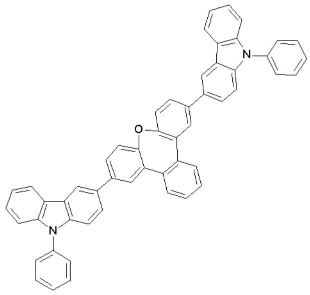
formule (A-260)

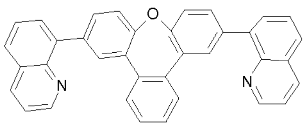
formule (A-261)

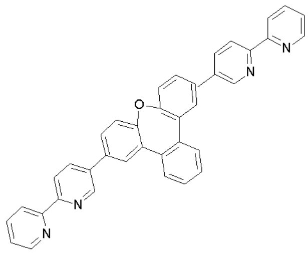
formule (A-262)

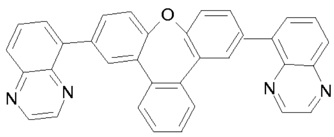
formule (A-263)

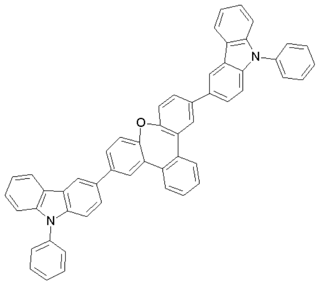
formule (A-264)

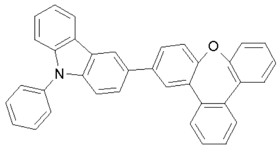
formule (A-265)

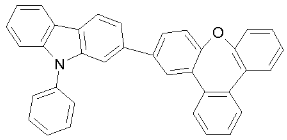
formule (A-266)

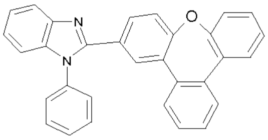
formule (A-267)

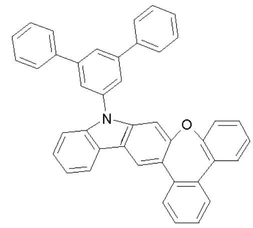
formule (A-268)

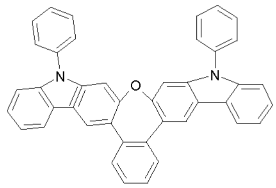
formule (A-269)

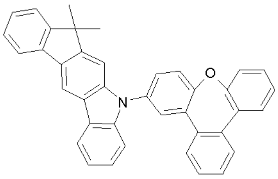
formule (A-270)

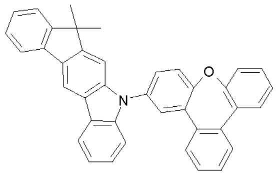
formule (A-271)

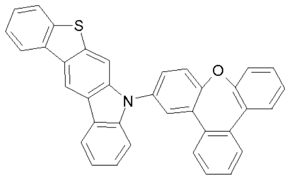
formule (A-272)

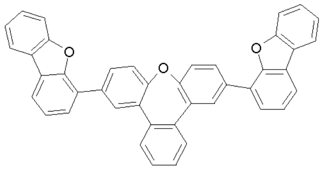
formule (A-273)

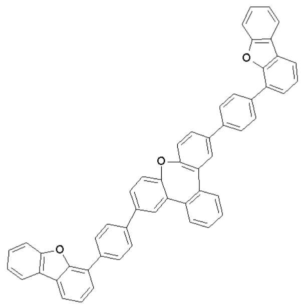
formule (A-274)

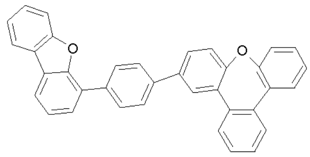
formule (A-275)

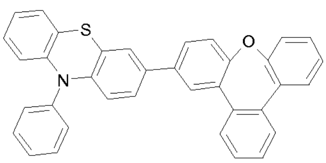
formule (A-276)

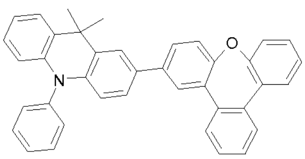
formule (A-277)

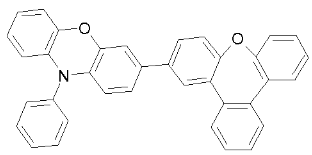
formule (A-278)

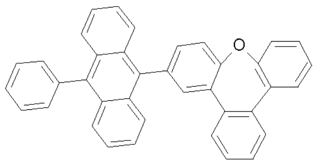
formule (A-279)

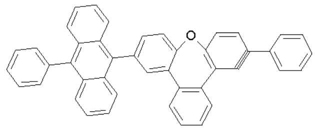
formule (A-280)

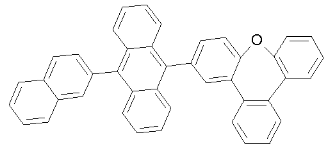
formule (A-281)

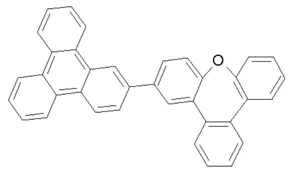
formule (A-282)

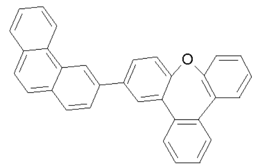
formule (A-283)

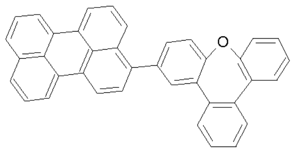
formule (A-284)

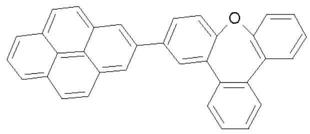
formule (A-285)

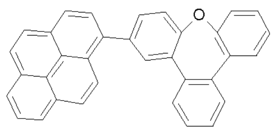
formule (A-286)

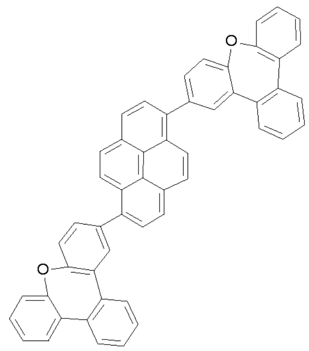
formule (A-287)

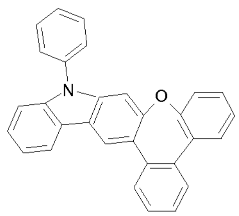
formule (A-288)

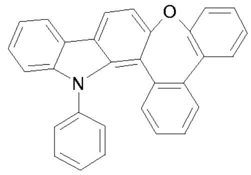
formule (A-289)

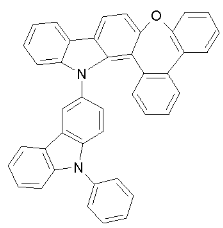
formule (A-290)

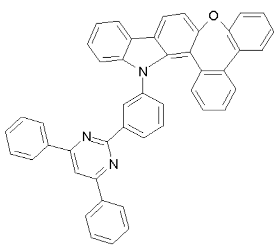
formule (A-291)

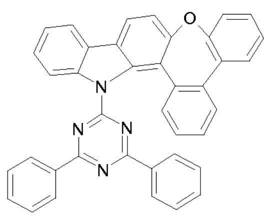
formule (A-292)

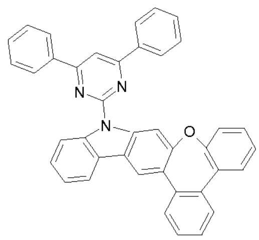
formule (A-293)

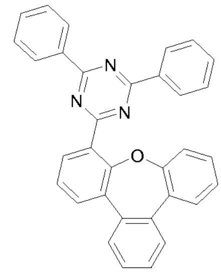

INV-5

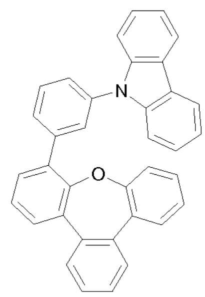

INV-6

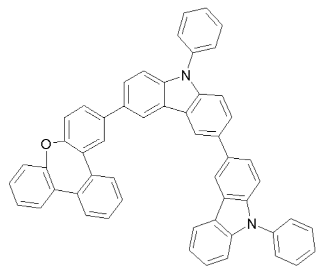

INV-7

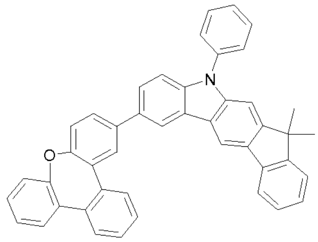

INV-8

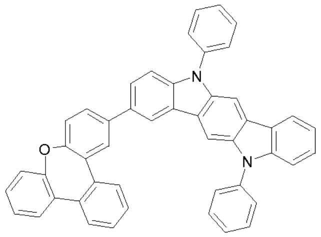

INV-9

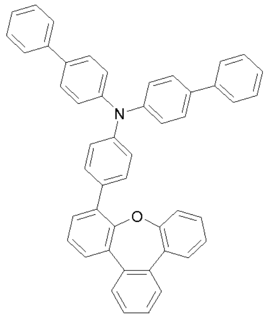

INV-10

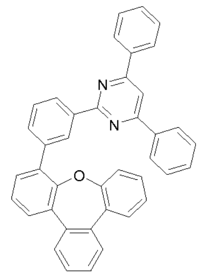

INV-11

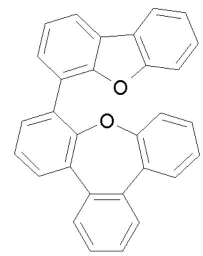

INV-12

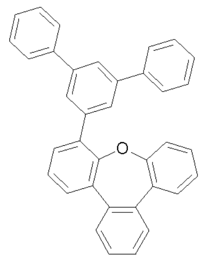

INV-13

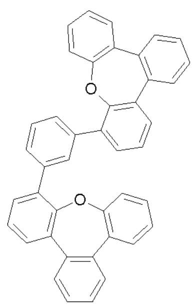

INV-14

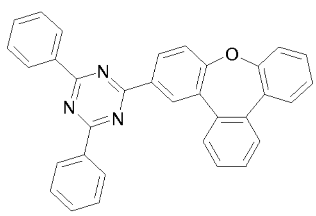

INV-15

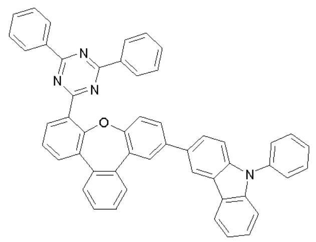

INV-19

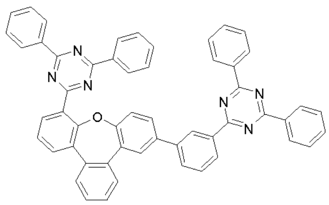
INV-20

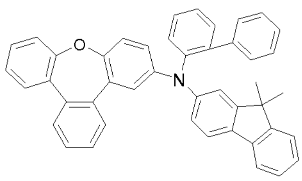
INV-21

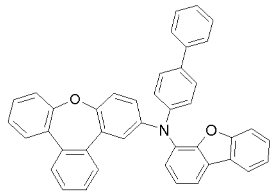
INV-22

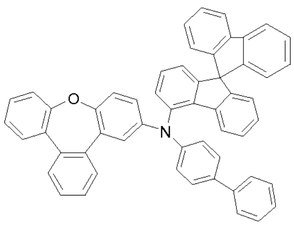
INV-23

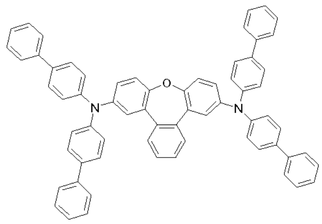
INV-24

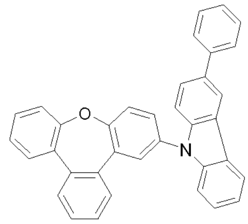
INV-25

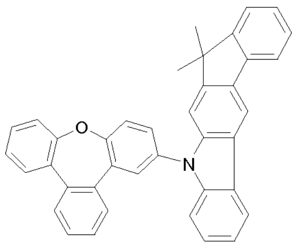
INV-26

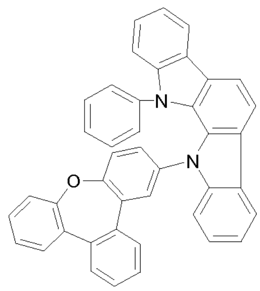
INV-27

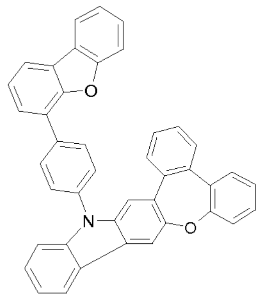
INV-28.

**18.** Procédé pour la préparation du composé selon une ou plusieurs des revendications 13 à 17 à l'aide d'un couplage de Suzuki.

**19.** Procédé pour la préparation du composé selon une ou plusieurs des revendications 13 à 17 à l'aide d'un couplage de Buchwald ou de Ullmann.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4539507 A
- US 5151629 A
- EP 0676461 A
- WO 9827136 A
- WO 2010119274 A
- WO 2005086251A2 A
- WO 2012175535 A1
- WO 2012175219 A1
- WO 2012168358 A1
- WO 2012031735 A1
- EP 1837926 A1
- WO 2007107306 A1
- EP 2452946 A1
- EP 2463927 A1
- WO 2009000237 A1
- US 2007145355 A1
- EP 1538684 A1
- WO 2006081780 A1
- WO 2009003455 A1
- WO 2010097433 A1
- EP 1988587 A1
- US 2010102709 A1
- EP 2180029 A1
- WO 2011131185 A1
- WO 2011134458 A1
- US 2012223296 A1
- WO 2007134873 A1
- WO 2008061517 A2
- WO 2008061518 A2
- DE 102008051737 A1
- WO 2009089821 A1
- US 2010096600 A1
- WO 2008138580 A1
- WO 2008058525 A2
- WO 2007115540 A1
- DE 102010046040 A1
- WO 2008128519 A2
- WO 2010108579 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2006108497 A
- WO 2006122630 A
- WO 2008006449 A
- WO 2007140847 A
- WO 2010012328 A
- EP 676461 A
- WO 2004081017 A
- WO 2004058911 A
- WO 2005084081 A
- WO 2005084082 A
- WO 2006048268 A
- WO 2006117052 A
- WO 2008145239 A
- US 20050069729 A
- WO 2005039246 A
- JP 2004288381 A
- EP 1205527 A
- WO 2008086851 A
- WO 2011088877 A
- WO 2011128017 A
- WO 2010136109 A
- WO 2011000455 A
- EP 1617710 A
- EP 1617711 A
- EP 1731584 A
- JP 2005347160 A
- WO 2007063754 A
- WO 2008056746 A
- WO 2004093207 A
- WO 2010006680 A
- WO 2005003253 A
- WO 2007137725 A
- WO 2005111172 A
- WO 2010015306 A
- EP 652273 A
- WO 2009062578 A
- WO 2010054729 A
- WO 2010054730 A
- WO 2005011013 A
- JP 2000053957 A
- WO 2003060956 A
- WO 2004028217 A
- WO 2004080975 A
- WO 2010072300 A
- WO 2006100896 A
- EP 1661888 A
- WO 01049806 A
- US 5061569 A
- WO 9509147 A

- WO 08006449 A
- WO 2012034627 A
- EP 12000929 A
- EP 12005369 A
- EP 12005370 A
- EP 12005371 A
- EP 11009127 A
- EP 11007067 A
- WO 04058911 A


**In der Beschreibung aufgeführte Nicht-Patentliteratur**


- **M. A. BALDO et al.** *Appl. Phys. Lett.,* 1999, vol. 75, 4-6
- **T. MATSUMOTO ; T. NAKADA ; J. ENDO ; K. MORI ; N. KAWAMURA ; A. YOKOI ; J. KIDO.** Multiphoton Organic EL Device Having Charge Generation Layer. *IDMC,* 2003
- **Y. SHIROTA et al.** *Chem. Rev.,* 2007, vol. 107 (4), 953-1010
- **M. S. ARNOLD et al.** *Appl. Phys. Lett.,* 2008, vol. 92, 053301
- *J. Org. Chem.,* 1985, vol. 50, 1486-1496